# EUROPEAN PATENT APPLICATION

(11) **EP 4 108 676 A1**
(43) Date of publication of application: **28.12.2022**
(21) Application number: 21756644.7
(22) Date of filing: 22.02.2021
(51) Int. Cl.: C07K 7/08, A61K 45/00, A61K 47/64, A61K 49/00, A61P 25/00, C07K 19/00

(54) **HUMAN TRANSFERRIN RECEPTOR BINDING PEPTIDE**

(30) Priority: 22.02.2020 JP 2020028879
(71) Applicant: JCR Pharmaceuticals Co., Ltd., Ashiya-shi, Hyogo 659-0021 (JP); PeptiDream Inc., Kawasaki-shi, Kanagawa 210-0821 (JP)
(72) Inventor: TAKAHASHI, Kenichi, Kobe-shi, Hyogo 651-2241 (JP); YODEN, Eiji, Kobe-shi, Hyogo 651-2241 (JP); HASHIMOTO, Hidehiko, Kobe-shi, Hyogo 651-2241 (JP); FUJIYAMA, Saki, Kobe-shi, Hyogo 651-2241 (JP); OHUCHI, Masaki, Kawasaki-shi, Kanagawa 210-0821 (JP); NAKAMURA, Naoko, Kawasaki-shi, Kanagawa 210-0821 (JP); BASHIRUDDIN, Nasir Kato, Kawasaki-shi, Kanagawa 210-0821 (JP); SAWAI, Naoki, Kawasaki-shi, Kanagawa 210-0821 (JP); EHARA, Takeru, Kawasaki-shi, Kanagawa 210-0821 (JP); TAKUWA, Masatoshi, Kawasaki-shi, Kanagawa 210-0821 (JP); MASUYA, Keiichi, Kawasaki-shi, Kanagawa 210-0821 (JP); INABA, Shinnosuke, Kawasaki-shi, Kanagawa 210-0821 (JP)
(74) Representative: Titmus, Craig Edward
(86) International application number: PCT/JP2021/006709
(87) International publication number: WO 2021/167107

(57) **Abstract**

[Problem] To provide a peptide, etc., capable of passing through the blood-brain barrier (BBB) by binding to a human transferrin receptor (hTfR). [Solution] A peptide, etc., having the amino acid sequence shown in SEQ ID NO: 1 (Ala-Val-Phe-Val-Trp-Asn-Tyr-Tyr-Ile-Ile-Ser-Cys) or an amino acid sequence having substitutions, deletions, additions, and/or insertions of 1 to 10 amino acid residues (inclusive) in the amino acid sequence shown in SEQ ID NO: 1.

## Description

### Technical Field

This invention relates to a peptide capable of binding to a human transferrin receptor (hTfR) . This invention relates to a peptide capable of passing through the blood-brain barrier (BBB), a peptide having directivity to a muscle tissue, and a peptide having a cell-penetrating property. Further, the present invention relates to a method for delivering a substance into the brain, a method for delivering a substance to a muscle tissue, and the like using these peptides.

### Background Art

The capillaries that supply the blood to most of the brain tissues, except some regions including the circumventricular organs (the pineal gland, the pituitary gland, the area postrema, etc.), are different from the capillaries present in other tissues, such as muscles, in that the endothelial cells forming the endothelium of the capillaries join together tightly through strong intercellular junctions. Thus, the passive transfer of substances from the blood to the brain is prevented, and although there are some exceptions, substances rarely migrate from the capillaries to the brain, except highly fat-soluble substances or substances with a small molecular weight (200 to 500 daltons or less) and that are electrically neutral at close to a physiological pH. This mechanism, which restricts the exchange of substances between the blood and the tissue fluid of the brain through the capillary endothelium in the brain, is called the blood-brain barrier (BBB). Further, the blood-brain barrier restricts the exchange of substances between not only the blood and the brain, but also the blood and the tissue fluids of the central nervous system, including the brain and the spinal cord. Due to the presence of the blood-brain barrier, most of the cells in the central nervous system can maintain their biochemical homeostasis without being influenced by changes in concentration of substances in the blood, such as hormones and lymphokines.

As a method for delivering high molecular weight substances into the brain through the blood-brain barrier, there have been reported various methods of modifying the high molecular weight substances for conferring affinity to a transferrin receptor, which is a membrane protein present on the surface of the capillary endothelial cells in the brain (Patent Literatures 1 to 3). For example, Patent Literature 1 describes a blood-brain barrier shuttle having affinity to a transferrin receptor and capable of binding to the receptor.

### Citation List

### Patent Literature

Patent Literature 1: JP-T-2015-528452
Patent Literature 2: JP-A-H06-228199
Patent Literature 3: WO2016/208695
Patent Literature 4: WO2019/151539

### Summary of Invention

### Technical Problem

One aspect of the invention described in this specification has an object of providing a novel peptide that binds to a human transferrin receptor (hTfR).

Another aspect of the invention has an object of further providing a peptide capable of passing through the blood-brain barrier (BBB), a peptide having directivity to a muscle tissue and capable of efficiently migrating to the muscle tissue, and a peptide having a cell-penetrating property.

Another aspect of the invention has an object of providing various applications of the above-mentioned novel peptides. Solution to Problem

One aspect of the invention described in this specification is related to a peptide that binds to a transferrin receptor.

This peptide has an amino acid sequence represented by SEQ ID NO: 1
(Ala-Val-Phe-Val-Trp-Asn-Tyr-Tyr-Ile-Ile-Ser-Cys), or an amino acid sequence having substitutions, deletions, additions, and/or insertions of 1 or more and 10 or less amino acid residues in the amino acid sequence represented by SEQ ID NO: 1. Advantageous Effects of Invention

According to the invention described in this specification, as proved in the Examples, it is possible to provide a peptide that binds to the human transferrin receptor (hTfR), a peptide capable of passing through the blood-brain barrier (BBB), a peptide having directivity to the muscle tissue, a peptide having a cell-penetrating property, and the like.

### Brief Description of Drawings

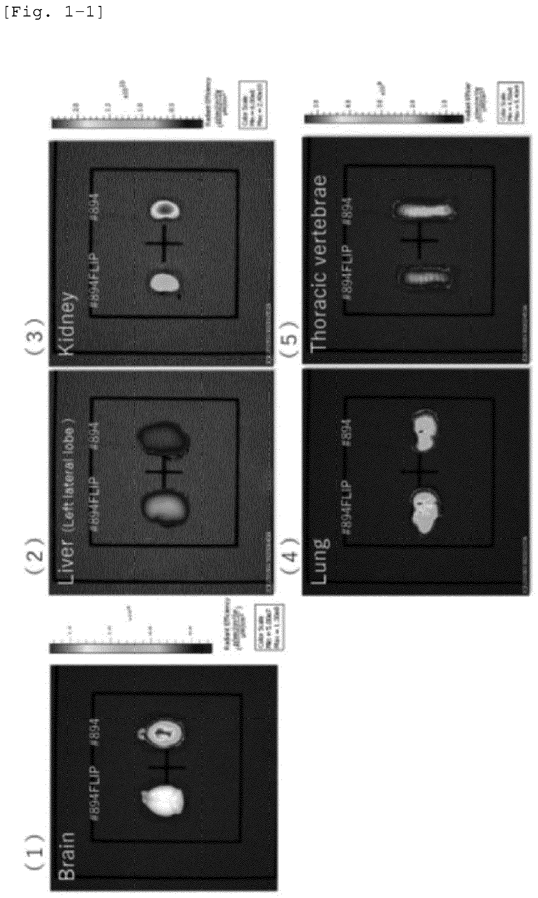

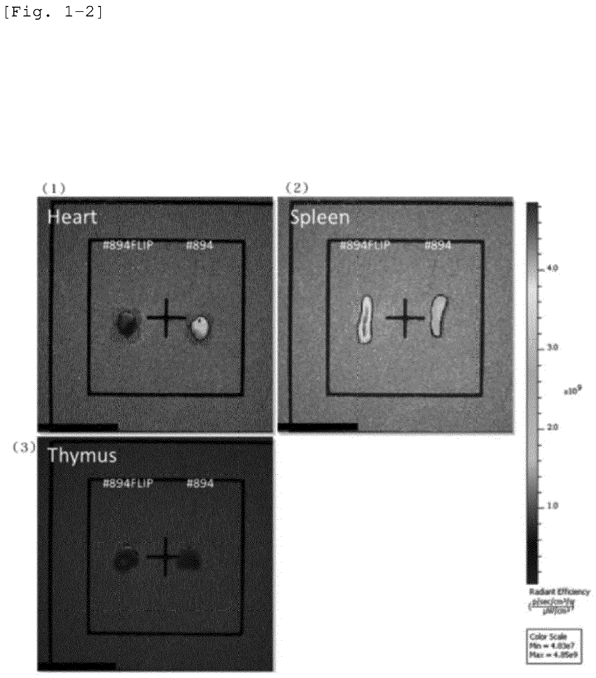

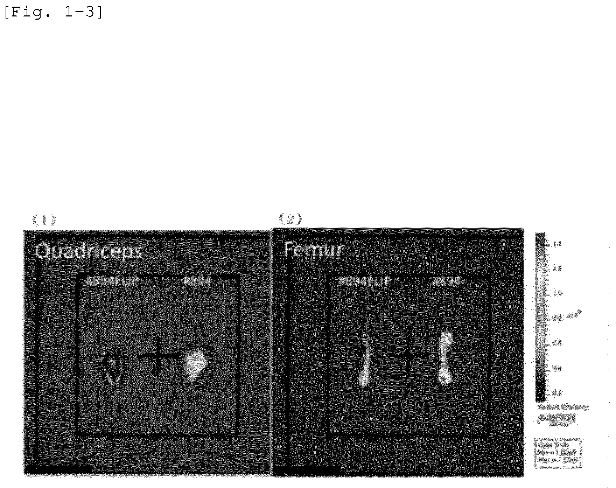

Fig. 1 is a photograph in place of a drawing, showing a fluorescence intensity measurement result in each tissue.
Fig. 1-2 is a photograph in place of a drawing, showing the fluorescence intensity measurement result in each tissue.
Fig. 1-3 is a photograph in place of a drawing, showing the fluorescence intensity measurement result in each tissue.
Fig. 2 is a photograph in place of a drawing, showing the fluorescence intensity measurement result in the enlarged brain.
Fig. 3 is a photograph in place of a drawing, showing a result of a mouse intracerebral localization confirmation test (single dose).
Fig. 4 is a photograph in place of a drawing, showing a result of a mouse intracerebral localization confirmation test (multiple dose).
Fig. 5 is a fluorescence microscope photograph in place of a drawing, showing migration to human breast cancer cells. Description of Embodiments

Hereinafter, embodiments of the present invention will be described using the drawings. The present invention is not limited to the embodiments described below and includes modifications made as appropriate based on the following embodiments within a scope obvious to those skilled in the art.

One aspect of the invention described in this specification relates to a peptide that binds to a transferrin receptor and is capable of passing through the blood-brain barrier.

### Transferrin receptor

Transferrin receptor refers to a receptor that binds to transferrin, a protein present in blood plasma that binds to an iron ion, and that has a function of taking the transferrin into cells. The transferrin receptor is expressed on the surface of various cells, such as reticulocytes, trophoblasts of the placenta, and lymphocytes, and it is suggested that the transferrin receptor is particularly expressed in tumor cells. Further, since the transferrin receptor has a property of triggering endocytosis of cells upon stimulation due to binding to iron ions in the blood plasma, studies have been conducted on allowing substances to pass through the BBB using an antibody or the like that binds to the transferrin receptor as a DDS. In the specification of the present application, a human-derived transferrin receptor is referred to as human TfR, hTfR, or simply TfR, unless otherwise specified.

### Peptides binding to transferrin receptor

Binding to the transferrin receptor (also referred to as having binding activity or having affinity) means specifical binding to the transferrin receptor.

Affinity is represented by an Equilibrium constant (KD) of dissociation between the transferrin receptor and a binding peptide. The Equilibrium constant (KD) is an index representing a binding strength between the transferrin receptor and an antigen-binding site on the binding peptide. As a KD value becomes smaller, the binding strength between the transferrin receptor and the binding peptide becomes stronger (alternately, the affinity can also be represented as an affinity constant (KA) defined by 1/KD) . As is obvious to those skilled in the art, the affinity can be determined by a publicly known method (e.g., based on the further disclosure in the present specification) in accordance with particular target antigens. The binding activity is an index for the binding strength between the transferrin receptor and the binding peptide. The binding activity is related to both the affinity between the transferrin receptor and a binding site thereof on the binding peptide and a number of the relevant binding sites present on the binding molecule.

The specifical binding between the transferrin receptor and the binding peptide can be determined by any appropriate publicly known method, including, for example, a surface plasmon resonance (SPR) assay described in the present specification, Scatchard analysis and/or a radioimmunoassay (RIA), an enzyme immunoassay (EIA), a competition binding assay such as a sandwich competition assay, and different variants thereof publicly known in the technical field. Preferably, the affinity between the peptide of the present invention and the transferrin receptor in terms of KD is less than 100 nM, and preferably less than 50 nM.

### Capable of passing through blood-brain barrier (BBB)

Capable of passing through the BBB means, for example, that substances can be caused to pass through the BBB into the brain, thus making it possible to detect the substances or metabolites thereof at any site in the brain at a certain time after administration, or it becomes possible to gain an insight that allows an analogical inference into an effect that may be exerted by the substances in the brain.

### Brain-related diseases

Brain-related diseases are diseases caused by some kinds of abnormality in the brain and include, for example, central nervous system (CNS) diseases. Examples of the brain-related diseases include, but are not limited to, Alzheimer's disease, Parkinson's disease, prion diseases, Huntington's disease, lysosomal storage diseases, central nervous system disorders, tumors in the central nervous system such as brain tumors, cerebral ischemia, diseases associated with brain damage, traumatic central nervous system injuries, viral and bacterial central nervous system diseases, and mental disorders such as schizophrenia and clinical depression.

### Having directivity to muscle tissue

The muscle tissue may be any of cardiac muscle, skeletal muscle, and smooth muscle. Particularly preferable as the muscle tissue is cardiac muscle or skeletal muscle. Having directivity to muscle tissue means having a property of specifically and efficiently migrating to the muscle tissue.

### Neuromuscular diseases

Neuromuscular diseases are diseases that cause movement disorders such as a loss in muscle strength due to lesions to nerves, such as the brain, the spinal cord, and peripheral nerves, or muscles. Examples of the neuromuscular diseases include, but are not limited to, spinocerebellar ataxia, amyotrophic lateral sclerosis, myasthenia gravis, muscular dystrophy, polymyositis, hereditary myopathy, neuromuscular disorders, muscular atrophy, drug-induced myopathy, acute heart failure, chronic heart failure, myocardial infarction, chronic fatigue syndrome, mitochondrial disease, mitochondrial respiratory chain complex disorders, and Guillain-Barré syndrome.

### Peptide having cell-penetrating property

Peptides having a cell-penetrating property are publicly known, as is described in, for example, Japanese Patent No. 6478632 and Japanese Patent No. 6708770 (cell penetrating peptide). Further, as shown in Examples, the peptide of the present invention binds to the transferrin receptor and is taken into a cell. Thus, using the peptide of the present invention and a conjugate thereof makes it possible to deliver an active target ingredient into cells, for example, making it possible to deliver an oligonucleotide therapeutic into cells.

### Peptides

A peptide refers to a structure of multiple consecutive amino acids, with polypeptides and proteins also included in that definition. Note that amino acids in the present application not only include naturally present amino acids (natural amino acids) taken into a peptide chain when mRNA is translated in a cell, but also amino acids not present in nature (non-natural amino acids) that can constitute a part of a peptide chain through a peptide bond. The amino acid may be artificially synthesized, or it may be one that is present in the nature world.

Further, in the present application, peptides with a circular part formed by cyclization after synthesis (also referred to as a cyclic peptide), peptides obtained by further chemically modifying the peptide, conjugates of a peptide and a substance bound to the peptide, and conjugates in which a peptide and a substance are bound to each other via a linker are also included in the peptides and the conjugates of the peptide and the substance of the present invention.

In the present specification, cyclic peptide refers to a peptide in which two amino acids separated from each other via one or more amino acid residues are bound to each other, causing the amino acid sequence to be wholly or partially cyclic. Note that a type of bonding between the two amino acids is not particularly limited, and examples of the cyclic peptide include those in which a cyclic structure is formed by an amide bond between a carboxyl group of one amino acid and an amino group of another amino acid, a thioether bond between a carboxyl group of one amino acid and a thiol group of another amino acid, a thiol bond between a thiol group of one amino acid and a thiol group of another amino acid, or lactam ring formation or a macrocyclization reaction, those having a lasso peptide-like structure, and the like. However, when the two amino acids are bonded to each other by an amide bond, the amide bond is not limited to the one formed by the bond of a carboxyl group of one amino acid and an amino group of another amino acid, but may be bound by an amide bond resulting from a synthesis reaction. The same also applies to other types of bonds.

That is, the cyclic peptide in the present application may be one in which a part thereof forms the cyclic structure, and it may also have a linear portion.

Note that, in the present specification, there are cases in which a part of an amino acid is modified for cyclizing the peptide. Such kinds of partially modified amino acids are also included. A case such as performing cyclization by adding a chloroacetyl group to the amino acid located at an N-terminus and binding it to a cysteine residue in the peptide is given as an example, and various types of (natural/non-natural) amino acids to which the chloroacetyl group is added are also included in the amino acids of the present application.

Non-natural amino acids refer to compounds that have the characteristics of amino acids, other than the natural amino acids. Examples of the non-natural amino acids include, but are not limited to, β-amino acid, γ-amino acid, and L-amino acid; D-amino acid (also referred to as D-configuration amino acid) ; a chemically modified amino acid such as an amino acid variant or an amino acid derivative; an amino acid that is not a constituent material of protein in vivo such as norleucine, β -alanine, or ornithine; and the like. N-methyl amino acid, N-ethyl amino acid, D-amino acid, a histidine-like amino acid, an amino acid having a structure in which additional methylene or an aromatic ring is added to a side chain, an amino acid derivative having a structure in which a carboxylic acid functional group in the side chain is replaced with a sulfonic acid group, and the like are also included.

Examples of the non-natural amino acids and abbreviations thereof are indicated in the present specification as follows. The CAS reference number or supplier company name is indicated in parentheses, and newly synthesized amino acids are indicated by the synthesis example number. Note that while the CAS number represents the non-natural amino acid alone or the non-natural amino acid to which a protective group is bound, special amino acids are not limited to these, and examples of the special amino acids include those having a structure in which one or a plurality of hydrogen atoms in these molecules are replaced with an alkyl group. When the hydrogen atom is replaced with an alkyl group, the alkyl group is preferably a methyl group or an ethyl group, and more preferably a methyl group. Note that in the present specification, "Me" or "N-Me-" denoted at the front of an amino acid name represents N-methyl amino acid, unless otherwise specified. For example, N-methylated amino acid of alanine (Ala or A) is represented by MeAla, N-MeAla, MeA, or N-MeA. Further, a single letter code of an amino acid designation with a "d" denoted in front represents D-amino acid. For example, D-amino acid of alanine (Ala or A) is represented by "da". Reagents without a CAS number or supplier listed can be purchased as general reagents . Note that the following amino acids can be used for peptide synthesis by protecting their alpha amino groups with Fmoc using a known method.
Yph (S)-2-amino-3-(4-phenoxyphenyl)propanoic acid (CAS number: 180414-93-1)
W7OMe (S)-2-amino-3-(7-methoxy-1H-indole-3-yl)propanoic acid (CAS number: 2416720-26-6)
W7N
   (*S*)-2-amino-3-(1H-pyrrolo[2,3-β]pyridin-3-yl)propanoi
c acid (CAS number: 737007-45-3)
W7F (S)-2-amino-3-(7-fluoro-1H-indole-3-yl)propanoic acid (CAS number: 1956434-65-3)
W6N
   (S)-2-amino-3-(1H-pyrrolo[2,3-c]pyridin-3-yl)propanoi c acid (Kishida Chemical)
W6F (S)-2-amino-3-(6-fluoro-1H-indole-3-yl)propanoic acid (CAS number: 908847-01-8)
W5OMe 5-methoxy-L-tryptophan (CAS number: 460751-69-3)
W5F (S)-2-amino-3-(5-fluoro-1H-indole-3-yl)propanoic acid (CAS number: 908846-88-8)
W4OMe 4-methoxy-L-tryptophan (CAS number: 1205553-56-5)
W4N (S)-2-amino-3-(1H-pyrrolo
[3,2-β]pyridin-3-yl)propanoic acid (CAS number: 149818-23-5) W4F
   (S)-2-((((9H-fluorene-9-yl)-methoxy)carbonyl)amino)-3 -(1-(tert-butoxycarbonyl)-4-fluoro-1H-indol-3-yl)propanoic acid (CAS number: 2244532-65-6)
W4C (S)-2-amino-3-(4-chloro-1H-indole-3-yl)propanoic acid (CAS number: 2244532-68-9)
W2N (S)-2-amino-3-(1H-indole-3-yl)propanoic acid (CAS number: 2305185-20-8)
WliPr 1-isopropyl-L-tryptophan (CAS number: 1496563-42-8) W1Et7Cl
   (S)-2-amino-3-(7-chloro-1-ethyl-1H-indole-3-yl)propan oic acid (Synthesis Example 2-1)
W1Et 1-ethyl-L-tryptophan (CAS number: 168471-23-6)
Tbg (S)-2-amino-3,3-dimethylbutanoic acid (CAS number: 33105-81-6)
pHPeG N-(4-hydroxyphenethyl)glycine (CAS number: 258332-56-8)
PeG N-(2-phenylethyl)-glycine (CAS number: 540483-58-7)
Nva L-norvaline (CAS number: 6600-40-4)
Nle N-α-chloroacetyl-L-norleucine (CAS number: 688-12-0)
Nal2 β-(2-naphthyl)L-alanine (CAS number: 58438-03-2)
Nal1 β-(1-naphthyl)L-alanine (CAS number: 2353616-32-5)
MeoBph N-α-methyl-2-phenyl-L-phenylalanine
MeNal2 N-α-methyl-β-(2-naphthyl)-L-alanine (CAS number: 179385-30-9)
MeNal1 N-α-methyl-β-(1-naphthyl)-L-alanine (CAS number: 1380327-68-3)
MemBph N-α-methyl-3-phenyl-L-phenylalanine
Hph L-homophenylalanine (CAS number: 943-73-7)
Hly (S)-2,7-diaminoheptanoic acid (CAS number: 498-56-6)
F4OMe (S)-2-amino-3-(4-methoxyphenyl)propanoic acid (CAS number: 7635-29-2)
F4G (4-guanidyl)-L-phenylalanine (CAS number: 59574-11-7)
F4F 4-fluoro-L-phenylalanine (CAS number: 1132-68-9)
F4C N-α-chloroacetyl-4-chloro-L-phenylalanine (CAS number: 14173-39-8)
F3OMe (S)-2-amino-3-(3-methoxyphenyl)propanoic acid (CAS number: 98813-19-5)
F3F 3-fluoro-L-phenylalanine (CAS number: 19883-77-3)
F3C N-α-chloroacetyl-3-chloro-L-phenylalanine (CAS number: 80126-51-8)
F2OMe (S)-2-amino-3-(2-methoxyphenyl)propanoic acid (CAS number: 206060-41-5)
F2C (S)-2-amino-3-(2-chlorophenyl)propanoic acid (CAS number: 198560-41-7)
MeF4OMe
   (S)-3-(4-methoxyphenyl)-2-(methylamino)propanoic acid (CAS number 1260595-45-6)
MeF4F N-α-methyl-4-fluoro-L-phenylalanine (CAS number: 1979176-87-8)
MeF3F N-α-methyl-3-fluoro-L-phenylalanine (CAS number: 1820567-10-9)
MeF3C N-α-methyl-3-chlorofluoro-L-phenylalanine (CAS number: 1446478-28-9)
MeBph N-α-methyl-4-phenyl-L-phenylalanine
Me4Py N-α-methyl-4-pyridyl-L-alanine
Me3Py N-α-methyl-3-pyridyl-L-alanine
dr D-arginine
dp D-proline
dc D-cysteine
dk D-lysine
Dap L-α,β-diaminopropanoic acid (CAS number: 515-94-6)
Dab (S)-4-amino-2-(2-chloroacetamide)butanoic acid (CAS number: 25691-37-6)
Cit 2-amino-5-ureidopentanoic acid (CAS number: 627-77-0)
Cha β-cyclohexyl-L-alanine (CAS number: 4441-50-3)
CeG N-(2-carboxyethyl)-glycine (CAS number: 174799-89-4)
Cbg (S)-2-amino-2-cyclobutylacetic acid (CAS number: 1391630-31-1)
Cba Cyclobutylalanine (CAS number: 478183-62-9)
aMeY α-methyl-L-tyrosine (CAS number: 658-48-0)
aMeW α-methyl-tryptophan (CAS number: 153-91-3)
aMeK α-methyl-lysine (CAS number: 111717-28-3)
aMeC α-methyl-L-cysteine (CAS number: 441317-73-3)
Aib α-methylalanine (CAS number: 62-57-7)
Ahp/Alahp (S)-2-aminoheptanoic acid (CAS number: 1115-90-8)
Abu L-α-aminobutanoic acid (CAS number: 1492-24-6)
A4paa
   (S)-2-amino-3-(1-(carboxymethyl)piperazine-4-yl)propa noic acid (Kishida Chemical)
5Ind (S)-2-amino-3-(1H-indole-5-yl)propanoic acid (CAS number: 1655518-66-3)
4Py2NH2 (S)-2-amino-3-(2-aminopyridine-4-yl)propanoic acid (Kishida Chemical)
4Py 4-pyridyl-L-alanine (CAS number: 1956-21-4)
3Py6NH2 2-amino-3-(6-aminopyridine-3-yl)propanoic acid

### (Synthesis Example 2-3)

3Py 3-pyridyl-L-alanine (CAS number: 17470-24-5)
Wlaa 1-(carboxymethyl)-L-tryptophan (CAS number: 773823-50-0)
KCOpipzMe N6-(4-methylpiperazine-1-carbonyl)-L-lysine (Kishida Chemical)
W1mCON 1-(2-amino-2-oxoethyl)-L-tryptophan (Synthesis Example 2-5)
W1EtOH 1-(2-hydroxyethyl)-L-tryptophan (Synthesis Example 2-9)
3Py6OMe (S)-2-amino-3-(6-methoxypyridin-3-yl)propanoic acid (CAS number: 1270317-99-1)
Epyrl2RCOO
   2-(5-((R)-2-((allyloxy)carbonyl)pyrrolidine-1-yl)-5-o xopentanoic acid
Dpyrl2RCOO
   2-(4-((R)-2-((allyloxy)carbonyl)pyrrolidine-1-yl)-4-o xobutanoic acid (Example 9-16)
MeF3COO 3-carboxy-N-methyl-phenylalanine (CAS number: 1499826-56-0)
3Imp 2-amino-3-(imidazo [1,2-a]pyridine-3-yl)propanoic acid (CAS number: 2276942-95-9)
KaAc N6-glycyl-L-lysine (Synthesis Example 2-8)
A1Me4pip 4-amino-1-methylpiperazine-4-carboxylic acid (CAS number: 15580-66-2)
Har N6-carbamimidoyl-L-lysine (CAS number: 156-86-5)
Acpr (S)-2-amino-3-cyclopropylpropanoic acid (CAS number: 1492156-90-7)
Atb (S)-2-amino-4,4-dimethylpentanoic acid (CAS number: 1934633-35-8)
MeF35dC
   (S)-3-(3,5-dichlorophenyl)-2-(methylamino)propanoic acid (CAS number: 1542508-65-5)
   - Adod: 12-aminododecanoic acid
   - Hly: L-homolysine
   - W5C: 5-chloro-L-tryptophan
   - F3COO: L-3-carboxyphenylalanine
   - F3CON: L-3-carbamoylphenylalanine
   - Hgl: L-2-aminoadipic acid
   - Ndm: N,N-dimethyl-L-asparagine
   - KN3 or LysN3: 6-azido-L-norleucine
   - KAc: N6-acetyl-L-lysine
   - dorn: D-ornithine
   - Nle: L-norleucine
   - F3H: 3-hydroxy-L-phenylalanine
   - Yae: O-(2-aminomethyl)-L-tyrosine
   - F4aao: O-(2-carboxymethyl)-L-tyrosine
   - F4OEt: O-ethyl-L-tyrosine
   - F34dOMe: 3,4-dimethoxy-L-phenylalanine
   - alT: L- allothreonine
   - all: L-alloisoleucine
   - MeK: N-methyl-L-lysine
   - Tbg: (S)-2-amino-3,3-dimethylbutyric acid
   - Nva: L-norvaline
   - Abu: (S)-(+)-2-aminobutyric acid
   - da: D-alanine
   - Bph: 4-phenyl-L-phenylalanine
   - de: D-glutamic acid
   - MeA: N-methyl-L-alanine
   - PEG4c or PEG3: 1-amino-3,6,9,12-tetraoxapentadecane-15-oic acid
   - MeR: N-methyl-L-arginine
   - MeW: N-methyl-L-tryptophan
   - PEG8c: 1-amino-3,6,9,12,15,18,21,24-octaoxaheptacosane-27-oi c acid
   - PEG12c or PEG11 or PEG12: 1-amino-3,6,9,12,15,18,21,24,27,30,33,36-dodecaoxanon atriacontane-39-oic acid

Note that newly synthesized amino acids are useful as they have the potential to add new functions to various peptides when various peptide derivatives are produced.

The peptide of the present invention has an amino acid sequence represented by SEQ ID NO: 1
(Ala-Val-Phe-Val-Trp-Asn-Tyr-Tyr-Ile-Ile-Ser-Cys), or an amino acid sequence having substitutions, deletions, additions, and/or insertions of 1 or more and 10 or less amino acid residues in the amino acid sequence represented by SEQ ID NO: 1.

In respect of peptide sequences

The number of amino acids subjected to amino acid substitution, deletion, addition, and/or insertion may be 1 or more and 10 or less, with a lower limit of 1. An upper limit is 10, 9, 8, 7, 6, 5, 4, 3, or 2, with a minimum of 1. The amino acid substitution is suitably a conservative amino acid substitution.

Conservative amino acid substitution

Conservative amino acid substitution refers to substitution with a functionally Equivalent or similar amino acid. The conservative amino acid substitution in a peptide causes a static change in the amino acid sequence of the peptide. For example, one or two or more amino acids having a same polarity act functionally Equivalently in causing a static change in the amino acid sequence of the peptide. In general, a substitution within a certain group can be considered to be conservative in terms of structure and function. However, as is obvious to those skilled in the art, a role of a particular amino acid residue can be determined in the context of a three-dimensional structure of a molecule containing the particular amino acid. For example, a cysteine residue can take an oxidized (disulfide) form having lower polarity than a reduced (thiol) form. A long aliphatic moiety in an arginine side chain can constitute structurally and functionally important features. Further, an aromatic ring-containing side chain (tryptophan, tyrosine, and phenylalanine) can contribute to ion-aromatic interaction or cation-pi interaction. In such a case, substitution of the amino acids having these side chains with amino acids belonging to an acidic or non-polar group can be structurally and functionally conservative. Residues of proline, glycine, cysteine (disulfide form), and the like may have a direct effect on a three-dimensional structure of the main chain, which often makes it not possible to substitute these amino acids without causing structural distortion.

The conservative amino acid substitution includes, as shown below, specific substitution based on side chain similarity (L. Lehninger, Biochemistry, 2nd edition, pp. 73-75, Worth Publisher, New York (1975)) and typical substitution.

Preferable examples of this peptide include the amino acid sequence having one or more substitutions selected from the following groups:
(I) substitution of the 1st alanine residue of SEQ ID NO: 1 with an aliphatic amino acid or a methylated aliphatic amino acid;
(II) substitution of the 2nd valine residue of SEQ ID NO: 1 with a basic amino acid residue or a methylated basic amino acid residue;
(III) substitution of the 3rd phenylalanine residue of SEQ ID NO: 1 with an aromatic amino acid residue, a methylated aromatic amino acid residue, an amino acid residue with an aromatic ring added, or an amino acid residue with a condensed ring added;
(IV) substitution of the 4th valine residue of SEQ ID NO: 1 with a methylated valine residue;
(V) substitution of the 5th tryptophan residue of SEQ ID NO: 1 with an aromatic amino acid residue, a methyltryptophan residue, a methylated aromatic amino acid residue, an amino acid residue with an aromatic ring added, or an amino acid residue with a condensed ring added;
(VI) substitution of the 6th asparagine residue of SEQ ID NO: 1 with a neutral amino acid or a methylated neutral amino acid;
(VII) substitution of the 7th and 8th tyrosine residues of SEQ ID NO: 1 with aromatic amino acid residues, methylated aromatic amino acid residues, amino acid residues with aromatic rings added, or amino acid residues with condensed rings added;
(VIII) substitution of the 9th isoleucine residue of SEQ ID NO: 1 with an aliphatic amino acid residue, a methylated aliphatic amino acid residue, or an amino acid residue having a branched chain structure;
(IX) substitution of the 10th isoleucine residue of SEQ ID NO: 1 with any amino acid; and
(X) substitution of the 11th serine residue of SEQ ID NO: 1 with a neutral amino acid residue.

(1) Nonpolar amino acid groups: alanine (hereinafter, referred to as "Ala" or simply as "A"), valine (hereinafter, referred to as "Val" or simply as "V"), leucine (hereinafter, referred to as "Leu" or simply as "L"), isoleucine (hereinafter, referred to as "Ile" or simply as "I"), proline (hereinafter, referred to as "Pro" or simply as "P"), phenylalanine (hereinafter, referred to as "Phe" or simply as "F"), tryptophan (hereinafter, referred to as "Trp" or simply as "W"), and methionine (hereinafter, referred to as "Met" or simply as "M")
(2) Uncharged polar amino acid groups: glycine (hereinafter, referred to as "Gly" or simply as "G"), serine (hereinafter, referred to as "Ser" or simply as "S"), threonine (hereinafter, referred to as "Thr" or simply as "T"), cysteine (hereinafter, referred to as "Cys" or simply as "C"), tyrosine (hereinafter, referred to as "Tyr" or simply as "Y"), asparagine (hereinafter, referred to as "Asn" or simply as "N"), and glutamine (hereinafter, referred to as "Gln" or simply as "Q")
(3) Acidic amino acid groups: aspartic acid (hereinafter, referred to as "Asp" or simply as "D") and glutamic acid (hereinafter, referred to as "Glu" or simply as "E")
(4) Basic amino acid groups: lysine (hereinafter, referred to as "Lys" or simply as "K"), arginine (hereinafter, referred to as "Arg" or simply as "R"), and histidine (hereinafter, referred to as "His" or simply as "H")

Further, the amino acids present in the natural world can be divided into the following groups based on their common side chain properties.
(1) Hydrophobic amino acid groups: Norleucine, Met, Ala, Val, Leu, and Ile
(2) Neutral hydrophilic amino acid groups: Cys, Ser, Thr, Asn, and Gln
(3) Acidic amino acid groups: Asp and Glu
(4) Basic amino acid groups: His, Lys, and Arg
(5) Groups of amino acids influencing direction of main chain: Gly and Pro
(6) Aromatic amino acid groups: Trp, Tyr, and Phe Note that each group also includes non-natural amino acids such as N-methylated amino acid and the like.

A preferable example of this peptide is a peptide having an amino acid sequence represented by SEQ ID NO: 2 (Ala-Val-Phe-Val-Trp-Asn-Tyr-Tyr-Ile-Ile-Arg-Arg-Tyr-MeTyr-Cys) .

Preferable examples of this peptide include the amino acid sequence having one or more substitutions selected from the following groups in the amino acid sequence represented by SEQ ID NO: 2:
(I) substitution of the 1st alanine residue of SEQ ID NO: 2 with an aliphatic amino acid or a methylated aliphatic amino acid;
(II) substitution of the 2nd valine residue of SEQ ID NO: 2 with a basic amino acid residue or a methylated basic amino acid residue;
(III) substitution of the 3rd phenylalanine residue of SEQ ID NO: 2 with an aromatic amino acid residue, a methylated aromatic amino acid residue, an amino acid residue with an aromatic ring added, or an amino acid residue with a condensed ring added;
(IV) substitution of the 4th valine residue of SEQ ID NO: 2 with a methylated valine residue;
(V) substitution of the 5th tryptophan residue of SEQ ID NO: 2 with an aromatic amino acid residue, a methylated aromatic amino acid residue, an amino acid residue with an aromatic ring added, or an amino acid residue with a condensed ring added;
(VI) substitution of the 6th asparagine residue of SEQ ID NO: 2 with a neutral amino acid or a methylated neutral amino acid;
(VII) substitution of the 7th and 8th tyrosine residues of SEQ ID NO: 2 with aromatic amino acid residues, methylated aromatic amino acid residues, amino acid residues with aromatic rings added, or amino acid residues with condensed rings added;
(VIII) substitution of the 9th isoleucine residue of SEQ ID NO: 2 with an aliphatic amino acid residue, a methylated aliphatic amino acid residue, or an amino acid residue having a branched chain structure;
(IX) substitution of the 10th isoleucine residue of SEQ ID NO: 2 with any amino acid;
   (XI) substitution of the 11th and 12th arginine residues of SEQ ID NO: 2 with basic amino acid residues;
(XII) substitution of the 13th tyrosine residue of SEQ ID NO: 2 with a hydrophilic amino acid residue;
(XIII) substitution of the 14th methyl tyrosine residue of SEQ ID NO: 2 with a tyrosine residue, an aromatic amino acid residue, or a methylated aromatic amino acid residue; and
(XIV) substitution of the 15th cysteine residue of SEQ ID NO: 2 with a methylated cysteine residue.

Another preferable example of this peptide is:
a peptide A; or
a peptide A having an amino acid sequence with substitutions, deletions, and/or insertions of 1 or more and 6 or less amino acid residues,
the peptide A having a peptide length of 10 or more and 17 or less and including the 1st to 10th amino acid sequence represented by SEQ ID NO: 18
   (Ala-Val-Phe-Val-Trp-Asn-Tyr-Tyr-Ile-Ile-Ser-Cys) .

An example of a preferable peptide in this specification is a peptide capable of binding to the human transferrin receptor (hTfR) in the same manner as the above-mentioned peptides. Further, preferable examples of the peptide include a peptide capable of passing through the blood-brain barrier (BBB), a peptide having directivity to the muscle tissue, or a peptide having a cell-penetrating property.

The peptide A may be a peptide having an amino acid sequence in which any of the 2nd, 3rd, 5th, 8th, and 10th amino acid residues of SEQ ID NO: 18 is substituted.

Amino acid residue is substituted means that a specific amino acid residue is substituted with another amino acid residue that may be modified.

The peptide A may be a peptide in which:
the 2nd amino acid residue of SEQ ID NO: 18 is valine (Val) that may be modified or glutamic acid (Glu) that may be modified;
the 3rd amino acid residue of SEQ ID NO: 18 is phenylalanine (Phe) that may be modified;
the 5th amino acid residue of SEQ ID NO: 18 is tryptophan (Trp) that may be modified;
the 8th amino acid residue of SEQ ID NO: 18 is tyrosine (Tyr) that may be modified; and/or
the 10th amino acid residue of SEQ ID NO: 18 is isoleucine (Ile) that may be modified or valine (Val) that may be modified.

May be modified means that a publicly known amino acid modification or change may be made. Examples of the modification include N-methylation, amino acid modifications with the below-mentioned abbreviations, modification (conversion) to a D-configuration, and conversion of the amino acids to their publicly known derivatives.

This peptide preferably has a peptide length of 11 or more and 13 or less.

This peptide is more preferably a peptide having the following amino acid sequence.

The peptide may be a peptide in which:
the 2nd amino acid residue of SEQ ID NO: 18 is Val or Glu;
the 3rd amino acid residue of SEQ ID NO: 18 is Phe or MeF3C;
the 5th amino acid residue of SEQ ID NO: 18 is Trp or MeTrp;
the 8th amino acid residue of SEQ ID NO: 18 is Tyr or F4OMe; and/or
the 10th amino acid residue of SEQ ID NO: 18 is Ile or Val.

Further, this peptide has the following amino acid sequence at the N-terminus of the peptide A.

The peptide may be a peptide in which:
the 11th amino acid residue of SEQ ID NO: 18 is Ser or His; and/or
the 12th amino acid residue of SEQ ID NO: 18 is Cys or Hgl.

Another preferable example of this peptide is:
a peptide B; or
a peptide B having an amino acid sequence with substitutions, deletions, and/or insertions of 1 or more and 5 or less amino acid residues,
the peptide B having a peptide length of 10 or more and 19 or less and including the 1st to 10th amino acid sequence represented by SEQ ID NO: 15
   (Ala-Val-Phe-Val-Trp-Asn-Tyr-Tyr-Ile-Val-Pro-Arg-Asp-Cys) .

The peptide B may be a peptide having an amino acid sequence in which any of the 2nd, 3rd, 5th, 8th, and 10th amino acid residues of SEQ ID NO: 15 is substituted.

The peptide B may be a peptide in which:
the 2nd amino acid residue of SEQ ID NO: 15 is valine (Val) that may be modified or glutamic acid (Glu) that may be modified;
the 3rd amino acid residue of SEQ ID NO: 15 is phenylalanine that may be modified (Phe) or tryptophan (Trp);
the 5th amino acid residue of SEQ ID NO: 15 is tryptophan (Trp) that may be modified;
the 8th amino acid residue of SEQ ID NO: 15 is tyrosine (Tyr) that may be modified; and/or
the 10th amino acid residue of SEQ ID NO: 15 is isoleucine (Ile) that may be modified or valine (Val) that may be modified.

These peptides may have a peptide length of 13 or more and 15 or less.

This peptide more preferably has the following amino acid sequence.

The peptide may be a peptide in which:
the 2nd amino acid residue of SEQ ID NO: 15 is Val or Glu;
the 3rd amino acid residue of SEQ ID NO: 15 is Phe, Trp, or MeF3C;
the 5th amino acid residue of SEQ ID NO: 15 is Trp or MeTrp;
the 8th amino acid residue of SEQ ID NO: 15 is Tyr, Phe, or F4OMe; and/or
the 10th amino acid residue of SEQ ID NO: 15 is Ile or Val.

Further, this peptide has the following amino acid sequence at the N-terminus of the peptide B.

The peptide may be a peptide in which:
the 11th amino acid residue of SEQ ID NO: 15 is Phe;
the 12th amino acid residue of SEQ ID NO: 15 is Arg;
the 13th amino acid residue of SEQ ID NO: 15 is Glu, Asn, Asp, His, Gln, or MeTrp; and/or
the 14th amino acid residue of SEQ ID NO: 15 is Cys.

Another preferable example of this peptide is:
a peptide C; or
a peptide C having an amino acid sequence with substitutions, deletions, and/or insertions of 1 or more and 5 or less amino acid residues,
the peptide C having a peptide length of 11 or more and 19 or less and including the 1st to 10th amino acid sequence represented by SEQ ID NO: 214
   (MeA-Val-MeF3C-Val-MeW-Asn-Tyr-F4OMe-Ile-Ile-Arg-Arg-Phe-Me Y-Cys).

The peptide C may have an amino acid sequence in which any of the 1st, 3rd, 5th, and 8th amino acid residues of SEQ ID NO: 214 is substituted.

The peptide C may be a peptide in which:
the 1st amino acid residue of SEQ ID NO: 214 is alanine (Ala) that may be modified or glutamic acid (Glu) that may be modified;
the 3rd amino acid residue of SEQ ID NO: 214 is phenylalanine (Phe) that may be modified;
the 5th amino acid residue of SEQ ID NO: 214 is tryptophan (Trp) that may be modified; and/or
the 8th amino acid residue of SEQ ID NO: 214 is phenylalanine (Phe) that may be modified.

These peptides preferably have a peptide length of 15 or more and 18 or less.

This peptide more preferably has the following amino acid sequence.

The peptide may be a peptide in which:
the 1st amino acid residue of SEQ ID NO: 214 is Ala, Aib, Abu, Glu, Gly, Ser, Phe, Pro, or MeA, and particularly preferably Ala or MeA;
the 3rd amino acid residue of SEQ ID NO: 214 is Phe, F3C, F2C, F2OMe, F4C, Cha, MeF, MeF35dC, MeF4F, MeF4Ome, MeNal1, Me3Py, Me4Py, Me3OMe, MeF3COO, MeF3F, Glu, Epyrl2RCOO, Dpyrl2RCOO, or MeF3C, and particularly preferably Phe, MeF, or MeF3C;
the 5th amino acid residue of SEQ ID NO: 214 is Trp, MeW, aMeW, dp, F3C, F3F, F3OMe, F4C, F4F, Hph, MemBph, MeNal1, MeNal2, MeoBph, W4OMe, W1Et, W1Et7Cl, W1iPr, Yph, W1Pr, W5C, W5F, W1aa, W1EtOH, W4OMe, W1mCON, or W6F, and particularly preferably Trp or MeW; and/or
the 8th amino acid residue of SEQ ID NO: 214 is Phe, Tyr, Typ, Ahp, MeY, F4OMe, 3Imp, 4Py, 3Py, 3Py6OMe, F3C, F3CON, F4C, F4aao, F4F, F4OEt, MeF34dOMe, Yae, Lys, Orn, or Nal1, and particularly preferably Tyr or F4OMe.

Further, this peptide has the following amino acid sequence at the N-terminus of the peptide C.

The peptide may be a peptide in which:
the 11th amino acid residue of SEQ ID NO: 214 is Arg, Ala, Asp, Gly, Glu, Lys, MeK, MeR, Dap, Dap, Abu, Aib, Hly, dorn, aMeK, A1Me4pip, KCOpipzMe, F4G, Nle, Nva, or Orn, and particularly preferably Lys or Arg;
the 12th amino acid residue of SEQ ID NO: 214 is Lys, Arg, dr, Tyr, F4G, Orn, Hly, da, Cit, Dap, or Dab, and particularly preferably Lys, Arg, or dr;
the 13th amino acid residue of SEQ ID NO: 214 is Ala, Phe, Asn, Tyr, or pHPeG, and particularly preferably Phe or Tyr;
the 14th amino acid residue of SEQ ID NO: 214 is MeTyr, Tyr, Phe, Ala, aMeY, Glu, Gly, Arg, Val, MeoBph, MeBph, MeF, MemBph, MeNal1, MeNal2, MeoBph, MeW, or pHPeG, and particularly preferably Phe or MeW; and/or
the 15th amino acid residue of SEQ ID NO: 214 is Cys or Hgl.

Another preferable example of this peptide is:
a peptide D; or
a peptide D having an amino acid sequence with substitutions, deletions, and/or insertions of 1 or more and 5 or less amino acid residues,
the peptide D having a peptide length of 11 or more and 19 or less and including the 1st to 10th amino acid sequence represented by SEQ ID NO: 219
   (Ala-Glu-Phe-Val-Trp-Asn-Tyr-Tyr-Ile-Ile-Arg-Arg-Tyr-MeY-Cy s) .

The peptide D may have the amino acid sequence in which any of the 2nd, 3rd, 5th, 8th, and 10th amino acid residues of SEQ ID NO: 219 is substituted.

The peptide D may be a peptide in which:
the 2nd amino acid residue of SEQ ID NO: 219 is valine (Val) that may be modified, glutamic acid (Glu) that may be modified, arginine (Arg) that may be modified, lysine (Lys) that may be modified, aspartic acid (Asp) that may be modified, or phenylalanine (Phe) that may be modified;

the 3rd amino acid residue of SEQ ID NO: 219 is phenylalanine (Phe) that may be modified;
the 5th amino acid residue of SEQ ID NO: 219 is tryptophan (Trp) that may be modified;
the 8th amino acid residue of SEQ ID NO: 219 is tyrosine (Tyr) that may be modified; and/or
the 10th amino acid residue of SEQ ID NO: 219 is isoleucine (Ile) that may be modified, glutamic acid (Glu) that may be modified, or lysine (Lys) that may be modified.

These peptides preferably have a peptide length of 15 or more and 18 or less.

This peptide more preferably has the following amino acid sequence.

The peptide may be a peptide in which:
the 2nd amino acid residue of SEQ ID NO: 219 is Val, Glu, Ala, Arg, Lys, Asp, Phe, Dap, Har, Abu, Nva, AcPr, Atb, Ahp, or Hgl, and particularly preferably Gln or Val;
the 3rd amino acid residue of SEQ ID NO: 219 is Phe, F3C, F2C, F2OMe, F4C, Cha, MeF, MeF35dC, MeF4F, MeF4Ome, MeNal1, Me3Py, Me4Py, Me3OMe, MeF3COO, MeF3F, Glu, Epyrl2RCOO, Dpyrl2RCOO, or MeF3C, and particularly preferably Phe, MeF, or MeF3C;
the 5th amino acid residue of SEQ ID NO: 219 is Trp, MeW, aMeW, dp, F3C, F3F, F3OMe, F4C, F4F, Hph, MemBph, MeNal1, MeNal2, MeoBph, W4OMe, W1Et, W1Et7Cl, W1iPr, Yph, W1Pr, W5C, W5F, W1aa, W1EtOH, W4OMe, W1mCON, or W6F, and particularly preferably Trp or MeW;
the 8th amino acid residue of SEQ ID NO: 219 is Phe, Tyr, Typ, Ahp, MeY, F4OMe, 3Imp, 4Py, 3Py, 3Py6OMe, F3C, F3CON, F4C, F4aao, F4F, F4OEt, MeF34dOMe, Yae, Lys, Orn, or Nal1, and particularly preferably Tyr or F4OMe; and/or
the 10th amino acid residue of SEQ ID NO: 219 is Ala, Abu, Acpr, Ahp, Aib, all, alT, Atb, Dab, Dap, dorn, Gln, Hly, Ile, Lys, KCOpipzMe, Leu, Nle, Nva, Pro, Arg, Ser, Thr, Tbg, Val, or Tyr, and particularly preferably Ile or alI.

Further, this peptide has the following amino acid sequence at the N-terminus of the peptide C.

The peptide may be a peptide in which:
the 11th amino acid residue of SEQ ID NO: 219 is Arg, Ala, Asp, Gly, Glu, Lys, MeK, MeR, Dap, Dap, Abu, Aib, Hly, dorn, aMeK, A1Me4pip, KCOpipzMe, F4G, Nle, Nva, or Orn, and particularly preferably Lys or Arg;
the 12th amino acid residue of SEQ ID NO: 219 is Lys, Arg, dr, Tyr, F4G, Orn, Hly, da, Cit, Dap, or Dab, and particularly preferably Lys, Arg, or dr;
the 13th amino acid residue of SEQ ID NO: 219 is Ala, Phe, Asn, Tyr, or pHPeG, and particularly preferably Phe or Tyr;
the 14th amino acid residue of SEQ ID NO: 219 is MeTyr, Tyr, Phe, Ala, aMeY, Glu, Gly, Arg, Val, MeoBph, MeBph, MeF, MemBph, MeNal1, MeNal2, MeoBph, MeW, or pHPeG, and particularly preferably Phe or MeW; and/or
the 15th amino acid residue of SEQ ID NO: 219 is Cys or Hgl.

Another preferable example of this peptide is:
a peptide E; or
a peptide E having an amino acid sequence with substitutions, deletions, and/or insertions of 1 or more and 5 or less amino acid residues,
the peptide E having a peptide length of 15 or more and 18 or less and including the 1st to 15th amino acid sequence represented by SEQ ID NO: 296
   (Ala-Val-MeF-Val-Trp-Asn-Tyr-Tyr-Ile-Ile-Arg-Arg-Tyr-MeY-Cy s) .

The peptide E may have the amino acid sequence in which any of the 3rd, 5th, 7th, 8th, 11th, 12th, and 13th amino acid residues of SEQ ID NO: 296 is substituted.

The peptide E may be a peptide in which:
the 3rd amino acid residue of SEQ ID NO: 296 is phenylalanine (Phe) that may be modified;
the 5th amino acid residue of SEQ ID NO: 296 is tryptophan (Trp) that may be modified;
the 7th amino acid residue of SEQ ID NO: 296 is tyrosine (Tyr) that may be modified;
the 8th amino acid residue of SEQ ID NO: 296 is tyrosine (Tyr) that may be modified;
the 11th amino acid residue of SEQ ID NO: 296 is arginine (Arg) that may be modified or alanine (Ala) that may be modified;
the 12th amino acid residue of SEQ ID NO: 296 is arginine (Arg) that may be modified or lysine (Lys) that may be modified; and/or
the 13th amino acid residue of SEQ ID NO: 296 is tyrosine (Tyr) that may be modified or phenylalanine (Phe) that may be modified.

Further, the peptide E may be a peptide in which:
the 3rd amino acid residue of SEQ ID NO: 296 is Phe, F3C, F2C, F2OMe, F4C, Cha, MeF, MeF35dC, MeF4F, MeF4Ome, MeNal1, Me3Py, Me4Py, Me3OMe, MeF3COO, MeF3F, Glu, Epyrl2RCOO, Dpyrl2RCOO, or MeF3C, and particularly preferably Phe, MeF, or MeF3C;
the 5th amino acid residue of SEQ ID NO: 296 is Trp, MeW, aMeW, dp, F3C, F3F, F3OMe, F4C, F4F, Hph, MemBph, MeNal1, MeNal2, MeoBph, W4OMe, W1Et, W1Et7Cl, W1iPr, Yph, W1Pr, W5C, W5F, W1aa, W1EtOH, W4OMe, W1mCON, or W6F, and particularly preferably Trp or MeW;

The 7th amino acid residue of SEQ ID NO: 296 is Tyr, 3Py6OMe, Ala, Ahp, Phe, F3H, F4C, Nal1, Arg or Trp, and particularly preferably Tyr;
the 8th amino acid residue of SEQ ID NO: 296 is Phe, Tyr, Typ, Ahp, MeY, F4OMe, 3Imp, 4Py, 3Py, 3Py6OMe, F3C, F3CON, F4C, F4aao, F4F, F4OEt, MeF34dOMe, Yae, Lys, Orn, or Nal1, and particularly preferably Tyr or F4OMe;
the 11th amino acid residue of SEQ ID NO: 296 is Arg, Ala, Asp, Gly, Glu, Lys, MeK, MeR, Dap, Dap, Abu, Aib, Hly, dorn, aMeK, A1Me4pip, KCOpipzMe, F4G, Nle, Nva, or Orn, and particularly preferably Lys or Arg;
the 12th amino acid residue of SEQ ID NO: 296 is Lys, Arg, dr, Tyr, F4G, Orn, Hly, da, Cit, Dap, or Dab, and particularly preferably Lys, Arg, or dr; and/or
the 13th amino acid residue of SEQ ID NO: 296 is Ala, Phe, Asn, Tyr, or pHPeG, and particularly preferably Phe or Tyr.

Further, this peptide has the following amino acid sequence at the N-terminus of the peptide C.

The peptide may be a peptide in which:
the 11th amino acid residue of SEQ ID NO: 296 is Arg, Ala, Asp, Gly, Glu, Lys, MeK, MeR, Dap, Dap, Abu, Aib, Hly, dorn, aMeK, A1Me4pip, KCOpipzMe, F4G, Nle, Nva, or Orn, and particularly preferably Lys or Arg;
the 12th amino acid residue of SEQ ID NO: 296 is Lys, Arg, dr, Tyr, F4G, Orn, Hly, da, Cit, Dap, or Dab, and particularly preferably Lys, Arg or dr;
the 13th amino acid residue of SEQ ID NO: 296 is Ala, Phe, Asn, Tyr, or pHPeG, and particularly preferably Phe or Tyr;
the 14th amino acid residue of SEQ ID NO: 214 is MeTyr, Tyr, Phe, Ala, aMeY, Glu, Gly, Arg, Val, MeoBph, MeBph, MeF, MemBph, MeNal1, MeNal2, MeoBph, MeW, or pHPeG, and particularly preferably Phe or MeW; and/or
the 15th amino acid residue of SEQ ID NO: 296 is Cys or Hgl.

Further, the peptide E may be a peptide in which:
the 3rd amino acid residue of SEQ ID NO: 296 is phenylalanine (Phe), methylated phenylalanine (MeF), or N-α-methyl-N-α-chloroacetyl-3-chloro-L-phenylalanine (MeF3C);
the 5th amino acid residue of SEQ ID NO: 296 is tryptophan (Trp) or methylated tryptophan (MeW);
the 7th amino acid residue of SEQ ID NO: 296 is tyrosine (Tyr);
the 8th amino acid residue of SEQ ID NO: 296 is tyrosine (Tyr) or (S)-2-amino-3-(4-methoxyphenyl)propanoic acid (F4OMe);
the 11th amino acid residue of SEQ ID NO: 296 is arginine (Arg) or lysine (Lys);
the 12th amino acid residue of SEQ ID NO: 296 is arginine (Arg) or D-arginine (dr); and/or
the 13th amino acid residue of SEQ ID NO: 296 is tyrosine (Tyr) or phenylalanine (Phe).

A preferable example of this peptide is a peptide consisting of an amino acid sequence of any of SEQ ID NOs: 3 to 200, or a peptide consisting of an amino acid sequence of any of SEQ ID NOs: 3 to 200, in which the N-terminus is chloroacetyl-Ala.

A preferable example of this peptide is a cyclic peptide that is any of the above-mentioned peptides.

A preferable example of this peptide has an amino acid sequence site having a cyclic structure, and which includes the 1st to 10th amino acid sequence of the amino acid sequence represented by any of SEQ ID NOs: 1 to 552, or a linker conjugate thereof.

A particularly preferable example of this peptide has an amino acid sequence site with a cyclic structure, and which consists of the 1st to 15th amino acid sequence of the amino acid sequence represented by any of SEQ ID NOs: 2, 9, 21 to 148, 159 to 200, 213 to 448, and 450 to 552, or a linker conjugate thereof.

### In respect of cyclic peptides

Cyclic peptides refer to a peptide in which two amino acids are bound to each other in the peptide, thereby causing the peptide to be wholly or partially cyclized. Note that in the present application, cyclic peptides include those in which amino acids form a cross-linking structure in the peptide, those in which a cyclic structure is formed by lactam ring formation or a macrocyclization reaction, those having a lasso peptide-like structure, and the like. That is, in the present application, the cyclic peptide may be one in which a part thereof forms a cyclic structure, and it may also have a linear portion.

Peptides have problems such as general poor metabolic stability in vivo and difficulty in penetrating cell membranes due to their large size. In response to such problems, a method of cyclizing the peptide has been adopted. It has been suggested that peptide cyclization improves protease resistance and metabolic stability, in addition to also restricting conformational change, thereby increasing rigidity and improving a membrane penetrating property and affinity to a target protein.

### Cyclization method

Peptide cyclization can be performed according to a publicly known method.

For example, a peptide designed to comprise two or more cysteine residues can form a cyclic structure via a disulfide bond after translation, although the invention is not limited thereto. Further, cyclization can also be achieved by synthesizing a peptide having a chloroacetyl group at the N-terminus and placing a cysteine residue in the peptide using a genetic code reprogramming technique according to the method by Goto et al. (Y. Goto et al., Acss Chem. Biol. 3 120-129 (2008)) . In this method, a mercapto group spontaneously performs a nucleophilic attack on the chloroacetyl group after translation, leading to cyclization of the peptide via a thioether bond. Cyclization may also be achieved by placing, in the peptide, a combination of other amino acids that are bonded to form a cycle by the genetic code reprogramming technique. Further, cyclization can also be achieved by synthesizing a peptide having cycloamide at the N-terminus and placing an Hgl residue in the peptide. As described above, as long as a publicly known cyclization method is used, cyclization can be performed without any particular limitations.

A preferable example of this peptide is any of the above-mentioned peptides consisting of 15 amino acid residues.

### Peptide length

While a number of amide bonds (a number/length of amino acids) in the peptide and the peptide site is not particularly limited, a total of the amino acid residues (when a substance bound to the peptide or a linker through which the substance is bound to the peptide comprises amino acids, these amino acids are not comprised) is preferably 20 residues or less. As for the amino acids, it is preferable that there are 6 or more, 7 or more, 8 or more, 9 or more, 10 or more, or 11 or more, and preferable that there are 19 or less, 18 or less, 17 or less, 16 or less, or 15 or less.

One aspect of the invention described in this specification relates to a conjugate. This conjugate contains any of the above-mentioned peptides, a linker bound to this peptide, and a substance bound to this linker. It is preferable that the conjugate is one in which at least the substance is capable of passing through the blood-brain barrier. The conjugate may also be capable of wholly passing through the blood-brain barrier. The conjugate preferably has directivity to the muscle tissue. It is preferable that the conjugate can deliver at least the substance to the muscle tissue. The conjugate preferably has a cell-penetrating property. It is preferable that this conjugate can deliver at least the substance to a cell.

An example of the linker is one having an amino acid length of 1 or more and 15 or less and comprising one or more of glycine (Gly) or serine (Ser).

A preferable example of this linker is one in which the N-terminus is cysteine (Cys) that may be modified or lysine (Lys) that may be modified.

Another example of the linker is one having an amino acid length of 1 or more and 5 or less and comprising either or both of D-configuration glutamic acid (de) and methylated glycine (MeG) .

A preferable example of this linker is one in which the N-terminus is cysteine (Cys) that may be modified or lysine (Lys) that may be modified, conjugate.

Another example of the linker is a PEG linker containing polyethylene glycol (PEG) or a derivative of polyethylene glycol. The derivative of polyethylene glycol includes all publicly known PEG linkers.

The PEG linker preferably further comprises glycine (Gly), serine (Ser), glutamic acid (Glu), arginine (Arg), or lysine (Lys).

A preferred example of this linker is one in which the N-terminus is cysteine (Cys) that may be modified or lysine (Lys) that may be modified, conjugate.

Another example of the linker is a linker having a sequence represented by any of SEQ ID NOs: 201 and 553 to 644.

A preferred example of this conjugate is one in which the linker is:
polyethylene glycol (PEG);
a G linker, which is a peptide linker consisting of Gly or MeG, or a GS linker, which is a peptide linker consisting of Gly or MeG and Ser; or
a linker having an amino acid sequence represented by any of SEQ ID NOs: 201 and 553 to 644.

In the present specification, the linker (also referred to as a cross linker) may be any linker that is publicly known or described in the present specification, as long as the linker mediates intermolecular linkage between a peptide that binds to the transferrin receptor and a substance desired to be delivered to the brain. In a specific embodiment, the above-mentioned linker is, for example, a chemical linker, a fatty acid linker, or a peptide linker (a polypeptide linker) . Further, the linker may be a conjugate of, for example, a chemical linker, a peptide linker, and the like. For example, the linker may have a linker structure having both PEG and amino acid residues or a peptide moiety, represented by SEQ ID NO: 616, SEQ ID NO: 627, or the like.

The linker may, for example, be dissociated or separated depending on the environment or conditions, or it may maintain a stable structure.

Chemical linker: in several embodiments, the linker may be a chemical linker. Examples of the chemical linker include, but are not limited to, substituted or unsubstituted alkylene, substituted or unsubstituted heteroalkylene, substituted or unsubstituted cycloalkylene, substituted or unsubstituted heterocycloalkylene, substituted or unsubstituted arylene, and/or substituted or unsubstituted heteroarylene. Further, the peptide and the linker can be conjugated via a sulfhydryl group, an amino group (amine), and/or a carbohydrate, or any suitable reactive group. Homobifunctional and heterobifunctional crosslinkers (conjugation agents) are available from many commercial sources. The crosslinker may contain a flexible arm, for example, 2, 3, 4, 5, 6, 7, 8, 9, 10, 11, 12, 13, 14 or 15 carbon atoms. Examples of the crosslinker include BSS3 ([bis(sulfosuccinimidyl)suberate]), NHSS/EDC (N-hydroxysuccinimide and N-ethyl(dimethylaminopropyl)carbodiimide, sulfo-EMCSS ([N-e-maleimidocaproic acid]hydrazide, hydrazide, and SSATA (N-succinimidyl-SS-acetylthioacetate) .

A preferable example of the chemical linker is a PEG polyethylene glycol) linker. For example, the PEG linker may be a PEG linker consisting of 1 to 24 ethylene glycol units.

Fatty acid linker: the linker may be a fatty acid linker comprising a divalent chemical moiety derived from a fatty acid. For example, the fatty acid linker may be a linker comprising 12-aminododecanoic acid.

Peptide linker: a peptide linker comprising at least one amino acid (e.g., a peptide having at least 2, 3, 4, 5, 6, 7, 10, 15, 20, 25, 40 or 50 amino acids) . In a specific embodiment, the linker is a single amino acid (e.g., any natural amino acid such as Cys). In another embodiment, a glycine-rich peptide such as a peptide having a sequence of [Gly-Gly-Gly-Gly-Ser]n (in the formula, n is 1, 2, 3, 4, 5, or 6) such as is described in U.S. Patent No. 7,271,149 or the like is used. In another embodiment, a serine-rich peptide linker such as is described in U.S. Patent No. 5,525,491 is used. Examples of the serine-rich peptide linker include those represented by a formula [X-X-X-X-Gly]y (in the formula, 2 Xs at most are Thr, with the rest of the Xs being Ser; y is 1 to 5) (e.g., Ser-Ser-Ser-Ser-Gly (in the formula, y is 2 or more)). In several cases, the linker is a single amino acid (e.g., any amino acid such as Gly or Ala).

One aspect of the invention described in this specification relates to a preventive or therapeutic agent for a brain-related disease. This preventive or therapeutic agent for a brain-related disease includes the above-mentioned conjugate, with the above-mentioned substance being an active ingredient.

The substance is a substance to be delivered to the brain. The substance may be any substance desired by those skilled in the art as long as it is intended to be delivered to the brain. However, since passing through the BBB depends on a mechanism such as endocytosis, transcytosis, or the like upon binding to the transferrin receptor, a substance that is too large to be transported by these mechanisms is not preferable. Examples of the substance include, but are not limited to, the following:
Compound: the compound may not only be small molecule compounds and medium molecule compounds but may also be any compound that can be introduced by a cell cytosis mechanism. Examples include a publicly known small molecule drug.
Peptide: the peptide may bind to an in vivo target to exhibit some kind of effect, and it may, for example, be a cyclic peptide.
RI: any may be used, as long as the RI is a compound that can be labeled with a radioisotope, such as a small or medium molecule compound, an antibody, or the like labeled with the radioisotope. Examples include a compound for PET examination.
Protein: any may be used, as long as the protein exhibits a useful function in vivo, such as an antibody, an enzyme, or the like. Examples include enzymes used for enzyme replacement therapy.
Nucleic acid: any may be used, as long as the nucleic acid contains a base sequence, such as DNA, RNA, or the like. Examples include a nucleic acid therapeutic.
DDS: a DDS molecule such as a liposome or a micelle may be used. The DDS molecule may also further comprise a compound, such as a pharmaceutical, therewithin.

Further, the substance may also be a conjugate of these above-mentioned substances.

One aspect of the invention described in this specification relates to a method for producing the preventive or therapeutic agent for a brain-related disease. This method for producing the preventive or therapeutic agent for a brain-related disease includes a step of obtaining the above-mentioned conjugate.

In a preferable example of this method, a linker is:
polyethylene glycol (PEG);
a G linker or a GS linker; or
a linker having an amino acid sequence represented by any of SEQ ID NOs: 201 and 553 to 644.

One aspect of the invention described in this specification relates to a diagnostic agent for a brain-related disease that includes the above-mentioned conjugate.

One aspect of the invention described in this specification is a preventive or therapeutic agent for a neuromuscular disease that includes the above-mentioned conjugate. In this case, the substance is an active ingredient of the preventive or therapeutic agent for a neuromuscular disease. One aspect of the invention described in this specification includes the above-mentioned conjugate. One aspect of the invention described in this specification relates to a diagnostic agent for a neuromuscular disease that includes the above-mentioned conjugate.

The peptide of the present invention can be produced by a publicly known peptide production method including a chemical synthesis method such as a liquid phase method, a solid phase method, a hybrid method that combines the liquid phase method and the solid phase method, and the like; a genetic recombination method; and the like.

In the solid phase method, for example, a hydroxyl group of a resin having the hydroxyl group and a carboxyl group of a first amino acid (normally the C-terminus amino acid of the target peptide) in which an α-amino group is protected by a protective group are subjected to an esterification reaction. A publicly known dehydration condensing agent such as 1-mesitylenesulfonyl-3-nitro-1,2,4-triazole (MSNT), dicyclohexylcarbodiimide (DCC), or diisopropylcarbodiimide (DIPCDI) can be used as an esterifying catalyst.

Next, the protective group of the α-amino group of the first amino acid is removed and a second amino acid in which all the functional groups other than a main chain carboxyl group are protected is added, followed by activation of the carboxyl group to allow the first amino acid and the second amino acid to bind to each other. Further, an α-amino group of the second amino acid is deprotected and a third amino acid in which all the functional groups other than a main chain carboxyl group are protected is added, followed by activation of the carboxyl group to allow the second amino acid and the third amino acid to bind to each other. This process is repeated, and when a peptide with an intended length has been synthesized, all the functional groups are deprotected.

Examples of the resin for solid phase synthesis include Merrifield resin, MBHA resin, Cl-Trt resin, SASRIN resin, Wang resin, Rink amide resin, HMFS resin, Amino-PEGA resin (Merck), HMPA-PEGA resin (Merck), and the like. These resins can be used after washing with a solvent (dimethylformamide (DMF), 2-propanol, methylene chloride, etc.).

Examples of the protective group of the α-amino group include a benzyloxycarbonyl (Cbz or Z) group, a tert-butoxycarbonyl (Boc) group, a fluorenylmethoxycarbonyl (Fmoc) group, a benzyl group, an allyl group, an allyloxycarbonyl (Alloc) group, and the like. The Cbz group can be deprotected by hydrofluoric acid, hydrogenation, or the like, the Boc group can be deprotected by trifluoroacetic acid (TFA), and the Fmoc group can be deprotected by a piperidine treatment.

A methyl ester, an ethyl ester, a benzyl ester, a tert-butyl ester, a cyclohexyl ester, or the like can be used to protect the α-carboxyl group.

As for other functional groups of the amino acid, a hydroxyl group of serine or threonine can be protected by a benzyl group or a tert-butyl group, and a hydroxyl group of tyrosine is protected by a 2-bromobenzyloxycarbonyl group or a tert-butyl group. An amino group of a lysine side chain and a carboxyl group of glutamic acid or aspartic acid can be protected in the same manner as that of the α-amino group and the α-carboxyl group.

The carboxyl group can be activated using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), 1-[bis(dimethylamino)methyl]-1H-benzotriazolium-3-oxide hexafluorophosphate (HBTU), or the like.

A peptide chain can be cleaved from the resin by treating with an acid such as TFA, hydrogen fluoride (HF), or the like.

Peptide production by a gene recombination method (a translation synthesis system) can be performed using nucleic acids that encode the peptide of the present invention. The nucleic acids that encode the peptide of the present invention may be DNA or RNA.

The nucleic acids that encode the peptide of the present invention can be prepared by a publicly known method, or a method based thereon. For example, synthesis can be performed by an automatic synthesizer. A restriction enzyme recognition site for inserting the obtained DNA into a vector may be added, and a base sequence encoding an amino acid sequence for cleaving the resulting peptide chain with an enzyme or the like may also be incorporated.

As described above, when the peptide of the present invention is fused to a membrane-penetrating peptide or the like, the above-mentioned nucleic acids also include nucleic acids that encode a membrane-penetrating peptide.

To suppress degradation by a host-derived protease, a chimeric protein expression method in which the target peptide is expressed as a chimeric peptide with other peptides can be used. In this case, nucleic acids that encode the target peptide and a peptide to be bound hereto are used as the above-mentioned nucleic acids.

Next, an expression vector is prepared using the nucleic acids that encode the peptide of the present invention. The nucleic acids can be inserted downstream of the promoter of the expression vector as they are, by digestion with a restriction enzyme, by addition of a linker, or the like. Examples of the vector include an Escherichia coli-derived plasmid (pBR322, pBR325, pUC12, pUC13, pUC18, pUC19, pUC118, pBluescript II, etc.), a Bacillus subtilis-derived plasmid (pUB110, pTP5, pC1912, pTP4, pE194, pC194, etc.), a yeast-derived plasmid (pSH19, pSH15, YEp, YRp, YIp, YAC, etc.), a bacteriophage (e phage, M13 phage, etc.), a virus (retrovirus, vaccinia virus, adenovirus, adeno-associated virus (AAV), cauliflower mosaic virus, tobacco mosaic virus, baculovirus, etc.), a cosmid, and the like.

The promoter can be selected as appropriate according to the host type. When the host is an animal cell, for example, an SV40 (simian virus 40)-derived promoter or a CMV (cytomegalovirus) -derived promoter can be used. When the host is Escherichia coli, a trp promoter, a T7 promoter, a lac promoter, or the like can be used.

Nucleic acids encoding a DNA replication origin (ori), a selection marker (antibiotic resistance, auxotrophy, etc.), an enhancer, a splicing signal, a poly A addition signal, or a tag (FLAG, HA, GST, GFP, etc.), or the like can be incorporated in the expression vector.

Next, suitable host cells are transformed with the above-mentioned expression vector. The host can be selected as appropriate in relation to the vector, and, for example, Escherichia coli, Bacillus subtilis, Bacillus spp., yeast, an insect or an insect cell, an animal cell, or the like is used. For example, HEK293T cells, CHO cells, COS cells, myeloma cells, HeLa cells, or Vero cells can be used as the animal cell. Transformation can be performed according to a publicly known method such as a lipofection method, a calcium phosphate method, an electroporation method, a microinjection method, a particle gun method, or the like, depending on the host type. A transformant is cultured according to a conventional method to express the target peptide.

The peptide is purified from a culture product of the transformant as follows. After cultured cells are collected and suspended in a suitable buffer liquid, the suspension is subjected to a method such as an ultrasonic treatment, freezing and thawing, or the like to crush the cells, followed by centrifugation separation or filtration to obtain a crude extract. When the peptide is secreted in the culture liquid, a supernatant is collected.

Purification from the crude extract or the culture supernatant can also be performed by a publicly known method, or a method based thereon (e.g., salting out, a dialysis method, an ultrafiltration method, a gel filtration method, an SDS-PAGE method, ion exchange chromatography, affinity chromatography, reverse-phase high-performance liquid chromatography, etc.).

The obtained peptide may be converted from a free form to a salt or from a salt to a free form by a publicly known method or a method based thereon.

The translation synthesis system may be a cell-free translation system. Cell-free translation systems include, for example, a ribosomal protein, an aminoacyl-tRNA synthetase (ARS), a ribosomal RNA, an amino acid, rRNA, GTP, ATP, translation initiation factor (IF), elongation factor (EF), and release factor (RF), ribosome recycling factor (RRF), and other factors necessary for translation. An Escherichia coli extract liquid or a wheat germ extract liquid may also be added to increase expression efficiency. In addition, a rabbit red blood cell extract liquid or an insect cell extract liquid may also be added.

By continuously supplying a system including the above-mentioned components with energy using dialysis, it is possible to produce several hundred micrograms per milliliter to several milligrams per milliliter of proteins. The system may include an RNA polymerase for performing transcription from gene DNA at the same time. Examples of the commercially available cell-free translation system that can be used include an Escherichia coli-derived system such as "RTS-100" (registered trademark) available from Roche Diagnostics "PURE system" available from Gene Frontier, "PURExpress In Vitro Protein Synthesis Kit" available from New England Biolabs; a system using a wheat germ extract liquid such as is available from ZoeGene or CellFree Sciences; and the like.

According to the cell-free translation system, an expression product can be obtained in a highly pure form without purification.

In the cell-free translation system, an artificial aminoacyl-tRNA in which a desired amino acid or hydroxy acid is linked (acylated) to tRNA may be used instead of an aminoacyl-tRNA synthesized into a natural aminoacyl-tRNA synthase. Such aminoacyl-tRNA can be synthesized using an artificial ribozyme.

Examples of such a ribozyme include a flexizyme (H. Murakami, H. Saito, and H. Suga, (2003), Chemistry & Biology, Vol. 10, 655-662; H. Murakami, D. Kourouklis, and H. Suga, (2003), Chemistry & Biology, Vol. 10, 1077-1084; H. Murakami, A. Ohta, H. Ashigai, H. Suga (2006) Nature Methods 3, 357-359, "The flexizyme system: a highly flexible tRNA aminoacylation tool for the synthesis of nonnatural peptides"; N. Niwa, Y. Yamagishi, H. Murakami, H. Suga (2009) Bioorganic & Medicinal Chemistry Letters 19, 3892-3894 "A flexizyme that selectively charges amino acids activated by a water-friendly leaving group"; WO2007/066627; etc.). The flexizyme is also known as original flexizyme (Fx), and dinitrobenzyl flexizyme (dFx), enhanced flexizyme (eFx), amino flexizyme (aFx), and the like, which are modified therefrom.

Using a tRNA linked to a desired amino acid or hydroxy acid produced by the flexizyme makes it possible to translate a desired codon in association with the desired amino acid or hydroxy acid. A special amino acid may be used as the desired amino acid. For example, the above-mentioned non-natural amino acid required for cyclization can also be introduced into the binding peptide by this method.

Chemical synthesis of a macrocyclic peptide of the present invention and an analog thereof can be performed using various methods commonly used in the technical field, including stepwise solid phase synthesis, semisynthesis of a peptide fragment through conformationally-assisted re-ligation, and chemical ligation. The synthesis of the peptide and the analog thereof described in the present specification is chemical synthesis using various solid phase techniques, such as is described in, for example, K. J. Jensen, P. T. Shelton, S. L. Pedersen, Peptide Synthesis and Applications, 2nd Edition, Springer, 2013. A preferable strategy is based on a combination of an Fmoc group capable of temporarily protecting an α-amino group and selectively removable by a base, and a protective group that temporarily protects a side chain functional group and is stable under Fmoc removal conditions. Selection of such a general peptide side chain is known in the above-mentioned Peptide Synthesis and Applications, 2nd edition and G. B. Fields, R. L. Noble, Solid phase Peptide Synthesis Utilizing 9-Fluorenylmethoxycarbonyl Amino Acids, Int. J. Peptide Protein Res. 35, 1990, 161-214, however examples of the preferable peptide side chain protective group include a Boc group and an Mtt group for lysine and other amino groups, a tert-butyl group for a carboxyl group of glutamic acid and aspartic acid, and a Trt group and an Mmt group for a thiol group of cysteine.

The peptide and the analog thereof described in the present invention can be synthesized on the above-mentioned solid phase resin using a stepwise method. The α-amino protective groups of the C-terminus amino acid in use and all amino acids and peptides used in synthesis need to be selectively removed during the synthetic process. It is preferable that the synthetic process is performed using the above-mentioned solid phase resin and initiated by converting a C-terminus carboxyl group of a peptide in which the N-terminus is suitably protected by Fmoc or the like, or a C-terminus carboxyl group of an amino acid protected by Fmoc to an activated ester using a suitable reagent and adding the activated ester to an amino group on the solid phase resin. Subsequent elongation of the peptide chain can be achieved by sequentially repeating the removal of the N-terminus protective group (the Fmoc group) and the following condensation of the protected amino acid derivative according to the amino acid sequence of the target peptide. Note that these target peptides can be released in the final stage. For example, releasing can be performed in a TFA solution containing water/silyl hydride/thiol as a scavenger in TFA, as described as a releasing condition in Teixeira, W. E. Benckhuijsen, P. E. de Koning, A. R. P. M. Valentijn, J. W. Drijfhout, Protein Pept. Lett., 2002, 9, 379-385 and the like. TFA/water/TIS/DODT (a volume ratio of 92.5:2.5:2.5:2.5) is mentioned as a typical example thereof.

The synthesis of the peptide analog described in the present specification can be performed using a single or multi-channel peptide synthesizer, such as the Liberty Blue synthesizer available from CEM or the Syro I synthesizer available from Biotage.

Activation of the carboxy group can be performed using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), 1-[bis(dimethyl amino)methyl]-1H-benzotriazolium-3-oxide hexafluorophosphate (HBTU), and the like.

Another embodiment disclosed in this specification relates to a pharmaceutical. This pharmaceutical includes the above-mentioned peptide and a pharmaceutically acceptable salt or solvate thereof (for simplicity, hereinafter these may be simply referred to as "peptides"). This pharmaceutical preferably includes an effective amount of the above-mentioned peptide as an active ingredient.

In the present specification, a route of administration of the pharmaceutical composition is not particularly limited, and the pharmaceutical composition may be administered either orally or parenterally. Examples of parenteral administration include administration by injection such as intramuscular injection, intravenous injection, or subcutaneous injection, transdermal administration, transmucosal administration (nasal, buccal, ocular, pulmonary, vaginal, or rectal administration), and the like.

Considering that polypeptides are an easily metabolizable and excretable substance, the above-mentioned pharmaceutical composition can be subjected to various modifications. For example, adding polyethylene glycol (PEG) or a sugar chain to the polypeptide can prolong the residence time of the peptide in the blood and reduce antigenicity of the peptide. Further, an emulsion, a nanoparticle, a nanosphere, or the like prepared using a biodegradable polymer compound such as polylactic acid-glycol (PLGA), porous hydroxyapatite, a liposome, a surface-modified liposome, or an unsaturated fatty acid may be used as a sustained-release base in which to encapsulate the polypeptide. In the case of transdermal administration, a weak electric current can be applied to the skin surface to allow the pharmaceutical composition to penetrate the stratum corneum (an iontophoresis method).

For the above-mentioned pharmaceutical composition, the active ingredient may be used as it is, or the pharmaceutical composition may be formulated by adding a pharmaceutically acceptable carrier, excipient, additive, or the like to the active ingredient. Examples of the dosage form include a liquid agent (e.g., an injected liquid), a dispersant, a suspension, a tablet, a pill, a powder, a suppository, a powdered drug, a fine granule, a granule, a capsule, a syrup, a troche, an inhalant, an ointment, an eye drop, a nasal drop, an ear drop, a cataplasm, and the like.

The formulation can be performed by a conventional method by using, for example, an excipient, a binder, a disintegrant, a lubricant, a solubilizing agent, a dissolution adjuvant, a colorant, a flavoring agent, a stabilizer, an emulsifier, an absorption promoter, a surfactant, a pH adjuster, an antiseptic, an antioxidant, or the like as appropriate.

Examples of components to be used in the formulation include, but are not limited to, purified water, saline, a phosphate buffer, dextrose, glycerol, a pharmaceutically acceptable organic solvent such as ethanol, animal or vegetable oil, lactose, mannitol, glucose, sorbitol, crystalline cellulose, hydroxypropyl cellulose, starch, corn starch, silicic anhydride, magnesium aluminum silicate, collagen, polyvinyl alcohol, polyvinylpyrrolidone, a carboxyvinyl polymer, carboxymethylcellulose sodium, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerol, glycerol, propylene glycol, vaseline, paraffin, octyldodecyl myristate, isopropyl myristate, a higher alcohol, stearyl alcohol, stearic acid, human serum albumin, and the like.

Considering that peptides are not easily absorbed through the mucosa, the above-mentioned pharmaceutical composition can include an absorption promoter to improve the absorption of a poorly absorbed pharmaceutical. Examples of a substance that can be used as such an absorption promoter include a surfactant such as a polyoxyethylene lauryl ether, sodium lauryl sulfate, or a saponin; a bile acid salt such as glycocholic acid, deoxycholic acid, or taurocholic acid; a chelating agent such as EDTA or a salicylic acid; a fatty acid such as caproic acid, capric acid, lauric acid, oleic acid, linoleic acid, or a mixed micelle; an enamine derivative; an N-acyl collagen peptide; an N-acyl amino acid; a cyclodextrin; a chitosan; a nitric oxide donor; and the like.

The pill or the tablet can also be coated with sugar or a gastro-soluble or enteric substance.

The injection liquid can comprise distilled water for injection, normal saline, propylene glycol, polyethylene glycol, vegetable oil, an alcohol, or the like. Further, a wetting agent, an emulsifier, a dispersant, a stabilizer, a solubilizing agent, a dissolution adjuvant, an antiseptic, or the like can be added.

When administered to a mammal (e.g., a human, a mouse, a rat, a guinea pig, a rabbit, a dog, a horse, a monkey, a pig, etc.), particularly a human, the dose to be administered of the pharmaceutical composition of the present invention is not particularly limited, and varies depending on a symptom, age, sex, weight, and sensitivity of a patient, an administration method, a dosing interval, a type of active ingredient, and a type of formulation. For example, the pharmaceutical composition can be administered once or split over several times at a dose of 30 µg to 100 g, 100 µg to 500 mg, or 100 µg to 100 mg. When administering by injection, the pharmaceutical composition may be administered once or split over several times at a dose of 1 µg/kg to 3000 µg/kg or 3 µg/kg to 1000 µg/kg, depending on a weight of a patient.

The preventative or therapeutic method for a brain-related disease using the peptide of the present invention can be performed with reference to the description in relation to the above-mentioned pharmaceutical composition.

The preventive or therapeutic agent for a neuromuscular disease including the above-mentioned conjugate may use the above-mentioned pharmaceutical composition.

Another embodiment disclosed in this specification relates to a brain-related disease detection agent. This brain-related disease detection agent includes the above-mentioned peptide, the salt thereof, or the solvate thereof.

### Brain-related disease detection agent and detection kit

The present invention also includes the brain-related disease detection agent including the peptide of the present invention. When used as the detection agent, the peptide of the present invention may be labelled to enable detection. Examples of substances used for peptide labelling include an enzyme such as peroxidase or alkaline phosphatase, a radioactive substance such as ¹²⁵I, ¹³¹I, ³⁵S, or ³H, a fluorescent substance such as fluorescein isothiocyanate, rhodamine, dansyl chloride, phycoerythrin, tetramethylrhodamine isothiocyanate, or a near-infrared fluorescent material, an antibody labeled with a luminescent substance such as luciferase, luciferin, aequorin, or the like. In addition, an antibody labeled with a nanoparticle such as colloidal gold or a quantum dot can also be detected. For example, a brain-related disease can be detected by creating a conjugate of an antibody that binds to a specific target related to the brain-related disease and the peptide of the present invention, labeling the antibody or the peptide of the present invention in the conjugate, and administering and detecting the resulting conjugate.

Further, in an immunoassay, the peptide of the present invention can be labeled with biotin and detected by allowing avidin or streptavidin labeled with an enzyme or the like to bind to the biotin.

Among immunoassays, an ELISA method using enzyme labeling is preferable since antigens can be measured easily and quickly. For example, an antibody is immobilized on a solid phase carrier and after a sample is added to the carrier to cause a reaction, the labeled peptide of the present invention is added to the carrier mixture to cause a reaction. After washing, the carrier mixture is reacted with an enzyme substrate for color development, and by measuring an absorbance, it is possible to detect the brain-related disease. After reacting the sample with the antibody immobilized on the solid phase carrier, the unlabeled peptide of the present invention may be added to the carrier mixture, then an antibody against the peptide of the present invention may be labeled with an enzyme and further added to the carrier mixture.

When the enzyme is a peroxidase, 3,3'-diaminobenzidine (DAB), 3,3',5,5'-tetramethylbenzidine (TMB), o-phenylenediamine (OPD), or the like can be used as the enzyme substrate, and when the enzyme is an alkaline phosphatase, p-nitropheny phosphate (NPP) or the like can be used.

In the present specification, the "solid phase carrier" is not particularly limited, as long as the carrier can immobilize antibodies, and examples include a microtiter plate made of glass, metal, resin, or the like, a substrate, a bead, a nitrocellulose membrane, a nylon membrane, a PVDF membrane, and the like. A target substance can be immobilized on these solid phase carriers in accordance with a publicly known method.

The detection kit according to the present invention includes a reagent and an instrument required for the above-mentioned detection (including, but not limited to, the peptide of the present invention, an antibody, a solid phase carrier, a buffer, an enzyme reaction terminator liquid, a microplate reader, etc.).

In another embodiment disclosed in this specification, it can also be considered that the invention is used as a brain-related disease detection kit including the above-mentioned brain-related detection agent or a tool for elucidating a brain-related disease and various cellular functions and biological phenomena associated with the brain-related disease.

This specification also provides a use of the peptide for producing a pharmaceutical for preventing or treating a brain-related disease. The peptide in this case may be any of the above-mentioned peptides.

Further, this specification also provides a use of the peptide for producing a pharmaceutical for preventing or treating a neuromuscular disease. The peptide in this case may be any of the above-mentioned peptides.

The present specification also provides a preventative or therapeutic method for a brain-related disease that includes a step of administering an effective amount of the peptide, the pharmaceutically acceptable salt or solvate thereof, or the conjugate thereof to a subject, which is a human, a non-human mammal, or a bird. The above-mentioned peptide can be used as appropriate as the peptide. Examples of the non-human mammal are a non-human primate, a pig, a cow, a dog, a cat, a horse, sheep, a rat, and a mouse.

This specification also provides a preventative or therapeutic method for a brain-related disease that includes a step of administering an effective amount of the peptide, the pharmaceutically acceptable salt or solvate thereof, or the conjugate thereof to a subject, which is a human, a non-human mammal, or a bird.

The abbreviations used in the present specification, especially in the below-mentioned representative examples, are well-known to those skilled in the art. Several of the abbreviations used are as follows:
Fmoc as 9-fluorenylmethyloxycarbonyl;
HOAt as 1-hydroxybenzotriazole;
HATU as
O-(7-azabenzotriazol-1-yl)-N,N,N',N',-tetramethyluronium hexafluorophosphate;
MeCN as acetonitrile;
DBU as 1,8-diazabicyclo[5.4.0]-7-undecene;
DIPEA as N,N-diisopropylethylamine;
DODT as 3,6-dioxa-1,8-octane-dithiol;
DMSO as dimethyl sulfoxide;
DMF as N,N-dimethylformamide;
Mtt as monomethyltrityl;
Mmt as monomethoxytrityl;
o-Ns as 2-nitrobenzenesulfonyl;
TFA as trifluoroacetic acid;
TIS as triisopropylsilane;
Trt as trityl;
mL as milliliter (unit);
M as molar (unit);
v/v as volume/volume;
Adod as 12-aminododecanoic acid

### [Example 1]

### Chemical synthesis

All raw materials, building blocks, reagents, acids, bases, solid phase resins, and solvents used in the chemical synthesis in the following examples are either commercially available products used as is, or products that can be synthesized by those skilled in the art using organic chemical methods. Note that for amino acids comprising protective groups, commercially available products were used as is unless otherwise specified.

Elongation of the peptide chain on the solid phase resin was performed using the resin described in each example as a starting raw material normally used under peptide coupling reaction conditions and Fmoc removal reaction conditions. The reaction was performed using Liberty Blue available from CEM, which is an automatic peptide synthesizer, according to the manufacturer's manual. General amino acids used are listed below, with side chain protective groups indicated in parentheses.

Fmoc-Trp(Boc)-OH; Fmoc-Thr(tBu)-OH; Fmoc-N-Me-Gly-OH; Fmoc-Asp(OtBu)-OH; Fmoc-N-Me-Phe-OH; Fmoc-Ala-OH; Fmoc-N-Me-Ala-OH; Fmoc-His(Trt)-OH; Fmoc-Tyr(tBu)-OH; Fmoc-Val-OH; Fmoc-HydPro(tBu)-OH; Fmoc-Cys(Trt)-OH; Fmoc-Lys(Mtt)-OH; Fmoc-Ser(tBu)-OH; Fmoc-N-Me-Ser(tBu)-OH

Introduction of a chloroacetyl group was performed using the solid phase resin holding the Fmoc-protected peptide obtained in the previous step by removing the Fmoc group of the α-amino group using the above-mentioned method, then adding chloroacetic acid (3 Eq), 3 Eq of an N,N'-diisopropylcarbodiimide-DMF solution (0.5 M), and 3 Eq of a HOAt-DMF solution (0.5 M) to the resin and shaking at room temperature for 40 minutes.

For deprotecting the side chain and cleaving from the solid phase resin, the resin obtained after the chloroacetyl group introduction step was first washed five times each with DMF and methylene chloride then dried under reduced pressure. Next, a reagent cocktail-A (a mixture of TFA/H₂O/TIS/DODT in a volume ratio of 92.5:2.5:2.5:2.5) was added to the reaction vessel containing the solid phase resin, followed by shaking at room temperature for 150 minutes. The reaction liquid was collected by frit filtration. The solid phase resin remaining in the reaction vessel was shaken again with a cleavage cocktail, then the solution component was collected by frit and mixed with the above-mentioned filtrate. When an excess of diethyl ether cooled to 0°C was added to this filtrate, a white cloudy precipitate was produced. This mixture was subjected to centrifugal separation (9000 rpm, 3 mins) and the resulting solution was subjected to decantation. After the obtained solid was washed again with a small amount of diethyl ether cooled to 0°C, the solid thus obtained was used for the subsequent cyclization reaction.

The cyclization reaction of the peptide was performed by dissolving the peptides in DMSO to a final concentration of 5 mM on the basis of the number of moles of the solid phase resin, then adding 6 Eq of triethylamine, followed by stirring at room temperature for about 16 hours. The reaction solution thus obtained was acidified with acetic acid and concentrated under reduced pressure using Biotage (registered trademark) V-10 (Biotage Japan).

As a method for purifying the obtained crudely purified peptides, reverse-phase preparative HPLC was used with the AutoPurification System with a single quadrupole mass spectrometer SQD2 available from Waters to elute the peptides while monitoring m/z ions derived from the target object. It was confirmed that a mass spectrum obtained using the ESI-positive scan mode and a mass spectrum including the multivalent ions calculated from the molecular formula of the target object matched within the error range of the mass spectrometer used. Note that purification conditions including the columns used were indicated in each example.

In the present invention, the purity of the chemically synthesized peptides was determined by any of the following analysis methods.

### (Analysis conditions)

### Analysis conditions A

Column; CORTECS (registered trademark) UPLC (registered trademark) C18 column (Nihon Waters), 90 Å, 1.6 µm, 2.1 × 100 mm
Mobile phase: 0.025% TFA in MeCN/0.025% TFA in H₂O
Temperature: 40°C
Gradient: 5 to 95% MeCN/0.025% TFA in H₂O in 5.56 mins; linear gradient
Flow rate 0.4 mL/min
Detection method: UV 220 nm

### Analysis conditions B

Column; Kinetex EVO C18 2.6 µm, 2.1 ID × 150 mm, 100 Å (Phenomenex)
Column temperature: 60°C
Mobile phase A: 0.025% TFA in H₂O
Mobile phase B: 0.025% TFA in CH₃CN
Gradient: described in each example
Flow rate: 0.25 mL/min
Detection: PDA (225 nm)

For determining a structure of the chemically synthesized peptides, the molecular weight calculated in consideration of the amino acids used according to the target sequence and the building blocks used as needed was confirmed by ESI-MS(+) in the mass spectrum analysis method. Note that the term "ESI-MS (+)" indicates an electrospray ionization mass spectrum analysis method performed using a positive ion mode. The detected mass was reported in units of m/z. Note that compounds with a molecular weight roughly greater than 1000 were frequently detected as divalent ions or trivalent ions.

### Chemical synthesis of special cyclic peptides binding to hTfR

The hTfR-binding peptides were also identified by the screening method disclosed in WO2014/119600, WO2012/033154, or WO2007/066627. The identified peptides were chemically synthesized for the purpose of confirming whether the peptides actually had binding activity to hTfR. Sequences of the synthesized peptides are shown in Table 1.

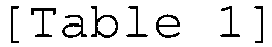

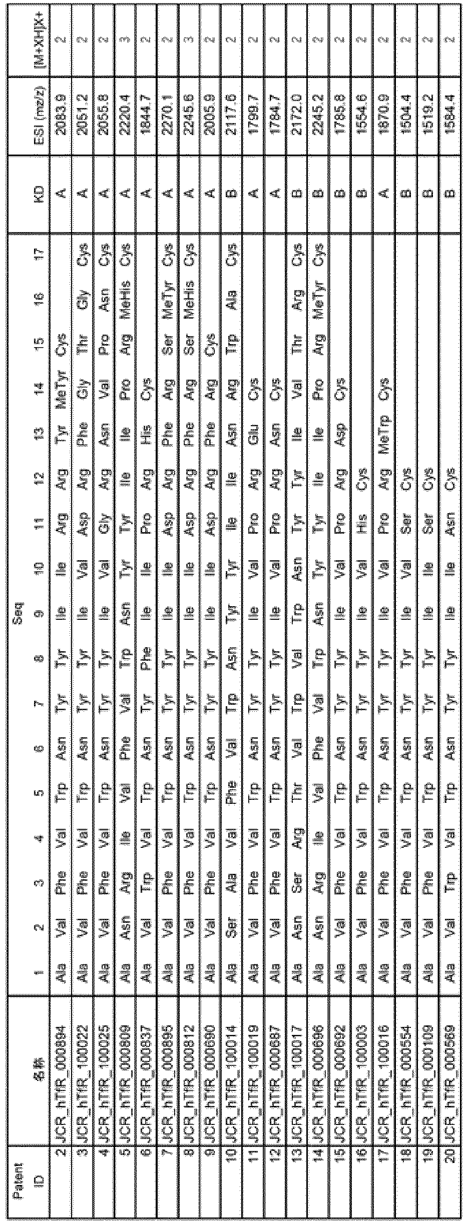

The target peptide was synthesized according to the above-mentioned general method, using Sieber amide resin (Watanabe Chemical Industries, 0.6 mmol/g, 0.33 g) and starting with the removal of Fmoc. In this process, the synthesis was performed using Liberty Blue HT available from CEM as a solid phase synthesizer according to the manufacturer's manual. The condensation reaction was performed at 75°C for 10 minutes as a basic condition. The removal of Fmoc was performed in 20% piperidine in DMF at 75°C for 3 minutes as a basic condition. Introduction of a chloroacetyl group was performed using the solid phase resin holding the Fmoc-protected peptide obtained in the previous step by removing the Fmoc group of the α-amino group by the above-mentioned method then adding 5 Eq of a chloroacetic acid-DMF solution (0.2 M), 5 Eq of an HATU-DMF solution (0.5 M), and 10 Eq of a DIPEA-DMF solution (1 M) and shaken at room temperature for 30 minutes. For deprotecting the side chain and cleaving from the solid phase resin, the resin obtained after the chloroacetyl group introduction step was first washed with DMF 5 times and methylene chloride 3 times, then dried under reduced pressure. Next, a reagent cocktail (a mixture of TFA/H₂O/TIS/DODT in a volume ratio of 92.5:2.5:2.5:2.5) was added to the reaction vessel containing the solid phase resin and shaken at room temperature for 90 minutes. The reaction liquid was collected by frit filtration. The solid phase resin remaining in the reaction vessel was shaken again with a cleavage cocktail, and the solution component was collected by frit and mixed with the above-mentioned filtrate. When an excess of diethyl ether/hexane (1/1) cooled to 0°C was added to this filtrate, a white cloudy precipitate was produced. This mixture was subjected to centrifugal separation (10000 rpm, 1 min) and the resulting solution was subjected to decantation. The obtained solid was washed again with a small amount of diethyl ether cooled to 0°C, then the solid thus obtained was dried and used for the subsequent cyclization reaction. The cyclization reaction of the peptides was performed by dissolving the peptides in DMSO to a final concentration of 5 mM on the basis of the number of moles of the solid phase resin, then adding 6 Eq of triethylamine, followed by stirring at room temperature for about 16 hours. The reaction solution thus obtained was concentrated under reduced pressure using a Savant Explorer SpeedVac.

The crudely purified product thus obtained was purified under the following conditions (Column; Waters Xbridge (registered trademark) C18 5 µm 50 × 150 mm: mobile phase; A = 0.1% TFA in H₂O, B = 0.1% TFA in MeCN: temperature 40°C: gradient (%B); 5 to 30% over 3 minutes, then 30 to 35% over 8 minutes, then 35 to 60% over 1 minute: flow rate 120 mL/min).

The obtained target product was analyzed under the above-mentioned analysis conditions A or analysis conditions B to confirm a structure by ESI-MS(+) in the mass spectrum analysis method. Table 1 shows the ESI-MS(+) observed values obtained and a value of X when X is shown as a number of added protons in (M+XH)^{X+} for each observed value.

### [Example 2]

### Intermolecular interaction evaluation test between transferrin receptor (hTfR) and peptide by surface plasmon resonance (SPR)

For each of the various synthesized peptides, the intermolecular interaction of the peptide with the transferrin receptor (hTfR) was tested by surface plasmon resonance (SPR) according to the following method. The specific test method is as follows.

### [SPR measurement]

An NTA sensor chip (Global Life Sciences Technologies Japan) was inserted in a Biacore T200 (Global Life Sciences Technologies Japan) and a prime operation was performed 3 times with a running buffer: 10 mM HEPES pH 8.0 (Nacalai Tesque), 150 mM NaCl (Nacalai Tesque), 0.05% Tween 20 (Nacalai Tesque), 0.1% BSA (Sigma-Aldrich), 1.0% DMSO (FUJIFILM Wako Pure Chemical) to create an Equilibration state at a flow rate of 30 µL/ min. After reacting with 350 mM EDTA solution for 60 seconds at a flow rate of 10 µL/min then reacting with 0.5 mM NiCl₂ solution (Kishida Chemical) for 60 seconds at a flow rate of 10 µL/min, the NTA sensor chip was washed with 3 mM EDTA solution (Nacalai Tesque) for 60 seconds at a flow rate of 10 µL/min. After mixing 50 µL each of 60 mM EDC solution (Global Life Sciences Technologies Japan) and 650 mM NHS solution (Global Life Sciences Technologies Japan), the mixture was reacted for 420 seconds at a flow rate of 10 µL/min. By diluting with the running buffer, 150 µL of 3.2 µM hTfR solution was prepared and used for reaction for 600 seconds at a flow rate of 10 µL/min, thereby immobilizing hTfR on the NTA sensor chip. After immobilization, capping was performed by reacting with 1.0 M ethanolamine aqueous solution (Global Life Sciences Technologies Japan) for 420 seconds at a flow rate of 10 µL/min. A peptide dissolution liquid prepared to 10 mM in the DMSO solution was diluted with the running buffer to a final concentration of 10 µM so as to become the peptide dissolution liquid, then peptide solutions of 100 nM, 50 nM, 25 nM, 10 nM and 5 nM were prepared. Kinetics of the peptide to hTfR was acquired by SPR measurement using the above-mentioned samples. Single-cycle kinetics was used as a kinetic evaluation model and curve fitting was performed using Biacore T200 Evaluation Software Version 3.0 (Global Life Sciences Technologies Japan) . An obtained sensorgram was subjected to curve fitting using the least-squares method, and the binding of the peptide to hTfR was evaluated by obtaining KD values. Table 1 shows the obtained KD values denoted as follows: KD value less than 1 nM: A, 1 nM or more and less than 100 nM: B, 100 nM or more and less than 1 µM: C, and 1 µM or more: D. The result showed that hTfR No. 894 and the cyclic peptides having an amino acid sequence similar to that of hTfR No. 894 had significant hTfR binding ability. A chemical structure of hTfR No. 894 is as follows.

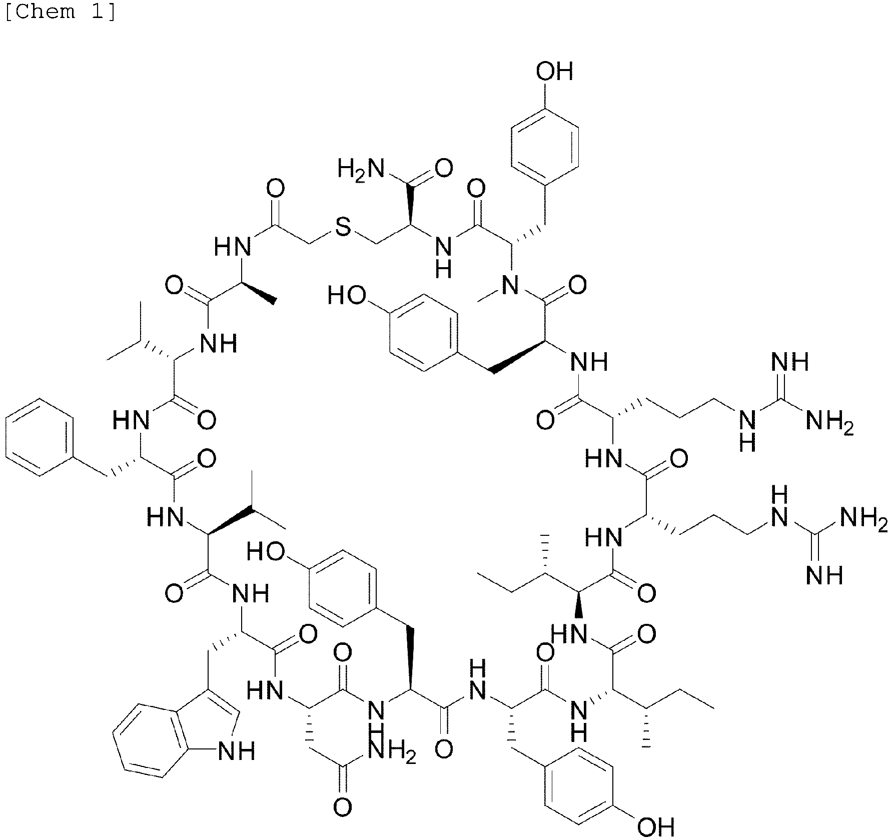

### [Example 3]

### Synthesis of hTfR-binding peptide-PEG11-vivotag750 conjugate (hTfRNo.894-vivotag750)

A compound was synthesized by binding hTfR No. 894 to VivoTag 750 (VivoTag-S (trademark) 750, PerkinElmer), which is a near-infrared fluorescent labeling substance serving as a payload, via a PEG11 linker (denoted as hTfRNo.894-vivotag750 or hTfR_000894_PEG11_(VivoTag)) (SEQ ID NO: 146). The chemical structure of the hTfR-binding peptide-PEG11 is as follows, and this compound was bound to VivoTag 750 to synthesize the title compound. Details thereof are described below.

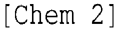

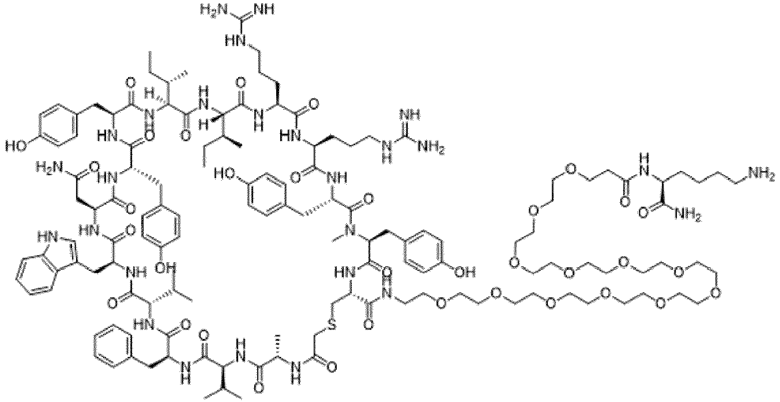

### Synthesis of hTfRNo.894-PEG11-vivotag750

The chemical synthesis was performed as follows:
According to the general method, the target peptide was synthesized using Fmoc-NH-SAL-PEG-resin 1500 to 2000 Da (Watanabe Chemical Industries, 0.38 mmol/g), starting with the removal of Fmoc. In this process, the synthesis was performed using Liberty Blue available from CEM as a solid phase synthesizer according to the manufacturer's manual. The condensation reaction was performed under a basic condition in which HATU was used in a condensing agent and a reaction was performed once at 75°C for 10 minutes. However, for the 11th residue and the 12th residue, the reaction was repeated twice at 25°C for 20 minutes. For the 13th residue and the 14th residue, the reaction was repeated twice at 75°C for 10 minutes. For the 15th residue, the reaction was performed once at 25°C for 20 minutes. For the 16th residue, the reaction was performed once at 25°C for 60 minutes. Further, the removal of Fmoc was performed under a basic condition in which a reaction was performed with a 20% piperidine-DMF solution at 75°C for 3 minutes. However, for the 13th residue and the 15th residue, the removal of Fmoc was performed by carrying out the reaction at 25°C for 5 minutes, then reacting for 10 minutes. The introduction of the chloroacetyl group was performed by adding 5 Eq of a chloroacetic acid-DMF solution (0.2 M), 5 Eq of a HATU-DMF solution (0.5 M), and 10 Eq of a DIPEA-DMF solution (1 M) to the solid phase resin obtained in the prior step, followed by shaking at room temperature for 30 minutes. For deprotecting the side chain and cleaving from the solid phase resin, the resin obtained after the chloroacetyl group introduction step was first washed with DMF 5 times and with methylene chloride 3 times, then dried under reduced pressure. Next, a reagent cocktail-A (a mixture of TFA/H₂O/TIS/DODT in a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above-mentioned filtrate . When an excess of a mixed solvent of diethyl ether and hexane cooled to 0°C was added to this filtrate, a white cloudy precipitate was produced. This mixture was subjected to centrifugal separation (10000 rpm, 1 min) and the supernatant was subjected to decantation. The obtained solid was washed with ice-cold diethyl ether then dried. The solid thus obtained was used for the subsequent cyclization reaction. The cyclization reaction of the peptide was performed by dissolving the peptide in DMSO to a final concentration of 5 mM on the basis of the number of moles of the solid phase resin, then adding 6 Eq of triethylamine to the DMSO solution, followed by shaking at room temperature overnight. The resulting reaction solution was concentrated under reduced pressure using a Savant Explorer SpeedVac, then purified by reverse-phase HPLC. The obtained peptides (26.1 mg, 9.30 µmol) were dissolved in DMSO/H₂O (9/1), then stirred for 45 minutes upon adding 0.91 Eq of VivoTag-NHS and 4.5 Eq of DIEA. AcOH was added to the resulting reaction solution for quenching.

The reaction liquid thus obtained was purified under the following conditions (Column; XSelect CSH C18 5µm 10 × 150 mm (Lot No. 147i362781): mobile phase; A = 0.1% TFA in H₂O, B = 0.1% TFA in MeCN: temperature 40°C: gradient (%B); 7 to 32% over 3 minutes, then 32 to 37% over 8 minutes, then 37 to 60% over 1 minute: flow rate 5 mL/min).

The purity of the target product was 97.1% calculated from the area ratio of the LC/MS (UV wavelength 225 nm) chromatogram under the analysis conditions B.

Analysis conditions B: retention time = 13.20 minutes: gradient (%B conc.); 20 to 60% over 20 minutes, then 60 to 95% over 1 minute, then 95% over 5 minutes.

ESI-MS(+) observed value m/z = 1226.4 (M+3H)³⁺

Further, when the hTfR binding ability of the above-mentioned synthesized conjugate was checked by SPR according to Example 2, it was confirmed as KD = 1.09 nM.

### [Example 4]

### Control peptide conjugate: synthesis of

### hTfRNo.894FLIP-vivotag750

As a control of hTfRNo.894-vivotag750, a conjugate in which a cyclic peptide having the following amino acid sequence with the sequence of No. 894 inverted and VivoTag 750 were bound to each other via the PEG11 linker was synthesized (shown as hTfRNo.894FLIP-vivotag750 or FLIP_000894_PEG11_K_FITC) . FLIP sequence:
ClAc-Ala-MeTyr-Tyr-Arg-Arg-Ile-Ile-Tyr-Tyr-Asn-Trp-Val-Phe-Val-Cys (SEQ ID NO: 202)

### Synthesis of hTfRNo.894FLIP-PEG11-vivotag750

The synthesis and purification of the peptide conjugate was performed in the same manner as in Example 3, except that Sieber amide resin (Watanabe Chemical Industries, 0.6 mmol/g, 0.33g) was used as a starting resin.

The purity of the target product was 95.5% calculated from the area ratio of the LC/MS (UV wavelength 225 nm) chromatogram under the analysis conditions B.

Analysis conditions B: retention time = 13.5 minutes: gradient (%B conc.); 20 to 60% over 20 minutes, then 60 to 95% over 1 minute, then 95% over 5 minutes.
ESI-MS (+) observed value m/z = 1226.4 (M+3H)³⁺

The N-terminus of the compound obtained as described above was labeled according to the protocol of VivoTag 750 (VivoTag-S (trademark) 750, PerkinElmer) to obtain the title compound.

Further, when the hTfR binding ability of the above-mentioned conjugate thus synthesized was checked by SPR according to Example 2, no binding to hTfR was recognized.

### [Example 5]

### Brain migration evaluation test of hTfRNo.894-vivotag750 using hTfR-KI mice

### [Preparation of test liquid]

Hydroxypropyl-β-cyclodextrin (Wako Pure Chemical Industries) was dissolved in water to a concentration of 20 w/v%, and this was used as a 20% hydroxypropyl-β-cyclodextrin solution.

### [Preparation of administration liquid]

hTfRNo.894-vivotag750 conjugate administration liquid (No. 894 administration liquid) : 4.8 µL of dimethyl sulfoxide (DMSO, Sigma Aldrich) was added to 6 µL of a 5 mM solution of the hTfRNo.894-vivotag750 conjugate synthesized in Example 2, 66 µL of the 20% hydroxypropyl-β-cyclodextrin solution was further added, and the mixture was mixed well. To this, 48 µL of polyethylene glycol 400 was further added and mixed to prepare a No. 894-administration liquid. The concentration of the hTfRNo.894-FITC conjugate in the No. 894 administration liquid has been adjusted to 250 µM.

hTfRNo.894FLIP-vivotag750 conjugate administration liquid (No.894NC administration liquid): 66 µL of the 20% hydroxypropyl-β-cyclodextrin solution was added to 6 µL of the above-mentioned 5 mM solution of the hTfR No.894FLIP-vivotag750 conjugate, and the mixture was mixed well. To this, 48 µL of polyethylene glycol 400 was further added and mixed to prepare a No. 894NC administration liquid. The concentration of the hTfRNo.894FLIP-vivotag750conjugate in the No. 894 FLIP administration liquid has been adjusted to 250 µM.

### [Creation of TfR-KI mice]

A targeting vector was created in accordance with the description of WO2016-208695 having a DNA fragment with a base sequence in which a neomycin resistance gene flanked with loxP sequences was placed on the 3' side of cDNA encoding a chimeric hTfR, in which an intracellular region was composed of an amino acid sequence of mouse hTfR and an extracellular region was composed of an amino acid sequence of human hTfR, a 5' arm sequence, and a 3' arm sequence. The targeting vector thus created was introduced into mouse ES cells using an electroporation method. After the gene introduction, the mouse ES cells were selectively cultured in the presence of neomycin, and the mouse ES cells in which the targeting vector was integrated into the chromosome by homologous recombination were selected. The obtained genetic recombinant mouse ES cells were injected into 8-cell stage embryos (host embryos) of ICR mice, and the resulting embryos were implanted into pseudo pregnant mice (recipient mice) obtained through mating with mice having undergone vasoligation. The offspring (chimeric mice) thus obtained were determined by hair color, and individuals in which the ES cells contributed to the development of the organism at a higher rate, that is, individuals having a higher proportion of white hairs relative to their total body hairs, were selected. These chimeric mice individuals were mated with C57BL6/J mice to obtain F1 mice. White-colored F1 mice were selected, DNA was extracted from their tail tissues and analyzed, and the mice in which the mouse transferrin receptor gene on the chromosome had been heterologously replaced with the chimeric hTfR were selected as the TfR-KI mice.

### [Brain migration evaluation

### test using TfR-KI mice]

Next, a brain migration evaluation test was performed using the TfR-KI mice. The TfR-KI mice (females, 12 weeks old) expressing hTfR were rapidly administered with 100 µL of the No. 894 administration liquid or the No. 894NC administration liquid via the tail vein. One hour after administration, the mice were anesthetized with isoflurane and subjected to a blood removal treatment by running normal saline from the left ventricle for 4 to 5 minutes . After this, tissues for measuring fluorescence intensity (brain, heart, lung, liver, spleen, kidney, quadriceps femoris muscle, thymus, thoracic vertebrae, and femur) were collected and stored in normal saline to prevent drying. The fluorescence intensity was measured using the IVIS Lumina III in vivo emission and fluorescence imaging system (PerkinElmer) and a filter set for the fluorescent dye VivoTag 750 according to the instruction manual.

### [Results of brain migration evaluation using hTfR-KI mice]

The fluorescence intensity measurement results for each tissue are shown in Fig. 1, Fig. 1-2, Fig. 1-3 and Fig. 2.

In the figures, the notation of "#894" indicates a hTfRNo.894-vivotag750 conjugate administration group and the notation of "#894FLIP" indicates a hTfRNo.894FLIP-vivotag750 conjugate administration group. Note that the bar on the right side of each photograph indicates an average radiation efficiency ([p/sec/cm²/sr]/[µW/cm²]).

In Figure 1-1, (1) Brain shows images of a brain, (2) Liver (left lateral lobe) shows images of an area of a left lateral lobe of a liver, (3) Kidney shows images of a kidney, (4) Lung shows images of a lung, and (5) Thoracic vertebrae shows images of thoracic vertebrae. A minimum value and a maximum value of the color scale in (1) is 5.00 × 10⁷ and 1.3 × 10⁸, respectively, a minimum value and a maximum value of the color scale in (2) and (3) is 6.00 × 10⁸ and 2.4 × 10¹⁰, respectively, and a minimum value and a maximum value of the color scale in (3) and (4) is 4.5 × 10⁸ and 5.4 × 10⁹, respectively. In Figure 1-2, (1) Heart shows images of a heart, (2) Spleen shows images of a spleen, and (3) Thymus shows images of a thymus. Note that the minimum value and the maximum value of the color scale is 4.83 × 10⁷ and 4.8 × 10⁹, respectively.

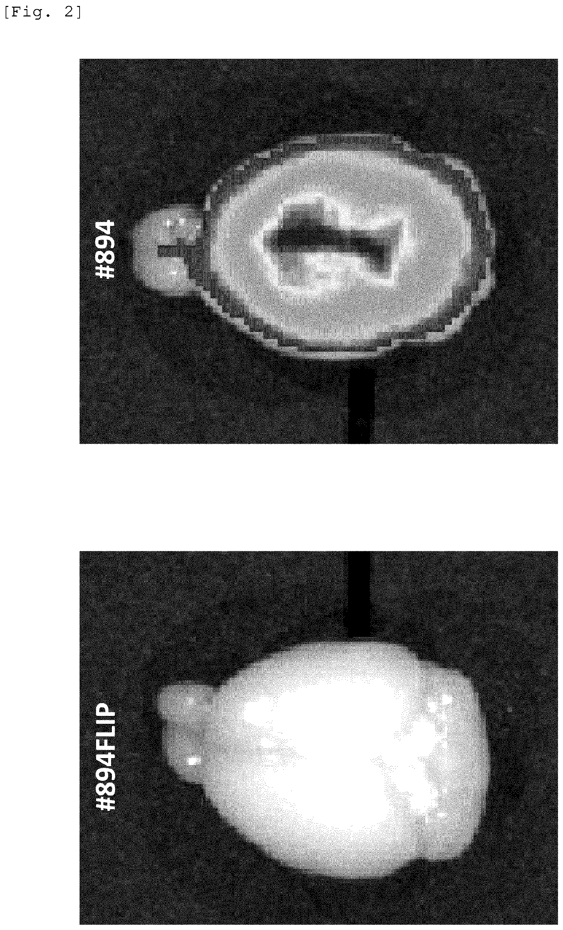

In Figure 1-3, (1) Quadriceps shows images of a quadriceps femoris muscle and (2) Femur shows images of a femur. Note that the minimum value and the maximum value of the color scale is 1.5 × 10⁸ and 1.5 × 10⁹, respectively. Fig. 2 is an enlarged photograph of Fig. 1-1 (1) Brain.

Figs. 1-1, 1-2, and 1-3 and Fig. 2 showed that the tissues in which a stronger fluorescence was detected in the hTfRNo.894-vivotag750 conjugate administration group than the hTfRNo.894FLIP-vivotag750 conjugate administration group were from the kidney, the thoracic vertebrae, the heart, the femur, the quadriceps femoris muscle, and the brain. The tissues in which a stronger fluorescence was detected in the hTfRNo.894FLIP-vivotag750 conjugate administration group than the hTfRNo.894-vivotag750 conjugate administration group were the liver and the spleen.

In both administration groups, the strongest fluorescence was detected in the kidney, and the second strongest fluorescence was detected in the liver, suggesting that both test substances were mainly metabolized and excreted by the kidney and the liver. Further, stronger fluorescence was observed in the thoracic vertebrae, the heart, the femur, the quadriceps femoris muscle, and the brain in the No. 894 administration group than in the No. 894NC administration group. Since these tissues had relatively high TfR expression levels, a difference in tissue distribution between both groups was considered to be due to the presence or absence of hTfR binding ability.

The present test confirmed that the hTfR-binding special cyclic peptide No. 894 migrated into the brain through the BBB by binding to TfR in the hTfR-KI mice and also migrated to each tissue, mainly muscle tissues.

### [Example 6]

### Mouse brain localization confirmation test using hTfR-binding special cyclic peptide-fluorescence labeled probe conjugate

### [Production of conjugate of hTfR-binding special cyclic peptide No. 894 and fluorescent substance FITC]

The conjugate of the hTfR-binding special cyclic peptide No. 894 and the fluorescent substance FITC was synthesized according to the protocol of the FITC labeling kit (Dojindo Laboratories) using the hTfR No. 894-PEG11 synthesized in Example 3. Further, when the hTfR binding ability of the above-mentioned synthesized conjugate was checked by SPR in accordance with Example 2, it was confirmed as KD = 0.28 nM.

### [Single dose test]

In the same manner as in Example 5, the conjugate of the hTfR-binding special cyclic peptide No. 894 and the fluorescent substance FITC was administered to the hTfR-KI mice. The dose was adjusted to 3.7 mg/kg.

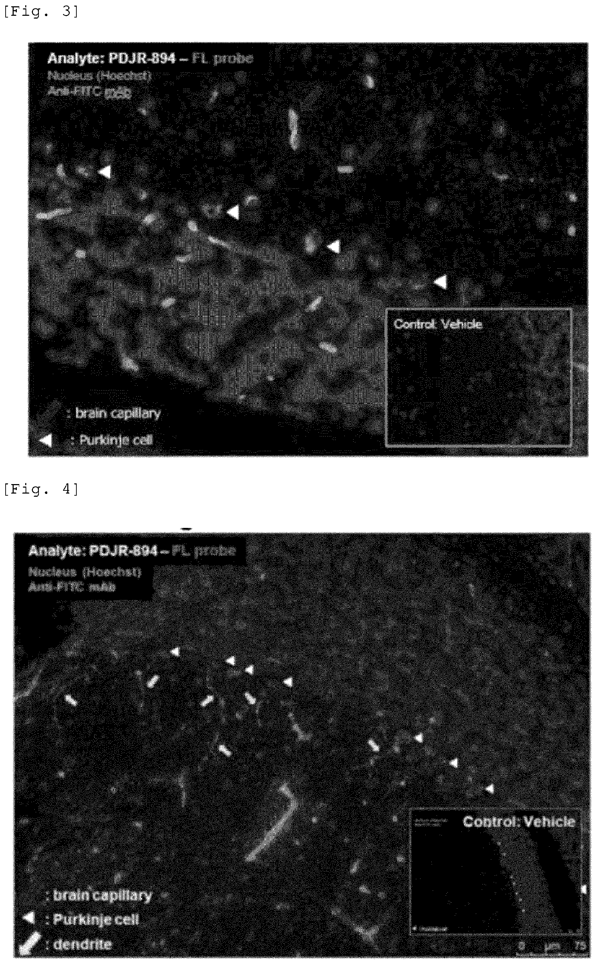

One hour after administration, the mice were anesthetized with isoflurane and subjected to a blood removal treatment by running normal saline from the left ventricle for 4 to 5 minutes . After that, the brain was collected for measuring the fluorescence intensity and stored in normal saline to prevent drying. This brain tissue was subjected to immunohistochemical staining using an anti-FITC antibody (MBL Life Science) . The immunohistochemical staining in the brain tissue using the anti-FITC antibody was performed with a publicly known method and the staining was observed using a fluorescence microscope. The result is shown in Fig. 3. Note that in the figure, red arrows indicate the cerebral capillaries and arrowheads indicate the Purkinje cells. (1) shows a photograph of the #894-FITC administration group and (2) shows a photograph of the group not administered with #894-FITC as a control.

### [Multiple dose test]

The conjugate of the hTfR-binding special cyclic peptide No. 894 and the fluorescent substance FITC was administered to the hTfR-KI mice in the same manner as the above-mentioned single dose except that the conjugate was administered in a dose of 3.7 mg/kg every 10 minutes for a total of 6 times. The localization of the above-mentioned conjugate in the brain tissue was confirmed by the immunohistochemical staining. The result is shown in Fig. 4. Note that, in the figure, red arrows indicate the cerebral capillaries, arrowheads indicate the Purkinje cells, and yellow arrows indicate dendrites. (1) shows a photograph of the #894-FITC administration group and (2) shows a photograph of the group not administered with #894-FITC as a control.

The present test confirmed that, in both dose tests, the conjugate of the hTfR-binding special cyclic peptide No. 894 and the fluorescent substance FITC was introduced into the cerebellum by passing through the BBB. Further, the result of the multiple dose test revealed that the conjugate reached as far as neurons such as the Purkinje cells.

### [Example 7]

### Evaluation test by SPR of intermolecular interaction between transferrin receptor (hTfR), and hTfR No. 894 variant peptides, linker-added peptides, and payload conjugates

### [Synthesis of hTfR No. 894 variants and linker-added peptides]

Peptides having a sequence in which several amino acids were inserted, deleted, or substituted in the amino acid sequence of the hTfR No. 894 (also referred to as variants) and the peptides to which various linkers were bound were synthesized, and the hTfR binding ability of these peptides was confirmed by SPR in the same manner. The variant peptides were synthesized according to Example 1 unless otherwise stated in Example 9 or 10. The linker-added peptides were synthesized according to Example 1, except that Fmoc-NH-SAL-PEG resin (Watanabe Chemical Industries) was used as a resin for the peptide synthesis when the linker was PEG. The peptide, to which a peptide, a fatty acid, PEG, or a conjugate thereof is added as a linker, was also synthesized according to Example 1, unless otherwise stated in Example 9 or 10. Table 2 to Table 4 show KD values obtained from the SPR measurement of these peptides with the results denoted as follows : KD value less than 1 nM: A, 1 nM or more and less than 100 nM: B, 100 nM or more and less than 1 µM: C, and 1 µM or more: D.

### Table 2: Peptides or linker-added peptides

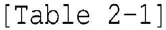

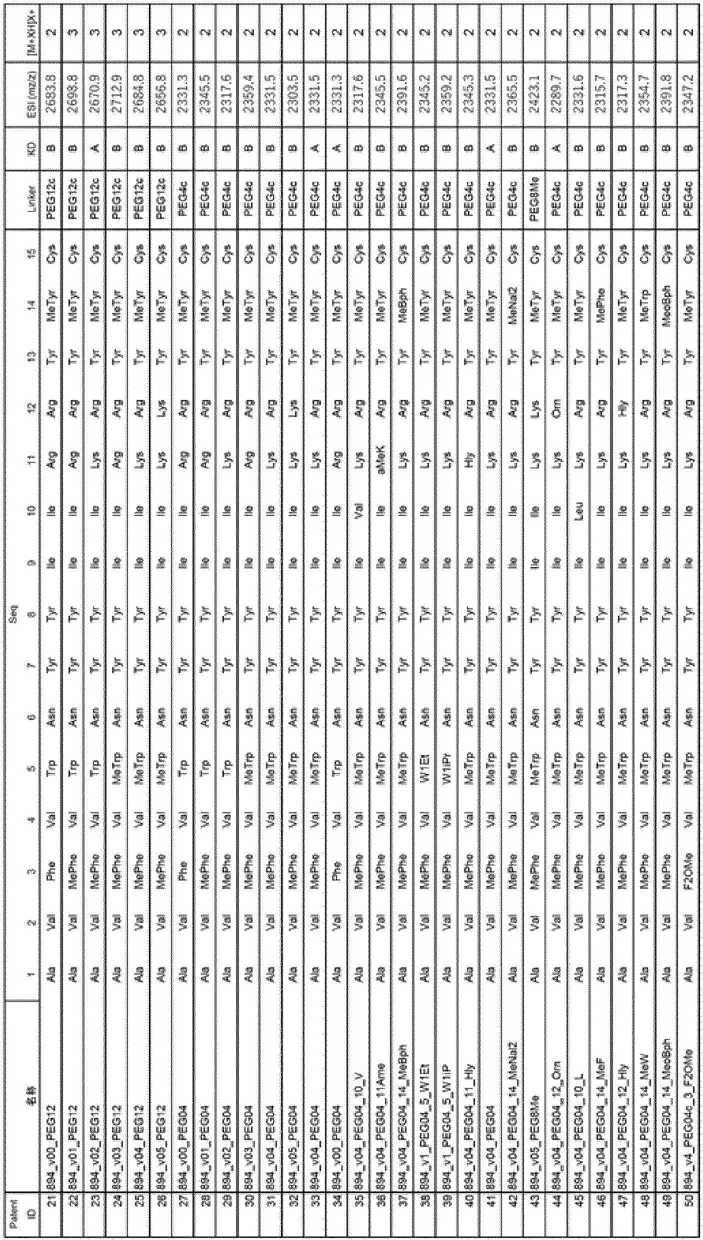

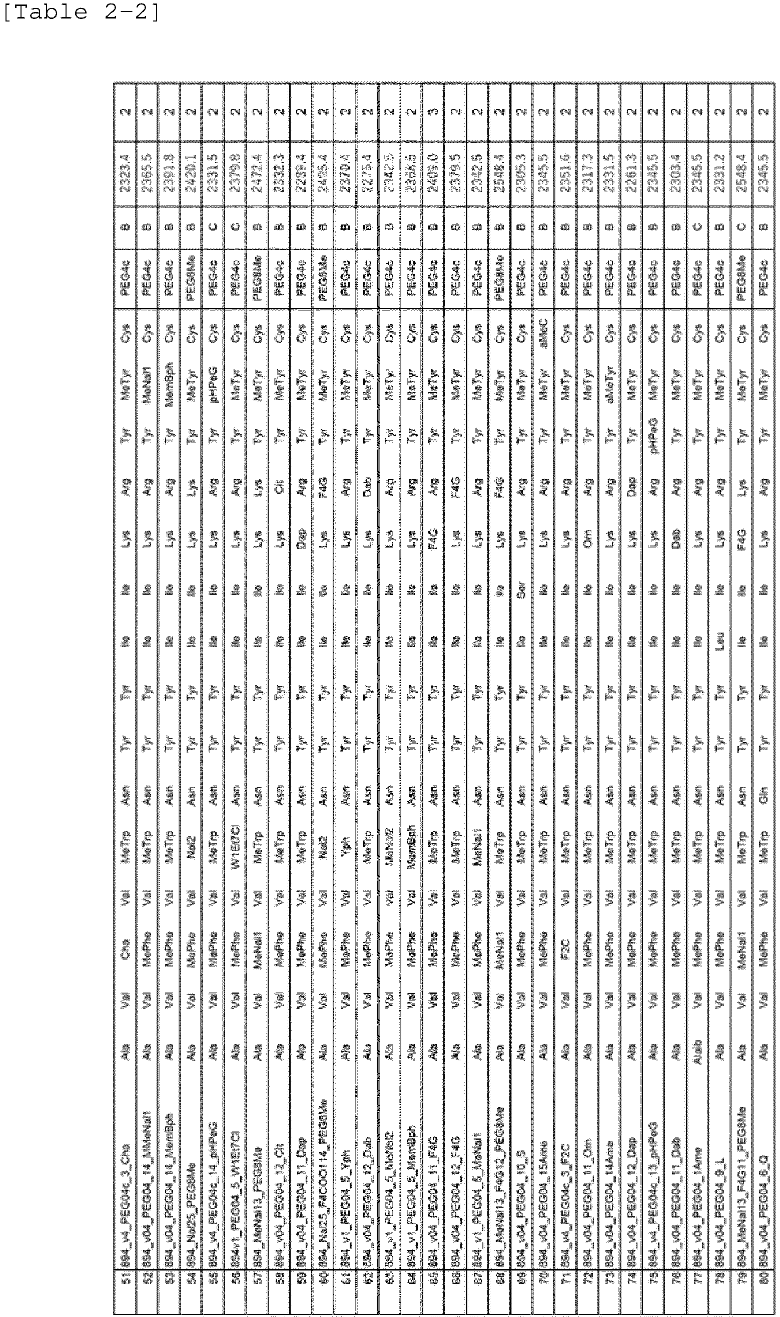

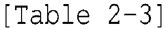

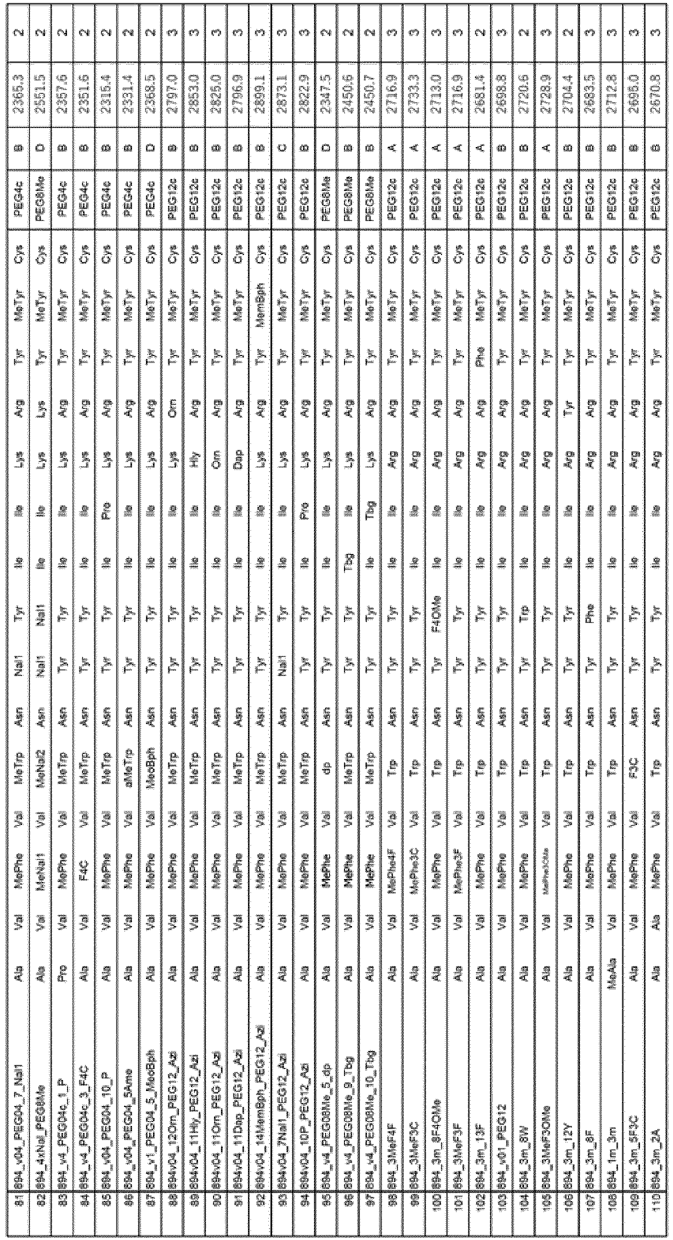

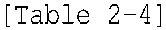

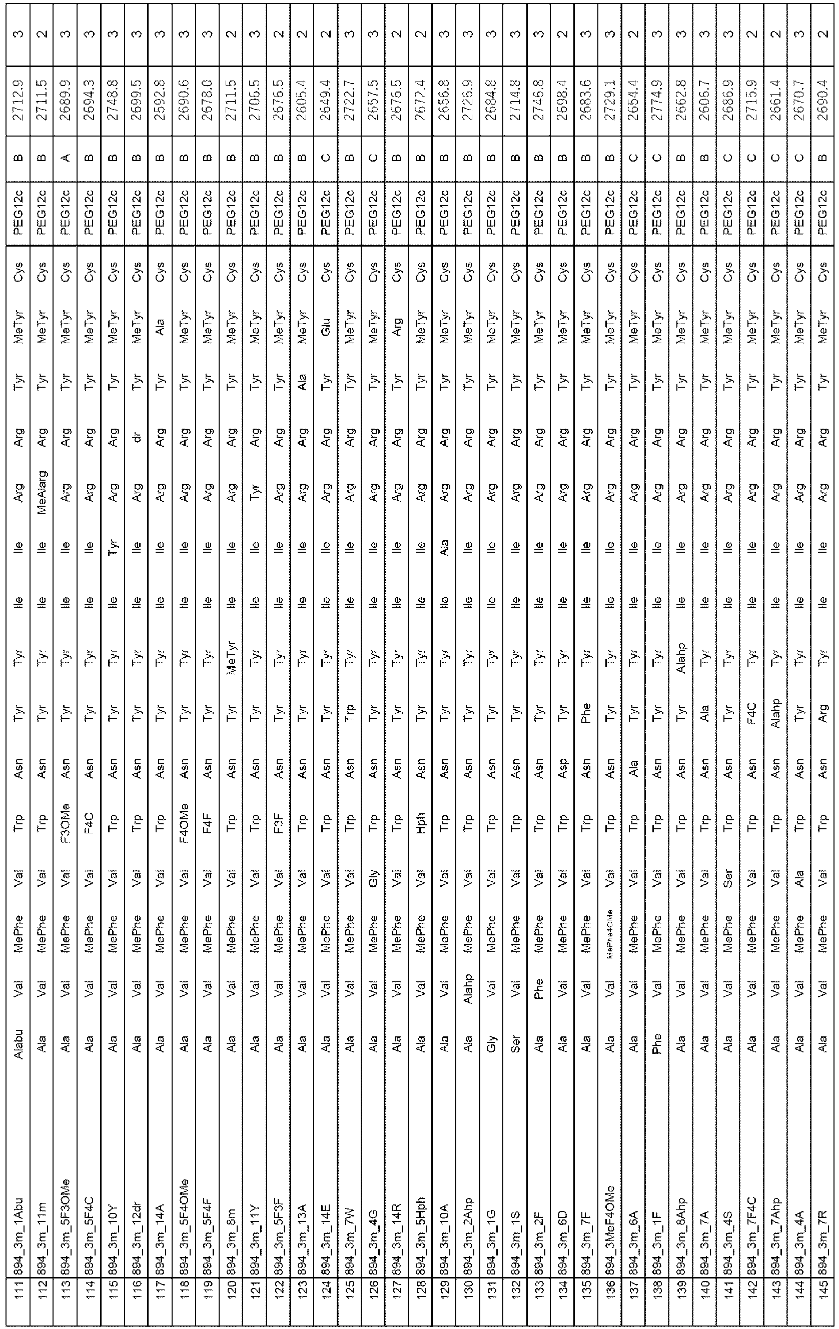

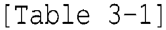

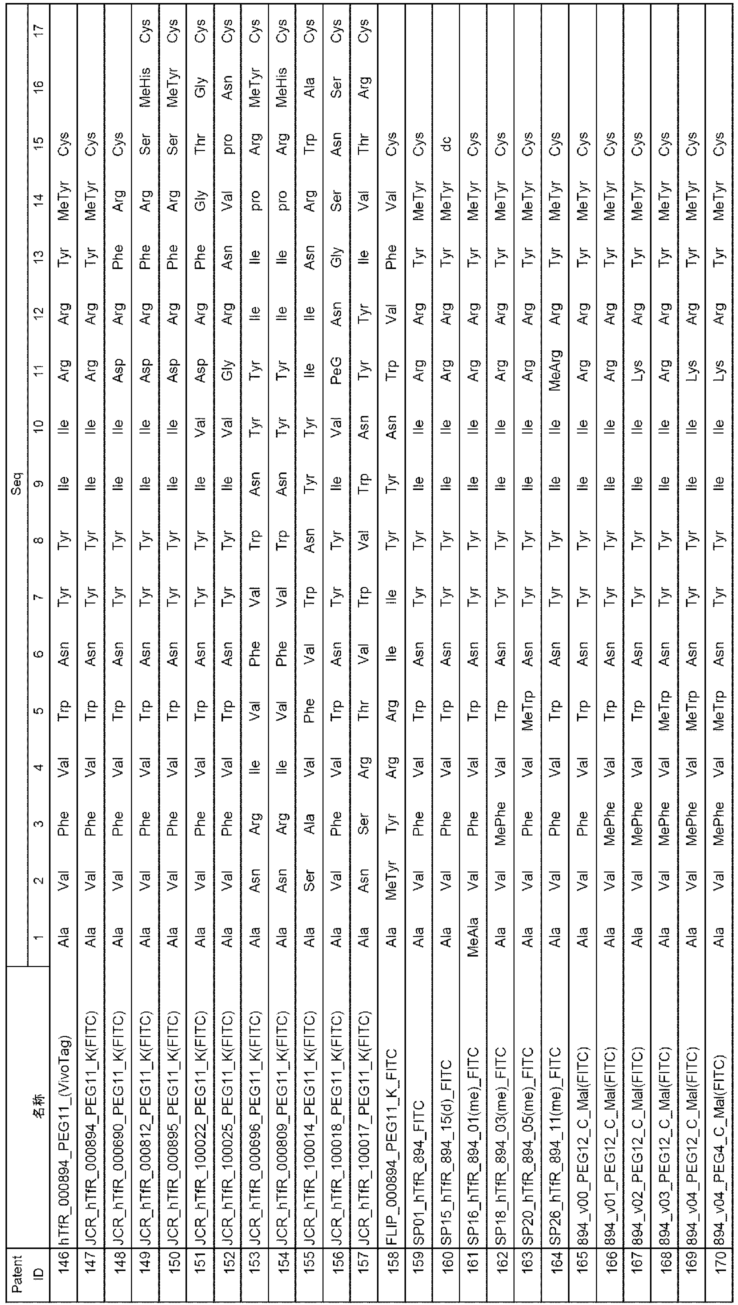

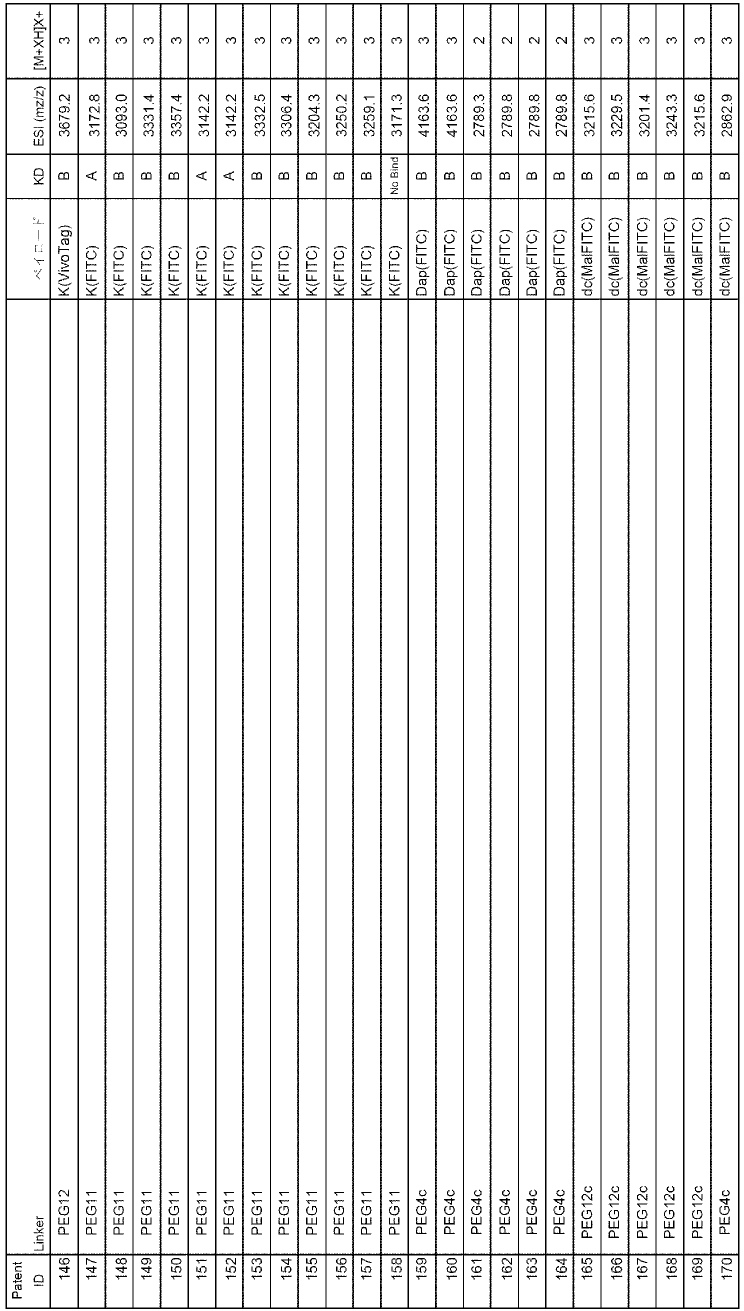

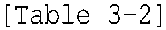

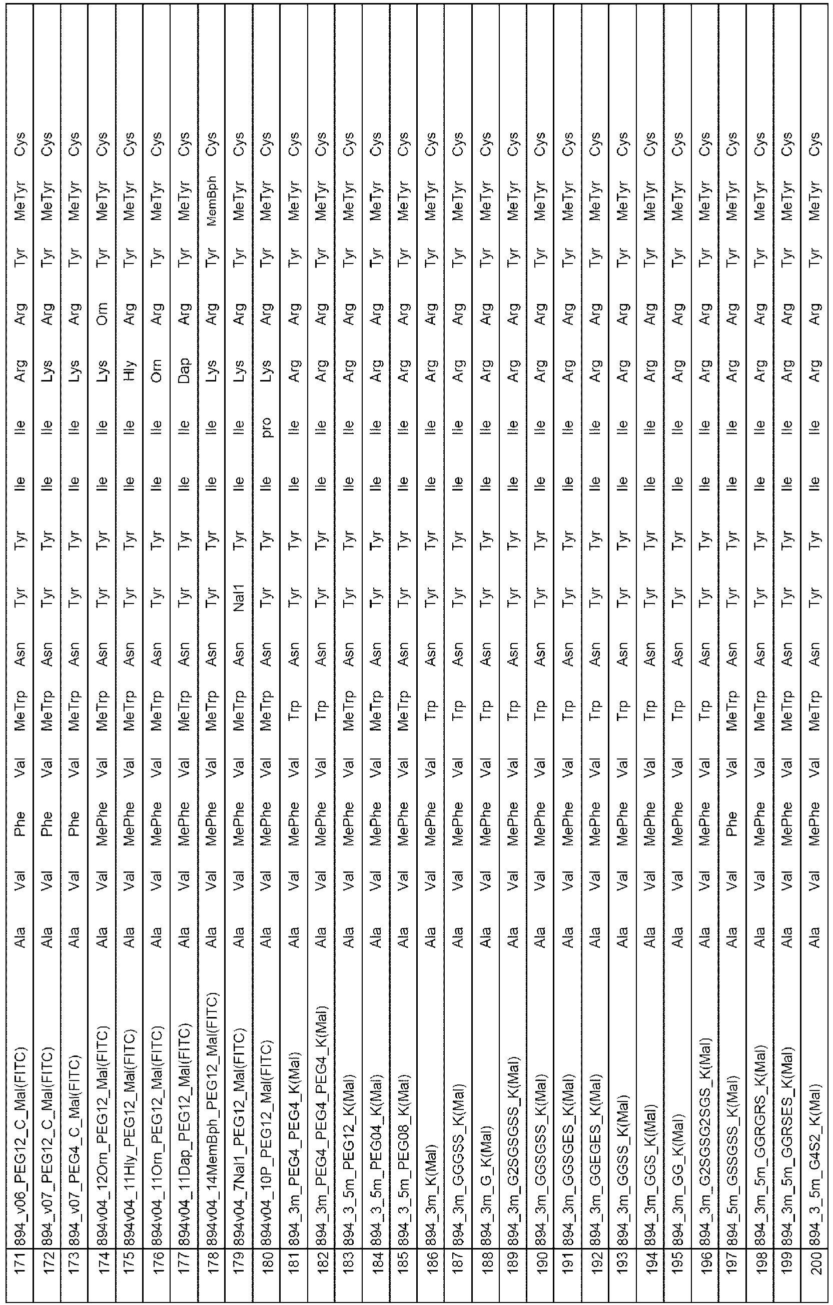

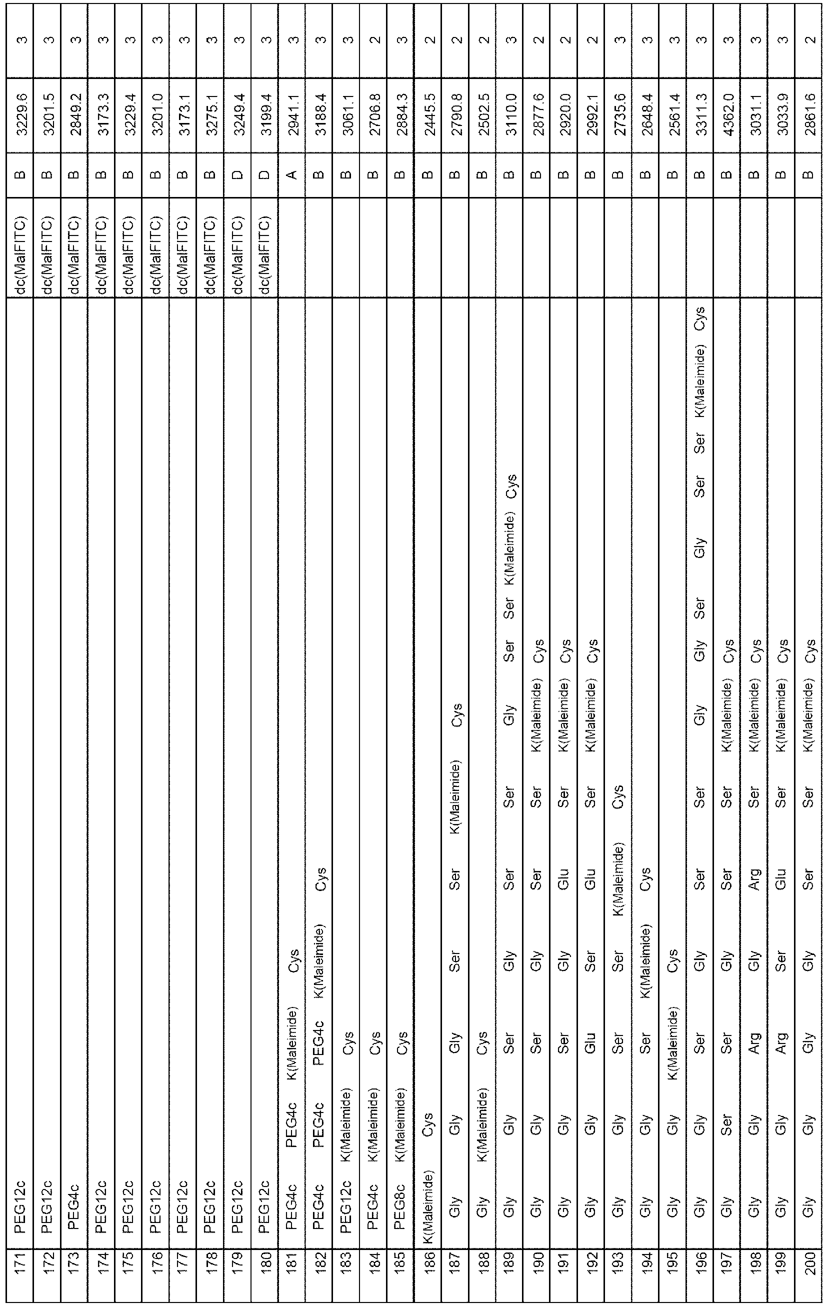

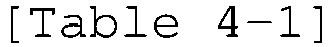

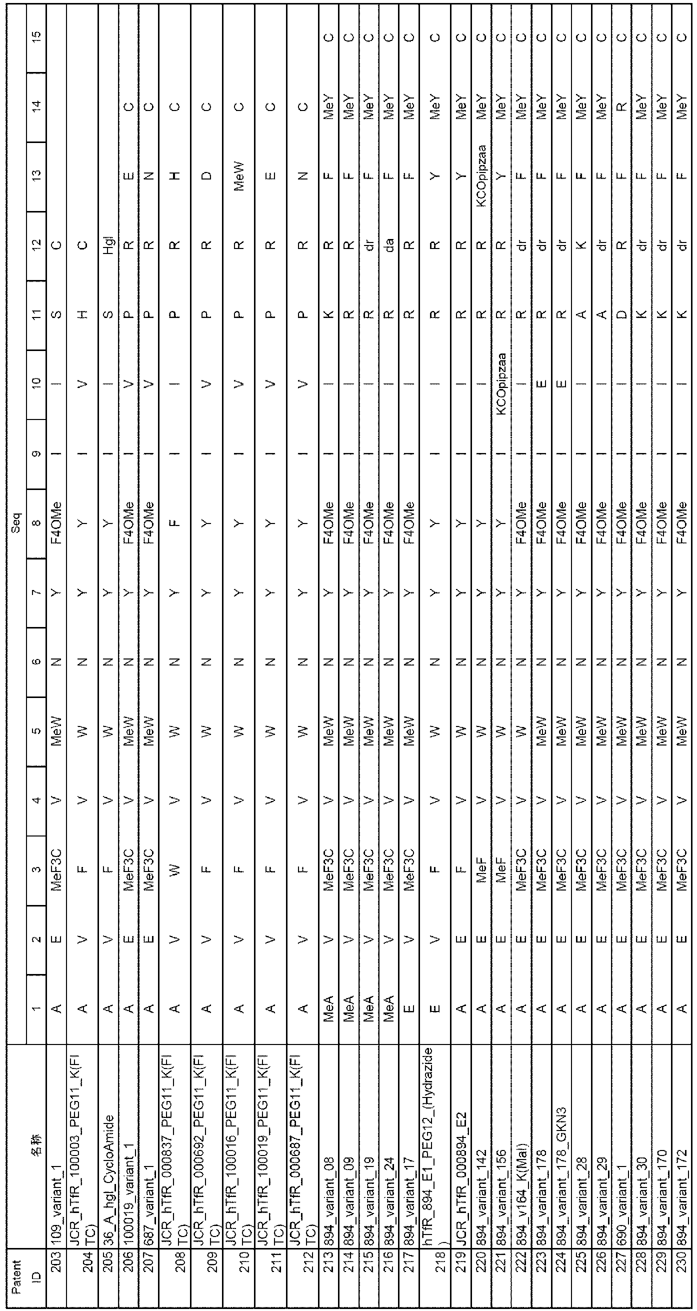

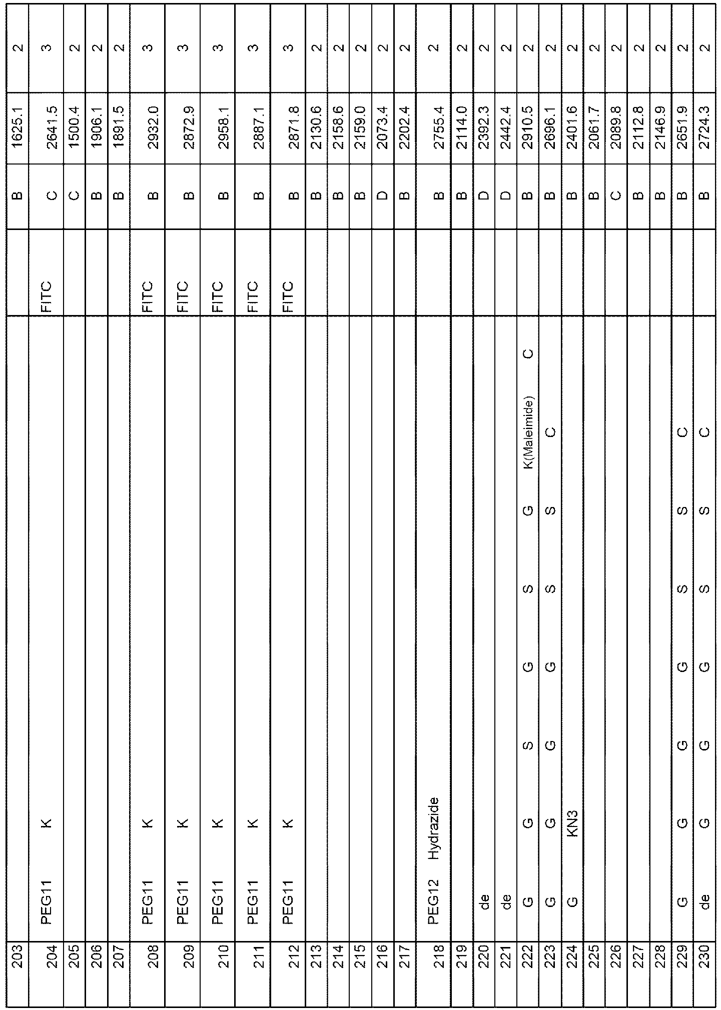

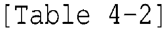

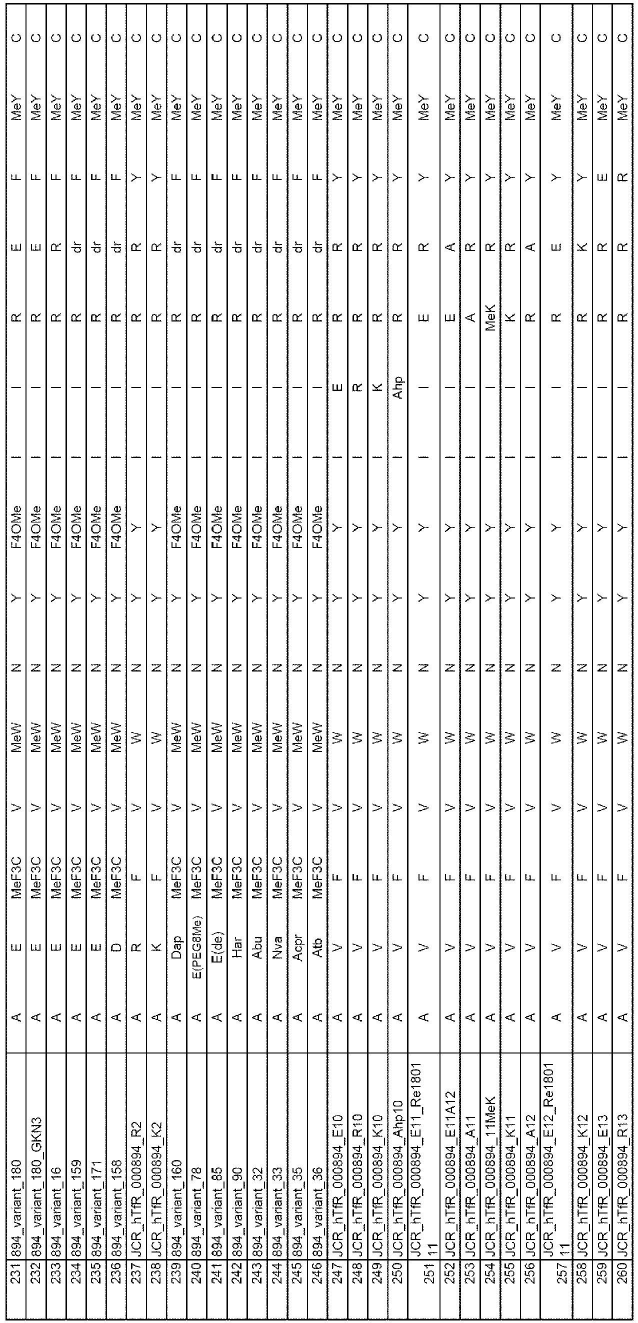

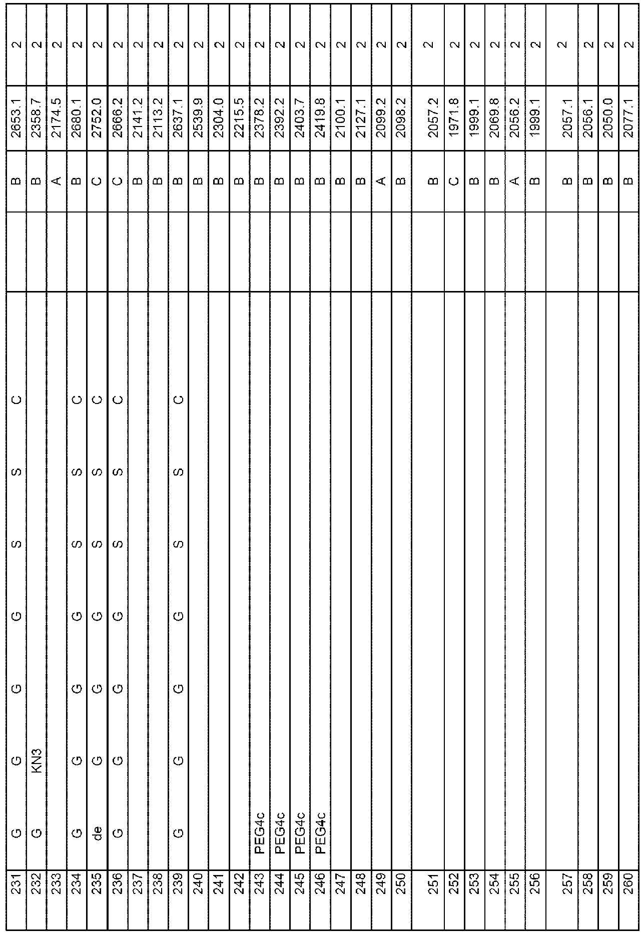

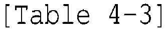

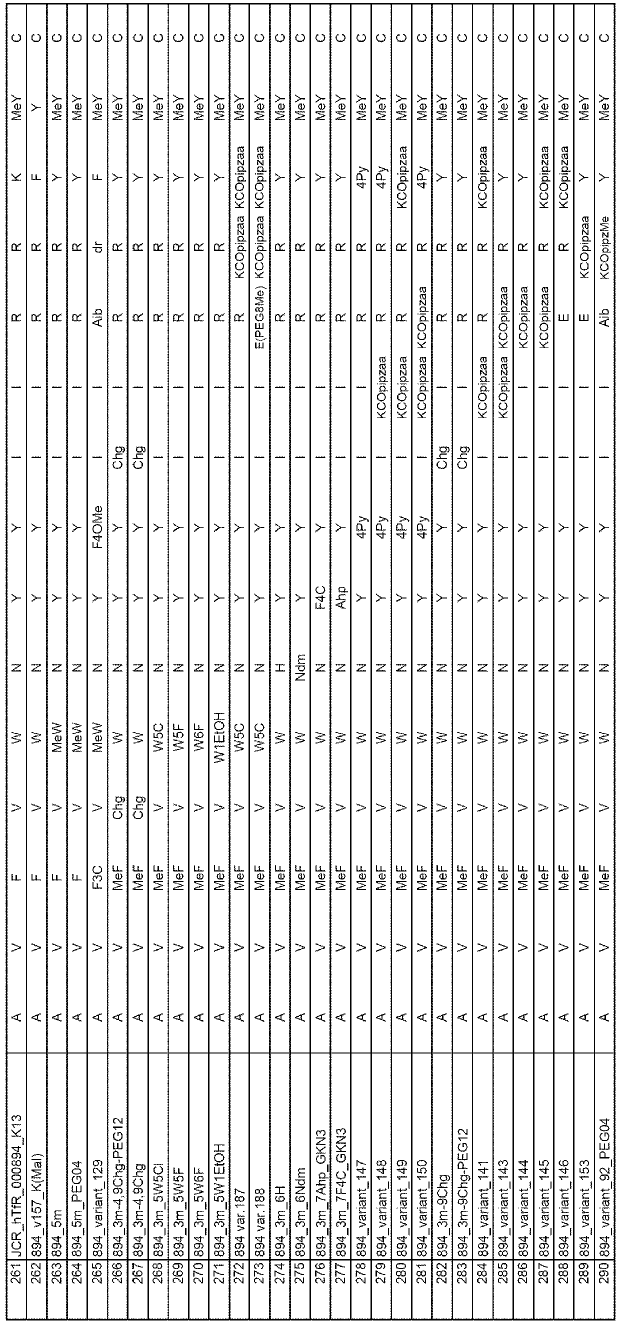

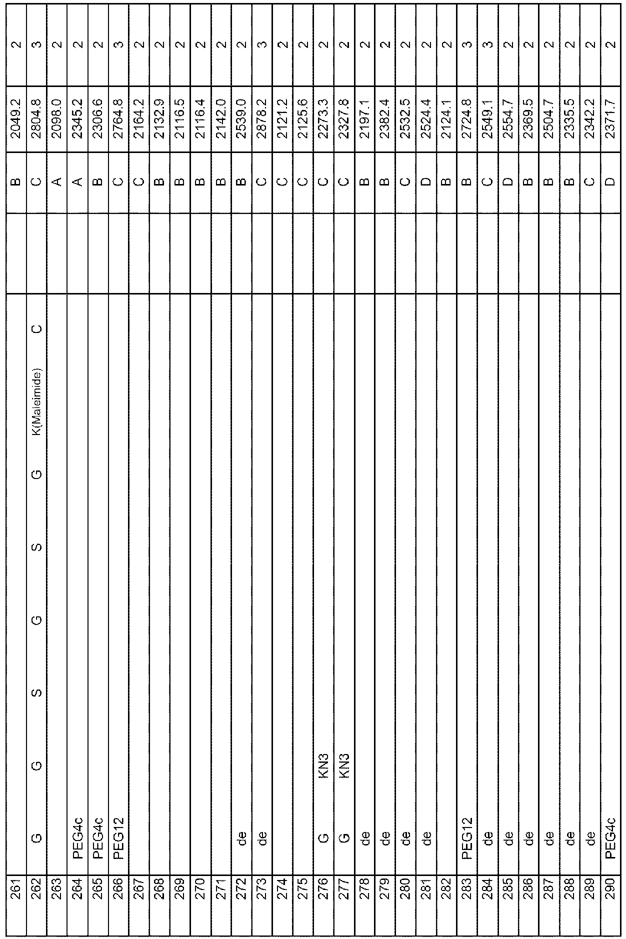

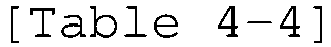

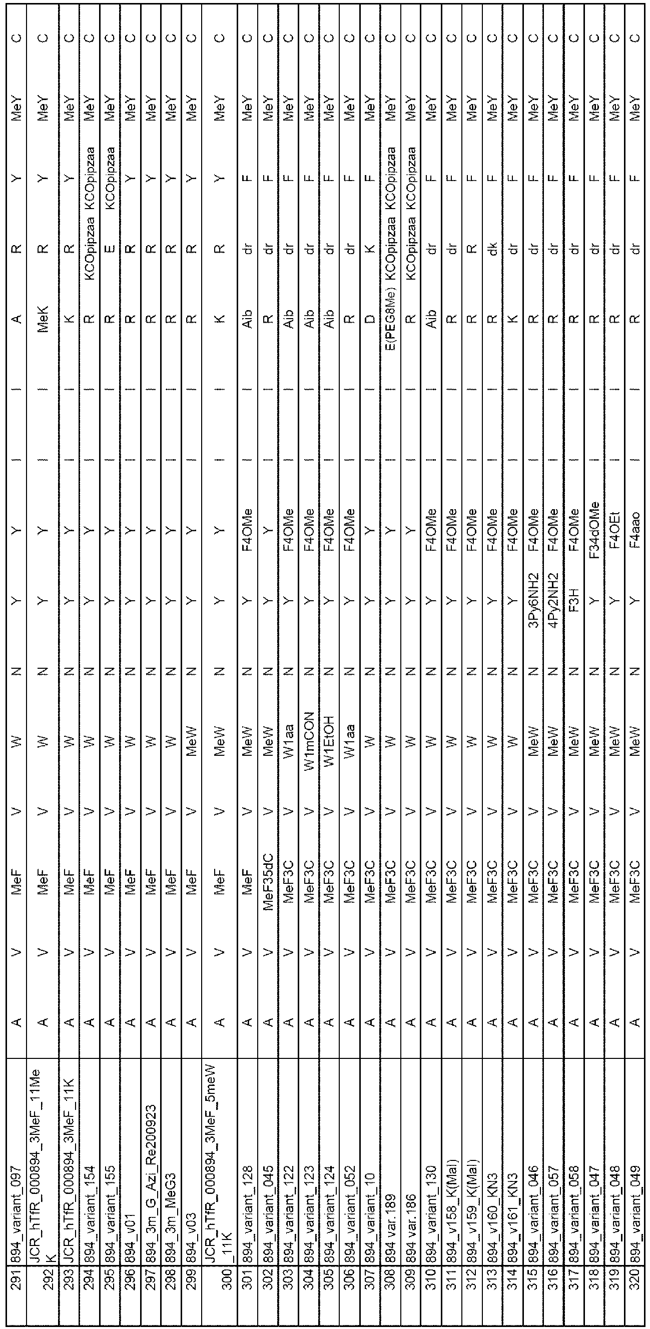

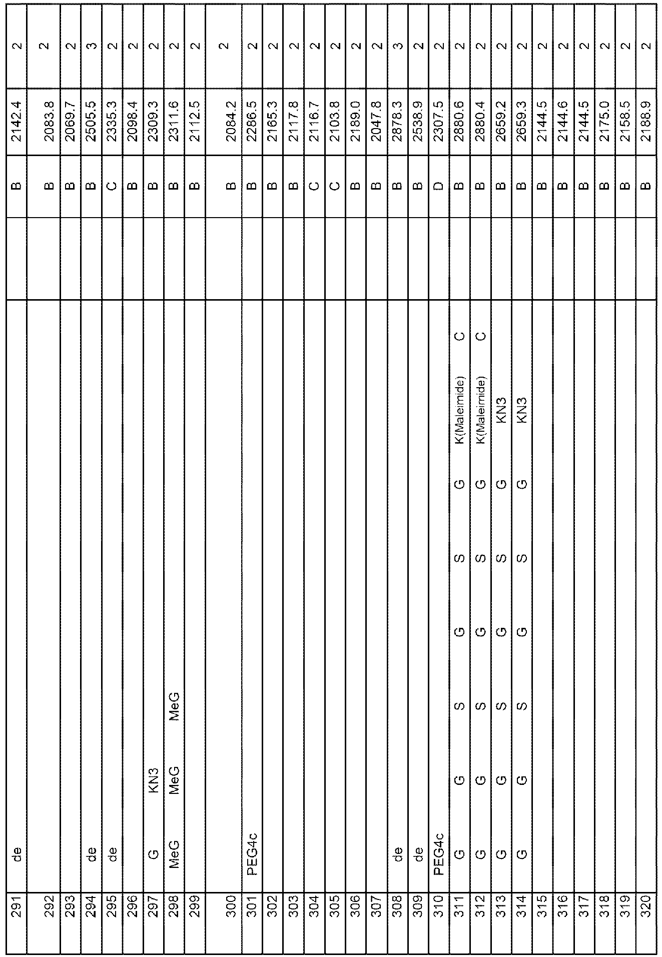

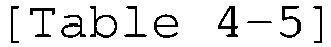

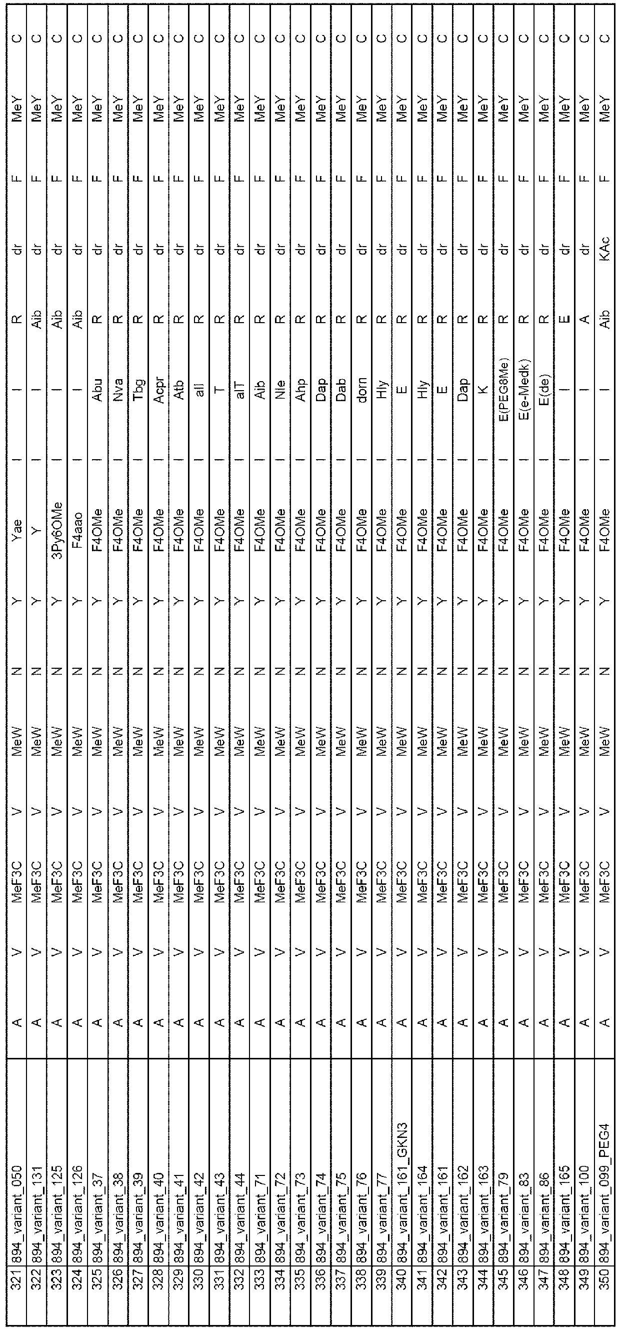

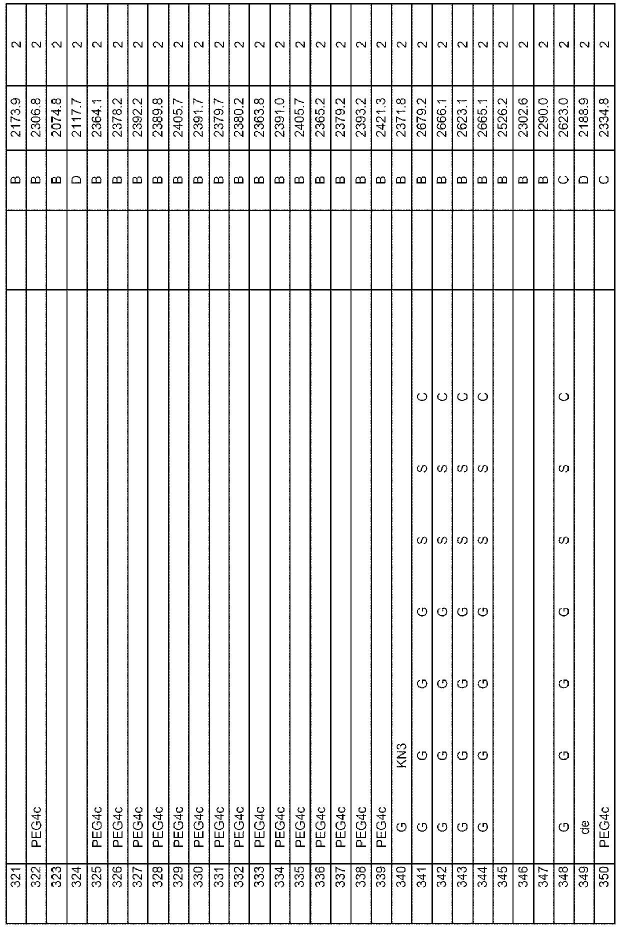

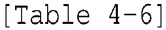

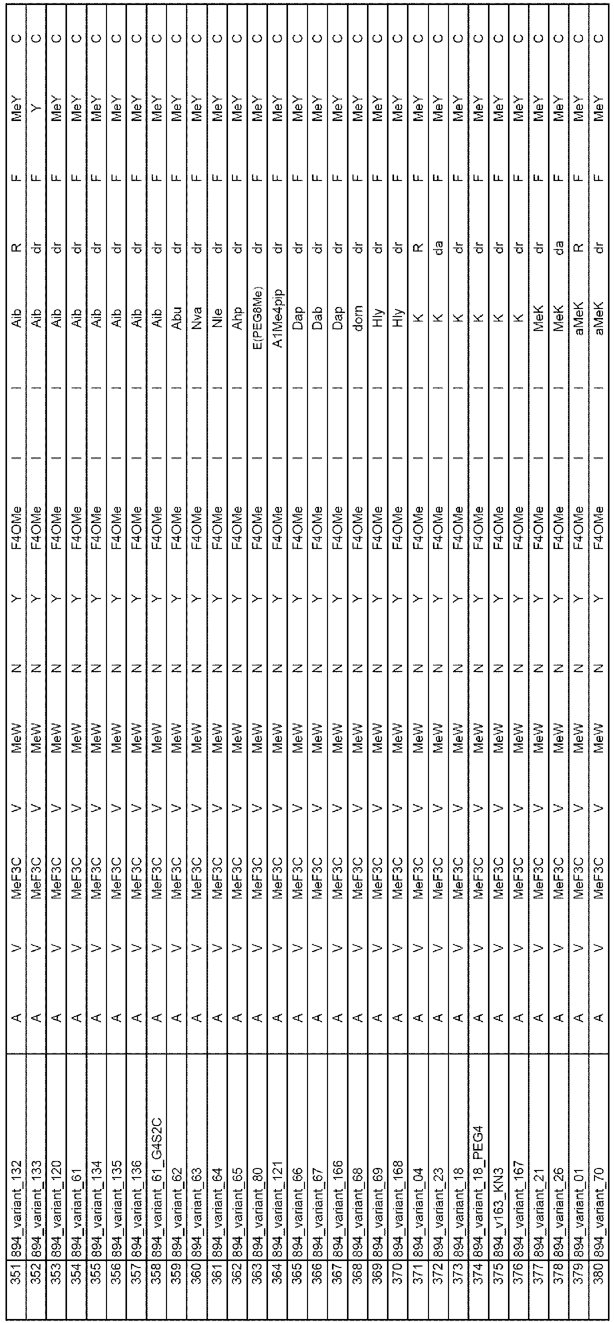

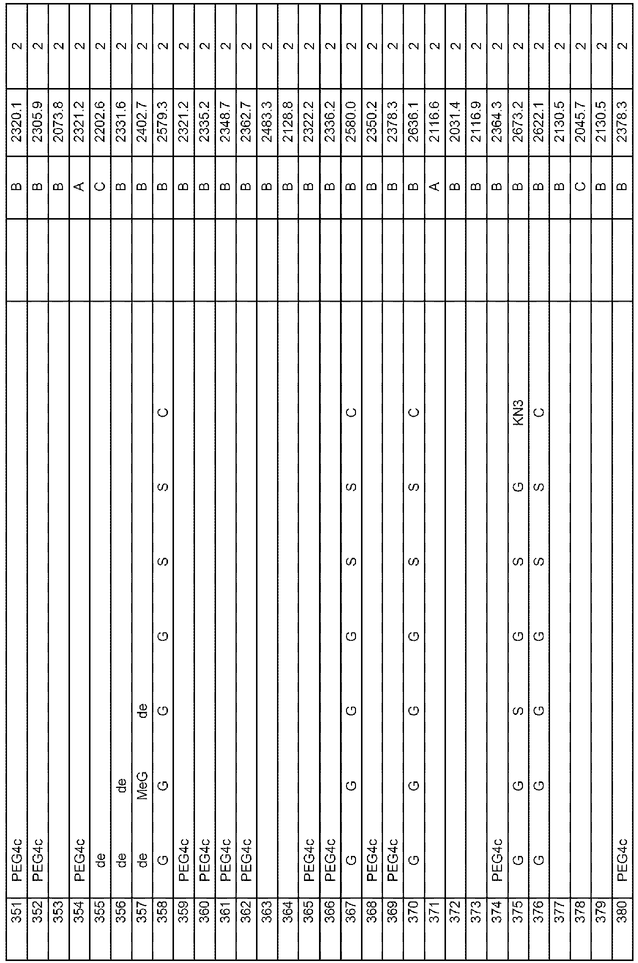

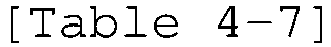

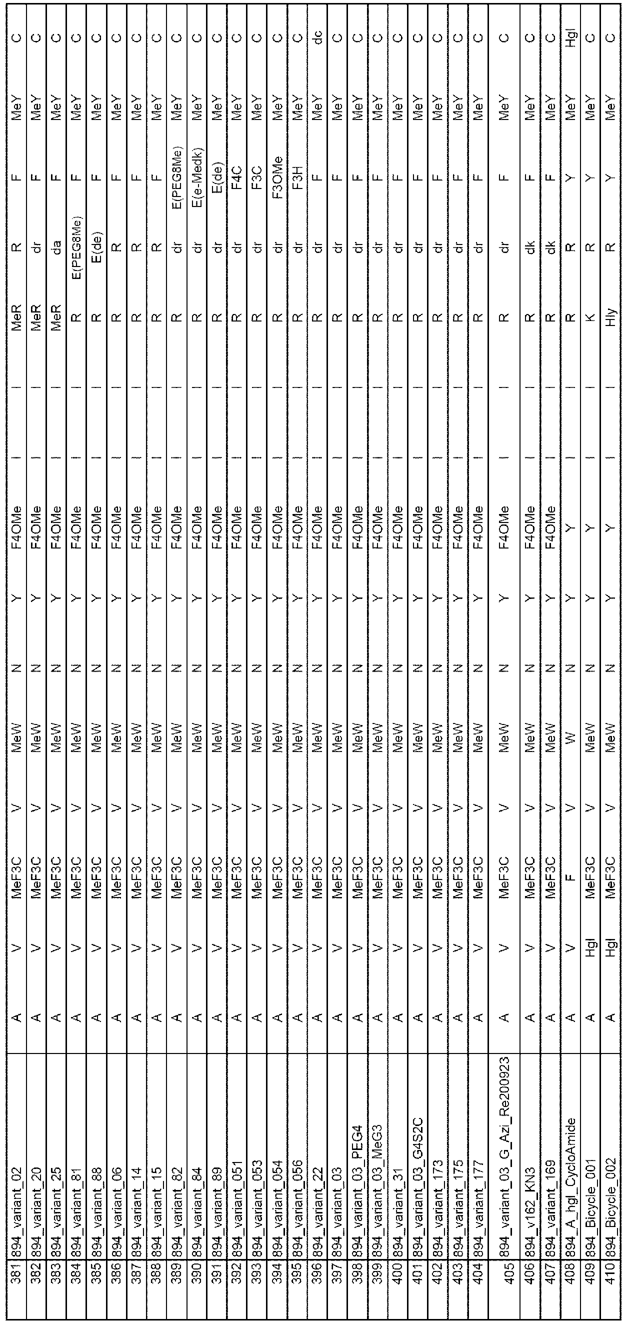

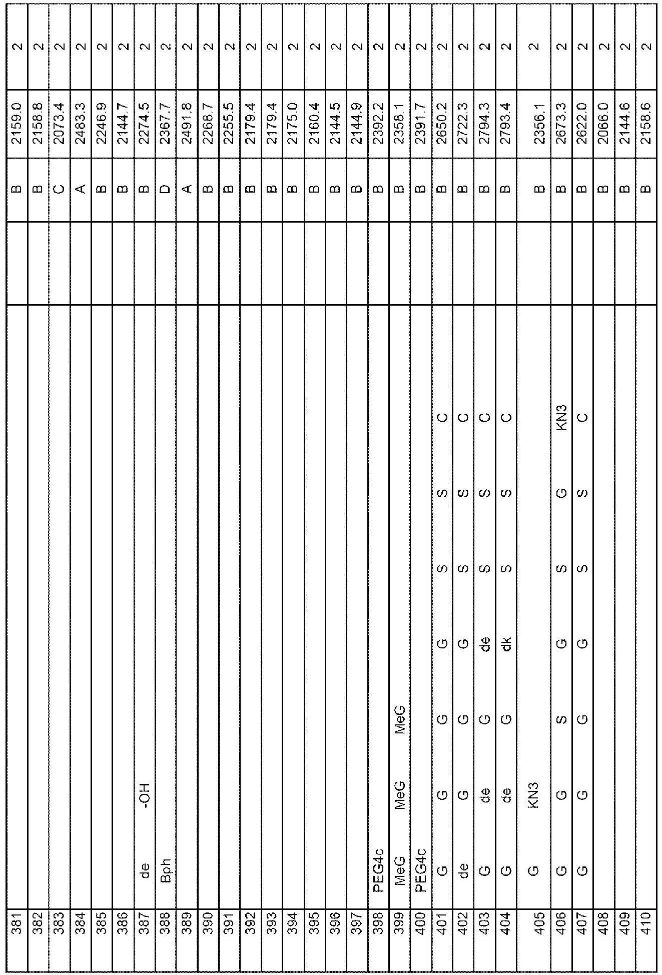

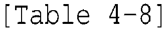

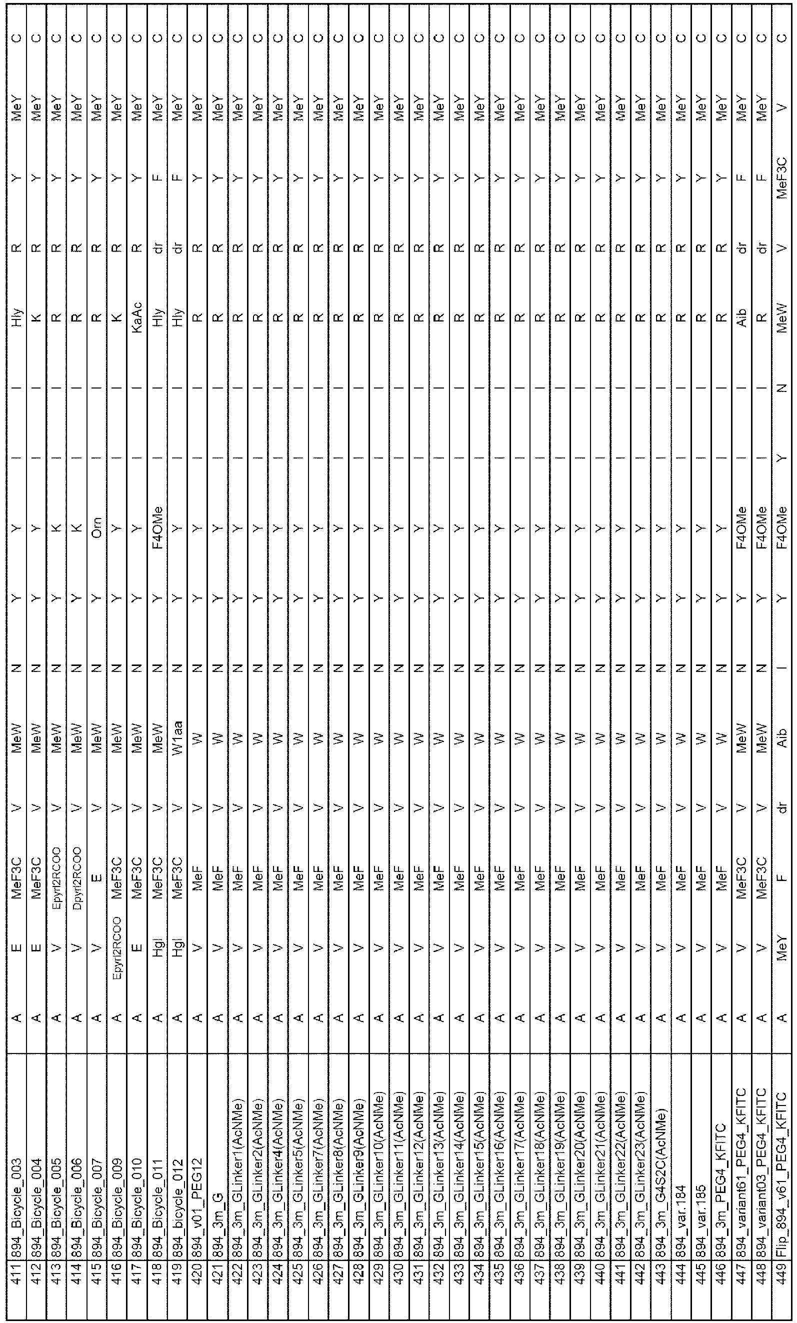

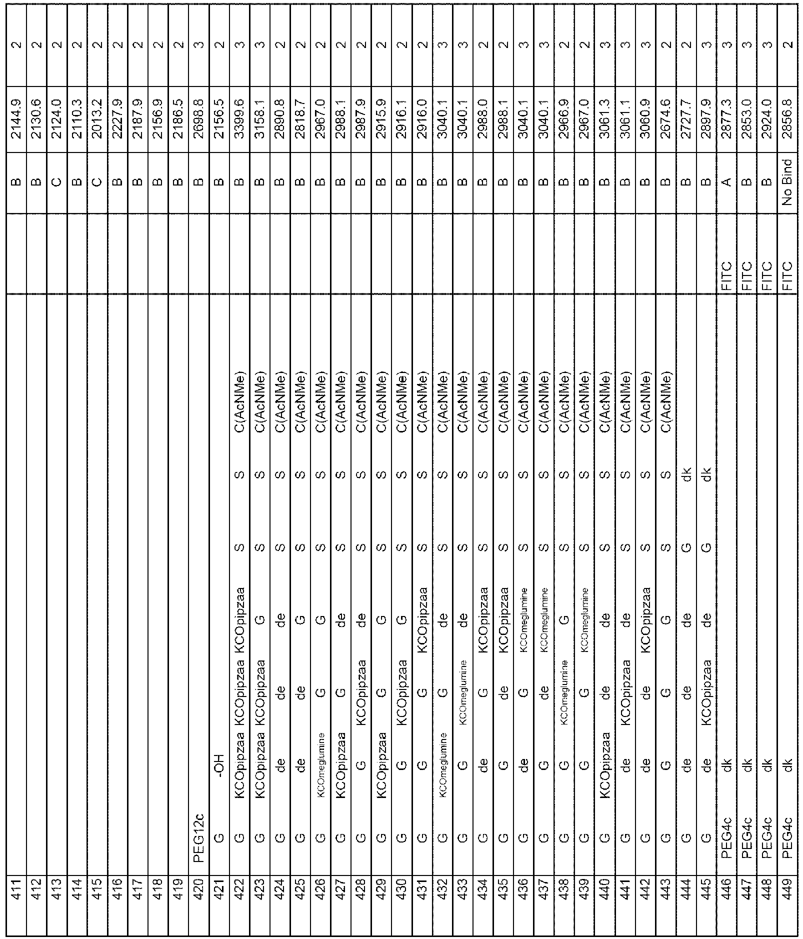

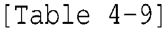

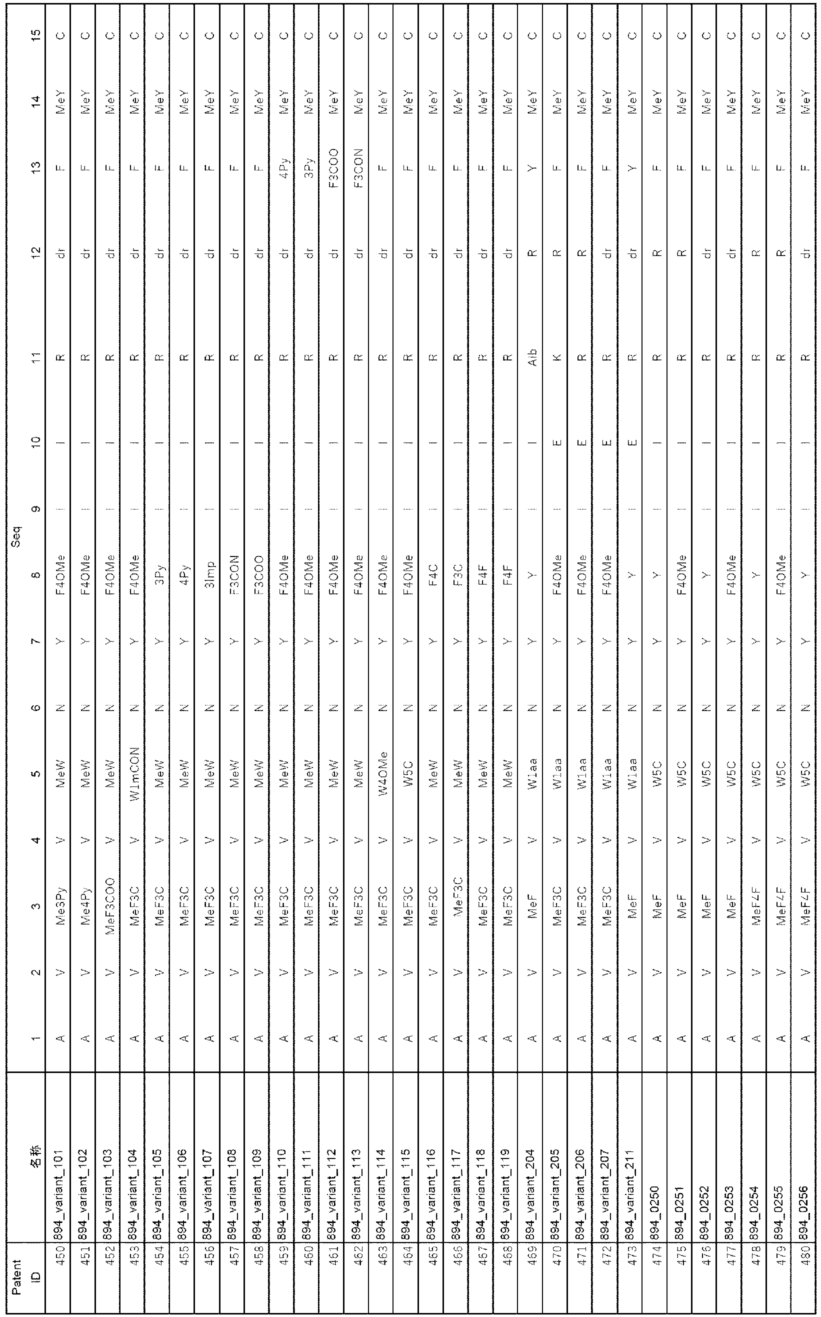

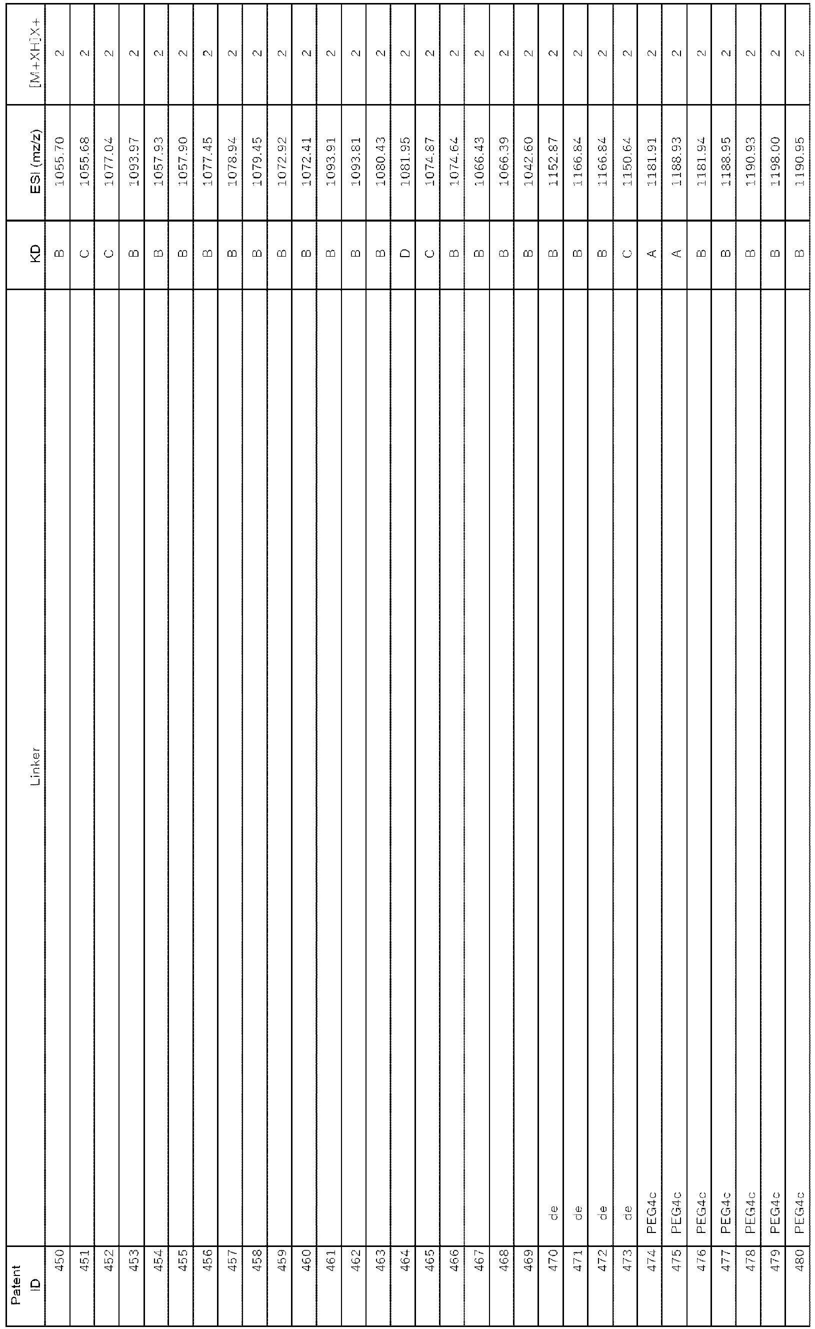

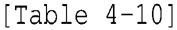

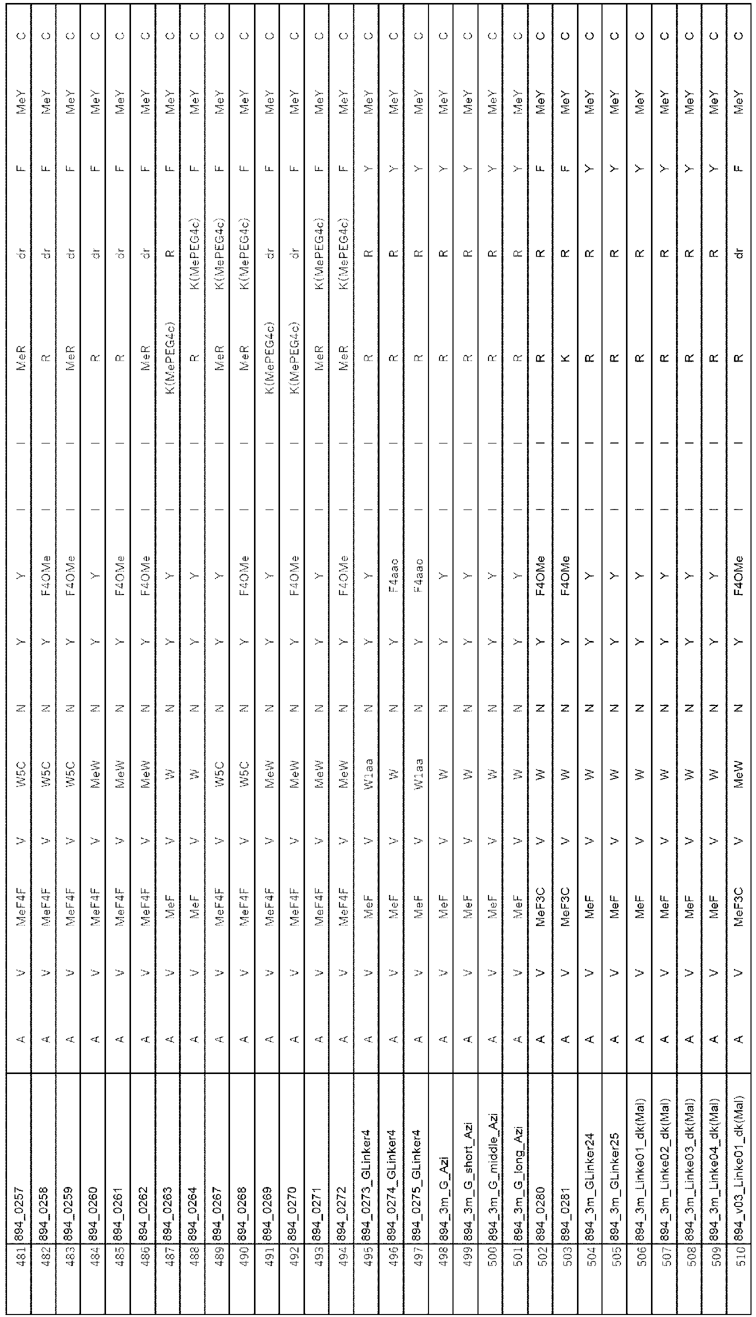

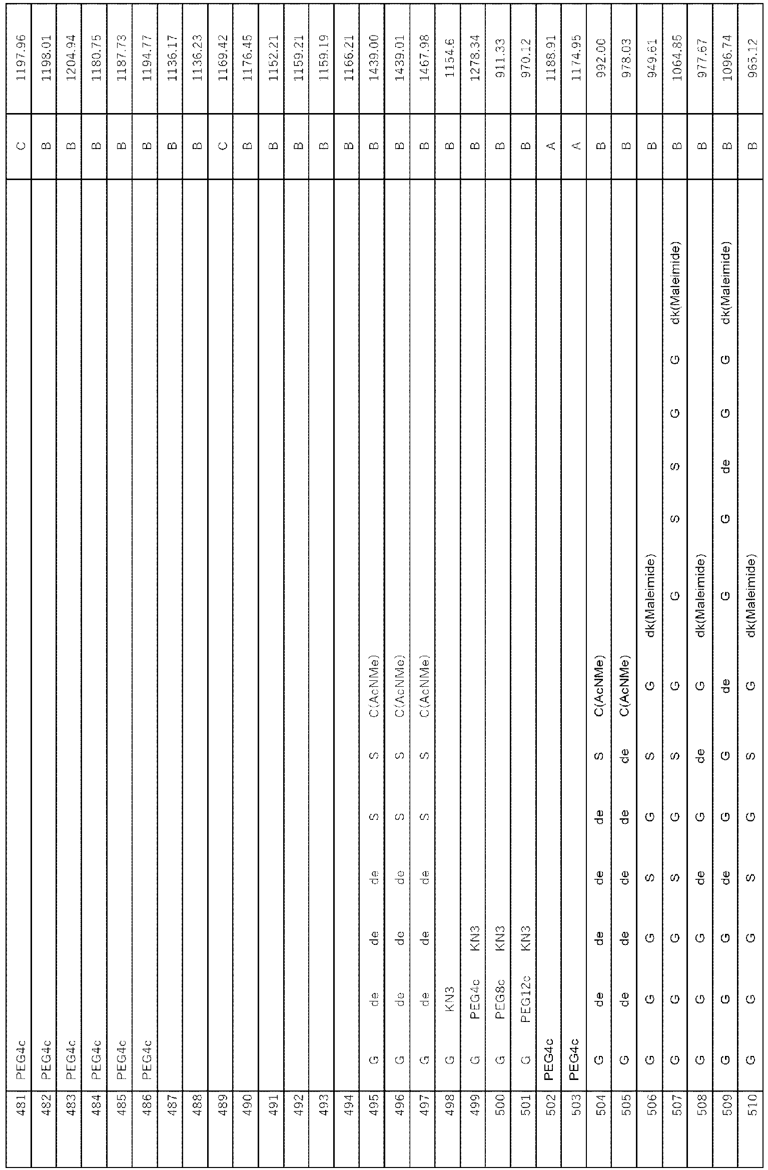

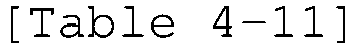

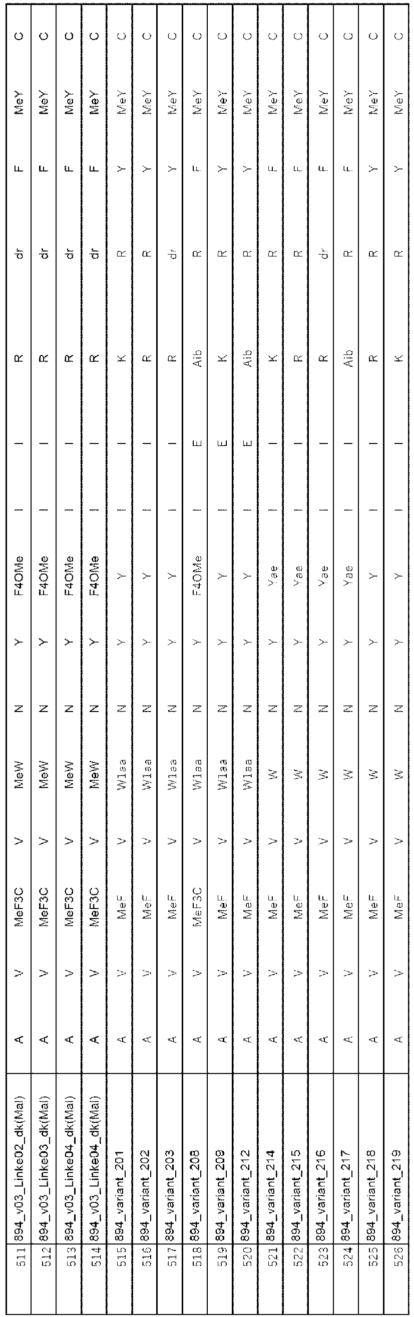

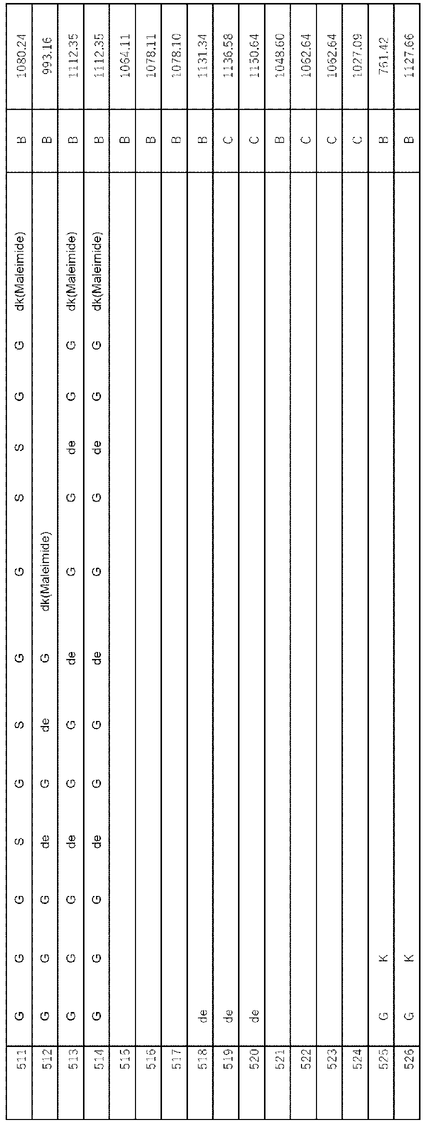

The results showed that hTfR No. 894, the peptides having the amino acid sequence of hTfR No. 894 in which several amino acids were inserted, deleted, or substituted, and the peptides to which various linkers were bound had the hTfR binding ability.

### [Example 8]

### [Cell culture]

Human breast cancer cells BT-549 (Cosmo Bio) were cultured using an RPMI-1640 medium (Thermo Fisher Scientific) containing 10% FBS and 2 mmol/L L-Glutamine. Culturing was performed under conditions of 37°C and 5% CO₂.

### [Cell inoculation]

Collagen Type I (Corning) was diluted to 50 µg/mL with 20 mmol/L acetic acid. A sterilized cover glass was placed in each well of the 24-well plate. After the diluted Collagen Type I solution was added to the plate, the plate was incubated at 37°C for 1 hour. The Collagen Type I solution was removed from the plate and the plate was washed 3 times with PBS. The human breast cancer cells BT-549 were inoculated in an amount of 1 × 10⁵ cells per 1 well and cultured overnight under conditions of 37°C and 5% CO₂.

### [Synthesis of various peptide conjugates]

Samples used were as follows: hTfR_894_3m_PEG4dk5FAM (SEQ ID NO: 446), hTfR_894_variant03_PEG4dk5FAM (SEQ ID NO: 448), hTfR_894_variant61_PEG4dk5FAM (SEQ ID NO: 447), and as a negative control, Flip894_variant61_PEG4dk5FA (SEQ ID NO: 449)M.

### Synthesis of hTfR_894_3m_PEG4dk5FAM

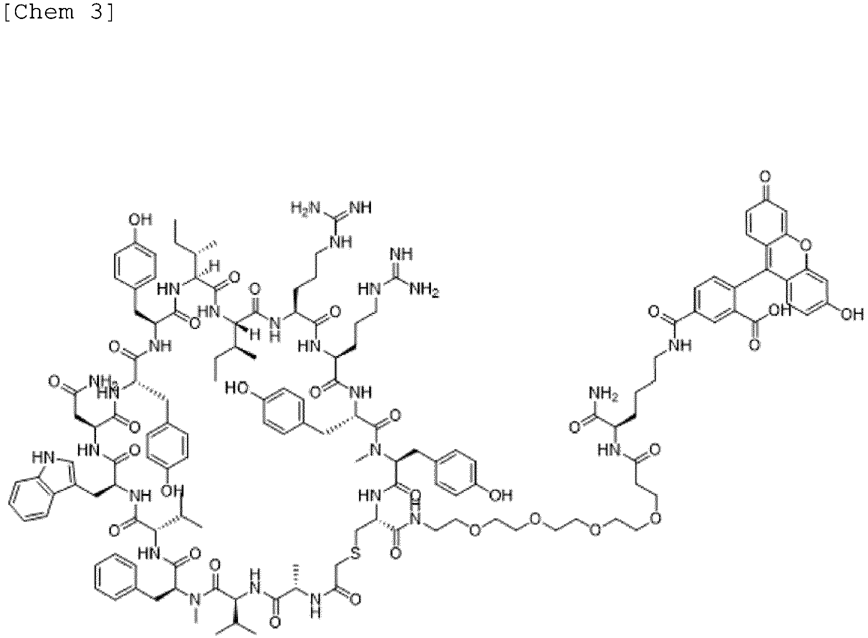

Starting from removal of Fmoc using Sieber amide resin (Watanabe Chemical Industries, Ltd., 0.47 mmol/g, 0.53 g), the target peptide was synthesized. For the synthesis, CEM's Liberty Blue HT was used as an automatic synthesizer, and the synthesis was performed according to the manufacturer's manual. The basic conditions for the introduction of each residue were such that using Fmoc-AA/DIC/Oxyma pure = 4.2 Eq/4 Eq/8 Eq per Eq of the resin, the reaction was performed at 75°C for 10 minutes. However, for the 2nd residue, the reaction was performed twice at 75°C for 30 minutes. For the 11th and 12th residues, the reaction was performed twice at 25°C for 20 minutes. For the 13th residue, the reaction was performed twice at 75°C for 10 minutes. For the 15th residue, the reaction was performed at 25°C for 20 minutes. The basic conditions for Fmoc removal were such that the reaction with a DMF solution of 20% piperidine was performed at 75°C for 3 minutes. However, the Fmoc groups of the 2nd and 13th residues were removed by reaction at 25°C for 5 minutes, and then reaction for 10 minutes. A chloroacetyl group was introduced in such a manner that after the Fmoc group of the α-amino group was removed by the method described above from the solid phase resin holding the Fmoc-protected peptide obtained in the previous step, 10 Eq of chloroacetic acid, 10 Eq of DIPCI, and 10 Eq of HOSu were stirred in DCM, and the same amount of DMF as DCM was added to prepare a DCM/DMF solution of ClAcOSu, which was then added to the solid phase resin and shaken at room temperature for 60 minutes. For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diethyl ether/hexane (1/1) cooled to 0°C, a white cloudy precipitate was formed. The mixture was subjected to centrifugation separation (9000 rpm, 2 min), and the solution was subjected to decantation. After the obtained solid was washed again with a small amount of diethyl ether cooled to 0°C, the obtained solid was used in the subsequent cyclization reaction. In the peptide cyclization reaction, after the solid was dissolved in DMSO so that the peptide final concentration was 5 mM based on the number of moles of the solid phase resin, 5 Eq of triethylamine was added and shaken at room temperature for about 14 hours. F-FAM-NHS prepared with 1.1 Eq of 5-FAM (Funakoshi Co., Ltd.), 1.2 Eq of EDC, and 1.2 Eq of HOSu was added to the reaction solution, followed by stirring at room temperature for 3 hours . The obtained reaction solution was concentrated under reduced pressure using Savant Explorer SpeedVac.

The obtained crude intermediate peptide was purified under the following conditions (column: Waters XBridge (registered trademark) C18 5 µm OBD (registered trademark) 50 x 150 mm; mobile phase: A = 0.1% TFA in H₂O, B = 0.1% TFA in MeCN; temperature: 40°C; gradient (%B): 9-34% over 3 minutes, then 34-39% over 8 minutes, and then 39-60% over 1 minute; flow rate: 120 mL/min). After freeze-drying, purification was performed again under the following conditions (column: COSMOSIL PBr 10 x 150 mm; mobile phase: A = 0.1% TFA in H₂O, B = 0.1% TFA in MeCN; temperature: 40°C; gradient (%B): 21-46% over 3 minutes, then 46-51% over 8 minutes, and then 51-60% over 1 minute; flow rate: 5 mL/min).

The purity of the target product was calculated from the area ratio of the LC/MS (UV wavelength: 225 nm) chromatogram under analysis conditions B, and the result was 95.3%. Analysis conditions B: retention time = 4.47 minutes; column: Kinetex EVO C18 2.6 µm 2.1 x 150 mm, 100Å; mobile phase: A = 0.025% TFA in H₂O, B = 0.025% TFA in MeCN: temperature: 60°C; gradient (%B conc) : 20-60% over 7.2 minutes, then 60-95% over 0.3 minutes, and then 95% over 1.6 minutes; flow rate: 0.5 mL/min.

ESI-MS(+) measured value m/z = 944.42 (M+3H)³⁺

### Synthesis of hTfR_894_variant03_PEG4dk5FAM, hTfR_894_variant61_PEG4dk5FAM, and Flip894_variant61_PEG4dk5FAM as negative control

To hTfR_894_3m_PEG4, hTfR_894_variant03_PEG4, hTfR_894_variant61_PEG4, and Flip894_variant61_PEG4, which were synthesized and cyclized in the same manner as for hTfR_894_3m_PEG4dk5FAM, F-FAM-NHS similarly prepared from 5-FAM was added to obtain the above conjugates.

The binding of the synthesized conjugates to hTfR was confirmed by SPR in the same manner as in Example 2. Only for Flip894_variant91_PEG4, no binding to hTfR was observed.

### [Preparation of sample solution and addition to cells]

For the dilution of the sample, a dilution medium (RPMI 1640 medium containing 0.5% bovine serum albumin and 20 µg/mL holo human transferrin) was used. The sample was diluted with the dilution medium to 100 nmol/L.

After it was confirmed that BT-549 cells cultured overnight in a 24-well plate were attached to the cover glass, the cells were washed twice with RPMI 1640 medium. RPMI 1640 medium containing 0.5% bovine serum albumin was added at 500 µL/well and allowed to stand on ice for 15 minutes, and then the diluted sample solution was added at 500 µL/well and allowed to stand under the conditions of 37°C and 5% CO₂ for 3 hours.

### [Cell immobilization, nuclear staining, and enclosure]

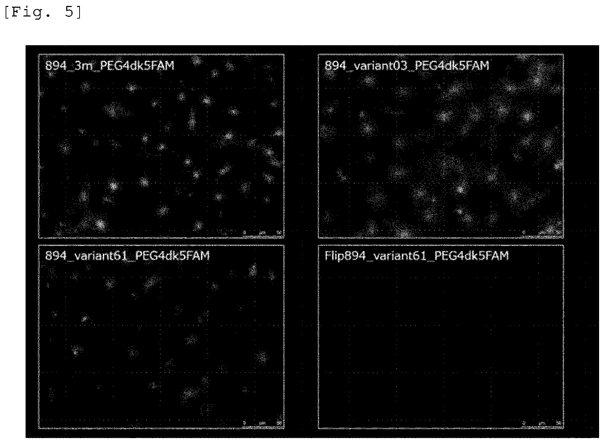

The sample solution was removed from the 24-well plate, and the BT-549 cells were washed three times with PBS. 4% paraformaldehyde phosphate buffer (FUJIFILM Wako Pure Chemical Corporation) was added at 500 µL/well, and the cells were allowed to stand at room temperature for 15 minutes and then washed three times with PBS. Further, Hoechst 33342 (Thermo Fisher Scientific) diluted with PBS to 2 µg/mL was added at 500 µL/well, and the cells were allowed to stand at room temperature under shading for 10 minutes and then washed three times with PBS. The cover glass was removed from the 24-well plate, and the cells were enclosed on a slide glass using fluorescent mounting medium (Agilent Technologies) and allowed to stand at room temperature under shading overnight. For the observation, an inverted fluorescence microscope DMI6000B (Leica Microsystems) was used, and the observation was performed at the wavelength for detecting FITC and DAPI. The results are shown in Fig. 5. The scale bar in the figures indicates 50 µm.

As can be confirmed in Fig. 5, migration of hTfR_894_3m_PEG4dk5FAM, hTfR_894_variant03_PEG4dk5FAM, and hTfR_894_variant61_PEG4dk5FAM into the cells was confirmed; however, Flip894_variant91_PEG4, which had a peptide without binding ability to hTfR, did not migrate into the cells. These results suggested that the variants with binding ability to hTfR_894 and hTfR migrated into the cells through the binding to hTfR.

### [Example 9]

The following peptides or linker-added peptides were synthesized.

### [Example 9-1]

### Synthesis of 894_v01_PEG12 (SEQ ID NO: 102)

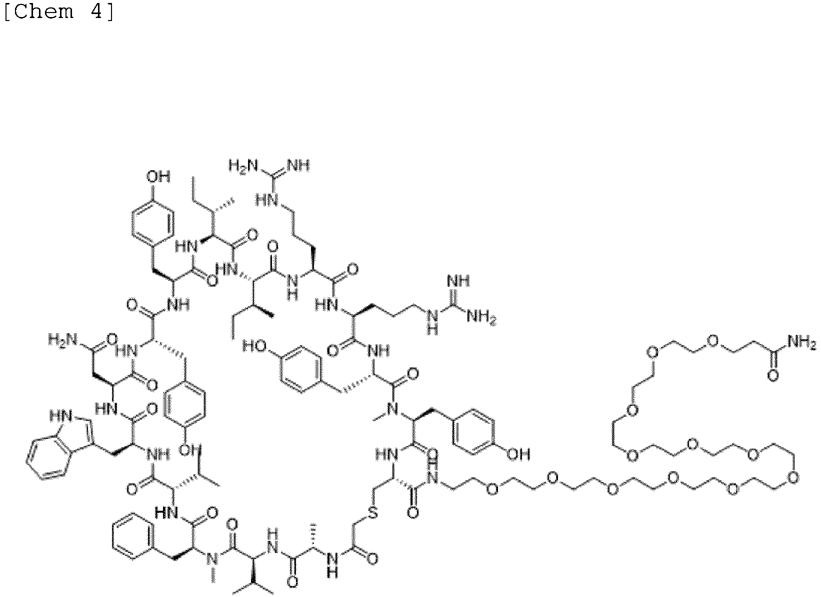

Starting from removal of Fmoc using Sieber amide resin (Watanabe Chemical Industries, Ltd., 0.52 mmol/g, 0.19 g), the target peptide was synthesized. For the synthesis, Biotage Syro I was used as an automatic synthesizer, and the synthesis was performed according to the manufacturer's manual. The basic conditions for the condensation reaction were such that the reaction was repeated twice at 75°C for 20 minutes . However, for the introduction of the 15th and 16th residues, the reaction was performed at room temperature for 60 minutes and 15 minutes, respectively. Further, for the introduction of PEG, the reaction was performed once, and for the introduction of the 11th residue amino acid, the condensation reaction was performed three times. The basic conditions for Fmoc removal were such that the reaction was performed in a DMF solution of piperazine (5%) and Oxima pure (0.2 M) at 50°C for 5 minutes, and the reaction was performed again for 15 minutes. However, the Fmoc groups of the 15th and 16th residues were removed by reaction at 25°C for 5 minutes, and then reaction again for 15 minutes. A chloroacetyl group was introduced in such a manner that after the Fmoc group of the α-amino group was removed by the method described above from the solid phase resin holding the Fmoc-protected peptide obtained in the previous step, a DMF solution of 5 Eq of ClAcOSu was added, followed by shaking at room temperature for 60 minutes. For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diethyl ether/hexane (1/1) cooled to 0°C, a white cloudy precipitate was formed. The mixture was subjected to centrifugation separation (10000 rpm, 1 min), and the solution was subjected to decantation. After the obtained solid was washed again with a small amount of diethyl ether cooled to 0°C, the obtained solid was used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by shaking at room temperature for about 1 hour. The obtained reaction solution was concentrated under reduced pressure using Savant Explorer SpeedVac.

Subsequently, using the obtained solid phase resin, deprotection of the side chain, cleavage from the solid phase resin, and the cyclization reaction were performed according to the general methods described above.

The obtained crude product was purified under the following conditions (column: Waters XBridge (registered trademark) C18 5 µm OBD (registered trademark) 50 x 150 mm; mobile phase: A = 0.1% TFA in H₂O, B = 0.1% TFA in MeCN; temperature: 40°C; gradient (%B): 8-33% over 3 minutes, then 33-38% over 8 minutes, and then 38-60% over 1 minute; flow rate : 120 mL/min).

The purity of the target product was calculated from the area ratio of the LC/MS (UV wavelength: 225 nm) chromatogram under analysis conditions B, and the result was 91.5%. Analysis conditions A: retention time = 3.57 minutes : ESI-MS (+) measured value m/z = theoretical value (M+3H)³⁺ Analysis conditions B: retention time = 12.8 minutes: gradient (%B conc) : 20-60% over 20 minutes, then 60-95% over 1 minute, and then 95% over 5 minutes.

ESI-MS(+) measured value m/z = 899.6 (M+3H)³⁺

### [Example 9-2]

### Synthesis of 894_v05_PEG8Me (SEQ ID NO: 43)

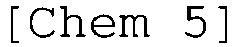

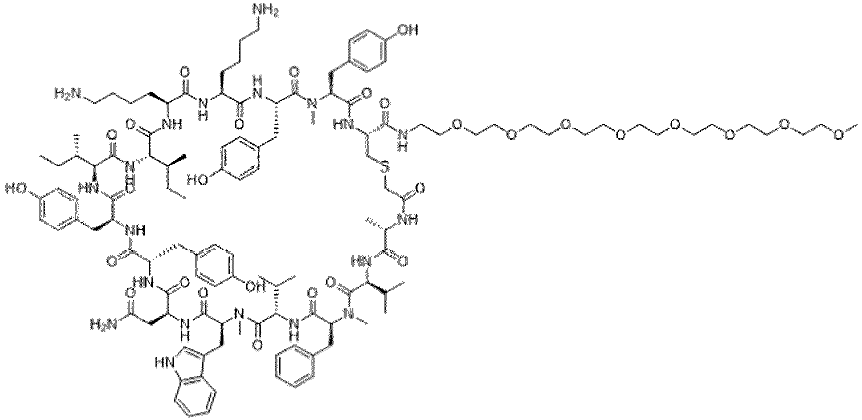

Cl-Trt(2-Cl) resin (1 g, 1.6 mmol, Watanabe Chemical Industries, Ltd., 1.6 mmol/g) was swollen with DCM (dehydrated, 10 mL, Nacalai Tesque, Inc.) for 10 minutes, and after filtration, the filtrate was washed twice with DCM (dehydrated, 10 mL). A DCM (dehydrated, 10 mL) solution of Fmoc-CyS (CH₂COOH)-PEG8Me (767 mg, 1 mmol) and DIEA (836 uL, 4.8 mmol, Watanabe Chemical Industries, Ltd.) were sequentially added to the swollen resin, followed by shaking at room temperature for 35 minutes. Further, after MeOH (1 mL, Kishida Chemical Co., Ltd.) was added, followed by shaking for 10 minutes, the resin was washed three times with DMF (10 mL, Kishida Chemical Co., Ltd.), three times with DCM (10 mL, Kishida Chemical Co., Ltd.), and three times with diethyl ether (10 mL, Kishida Chemical Co., Ltd.), and then dried under reduced pressure, thereby obtaining
Fmoc-CyS[CH₂COO-Trt(2-Cl)-resin]-PEG8Me (1.686 g, 94%). Starting from removal of Fmoc groups using the obtained resin by the general method described above, the target peptide was synthesized. For the synthesis, Biotage Syro I was used as a solid phase synthesizer, and the synthesis was performed according to the manufacturer's manual. For the introduction of each residue, using Fmoc-AA-OH/HATU/DIEA (3.2 Eq/3 Eq/6.3 Eq) per Eq of the resin, the reaction was repeated twice in DMF at room temperature for 30 minutes. However, for the introduction of the 1st, 2nd, and 4th residues, the reaction was performed three times at room temperature for 60 minutes. For the introduction of the 3rd residue, the reaction was repeated twice at 25°C for 60 minutes. For the introduction of the 9th residue, the reaction was performed three times at room temperature for 30 minutes. Further, Fmoc removal was performed by reaction with a DMF solution of 20% piperidine at room temperature for 5 minutes, and then reaction again for 15 minutes. A chloroacetyl group was introduced in such a manner that after the Fmoc group of the α-amino group was removed by the method described above from the solid phase resin holding the Fmoc-protected peptide obtained in the previous step, a DMF solution (0.45 M) of 3 Eq of chloroacetic acid, a DMF solution (0.43 M) of 3 Eq of HCTU, and a DMF solution (1.57 M) of 3 Eq of DIPEA were added to the solid phase resin, followed by shaking at room temperature for 30 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-HFIP/DCM (1/4) was added to the reaction vessel containing the solid phase resin, followed by shaking at room temperature for 90 minutes. The reaction liquid was collected by frit filtration. The solid phase resin remaining in the reaction vessel was shaken with a cleavage cocktail, and the solution component was collected by frit and mixed with the above filtrate; this operation was repeated three times. When the filtrate was concentrated under reduced pressure using Genevac EZ-2 Elite and then added to excess diisopropyl ether cooled to 0°C, a white cloudy precipitate was formed. The mixture was subjected to centrifugation separation (9000 rpm, 5 min), and the supernatant was subjected to decantation. The obtained solid was washed again with a small amount of diethyl ether cooled to 0°C, and then dried under reduced pressure. The obtained solid was used in the subsequent cyclization reaction. In the peptide cyclization reaction, after the solid was dissolved in DMF so that the peptide final concentration was 2.5 mM based on the number of moles of the solid phase resin, a DMF solution (0.43 M) of 1.1 Eq of HATU and 1.5 Eq of DIPEA were added, followed by shaking at room temperature for about 1 hour. 3 Eq of acetic acid was added to the obtained reaction solution at room temperature, followed by concentration under reduced pressure using Genevac EZ-2. Ice-cooled diisopropyl ether was added to the obtained residue, and a white precipitate was formed. The mixture was subjected to centrifugation separation (9000 rpm, 10 minutes), and the supernatant was subjected to decantation. The obtained solid was washed again with diethyl ether cooled to 0°C, and dried under reduced pressure. A reagent cocktail-A (a mixture of TFA/H₂O/TIS/DODT at a volume ratio of 92.5:2.5:2.5:2.5) was added to the obtained solid, followed by shaking at room temperature for 60 minutes. Excess ice-cooled diisopropyl ether was added to produce a precipitate. The mixture was subjected to centrifugation separation (9000 rpm, 5 min), and the supernatant was subjected to decantation. The obtained solid was washed again with ice-cooled diethyl ether and then dried under reduced pressure.

The obtained crude product was purified under the following conditions (column: Waters XBridge (registered trademark) C18 5 µm (registered trademark) 30 x 150 mm; mobile phase: A = 0.1% TFA in H₂O, B = 0.1% TFA in MeCN; temperature: 40°C; gradient (%B): 8-33% over 3 minutes, then 33-38% over 8 minutes, and then 38-60% over 1 minute; flow rate: 45 mL/min) .

The purity of the target product was calculated from the area ratio of the LC/MS (UV wavelength: 225 nm) chromatogram under analysis conditions B, and the result was 97.4%. Analysis conditions B: retention time = 11.93 minutes; gradient (%B conc) : 20-60% over 20 minutes, then 60-95% over 1 minute, and then 95% over 5 minutes
ESI-MS (+) measured value m/z = 1211.6 (M+2H)²⁺

### [Example 9-3]

### Synthesis of hTfR_894_E1_PEG12_(Hydrazide) (SEQ ID NO: 218)

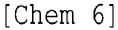

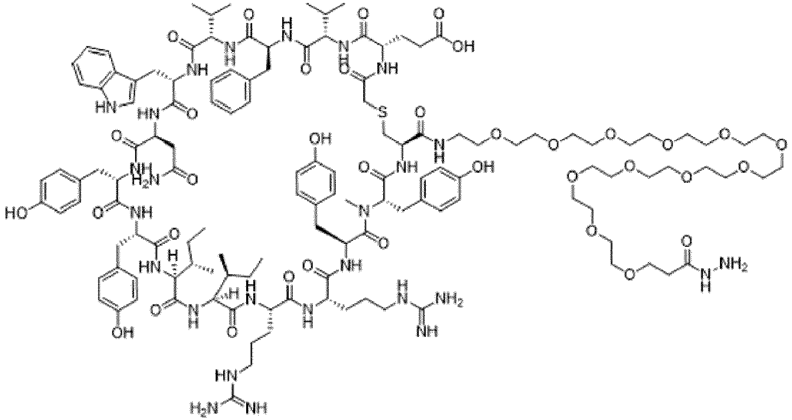

Starting from removal of Fmoc using NH2NH-Trt(2-Cl)-resin (Watanabe Chemical Industries, Ltd., 0.78 mmol/g, 0.13 g) according to the general method described above, the target peptide was synthesized. For the synthesis, CEM's Liberty Blue HT was used as a solid phase synthesizer, and the synthesis was performed according to the manufacturer's manual. The basic conditions for the introduction of each residue were such that using Fmoc-AA-OH/HATU/DIEA (5.3 Eq/5 Eq/10 Eq) per Eq of the resin, the condensation reaction was performed at 75°C for 10 minutes. For the introduction of the 11th and 12th residue amino acids, the condensation reaction was performed twice at 25°C for 30 minutes. For the 13th and 14th residues, the reaction was performed twice at 75°C for 10 minutes. For the 15th residue, the reaction was performed once at 25°C for 30 minutes. For the 16th residue, using Fmoc-AA-OH/HATU/DIEA (3 Eq/3 Eq/6 Eq), the reaction was performed once at 25°C for 120 minutes. The basic conditions for Fmoc removal were such that the reaction with 20% piperidine in DMF was performed at 75°C for 3 minutes. However, the Fmoc groups of the 13th and 15th residues were removed by reaction at 25°C for 5 minutes, and then reaction for 10 minutes. A chloroacetyl group was introduced in such a manner that after the Fmoc group of the α-amino group was removed by the method described above from the solid phase resin holding the Fmoc-protected peptide obtained in the previous step, 10 Eq of chloroacetic acid, 10 Eq of DIPCI, and 10 Eq of HOSu were stirred in DCM, and the same amount of NMP as DCM was added to prepare a DCM/NMP solution (0.2 M) of ClAcOSu, which was then added to the solid phase resin, followed by shaking at room temperature for 60 minutes. For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail (a mixture of TFA/H₂O/TIS/DODT at a volume ratio of 92.5:2.5:2.5:2.5) was added to the reaction vessel containing the solid phase resin, followed by shaking at room temperature for 60 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diethyl ether/hexane (1/1) cooled to 0°C, a white cloudy precipitate was formed. The mixture was subjected to centrifugation separation (9000 rpm, 2 min), and the solution was subjected to decantation. After the obtained solid was washed again with a small amount of diethyl ether cooled to 0°C, the obtained solid was dried and used in the subsequent cyclization reaction. In the peptide cyclization reaction, after the solid was dissolved in DMSO so that the peptide final concentration was 5 mM based on the number of moles of the solid phase resin, 5 Eq of triethylamine was added, followed by stirring at room temperature for about 14 hours. After quenching with acetic acid, the reaction solution was concentrated under reduced pressure using Biotage V-10.

The obtained crude product was purified under the following conditions (column: Waters XBridge (registered trademark) C18 5 µm (registered trademark) 30 x 150 mm; mobile phase: A = 0.1% TFA in H₂O, B = 0.1% TFA in MeCN; temperature: 40°C; gradient (%B): 5-29% over 3 minutes, then 29-34% over 8 minutes, and then 34-60% over 1 minute; flow rate: 45 mL/min.

The purity of the target product was calculated from the area ratio of the LC/MS (UV wavelength: 225 nm) chromatogram under analysis conditions B, and the result was 77.0%. Analysis conditions B: retention time = 10.57 minutes; column: Kinetex EVO C18 2.6 µm, 2.1 x 150 mm, 100Å; mobile phase: A = 0.025% TFA in H₂O, B = 0.025% TFA in MeCN; temperature: 40°C; gradient (%B conc): 20-60% over 20 minutes, then 60-95% over 1 minute, and then 95-95% over 5 minutes; flow rate: 0.25 mL/min.
ESI-MS (+) measured value m/z = 1378.48 (M+2H)²⁺

### [Example 9-4]

### Synthesis of 894_3m_G_Azi (SEQ ID NO: 297)

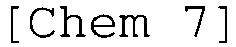

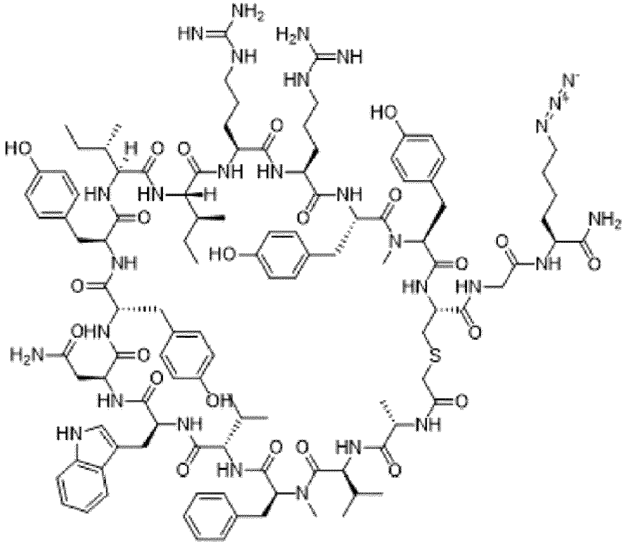

1.1 Eq of H-KN₃-NH₂ (316 mg, 1.52 mmol) synthesized by a known method, 1.2 Eq of HATU (632 mg, 1.66 mmol), and 5 Eq of DIEA (1.21 mL, 6.93 mmol) were added to separately synthesized 894_3m_G (3.3 g, 1.39 mmol), followed by stirring at room temperature for 30 minutes.

The obtained mixture was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 7-7% over 2 minutes, then 7-32% over 1 minute, then 32-37% over 8 minutes, and then 37-60% over 1 minute; flow rate: 20-20 mL/min over 1 minute, then 20-120 mL/min over 1 minute, and then 120 mL/min).

The purity of the target product was calculated from the area ratio of the LC/MS (UV wavelength: 225 nm) chromatogram under analysis conditions B, and the result was 97.1%. Analysis conditions B: retention time = 4.29 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min.
ESI-MS (+) measured value m/z = 1154.72 (M+2H)²⁺

### [Example 9-5]

### Synthesis of 894_variant_39 (SEQ ID NO: 327)

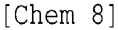

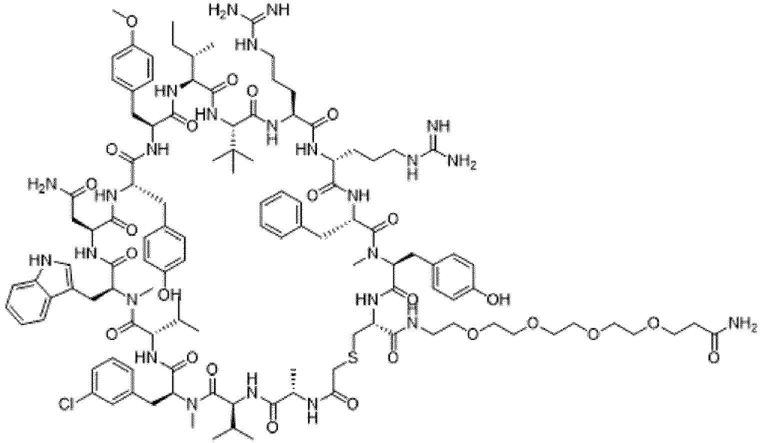

Starting from removal of Fmoc groups using Sieber amide resin (Watanabe Chemical Industries, Ltd., 0.47 mmol/g, 0.21 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA-OH/DIPCI/Oxima pure (5.3 Eq/10 Eq/5 Eq) per Eq of the resin, the reaction was performed once in DMF at 90°C for 3 minutes. However, for the introduction of the 2nd and 4th residues, the reaction was performed twice at 75°C for 30 minutes. For the 9th residue, the reaction was performed twice at 90°C for 10 minutes. For the 11th and 12th residues, the reaction was performed twice at 50°C for 15 minutes. For the 13th residue, the reaction was performed twice at 90°C for 3 minutes. For the 15th residue, the reaction was performed once at 50°C for 15 minutes. Further, the basic conditions for Fmoc removal were such that the reaction with a DMF solution of 20% piperidine was performed at 75°C for 3 minutes. However, the Fmoc groups of the 2nd, 4th, and 13th residues were removed by repeating the reaction twice at 25°C for 5 minutes. A chloroacetyl group was introduced in such a manner that after the Fmoc group of the α-amino group was removed by the method described above from the solid phase resin holding the Fmoc-protected peptide obtained in the previous step, 5 Eq of chloroacetic acid in DMF, 5 Eq of HATU in DMF, and 10 Eq of DIEA in DMF were added to the solid phase resin, followed by shaking at room temperature for 30 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 60 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether/hexane (1/1) cooled to 0°C, a white cloudy precipitate was formed. The mixture was subjected to centrifugation separation (9000 rpm, 1.5 min), and the solution was subjected to decantation. The obtained solid was washed again with a small amount of diethyl ether cooled to 0°C, and then dried under reduced pressure. The obtained solid was used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by shaking at room temperature for about 24 hours. The obtained reaction solution was concentrated under reduced pressure using Savant Explorer SpeedVac.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 11-36% over 3 minutes, then 36-41% over 8 minutes, and then 41-60% over 1 minute; flow rate: 120 mL/min.

The purity of the target product was calculated from the area ratio of the LC/MS (UV wavelength: 225 nm) chromatogram under analysis conditions B, and the result was 93.1%. Analysis conditions B: retention time = 12.76 minutes; column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc): 20-60% over 20 minutes, then 60-95% over 1 minute, and then 95% over 5 minutes; flow rate: 0.25 mL/min.
ESI-MS (+) measured value m/z = 1196.12 (M+2H)²⁺

### [Example 9-6]

### Synthesis of 894_variant_120 (SEQ ID NO: 353)

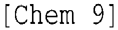

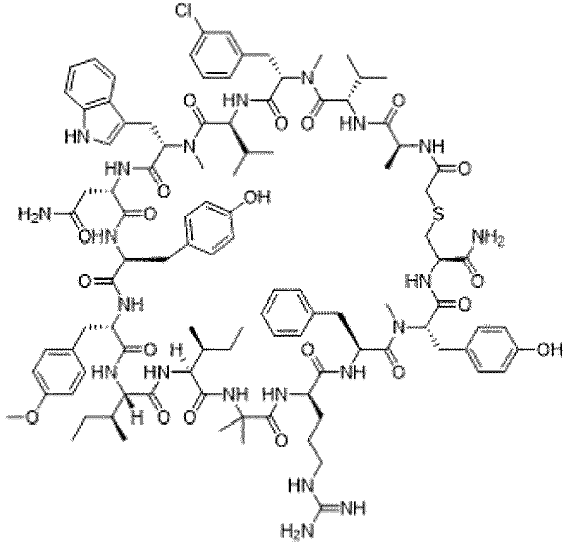

Starting from removal of Fmoc groups using Sieber amide resin (Watanabe Chemical Industries, Ltd., 0.47 mmol/g, 0.43 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA-OH/HATU/DIEA (5 Eq/5 Eq/10 Eq) per Eq of the resin, the reaction was performed once in DMF at 75°C for 10 minutes. However, for the 1st and 3rd residues, the reaction was performed once at 75°C for 20 minutes. For the 2nd and 4th residues, the reaction was performed twice at 75°C for 30 minutes. For the 9th, 10th, and 11th residues, the reaction was performed twice at 75°C for 20 minutes. For the 12th residue, the reaction was performed twice at 25°C for 30 minutes. For the 13th residue, the reaction was performed twice at 75°C for 10 minutes. For the 15th residue, the reaction was performed once at 25°C for 30 minutes. Further, the basic conditions for Fmoc removal were such that the reaction with a DMF solution of 20% piperidine was performed at 75°C for 3 minutes. However, the Fmoc groups of the 2nd, 4th, and 13th residues were removed by repeating the reaction twice at 25°C for 5 minutes. A chloroacetyl group was introduced in such a manner that after the Fmoc group of the α-amino group was removed by the method described above from the solid phase resin holding the Fmoc-protected peptide obtained in the previous step, 5 Eq of ClAcOH in DMF, 5 Eq of HATU in DMF, and 10 Eq of DIEA in DMF were added to the solid phase resin, followed by shaking at 25°C for 30 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 60 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diisopropyl ether/hexane (1/1) cooled to 0°C, a white cloudy precipitate was formed. The mixture was subjected to centrifugation separation (9000 rpm, 2 min), and the solution was subjected to decantation. The obtained solid was washed again with a small amount of diethyl ether cooled to 0°C, and then dried under reduced pressure . The obtained solid was used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by shaking at room temperature for about 16 hours. The obtained reaction solution was concentrated under reduced pressure using Savant Explorer SpeedVac.

The obtained crude product was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 16-41% over 3 minutes, then 41-46% over 8 minutes, and then 46-60% over 1 minute; flow rate: 120 mL/min) .

The purity of the target product was calculated from the area ratio of the LC/MS (UV wavelength: 225 nm) chromatogram under analysis conditions B, and the result was 97.5%. Analysis conditions B: retention time = 6.07 minutes; column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min.
ESI-MS (+) measured value m/z = 1036.90 (M+2H)²⁺

### [Example 9-7]

### Synthesis of 894_variant_61 (SEQ ID NO: 354)

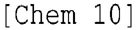

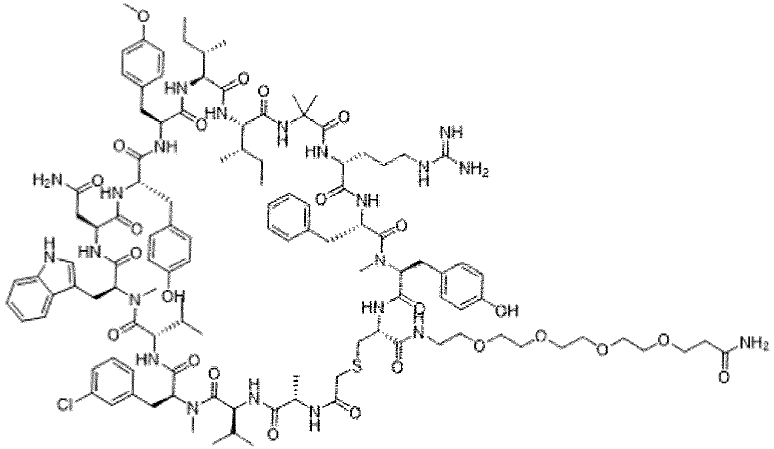

Starting from removal of Fmoc groups using Sieber amide resin (Watanabe Chemical Industries, Ltd., 0.47 mmol/g, 0.21 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA-OH/DIPCI/Oxima pure (5.3 Eq/10 Eq/5 Eq) per Eq of the resin, the reaction was performed once in DMF at 90°C for 3 minutes. However, for the 2nd and 4th residues, the reaction was performed twice at 75°C for 30 minutes. For the 10th and 11th residues, the reaction was performed twice at 90°C for 10 minutes . For the 12th residue, the reaction was performed twice at 50°C for 15 minutes. For the 13th residue, the reaction was performed twice at 90°C for 3 minutes. For the 15th residue, the reaction was performed once at 50°C for 15 minutes. Further, the basic conditions for Fmoc removal were such that the reaction with a DMF solution of 20% piperidine was performed at 75°C for 3 minutes. However, the Fmoc groups of the 2nd, 4th, and 13th residues were removed by repeating the reaction twice at 25°C for 5 minutes. A chloroacetyl group was introduced in such a manner that after the Fmoc group of the α-amino group was removed by the method described above from the solid phase resin holding the Fmoc-protected peptide obtained in the previous step, 10 Eq of chloroacetic acid, 5 Eq of DIPCI, and 5 Eq of HOSu were stirred in DCM, and the same amount of DMF as DCM was added to prepare a DCM/DMF solution (0.2 M) of ClAcOSu, which was then added to the solid phase resin, followed by shaking at room temperature for 60 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether/hexane (1/1) cooled to 0°C, a white cloudy precipitate was formed. The mixture was subjected to centrifugation separation (9000 rpm, 2 min), and the solution was subjected to decantation. The obtained solid was washed again with a small amount of diethyl ether cooled to 0°C, and then dried under reduced pressure. The obtained solid was used in the subsequent cyclization reaction. In the peptide cyclization reaction, after the solid was dissolved in DMSO so that the peptide final concentration was 5 mM based on the number of moles of the solid phase resin, 5 Eq of triethylamine was added, followed by shaking at room temperature for about 14 hours. The obtained reaction solution was concentrated under reduced pressure using Savant Explorer SpeedVac.

The obtained crude product was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 15-40% over 3 minutes, then 40-45% over 8 minutes, and then 45-60% over 1 minute; flow rate: 120 mL/min) .

The purity of the target product was calculated from the area ratio of the LC/MS (UV wavelength: 225 nm) chromatogram under analysis conditions B, and the result was 87.5%. Analysis conditions B: retention time = 16.50 minutes; column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc): 20-60% over 20 minutes, then 60-95% over 1 minute, and then 95% over 5 minutes; flow rate: 0.25 mL/min
ESI-MS (+) measured value m/z = 1160.58 (M+2H)²⁺

### [Example 9-8]

### Synthesis of 894_variant_14 (SEQ ID NO: 387)

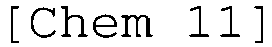

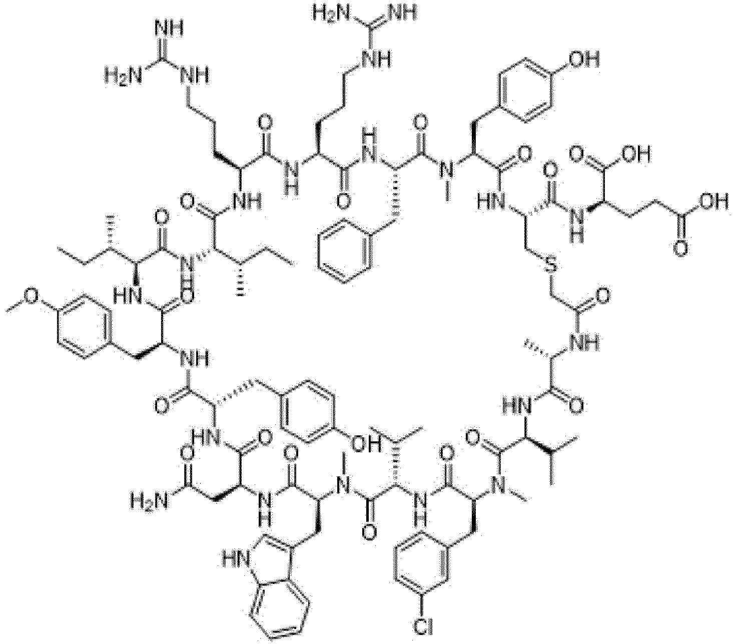

Starting from removal of Fmoc using Fmoc-D-Glu(OtBu)-wang resin (Watanabe Chemical Industries, Ltd., 0.68 mmol/g, 184 mg) according to a general method, the target peptide was synthesized. For the synthesis, CEM's Liberty Blue HT was used as a solid phase synthesizer, and the synthesis was performed according to the manufacturer's manual. The basic conditions for the condensation reaction were such that using HATU as a condensing agent, the reaction was performed twice at 75°C for 10 minutes. However, for the 2nd residue, the reaction was performed twice at 75°C for 30 minutes . For the 4th residue, the reaction was performed twice at 75°C for 30 minutes. For the 6th residue, the reaction was performed once at 75°C for 10 minutes. For the 7th residue, the reaction was performed once at 75°C for 10 minutes. For the 8th residue, the reaction was performed once at 75°C for 10 minutes. For the 10th residue, the reaction was performed once at 75°C for 10 minutes. For the 11th residue, the reaction was performed twice at 30°C for 30 minutes. For the 12th residue, the reaction was performed once at 30°C for 30 minutes . For the 15th residue, the reaction was performed once at 30°C for 30 minutes. Further, the basic conditions for Fmoc removal were such that the reaction with a DMF solution of 20% piperidine was performed at 75°C for 3 minutes. However, the Fmoc groups of the 2nd, 4th, and 13th residues were removed by performing the reaction twice at room temperature for 5 minutes. A chloroacetyl group was introduced in such a manner that chloroacetic acid (5 Eq), DIPCI (5 Eq), and HOSu (5 Eq) were stirred in DCM, and the same amount of DMF as DCM was added to prepare a DCM/DMF solution of ClAcOSu, which was then added to the solid phase resin obtained in the previous step, followed by shaking at room temperature for 60 minutes. For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diethyl ether/hexane (3/1) cooled to 0°C, a white cloudy precipitate was formed. The mixture was subjected to centrifugation separation (9500 rpm, 1 min), the supernatant was subjected to decantation, followed by washing with diethyl ether cooled to 0°C, and the obtained solid was used in the subsequent cyclization reaction. In the peptide cyclization reaction, after the solid was dissolved in DMSO so that the peptide final concentration was 4 mM based on the number of moles of the solid phase resin, 10 Eq of triethylamine was added, followed by shaking at room temperature for about 16 hours. The obtained reaction solution was concentrated under reduced pressure using Genevac EZ-2 Elite.

The obtained reaction liquid was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 11-36% over 3 minutes, then 36-41% over 8 minutes, and then 41-60% over 1 minute; flow rate: 120 mL/min) .

The purity of the target product was calculated from the area ratio of the LC/MS (UV wavelength: 225 nm) chromatogram under analysis conditions B, and the result was 88.60%. Analysis conditions B: retention time = 13.03 minutes; gradient (%B conc) : 20-60% over 20 minutes, then 60-95% over 1 minute, and then 95% over 5 minutes.
ESI-MS (+) measured value m/z = 1137.23 (M+2H)²⁺

### [Example 9-9]

### Synthesis of 894_variant84 (SEQ ID NO: 390)

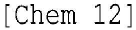

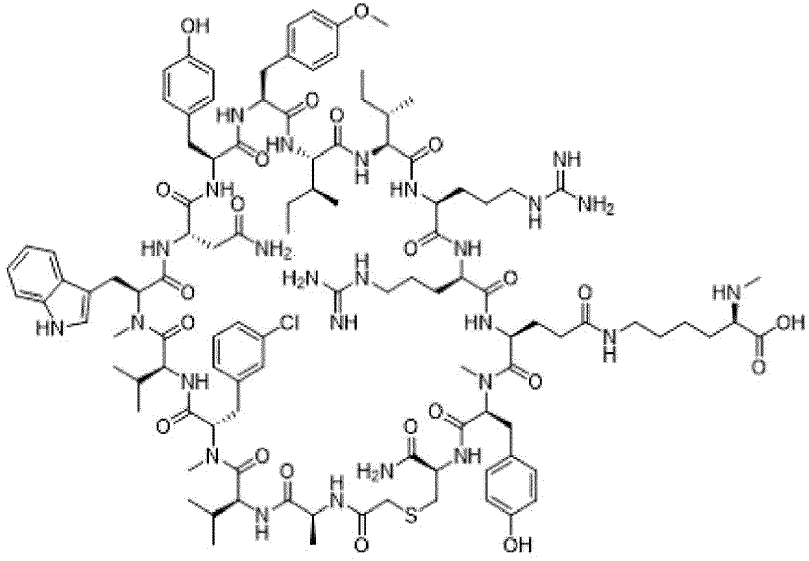

Starting from removal of Fmoc groups using Sieber amide resin (Watanabe Chemical Industries, Ltd., 0.47 mmol/g, 0.21 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA/DIPCI/Oxyma pure = 5.3 Eq/10 Eq/5 Eq per Eq of the resin, the reaction was performed once in DMF at 90°C for 3 minutes. However, for the 2nd and 4th residues, the reaction was performed twice at 75°C for 45 minutes. For the 11th and 12th residues, the reaction was repeated twice at 50°C for 15 minutes. For the introduction of the 13th residue, the reaction was performed twice at 90°C for 3 minutes. Further, the basic conditions for Fmoc removal were such that the reaction with a DMF solution of 20% piperidine was performed once at 75°C for 3 minutes. However, the Fmoc groups of the 2nd, 4th, and 13th residues were removed by repeating the reaction twice at 25°C for 5 minutes. After extension to the 1st residue, the resin was suspended in DCM, and Pd(PPh₃)_{4/}PhSiH₃ (0.2 Eq/10 Eq) were added, followed by shaking at room temperature for 1 hour. A chloroacetyl group was introduced in such a manner that after the solid phase resin holding the Fmoc-protected peptide obtained in the previous step was washed three times with DMF and three times with DCM, the Fmoc group of the α-amino group was removed by the method described above, and then 5 Eq of chloroacetic acid in DMF, 5 Eq of HATU in DMF, and 10 Eq of DIEA in DMF were added, followed by shaking at room temperature for 30 minutes . For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether/hexane (1/1) cooled to 0°C, a white cloudy precipitate was formed. The mixture was subjected to centrifugation separation (10000 rpm, 1 min), and the solution was subjected to decantation. The obtained solid was washed again with a small amount of diethyl ether cooled to 0°C, and then dried under reduced pressure . The obtained solid was used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by shaking at room temperature overnight. The reaction solution was concentrated under reduced pressure using Savant Explorer SpeedVac. The obtained mixture was dissolved in DMSO (4 mL), and HOSu (10 Eq) and EDC HCl (10 Eq) were added, followed by stirring at room temperature for 2 hours.

The obtained crude product was purified under the following conditions (column: Waters XBridge (registered trademark) C18 5 µm (registered trademark) 30 x 150 mm; mobile phase: A = 0.1% TFA in H₂O, B = 0.1% TFA in MeCN; temperature: 40°C; gradient (%B): 11-36% over 3 minutes, then 36-41% over 8 minutes, and then 41-60% over 1 minute; flow rate: 45 mL/min) .

The obtained cyclic peptide-NHS ester (25 mg, 11.3 µmol) was dissolved in DMF (225 µL), and Fmoc-MeK-OH hydrochloride (5 mg, 11.9 µmol) and DIEA (5.9 µL, 33.9 µmol) were added, followed by stirring. After 1 hour, Et₂NH (5.9 µL, 56.5 µmol) was added to the reaction liquid, followed by stirring. After 1 hour, the reaction was quenched with acetic acid.

The obtained crude product was purified under the following conditions (column: Waters XBridge (registered trademark) C18 5 µm (registered trademark) 19 x 150 mm; mobile phase: A = 0.1% TFA in H₂O, B = 0.1% TFA in MeCN; temperature: 60°C; gradient (%B): 8-33% over 3 minutes, then 33-38% over 8 minutes, and then 38-60% over 1 minute; flow rate: 17 mL/min.

The purity of the target product was calculated from the area ratio of the LC/MS (UV wavelength: 225 nm) chromatogram under analysis conditions B, and the result was 97.9%. Analysis conditions B: retention time = 12.33 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc): 20-60% over 20 minutes, then 60-95% over 1 minute, and then 95% over 5 minutes
ESI-MS (+) measured value m/z = 1134.34 (M+2H)²⁺

### [Example 9-10]

### Synthesis of 894_variant89 (SEQ ID NO: 391)

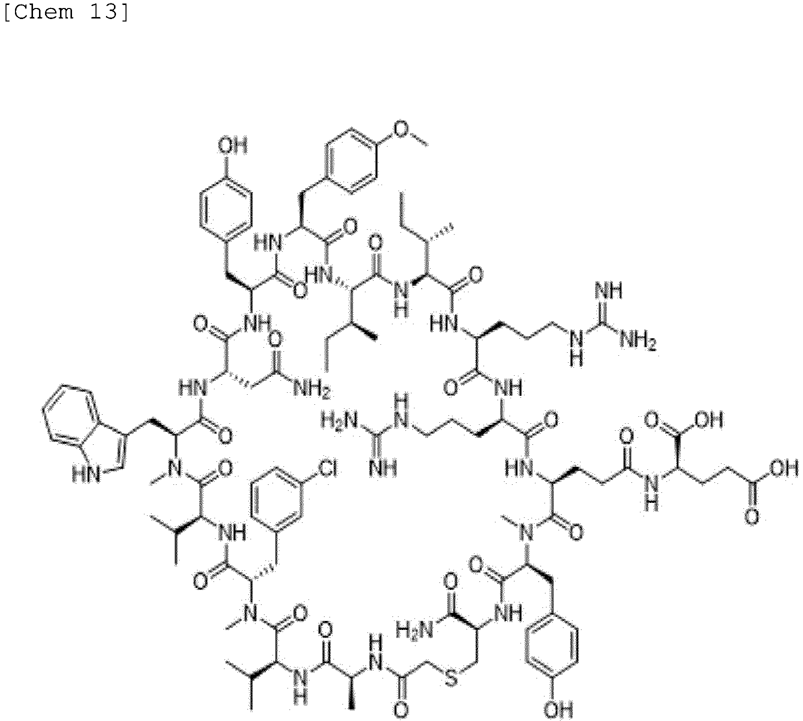

Starting from removal of Fmoc groups using Sieber amide resin (Watanabe Chemical Industries, Ltd., 0.47 mmol/g, 0.21 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA/DIPCI/Oxyma pure/DIEA (5.3 Eq/10 Eq/5 Eq) per Eq of the resin, the reaction was performed once in DMF at 90°C for 3 minutes. However, for the introduction of the 2nd and 4th residues, the reaction was performed twice at 75°C for 45 minutes. For the introduction of the 11th and 12th residues, the reaction was repeated twice at 50°C for 15 minutes. For the introduction of the 13th residue, the reaction was performed twice at 90°C for 3 minutes . Further, the basic conditions for Fmoc removal were such that the reaction with a DMF solution of 20% piperidine was performed once at 75°C for 3 minutes. However, the Fmoc groups of the 2nd, 4th, and 13th residues were removed by repeating the reaction twice at 25°C for 5 minutes. After extension to the 1st residue, the resin was suspended in DCM, and Pd(PPh₃)₄/PhSiH₃ (0.2 Eq/10 Eq) were added, followed by shaking at room temperature for 1 hour. The solid phase resin was washed three times with DMF and three times with DCM, and then dried under reduced pressure. The resin (60 µmol) was suspended in DMF, H-D-Glu(OtBu)-OtBu hydrochloride (71 mg, 0.24 mmol), 0.5 M Oxyma pure in DMF (0.48 mL, 0.24 mmol), DIPCI (37 µL, 0.24 mmol), and DIEA (42 µL, 0.24 mmol) were added and reacted twice under microwave irradiation at 75°C for 30 minutes. A chloroacetyl group was introduced in such a manner that after the Fmoc group of the α-amino group was removed by the method described above from the solid phase resin holding the Fmoc-protected peptide obtained in the previous step, 5 Eq of chloroacetic acid in DMF, 5 Eq of HATU in DMF, and 10 Eq of DIEA in DMF were added, followed by shaking at room temperature for 30 minutes . For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether/hexane (1/1) cooled to 0°C, a white cloudy precipitate was formed. The mixture was subjected to centrifugation separation (10000 rpm, 1 min), and the solution was subjected to decantation. The obtained solid was washed again with a small amount of diethyl ether cooled to 0°C, and then dried under reduced pressure . The obtained solid was used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by shaking at room temperature overnight. The reaction solution was concentrated under reduced pressure using Savant Explorer SpeedVac.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 8-33% over 3 minutes, then 33-38% over 8 minutes, and then 38-60% over 1 minute; flow rate: 120 mL/min) .

The purity of the target product was calculated from the area ratio of the LC/MS (UV wavelength: 225 nm) chromatogram under analysis conditions B, and the result was 94.9%. Analysis conditions B: retention time = 11.81 minutes; column: Kinetex EVO C18 2.6 µm 2.1 x 150 mm, 100Å; mobile phase: A = 0.025% TFA in H₂O, B = 0.025% TFA in MeCN; temperature: 40°C; gradient (%B conc): 20-60% over 20 minutes, then 60-95% over 1 minute, and then 95% over 5 minutes; flow rate: 0.25 mL/min.
ESI-MS (+) measured value m/z = 1127.76 (M+2H)²⁺

### [Example 9-11]

### Synthesis of 894_variant_03 (SEQ ID NO: 397)

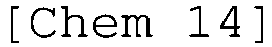

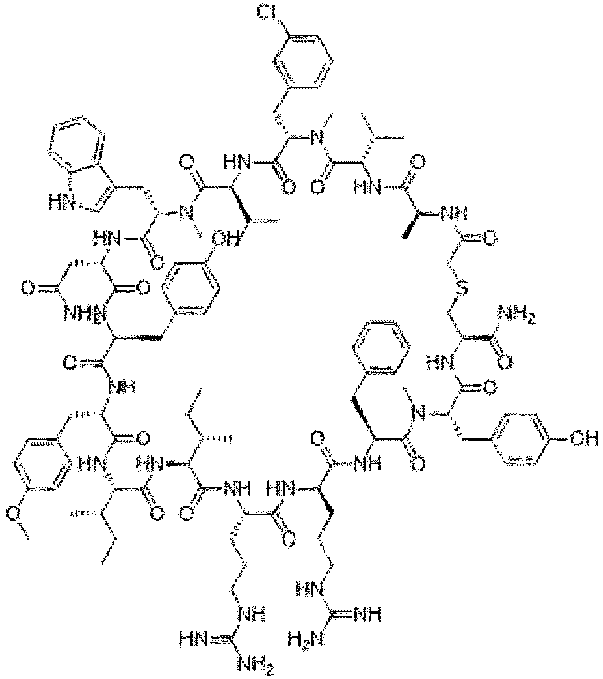

Starting from removal of Fmoc groups using Sieber amide resin (Watanabe Chemical Industries, Ltd., 0.47 mmol/g, 0.21 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA/DIPCI/Oxima pure (4.2 Eq/4 Eq/8 Eq) per Eq of the resin, the reaction was performed once in DMF at 75°C for 10 minutes. However, for the 2nd and 4th residues, the reaction was performed twice at 75°C for 30 minutes. For the 11th and 12th residues, the reaction was performed twice at 90°C for 10 minutes. For the 13th residue, the reaction was performed twice at 75°C for 10 minutes. For the 15th residue, the reaction was performed once at 25°C for 20 minutes . Further, the basic conditions for Fmoc removal were such that the reaction with a DMF solution of 20% piperidine was performed at 75°C for 3 minutes. However, the Fmoc groups of the 2nd, 4th, and 13th residues were removed by reaction at 25°C for 5 minutes, and then reaction at 25°C for 10 minutes. A chloroacetyl group was introduced in such a manner that after the Fmoc group of the α-amino group was removed by the method described above from the solid phase resin holding the Fmoc-protected peptide obtained in the previous step, chloroacetic acid (10 Eq), 10 Eq of DIPCI, and 10 Eq of HOSu were stirred in DCM, and the same amount of DMF as DCM was added to prepare a DCM/DMF solution (0.2 M) of ClAcOSu, which was then added to the solid phase resin, followed by shaking at room temperature for 60 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether/hexane (1/1) cooled to 0°C, a white cloudy precipitate was formed. The mixture was subjected to centrifugation separation (9000 rpm, 2 min), and the solution was subjected to decantation. The obtained solid was washed again with a small amount of diethyl ether cooled to 0°C, and then dried under reduced pressure. The obtained solid was used in the subsequent cyclization reaction. In the peptide cyclization reaction, after the solid was dissolved in a 50% aqueous MeCN solution so that the peptide final concentration was 5 mM based on the number of moles of the solid phase resin, 5 Eq of triethylamine was added, followed by shaking at room temperature for about 14 hours. The obtained reaction solution was concentrated under reduced pressure using Savant Explorer SpeedVac.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 10-35% over 3 minutes, then 35-40% over 8 minutes, and then 40-60% over 1 minute; flow rate: 120 mL /min.

The purity of the target product was calculated from the area ratio of the LC/MS (UV wavelength: 225 nm) chromatogram under analysis conditions B, and the result was 98.2%. Analysis conditions B: retention time = 13.18 minutes; column: Kinetex EVO C18 2.6 µm 2.1 x 150 mm, 100Å; mobile phase: A = 0.025% TFA in H₂O, B = 0.025% TFA in MeCN; temperature: 40°C; gradient (%B conc): 20-60% over 20 minutes, then 60-95% over 1 minute, and then 95-95% over 5 minutes; flow rate: 0.25mL/min.
ESI-MS (+) measured value m/z = 1072.49 (M+2H)²⁺

### [Example 9-12]

### Synthesis of 894_variant_31 (SEQ ID NO: 400)

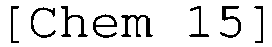

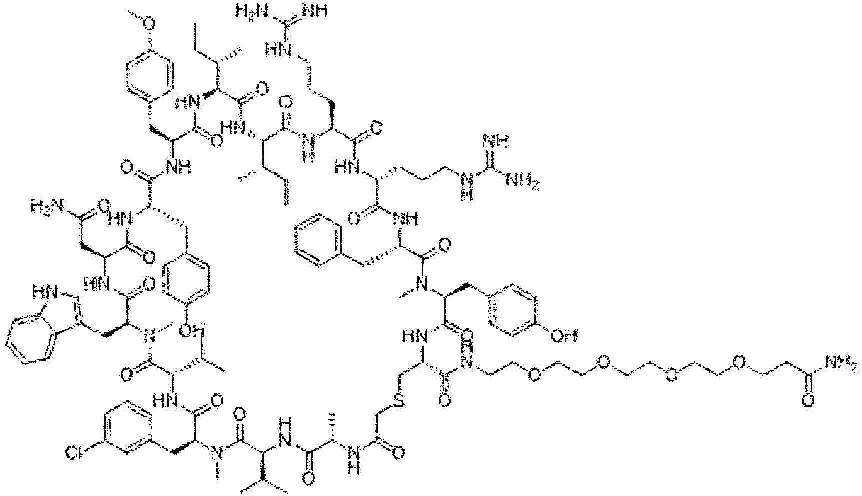

Starting from removal of Fmoc groups using Sieber amide resin (Watanabe Chemical Industries, Ltd., 0.47 mmol/g, 0.21 g) by the general method described above, the target peptide was synthesized. For the synthesis, CEM's Liberty Blue was used as a solid phase synthesizer, and the synthesis was performed according to the manufacturer's manual. For the introduction of each residue, using Fmoc-AA/ DIPCI/ Oxima pure (4.2 Eq/4 Eq/8 Eq) per Eq of the resin, the reaction was repeated twice in DMF at 90°C for 3 minutes. However, for the introduction of the 2nd and 4th residues, the reaction was performed twice at 75°C for 45 minutes. For the 3rd residue, the reaction was performed twice at 75°C for 30 minutes. For the 7th and 8th residues, the reaction was performed once at 90°C for 3 minutes. For the introduction of the 11th and 12th residues, the reaction was performed twice at 50°C for 15 minutes. For the 15th and 16th residues, the reaction was performed once at 50°C for 15 minutes . Further, the basic conditions for Fmoc removal were such that the reaction with a DMF solution of 20% piperidine was performed at 75°C for 3 minutes. However, the Fmoc groups of the 2nd, 4th, and 13th residues were removed by repeating the reaction twice at 25°C for 5 minutes. A chloroacetyl group was introduced in such a manner that after the Fmoc group of the α-amino group was removed by the method described above from the solid phase resin holding the Fmoc-protected peptide obtained in the previous step, chloroacetic acid (5 Eq), 5 Eq of DIPCI, and 5 Eq of HOSu were stirred in DCM, and the same amount of DMF as DCM was added to prepare a DCM/DMF solution (0.2 M) of ClAcOSu, which was then added to the solid phase resin, followed by shaking at room temperature for 60 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether cooled to 0°C, a white cloudy precipitate was formed. The mixture was subjected to centrifugation separation (9500 rpm, 1 min), and the solution was subjected to decantation. The obtained solid was washed again with a small amount of diethyl ether cooled to 0°C, and then dried under reduced pressure. The obtained solid was used in the subsequent cyclization reaction. In the peptide cyclization reaction, after the solid was dissolved in DMSO so that the peptide final concentration was 4 mM based on the number of moles of the solid phase resin, 7 Eq of triethylamine was added, followed by shaking at room temperature for about 12 hours. The obtained reaction solution was concentrated under reduced pressure using Genevac EZ-2 Elite.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 15-40% over 3 minutes, then 40-45% over 8 minutes, and then 45-60% over 1 minute; flow rate: 120 mL/min.

The purity of the target product was calculated from the area ratio of the LC/MS (UV wavelength: 225 nm) chromatogram under analysis conditions B, and the result was 95.4%. Analysis conditions B: retention time = 12.64 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc): 20-60% over 20 minutes, then 60-95% over 1 minute, and then 95% over 5 minutes; flow rate: 0.25 mL/min
ESI-MS (+) measured value m/z = 1195.87 (M+2H)²⁺

### [Example 9-13]

### Synthesis of 894_variant_03_G4S2C (SEQ ID NO: 401)

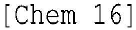

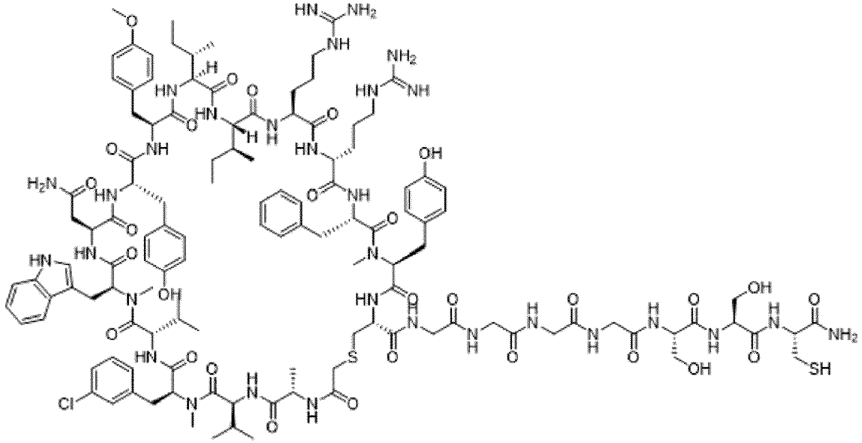

Starting from removal of Fmoc groups using Sieber amide resin (Watanabe Chemical Industries, Ltd., 0.73 mmol/g, 1.37 g) by the general method described above, the target peptide was synthesized. For the synthesis, CEM's Liberty Blue was used as a solid phase synthesizer, and the synthesis was performed according to the manufacturer's manual. For the introduction of each residue, using Fmoc-AA/HATU/DIEA (4.2 Eq/4 Eq/8 Eq) per Eq of the resin, the reaction was performed once in DMF at 75°C for 10 minutes. However, for the 2nd and 4th residues, the reaction was performed twice at 75°C for 30 minutes. For the 11th and 12th residues, the reaction was repeated twice at 25°C for 20 minutes. For the 13th residue, the reaction was performed twice at 75°C for 10 minutes. For the 15th and 22nd residues, the reaction was performed once at 25°C for 30 minutes . Further, the basic conditions for Fmoc removal were such that the reaction with a DMF solution of 20% piperidine was performed at 75°C for 3 minutes. However, the Fmoc groups of the 2nd, 4th, and 13th residues were removed by reaction at 25°C for 5 minutes, and then reaction at room temperature for 10 minutes. A chloroacetyl group was introduced in such a manner that after the Fmoc group of the α-amino group was removed by the method described above from the solid phase resin holding the Fmoc-protected peptide obtained in the previous step, 10 Eq of chloroacetic acid, 10 Eq of DIPCI, and 10 Eq of HOSu were stirred in DCM, and the same amount of DMF as DCM was added to prepare a DCM/DMF solution (0.2 M) of ClAcOSu, which was then added to the solid phase resin, followed by shaking at room temperature for 60 minutes . For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 90:2.5:2.5:5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diisopropyl ether/hexane (1/1) cooled to 0°C, a white cloudy precipitate was formed. The mixture was subjected to centrifugation separation (9500 rpm, 1 min), and the solution was subjected to decantation, followed by drying under reduced pressure. The obtained solid was used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by shaking at room temperature for about 15 hours. The obtained reaction solution was concentrated under reduced pressure using Genevac HT-12. The obtained mixture was dissolved in DMSO, and 3 Eq of silver acetate was added, followed by shaking for 3 hours. A 2 M DTT aqueous solution (11 Eq) was added, followed by centrifugation to collect the supernatant, and then concentration under reduced pressure, thereby obtaining a crude product.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 11-11% over 3 minutes, then 11-36% over 3 minutes, then 36-41% over 8 minutes, and then 41-60% over 1 minute; flow rate: 120 mL/min).

The purity of the target product was calculated from the area ratio of the LC/MS (UV wavelength: 225 nm) chromatogram under analysis conditions B, and the result was 94.8%. Analysis conditions B: retention time = 4.31 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min
ESI-MS (+) measured value m/z = 1324.94 (M+2H)²⁺

### [Example 9-14]

### Synthesis of 894_A_hgl_CyCloamide (SEQ ID NO: 408)

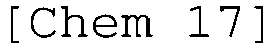

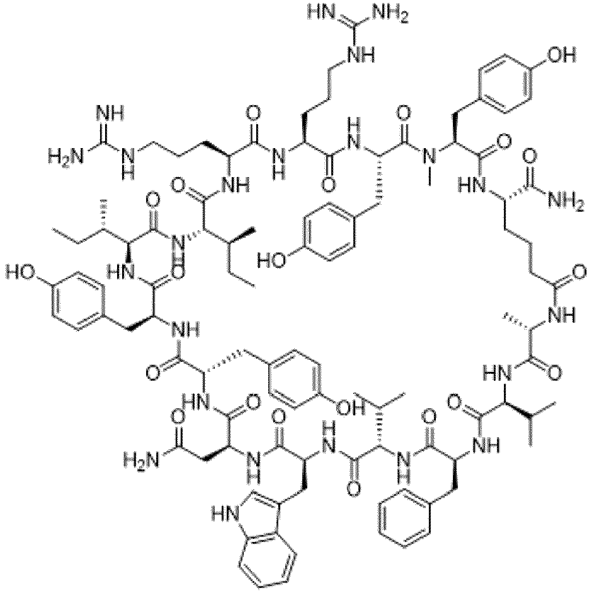

Starting from removal of Fmoc using Sieber amide resin (Watanabe Chemical Industries, Ltd., 0.47 mmol/g) according to a general method, the target peptide was synthesized. For the synthesis, CEM's Liberty Blue HT was used as a solid phase synthesizer, and the synthesis was performed according to the manufacturer's manual. The basic conditions for the condensation reaction were such that using DIPCI and Oxyma pure as condensing agents, the reaction was performed once at 90°C for 3 minutes. However, for the 11th and 12th residues, the reaction was repeated twice at 50°C for 15 minutes. For the 13th residue, the reaction was repeated twice at 90°C for 3 minutes. Further, the basic conditions for Fmoc removal were such that the reaction with a DMF solution of 20% piperidine was performed at 75°C for 3 minutes. However, the Fmoc group at the 13th residue was removed by performing the reaction twice at 25°C for 5 minutes. The obtained resin was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diisopropyl ether and hexane cooled to 0°C, a white cloudy precipitate was formed. The mixture was subjected to centrifugation separation (9000 rpm, 2 min), and the supernatant was subjected to decantation. The obtained solid was washed with ice-cooled diethyl ether and then dried. The obtained solid was used in the subsequent cyclization reaction. In the peptide cyclization reaction, after the solid was dissolved in DMF so that the peptide final concentration was 5 mM based on the number of moles of the solid phase resin, 1.5 Eq of HATU and 3 Eq of triethylamine were added, followed by shaking at room temperature for about 1 hour. The obtained reaction solution was concentrated under reduced pressure using Savant Explorer SpeedVac.

The obtained crude product was purified under the following conditions (column: Waters XBridge (registered trademark) C18 5 µm (registered trademark) 30 x 150 mm; mobile phase: A = 0.1% TFA in H₂O, B = 0.1% TFA in MeCN; temperature: 40°C; gradient (%B): 6-31% over 3 minutes, then 31-36% over 8 minutes, and then 36-60% over 1 minute; flow rate: 45 mL/min) .

The purity of the target product was calculated from the area ratio of the LC/MS (UV wavelength: 225 nm) chromatogram under analysis conditions B, and the result was 96.8%. Analysis conditions B: retention time = 10.03 minutes; gradient (%B conc) : 20-60% over 20 minutes, then 60-95% over 1 minute, and then 95% over 5 minutes
ESI-MS (+) measured value m/z = 1033.0 (M+2H)²⁺

### [Example 9-15]

### Synthesis of 894_BiCyCle 001 (SEQ ID NO: 409)

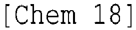

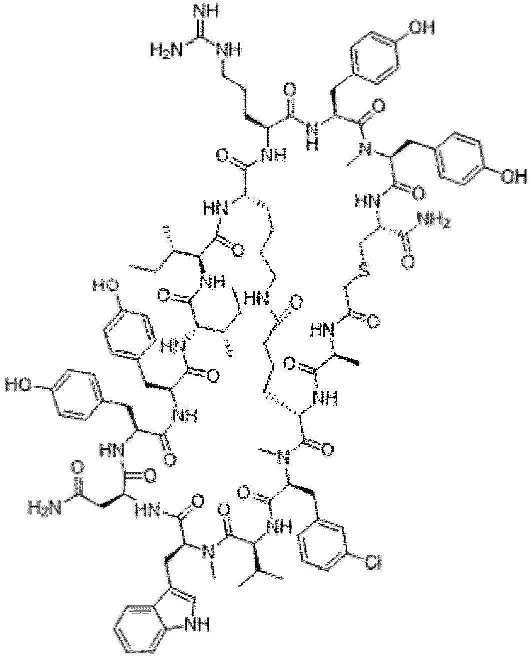

Starting from removal of Fmoc groups using Sieber amide resin (Watanabe Chemical Industries, Ltd., 0.47 mmol/g, 0.53 mg) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA/HATU/DIEA (4.2 Eq/4 Eq/8 Eq) per Eq of the resin, the reaction was performed twice in DMF at 75°C for 10 minutes. However, for the 1st, 6th, and 10th residues, the reaction was performed once at 75°C for 10 minutes. For the 2nd and 3rd residues, the reaction was performed twice at 75°C for 20 minutes. For the 4th residue, the reaction was performed twice at 75°C for 30 minutes. For the 11th and 14th residues, the reaction was performed once at 75°C for 20 minutes. For the 12th and 15th residues, the reaction was performed once at 30°C for 30 minutes. Further, the basic conditions for Fmoc removal were such that the reaction with a DMF solution of 20% piperidine was performed once at 75°C for 3 minutes. However, the Fmoc groups of the 2nd, 4th, and 13th residues were removed by repeating the reaction twice at 25°C for 5 minutes. A chloroacetyl group was introduced in such a manner that after the Fmoc group of the α-amino group was removed by the method described above from the solid phase resin holding the Fmoc-protected peptide obtained in the previous step, 5 Eq of chloroacetic acid, 5 Eq of DIPCI, and 5 Eq of HOSu were stirred in DCM, and the same amount of DMF as DCM was added to prepare a DCM/DMF solution (0.2 M) of ClAcOSu, which was then added to the solid phase resin, followed by shaking at room temperature for 75 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether cooled to 0°C, a white cloudy precipitate was formed. The mixture was subjected to centrifugation separation (9500 rpm, 1 min), and the solution was subjected to decantation. The obtained solid was washed again with a small amount of diethyl ether cooled to 0°C, and then dried under reduced pressure. The obtained solid was used in the subsequent cyclization reaction. In the peptide cyclization reaction, after the solid was dissolved in 5% water-containing DMSO so that the peptide final concentration was 4 mM based on the number of moles of the solid phase resin, 10 Eq of triethylamine was added, followed by shaking at room temperature for about 12 hours. The obtained reaction solution was concentrated under reduced pressure using Genevac EZ-2 Elite. The obtained mixture was dissolved in DMF so that the peptide final concentration was 25 mM based on the number of moles of the solid phase resin, and 1 Eq of HATU and 3 Eq of DIEA were added, followed by shaking at room temperature for about 30 minutes.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 11-36% over 3 minutes, then 36-41% over 8 minutes, and then 41-60% over 1 minute; flow rate: 120 mL/min

The purity of the target product was calculated from the area ratio of the LC/MS (UV wavelength: 225 nm) chromatogram under analysis conditions B, and the result was 94.8%. Analysis conditions B: retention time = 13.78 minutes; column: Kinetex EVO C18 2.6 µm 2.1 x 150 mm, 100Å; mobile phase: A = 0.025% TFA in H₂O, B = 0.025% TFA in MeCN: temperature: 60°C; gradient (%B conc): 20-60% over 20 minutes, then 60-95% over 1 minute, and then 95-95% over 5 minutes; flow rate: 0.25%
ESI-MS (+) measured value m/z = 1072.26 (M+2H)²⁺

### [Example 9-16]

### Synthesis of 894_BiCyCle 006 (SEQ ID NO: 414)

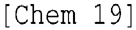

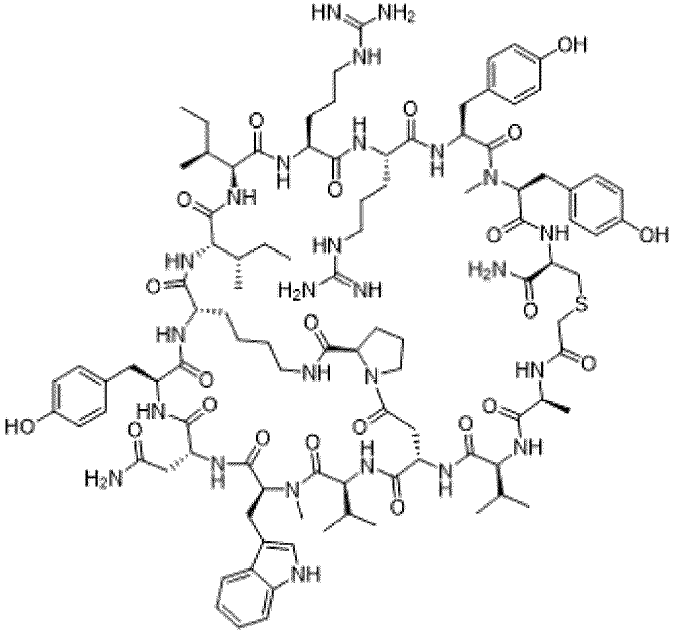

Starting from removal of Fmoc groups using Sieber amide resin (Watanabe Chemical Industries, Ltd., 0.47 mmol/g, 0.32 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA/DIPCI/Oxyma pure/DIEA (5.3 Eq/10 Eq/5 Eq) per Eq of the resin, the reaction was performed once in DMF at 90°C for 3 minutes. However, for the introduction of the 2nd, 4th, 11th, and 13th residues, the reaction was performed twice at 90°C for 3 minutes. For the introduction of the 12th residue, the reaction was performed twice at 50°C for 15 minutes. For the introduction of the 15th residue, the reaction was performed once at 50°C for 15 minutes. Further, the basic conditions for Fmoc removal were such that the reaction with a DMF solution of 20% piperidine was performed once at 75°C for 3 minutes. However, the Fmoc groups of the 2nd, 4th, and 13th residues were removed by repeating the reaction twice at 25°C for 5 minutes. The solid phase resin was washed sequentially with DMF and DCM, dried under reduced pressure, and then swollen with DCM, after which 10 Eq of PhSiH₃ and 0.2 Eq of Pd(PPh₃)₄ were added, followed by shaking at room temperature for 1 hour. The solid phase resin was washed five times with DCM, five times with DMF, and three times with diethyl ether, and dried. The solid phase resin was swollen with NMP, and 5 Eq of HATU and 5 Eq of DIEA were added, followed by shaking at room temperature for 30 minutes . After the resin was washed, 5 Eq of HATU and 5 Eq of DIEA were added again, followed by shaking at room temperature for 30 minutes. The solid phase resin was washed five times with DCM, five times with DMF, and three times with diethyl ether. A chloroacetyl group was introduced in such a manner that after the Fmoc group of the α-amino group was removed by the method described above from the solid phase resin holding the Fmoc-protected peptide obtained in the previous step, 5 Eq of chloroacetic acid, 5 Eq of DIPCI, and 5 Eq of HOSu were stirred in DCM, and the same amount of DMF as DCM was added to prepare a DCM/DMF solution (0.2 M) of ClAcOSu, which was then added to the solid phase resin, followed by shaking at room temperature for 60 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether cooled to 0°C, a white cloudy precipitate was formed. The mixture was subjected to centrifugation separation (9500 rpm, 1 min), and the solution was subjected to decantation. The obtained solid was washed again with a small amount of diethyl ether cooled to 0°C, and then dried under reduced pressure . The obtained solid was used in the subsequent cyclization reaction. In the peptide cyclization reaction, after the solid was dissolved in 5% water-containing DMSO so that the peptide final concentration was 3.3 mM based on the number of moles of the solid phase resin, 7 Eq of triethylamine was added, followed by shaking at room temperature for about 12 hours. The obtained reaction solution was concentrated under reduced pressure using Genevac EZ-2 Elite.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN: temperature: 40°C; gradient (%B): 7-32% over 3 minutes, then 32-37% over 8 minutes, and then 37-60% over 1 minute; flow rate: 120 mL/min), then freeze-dried, and purified again under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 11-36% over 3 minutes, then 36-41% over 8 minutes, and then 41-60% over 1 minute; flow rate: 120 mL/min)

The purity of the target product was calculated from the area ratio of the LC/MS (UV wavelength: 225 nm) chromatogram under analysis conditions B, and the result was 89.0%. Analysis conditions B: retention time = 3.67 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min
ESI-MS (+) measured value m/z = 1055.13 (M+2H)²⁺

### [Example 9-17]

### Synthesis of 894_BiCyCle_012 (SEQ ID NO: 419)

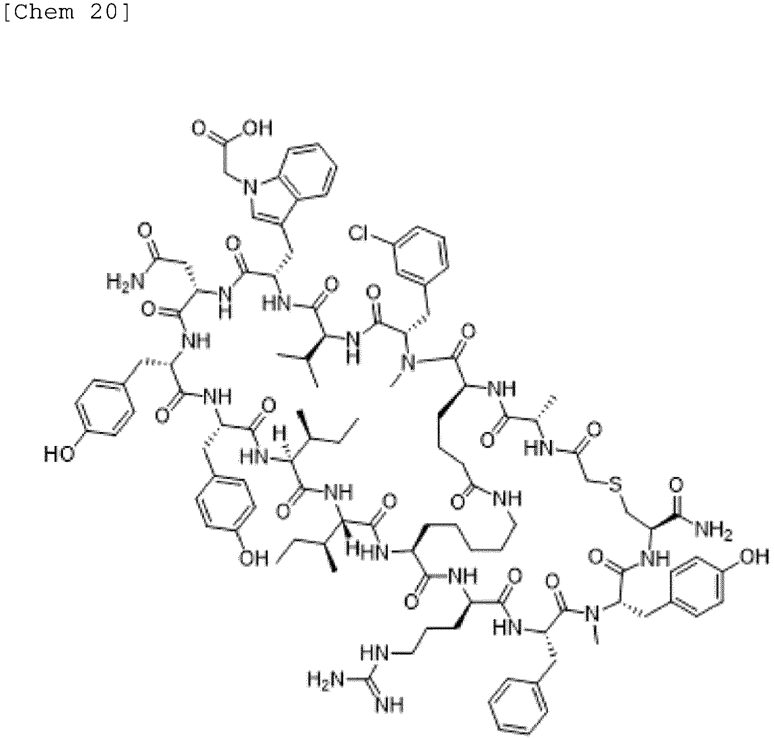

Starting from removal of Fmoc groups using Sieber amide resin (Watanabe Chemical Industries, Ltd., 0.47 mmol/g, 213 mg) by the general method described above, the target peptide was synthesized. For the synthesis, CEM's Liberty Blue was used as a solid phase synthesizer, and the synthesis was performed according to the manufacturer's manual. A crude product was obtained in the same manner with reference to the synthesis of Bicycle_01 and Bicycle_06, except that Fmoc-Wlaa(Allyl)-OH, Fmoc-Hgl (tBu) -OH, and Fmoc-Hly (Boc) -OH were used as amino acid raw materials.

The obtained crude product was purified under the following conditions (column: Waters XBridge (registered trademark) C18 5 µm (registered trademark) 19 x 150 mm; mobile phase: A = 0.1% TFA in H₂O, B = 0.1% TFA in MeCN; temperature: 60°C; gradient (%B): 11-36% over 3 minutes, then 36-41% over 8 minutes, and then 41-60% over 1 minute; flow rate: 17 mL/min

The purity of the target product was calculated from the area ratio of the LC/MS (UV wavelength: 225 nm) chromatogram under analysis conditions B, and the result was 94.8%. Analysis conditions B: retention time = 4.76 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95% over 1.6 minutes
ESI-MS (+) measured value m/z = 1093.23 (M+2H)²⁺

### [Example 9-18]

### Synthesis of 894 3m G (SEQ ID NO: 421)

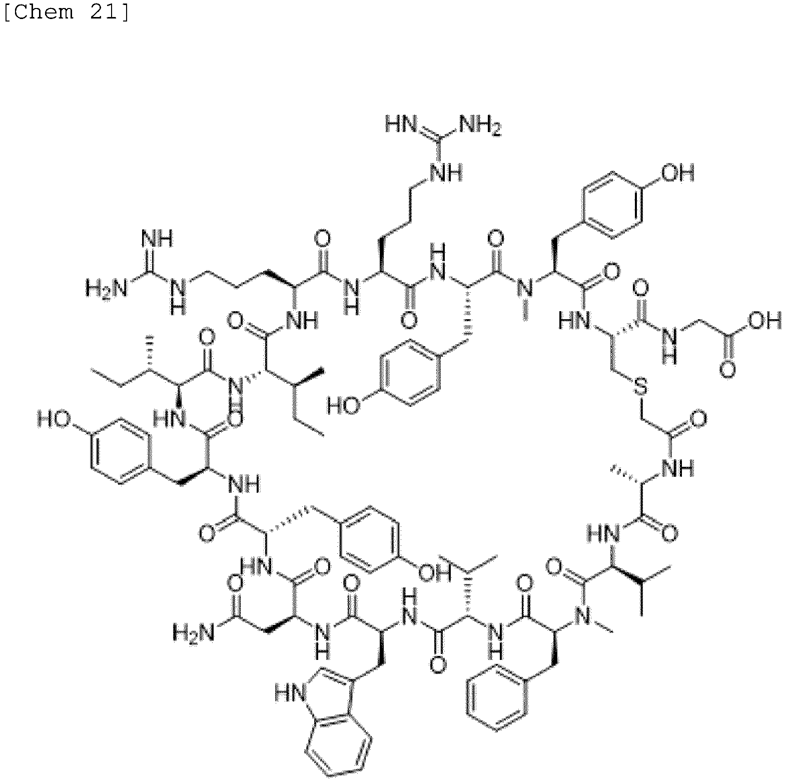

Starting from removal of Fmoc groups using Fmoc-Gly-wang resin (Watanabe Chemical Industries, Ltd., 0.78 mmol/g, 1.31 g) by the general method described above, the target peptide was synthesized. For the synthesis, CEM's Liberty Blue was used as a solid phase synthesizer, and the synthesis was performed according to the manufacturer's manual. For the introduction of each residue, using Fmoc-AA/HATU/DIEA (4.2 Eq/4 Eq/8 Eq) per Eq of the resin, the reaction was repeated twice in DMF at 60°C for 15 minutes. However, for the introduction of the 15th residue, the reaction was performed once at 25°C for 30 minutes. For the introduction of the 11th and 12th residues, the reaction was repeated twice at 25°C for 20 minutes. For the introduction of the 10th residue, the reaction was performed once at 60°C for 15 minutes. For the introduction of the 7th residue, the reaction was performed once at 60°C for 15 minutes. For the introduction of the 5th residue, the reaction was performed once at 60°C for 15 minutes. For the introduction of the 3rd residue, the reaction was performed twice at 60°C for 45 minutes. Further, the basic conditions for Fmoc removal were such that the reaction with a DMF solution of 20% piperidine was performed at 75°C for 3 minutes. However, the Fmoc groups of the 2nd and 13th residues were removed by repeating the reaction twice at 25°C for 5 minutes. A chloroacetyl group was introduced in such a manner that after the Fmoc group of the α-amino group was removed by the method described above from the solid phase resin holding the Fmoc-protected peptide obtained in the previous step, chloroacetic acid (5 Eq), 5 Eq of DIPCI, and 5 Eq of HOSu were stirred in DCM, and the same amount of DMF as DCM was added to prepare a DCM/DMF solution (0.2 M) of ClAcOSu, which was then added to the solid phase resin, followed by shaking at room temperature for 240 minutes . For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether cooled to 0°C, a white cloudy precipitate was formed. The mixture was subjected to centrifugation separation (9500 rpm, 1 min), and the solution was subjected to decantation. The obtained solid was washed again with a small amount of diethyl ether cooled to 0°C, and then dried under reduced pressure . The obtained solid was used in the subsequent cyclization reaction. In the peptide cyclization reaction, after the solid was dissolved in DMSO so that the peptide final concentration was 4 mM based on the number of moles of the solid phase resin, 10 Eq of triethylamine was added, followed by shaking at room temperature for about 12 hours. The obtained reaction solution was concentrated under reduced pressure using Genevac EZ-2.

The obtained crude product was purified under the following conditions (column: Waters XBridge (registered trademark) C18 5 µm (registered trademark) 50 x 250 mm; mobile phase: A = 0.1% TFA in H₂O, B = 0.1% TFA in MeCN; temperature: 60°C; gradient (%B): 0-0% over 5 minutes, then 0-4.2% over 2 minutes, then 4.2-28.6% over 3 minutes, then 28.6-33.7% over 15.5 minutes, and then 33.7-60% over 1.5 minutes; flow rate: 18 mL/min for 5 minutes, then 18 mL/min-118 mL/min over 2 minutes, and then 118 mL/min).

The purity of the target product was calculated from the area ratio of the LC/MS (UV wavelength: 225 nm) chromatogram under analysis conditions B, and the result was 92.2%. Analysis conditions B: retention time = 11.52 minutes; gradient (%B conc) : 20-60% over 20 minutes, then 60-95% over 1 minute, and then 95% over 5 minutes
ESI-MS (+) measured value m/z = 1078.24 (M+2H)²⁺

### [Example 9-19]

### Synthesis of 894_3m_G4S2C(ACNMe) (SEQ ID NO: 443)

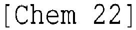

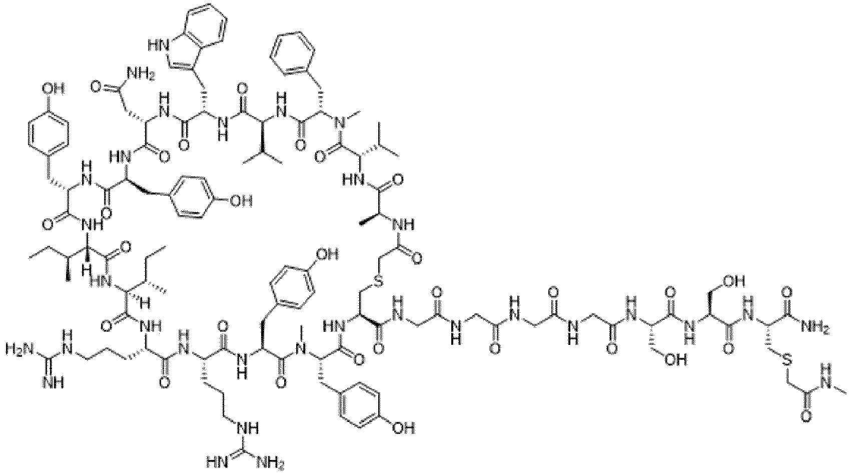

Separately synthesized 894_3m_G4S2C (20 mg, 7.07 µmol) was dissolved in DMF (0.1 mL), and 4 Eq of TEA (3.94 µL, 28.3 µmol) in DMF (39 µL) and 1.1 Eq of 2-iodo-N-methylacetamide (39.1 mg, 0.25 mmol) in DMF (15 µL) were added, followed by stirring at room temperature for 1 hour.

The obtained mixture was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 60°C; gradient (%B): 6-31 % over 3 minutes, then 31-36% over 8 minutes, and then 36-60% over 1 minute; flow rate: 17 mL/min).

The purity of the target product was calculated from the area ratio of the LC/MS (UV wavelength: 225 nm) chromatogram under analysis conditions B, and the result was 97.3%. Analysis conditions B: retention time = 3.53 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min
ESI-MS (+) measured value m/z = 1337.32 (M+2H)²⁺

### [Example 9-20]

### Synthesis of 894 0263 (SEQ ID NO: 487)

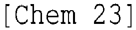

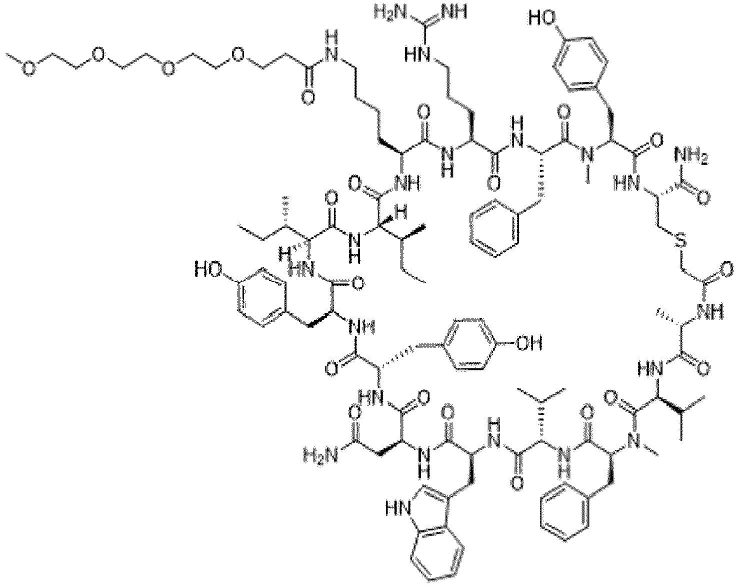

Starting from removal of Fmoc groups using Sieber amide resin (Watanabe Chemical Industries, Ltd., 0.48 mmol/g, 0.26 g) by the general method described above, the target peptide was synthesized. For the synthesis, CEM's Liberty Blue HT was used as a solid phase synthesizer, and the synthesis was performed according to the manufacturer's manual. For the introduction of each residue, the reaction was performed using Fmoc-AA/DIPCI/Oxyma pure/DIEA (4.2 Eq/8 Eq/4 Eq) per Eq of the resin. Further, Fmoc removal was performed by reaction with a DMF solution of 20% piperidine. A chloroacetyl group was introduced in such a manner that after the Fmoc group of the α-amino group was removed by the method described above from the solid phase resin holding the Fmoc-protected peptide obtained in the previous step, 5 Eq of chloroacetic acid in DMF, 5 Eq of HATU in DMF, and 10 Eq of DIEA in DMF were added, followed by shaking at 25°C for 30 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 60 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diisopropyl ether/hexane (1/1) cooled to 0°C, a white cloudy precipitate was formed. The mixture was subjected to centrifugation separation (9000 rpm, 2 min), and the solution was subjected to decantation. The obtained solid was washed again with a small amount of diethyl ether cooled to 0°C, and then dried under reduced pressure. The obtained solid was used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by shaking at room temperature overnight. The reaction solution was concentrated under reduced pressure using Savant Explorer SpeedVac. After the obtained solid was dissolved in DMSO so that the peptide final concentration was 5 mM based on the number of moles of the solid phase resin, MePEG4c (1.2 Eq), HATU (1.1 Eq), and DIEA (3 Eq) were added, followed by shaking at room temperature for 1 hour.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 13-38% over 3 minutes, then 38-43% over 8 minutes, and then 43-60% over 1 minute; flow rate: 120 mL/min.

The purity of the target product was calculated from the area ratio of the LC/MS (UV wavelength: 225 nm) chromatogram under analysis conditions B, and the result was 92.3%. Analysis conditions B: retention time = 5.28 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min
ESI-MS (+) measured value m/z = 1136.17 (M+2H)²⁺

The following conjugates of peptides, linkers, and payloads were synthesized.

### [Example 10-1]

### Synthesis of JCR_hTfR_000894_PEG11_K_FITC (SEQ ID NO: 147)

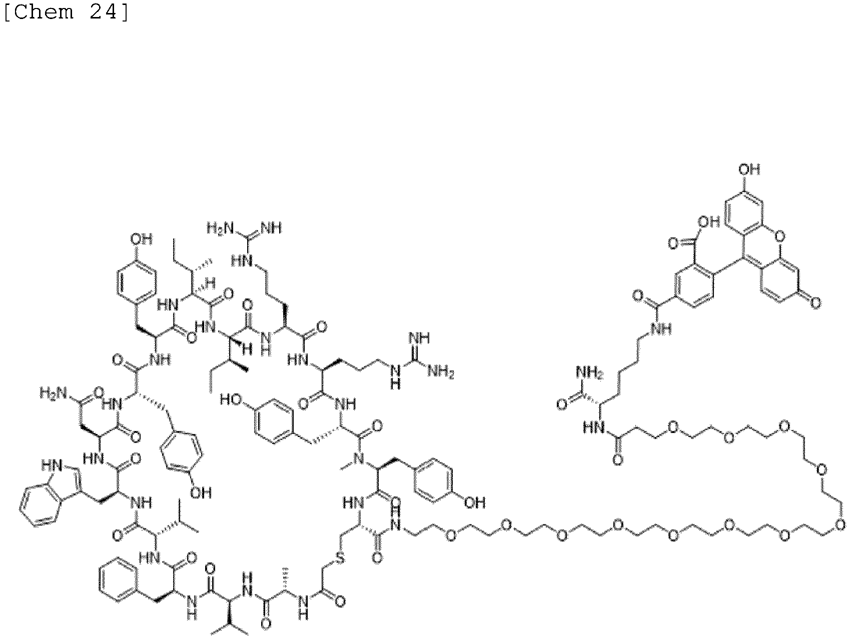

Starting from removal of Fmoc using Fmoc-NH-SAL-PEG-resin 1500-2000Da (Watanabe Chemical Industries, Ltd., 0.38 mmol/g) according to a general method, the target peptide was synthesized. For the synthesis, CEM's Liberty Blue was used as a solid phase synthesizer, and the synthesis was performed according to the manufacturer's manual. The basic conditions for the condensation reaction were such that using HATU as a condensing agent, the reaction was performed once at 75°C for 10 minutes. However, for the 11th and 12th residues, the reaction was repeated twice at 25°C for 20 minutes. For the 13th and 14th residues, the reaction was repeated twice at 75°C for 10 minutes. For the 15th residue, the reaction was performed once at 25°C for 20 minutes. For the 16th residue, the reaction was repeated once at 25°C for 60 minutes . Further, the basic conditions for Fmoc removal were such that the reaction with a DMF solution of 20% piperidine was performed at 75°C for 3 minutes. However, the Fmoc groups of the 13th and 15th residues were removed by reaction at 25°C for 5 minutes, and then reaction for 10 minutes. A chloroacetyl group was introduced in such a manner that a DMF solution (0.2 M) of 5 Eq of chloroacetic acid, a DMF solution (0.5 M) of 5 Eq of HATU, and a DMF solution (1 M) of 10 Eq of DIPEA were added to the solid phase resin obtained in the previous step, followed by shaking at room temperature for 30 minutes . For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether and hexane cooled to 0°C, a white cloudy precipitate was formed. The mixture was subjected to centrifugation separation (10000 rpm, 1 min), and the supernatant was subjected to decantation. The obtained solid was washed with ice-cooled diethyl ether and then dried. The obtained solid was used in the subsequent cyclization reaction. In the peptide cyclization reaction, after the solid was dissolved in DMSO so that the peptide final concentration was 5 mM based on the number of moles of the solid phase resin, 6 Eq of triethylamine was added, followed by shaking at room temperature overnight. A DMSO solution (0.71 M) of 1.1 Eq of FAM-OSu was added to the obtained reaction solution, followed by stirring for 30 minutes. AcOH was added to the reaction solution for quenching, and the solvent was concentrated under reduced pressure.

The obtained crude product was purified under the following conditions (column: XSelect C18 30 x 150 mm (Lot No.15113629811308) ; mobile phase: A = 0.1% TFA in H₂O, B = 0.1% TFA in MeCN; temperature: 40°C; gradient (%B): 6-31% over 3 minutes, then 31-36% over 8 minutes, and then 36-60% over 1 minute; flow rate: 45 mL/min).

The purity of the target product was calculated from the area ratio of the LC/MS (UV wavelength: 225 nm) chromatogram under analysis conditions B, and the result was 97.1%. Analysis conditions B: retention time = 13.20 minutes; gradient (%B conc) : 20-60% over 20 minutes, then 60-95% over 1 minute, and then 95% over 5 minutes
ESI-MS (+) measured value m/z = 793.2, theoretical value 792.6 (M+4H)⁴⁺

### [Example 10-2]

### Synthesis of 894_v01_PEG12_C_Mal(FITC) (SEQ ID NO: 166)

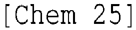

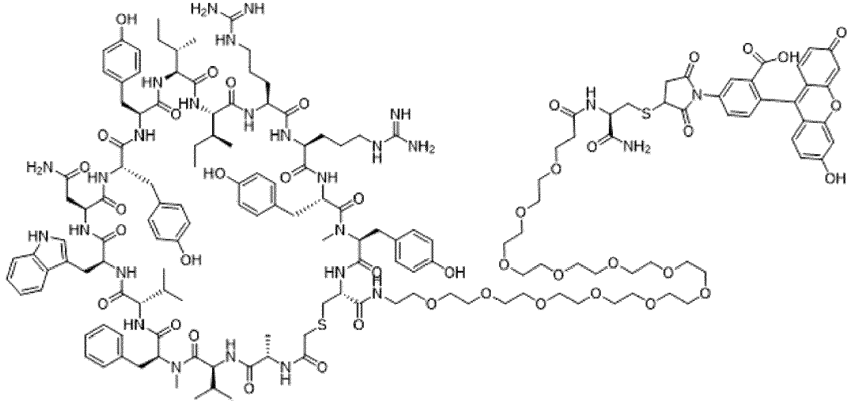

Starting from removal of Fmoc groups using Sieber amide resin (Watanabe Chemical Industries, Ltd., 0.6 mmol/g, 0.19 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA/HATU/DIEA (5.3 Eq/5 Eq/10 Eq) per Eq of the resin, the reaction was performed once in DMF at 75°C for 10 minutes. However, for the 2nd and 13th residues, the reaction was repeated twice at 75°C for 10 minutes. For the 11th and 12th residues, the reaction was repeated twice at 25°C for 20 minutes. For the 15th and 17th residues, the reaction was performed once at 25°C for 30 minutes. Further, the basic conditions for Fmoc removal were such that the reaction with a DMF solution of 20% piperidine was performed at 75°C for 3 minutes. However, the Fmoc groups of the 2nd, 13th, 15th, and 17th residues were removed by reaction at 25°C for 5 minutes, and then reaction at 25°C for 10 minutes. A chloroacetyl group was introduced in such a manner that after the Fmoc group of the α-amino group was removed by the method described above from the solid phase resin holding the Fmoc-protected peptide obtained in the previous step, 5 Eq of chloroacetic acid in DMF, 5 Eq of HATU in DMF, and 10 Eq of DIEA in DMF were added to the solid phase resin, followed by shaking at room temperature for 30 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether/hexane (1/1) cooled to 0°C, a white cloudy precipitate was formed. The mixture was subjected to centrifugation separation (10000 rpm, 1 min), the solution was subjected to decantation, and the solid was washed with diethyl ether cooled to 0°C and then dried under reduced pressure. The obtained solid was used in the subsequent cyclization reaction. In the peptide cyclization reaction, after the solid was dissolved in DMSO so that the peptide final concentration was 5 mM based on the number of moles of the solid phase resin, 6 Eq of triethylamine was added, followed by shaking at room temperature overnight. The obtained reaction solution was concentrated under reduced pressure using Savant Explorer SpeedVac. The obtained mixture was dissolved in DMSO, and 3 Eq of silver acetate was added, followed by shaking for 3 hours . 10 Eq of DTT was added, followed by centrifugation to collect the supernatant.

The obtained crude intermediate peptide was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 9-34% over 3 minutes, then 34-39% over 8 minutes, and then 39-60% over 1 minute; flow rate : 120 mL/min).

The purified intermediate peptide (29.8 mg, 10.6 µmol) was dissolved in DMSO (424 µL), and 1.1 Eq of fluorescein-5-maleimide and 5 Eq of DIEA were added, followed by stirring for 1 hour, after which the reaction was quenched with acetic acid.

The obtained crude product was purified under the following conditions (column: COSMOSIL PBr 10 x 150 mm; mobile phase: A = 0.1% TFA in H₂O, B = 0.1% TFA in MeCN; temperature: 40°C; gradient (%B): 23-48% over 3 minutes, then 48-53% over 8 minutes, and then 53-60% over 1 minute; flow rate: 5 mL/min) .

The purity of the target product was calculated from the area ratio of the LC/MS (UV wavelength: 225 nm) chromatogram under analysis conditions B, and the result was 82.8%. Analysis conditions B: retention time = 13.93 minutes; column: Kinetex EVO C18 2.6 µm 2.1 x 150 mm, 100Å; mobile phase: A = 0.025% TFA in H₂O, B = 0.025% TFA in MeCN; temperature: 40°C; gradient (%B conc): 20-60% over 20 minutes, then 60-95% over 1 minute, and then 95% over 5 minutes; flow rate: 0.25 mL/min
ESI-MS (+) measured value m/z = 1076.51, theoretical value (M+2H)²⁺

### [Synthesis Example 10-3]

### Synthesis of 894_variant_61_G4S2C (SEQ ID NO: 358)

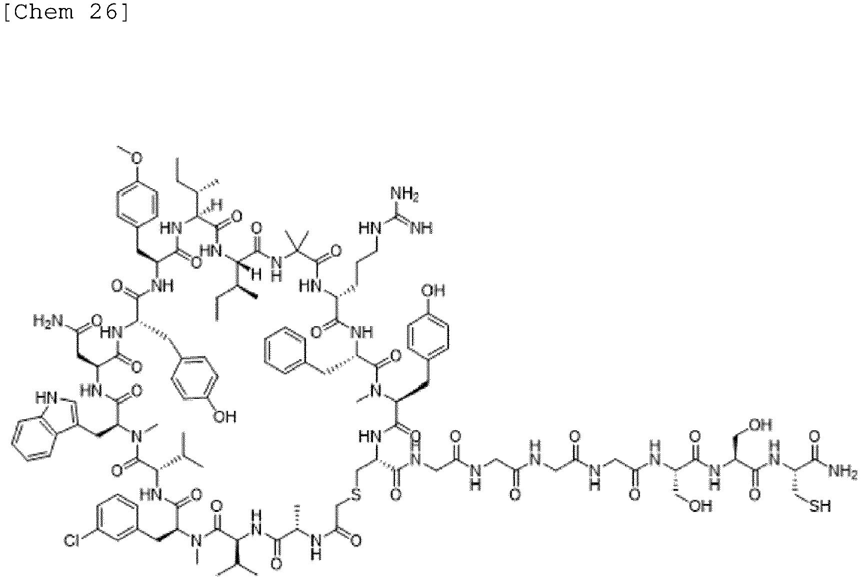

Starting from removal of Fmoc groups using Sieber amide resin (Watanabe Chemical Industries, Ltd., 0.73 mmol/g, 1.37 g) by the general method described above, the target peptide was synthesized. For the synthesis, CEM's Liberty Blue was used as a solid phase synthesizer, and the synthesis was performed according to the manufacturer's manual. For the introduction of each residue, using Fmoc-AA/HATU/DIEA (4.2 Eq/4 Eq/8 Eq) per Eq of the resin, the reaction was performed once in DMF at 75°C for 10 minutes. However, for the 2nd and 4th residues, the reaction was performed twice at 75°C for 30 minutes. For the 12th residue, the reaction was repeated twice at 25°C for 20 minutes. For the 13th residue, the reaction was performed twice at 75°C for 10 minutes. For the 15th and 22nd residues, the reaction was performed once at 25°C for 30 minutes. Further, the basic conditions for Fmoc removal were such that the reaction with a DMF solution of 20% piperidine was performed at 75°C for 3 minutes. However, the Fmoc groups of the 4th and 13th residues were removed by reaction at 25°C for 5 minutes, and then reaction at room temperature for 10 minutes. A chloroacetyl group was introduced in such a manner that after the Fmoc group of the α-amino group was removed by the method described above from the solid phase resin holding the Fmoc-protected peptide obtained in the previous step, 10 Eq of chloroacetic acid, 10 Eq of DIPCI, and 10 Eq of HOSu were stirred in DCM, and the same amount of DMF as DCM was added to prepare a DCM/DMF solution (0.2 M) of ClAcOSu, which was then added to the solid phase resin, followed by shaking at room temperature for 60 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 90:2.5:2.5:5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diisopropyl ether/hexane (1/1) cooled to 0°C, a white cloudy precipitate was formed. The mixture was subjected to centrifugation separation (9500 rpm, 1 min), and the solution was subjected to decantation, followed by drying under reduced pressure. The obtained solid was used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by shaking at room temperature for about 15 hours. The reaction liquid was quenched with acetic acid and concentrated under reduced pressure using Genevac HT-12. The obtained mixture was dissolved in DMSO (20 mL), and 3 Eq of silver acetate was added, followed by shaking for 3 hours. After a 2 M DTT aqueous solution (11 Eq) was added, centrifugation was performed to collect the supernatant, followed by concentration under reduced pressure, thereby obtaining a crude product.

The obtained crude product was purified under the following conditions (column: Waters XBridge (registered trademark) C18 5 µm (registered trademark) 50 × 250 mm; mobile phase: A = 0.1% TFA in H₂O, B = 0.1% TFA in MeCN; temperature: 60°C; gradient (%B): 6.7-6.7% over 5 minutes, then 6.7-10.3% over 2 minutes, then 10.3-35.8% over 3 minutes, then 35.8-40.8% over 15.5 minutes, and then 40.8-60% over 1.5 minutes; flow rate: 18 mL/min-18 mL/min over 5 minutes, then 18 mL/min-118 mL/min over 2 minutes, and then 118 mL/min).

The purity of the target product was calculated from the area ratio of the LC/MS (UV wavelength: 225 nm) chromatogram under analysis conditions B, and the result was 94.8%. Analysis conditions B: retention time = 5.45 minutes; column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min
ESI-MS (+) measured value m/z = 1289.65 (M+2H)²⁺

Peptides or linker-added peptides were synthesized as described below. The synthesized peptides and linker-added peptides are shown in Table 5, and the linkers are shown in Table 6.

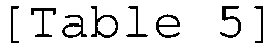

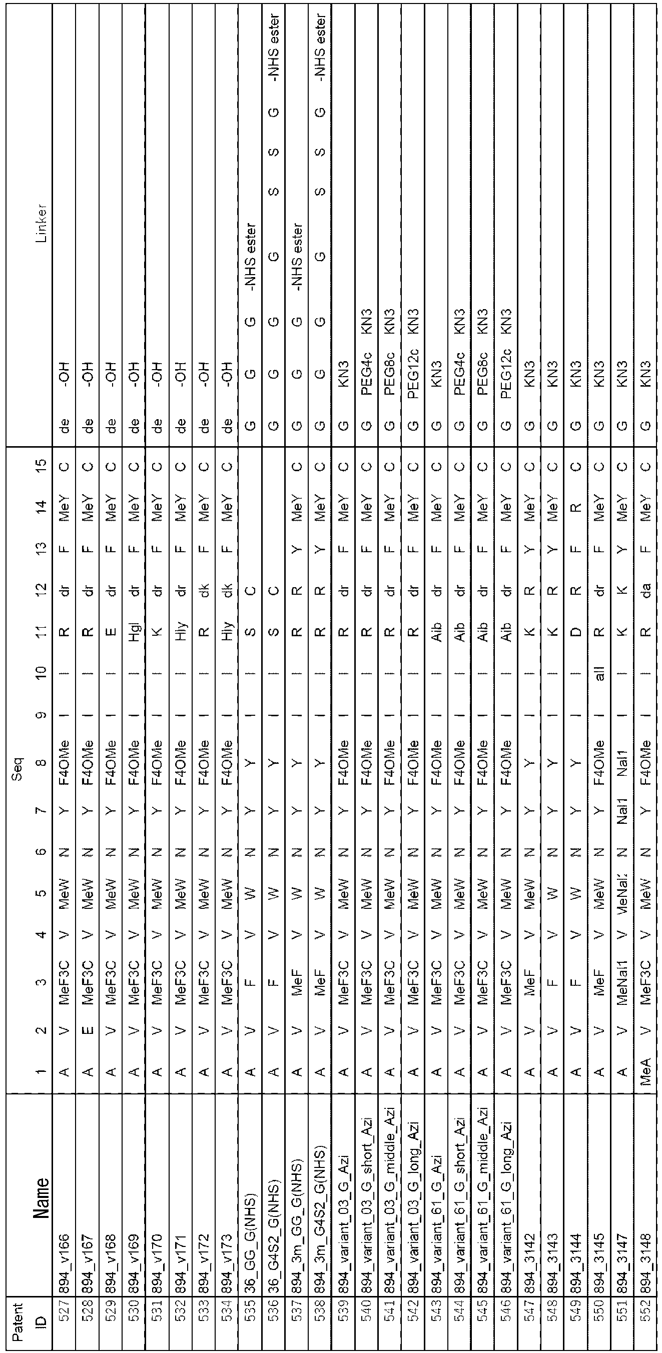

**[Table 6-1]**

| Patent ID | Seq | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | 13 |
| 553 | G | | | | | | | | | | | | |
| 554 | G | KN3 | | | | | | | | | | | |
| 555 | G | OH | | | | | | | | | | | |
| 556 | G | K(Maleimide) | C | | | | | | | | | | |
| 557 | G | G | K(Maleimide) | C | | | | | | | | | |
| 558 | G | G | S | K(Maleimide) | C | | | | | | | | |
| 559 | G | G | S | S | K(Maleimide) | C | | | | | | | |
| 560 | G | G | G | S | S | K(Maleimide) | C | | | | | | |
| 561 | G | G | G | G | S | S | | | | | | | |
| 562 | G | G | S | G | S | G | | | | | | | |
| 563 | de | G | G | G | S | S | | | | | | | |
| 564 | G | de | G | de | S | S | | | | | | | |
| 565 | G | dk | G | dk | S | S | | | | | | | |
| 566 | G | de | G | dk | S | S | | | | | | | |
| 567 | G | G | G | G | S | S | | | | | | | |
| 568 | G | G | R | G | R | S | K(Maleimide) | | | | | | |
| 569 | G | G | G | G | S | S | K(Maleimide) | | | | | | |
| 570 | G | MeG | S | MeG | S | S | K(Maleimide) | | | | | | |
| 571 | G | G | G | G | S | S | KTrzMal | | | | | | |
| 572 | G | MeG | S | MeG | S | S | K(Maleimide) | | | | | | |
| 573 | G | G | G | G | S | S | K(Maleimide) | | | | | | |
| 574 | G | G | S | G | S | S | K(Maleimide) | C | | | | | |
| 575 | G | G | S | G | E | S | K(Maleimide) | C | | | | | |
| 576 | G | G | E | S | E | S | K(Maleimide) | C | | | | | |
| 577 | G | S | S | G | S | S | K(Melaimide) | C | | | | | |
| 578 | G | G | R | G | R | S | K(Maleimide) | C | | | | | |
| 579 | G | G | R | S | E | S | K(Maleimide) | C | | | | | |
| 580 | G | G | G | G | S | S | K(Maleimide) | C | | | | | |
| 581 | G | G | G | G | S | S | C(AcNMe) | | | | | | |
| 582 | G | KCOpipzaa | KCOpipzaa | KCOpipzaa | S | S | C(AcNMe) | | | | | | |

**[Table 6-2]**

| | | | | | | | | | | | | | |
|---|---|---|---|---|---|---|---|---|---|---|---|---|---|
| 583 | G | KCOpipzaa | KCOpipzaa | G | S | S | C(AcNMe) | | | | | | |
| 584 | G | de | de | de | S | S | C(AcNMe) | | | | | | |
| 585 | G | de | de | G | S | S | C(AcNMe) | | | | | | |
| 586 | G | KCOmeglumine | G | G | S | S | C(AcNMe) | | | | | | |
| 587 | G | KCOpipzaa | G | de | S | S | C(AcNMe) | | | | | | |
| 588 | G | G | KCOpipzaa | de | S | S | C(AcNMe) | | | | | | |
| 589 | G | KCOpipzaa | G | G | S | S | C(AcNMe) | | | | | | |
| 590 | G | G | KCOpipzaa | G | S | S | C(AcNMe) | | | | | | |
| 591 | G | G | G | KCOpipzaa | S | S | C(AcNMe) | | | | | | |
| 592 | G | KCOmeglumine | G | de | S | S | C(AcNMe) | | | | | | |
| 593 | G | G | KCOmeglumine | de | S | S | C(AcNMe) | | | | | | |
| 594 | G | de | G | KCOpipzaa | S | S | C(AcNMe) | | | | | | |
| 595 | G | G | de | KCOpipzaa | S | S | C(AcNMe) | | | | | | |
| 596 | G | de | G | KCOmeglumine | S | S | C(AcNMe) | | | | | | |
| 597 | G | G | de | KCOmeglumine | S | S | C(AcNMe) | | | | | | |
| 698 | G | G | KCOmeglumine | G | S | S | C(AcNMe) | | | | | | |
| 599 | G | G | G | KCOmeglumine | S | S | C(AcNMe) | | | | | | |
| 600 | G | KCOpipzaa | de | de | S | S | C(AcNMe) | | | | | | |
| 601 | G | de | KCOpipzaa | de | S | S | C(AcNMe) | | | | | | |
| 602 | G | de | de | KCOpipzaa | S | S | C(AcNMe) | | | | | | |
| 603 | G | de | de | de | S | S | C(AcNMe) | | | | | | |
| 604 | G | G | S | G | S | S | G | G | S | G | | | |
| 605 | G | G | S | G | S | S | G | S | S | K(Maleimide) | | | |
| 606 | G | G | R | G | R | S | G | G | R | G | R | S | |
| 607 | G | G | Q | G | Q | S | G | G | Q | G | Q | S | |
| 608 | de | | | | | | | | | | | | |
| 609 | de | de | | | | | | | | | | | |
| 610 | de | MeG | de | | | | | | | | | | |
| 611 | MeG | MeG | MeG | | | | | | | | | | |
| 612 | PEG4c | dk | | | | | | | | | | | |

**[Table 6-3]**

| | | | | | | | | | | | | | |
|---|---|---|---|---|---|---|---|---|---|---|---|---|---|
| 613 | PEG12c | dk | | | | | | | | | | | |
| 614 | PEG4c | de | PEG4c | dk | | | | | | | | | |
| 615 | PEG4c | dr | PEG4c | dk | | | | | | | | | |
| 616 | PEG4c | de | PEG4c | de | PEG4c | dk | | | | | | | |
| 617 | PEG4c | dr | PEG4c | dr | PEG4c | dk | | | | | | | |
| 618 | PEG4c | dk | | | | | | | | | | | |
| 619 | PEG12c | dk | | | | | | | | | | | |
| 620 | PEG4c | de | PEG4c | dk | | | | | | | | | |
| 621 | PEG4c | dr | PEG4c | dk | | | | | | | | | |
| 622 | PEG4c | de | PEG4c | de | PEG4c | dk | | | | | | | |
| 623 | PEG4c | dr | PEG4c | dr | PEG4c | dk | | | | | | | |
| 624 | PEG4c | KTrzMal | | | | | | | | | | | |
| 625 | PEG8c | KTrzMal | | | | | | | | | | | |
| 626 | PEG12c | KTrzMal | | | | | | | | | | | |
| 627 | PEG4c | K(Maleimide) | | | | | | | | | | | |
| 628 | PEG8c | K(Maleimide) | | | | | | | | | | | |
| 629 | PEG12c | K(Maleimide) | | | | | | | | | | | |
| 630 | KN3 | | | | | | | | | | | | |
| 631 | PEG4c | KN3 | | | | | | | | | | | |
| 632 | PEG8c | KN3 | | | | | | | | | | | |
| 633 | PEG12c | KN3 | | | | | | | | | | | |
| 634 | G | PEG12c | gAbu | | | | | | | | | | |
| 635 | G | PEG4c | gAbu | | | | | | | | | | |
| 636 | G | PEG4c | PEG4c | PEG4c | gAbu | | | | | | | | |
| 637 | G | G | R | G | R | S | gAbu | | | | | | |
| 638 | G | PEG4c | R | PEG4c | R | PEG4c | gAbu | | | | | | |
| 639 | G | G | Q | G | Q | S | gAbu | | | | | | |
| 640 | G | G | G | G | S | S | G | G | G | G | S | S | gAbu |
| 641 | G | G | G | G | S | S | dk(CHO4PhCO) | NH2 | | | | | |
| 642 | PEG12c | dk(CHO4PhCO) | NH2 | | | | | | | | | | |
| 643 | G | G | G | G | | | | | | | | | |
| 644 | G | G | G | G | S | S | C | | | | | | |

### [Synthesis Example 1-1]

### Synthesis of 894_3142 (SEQ ID NO: 547)

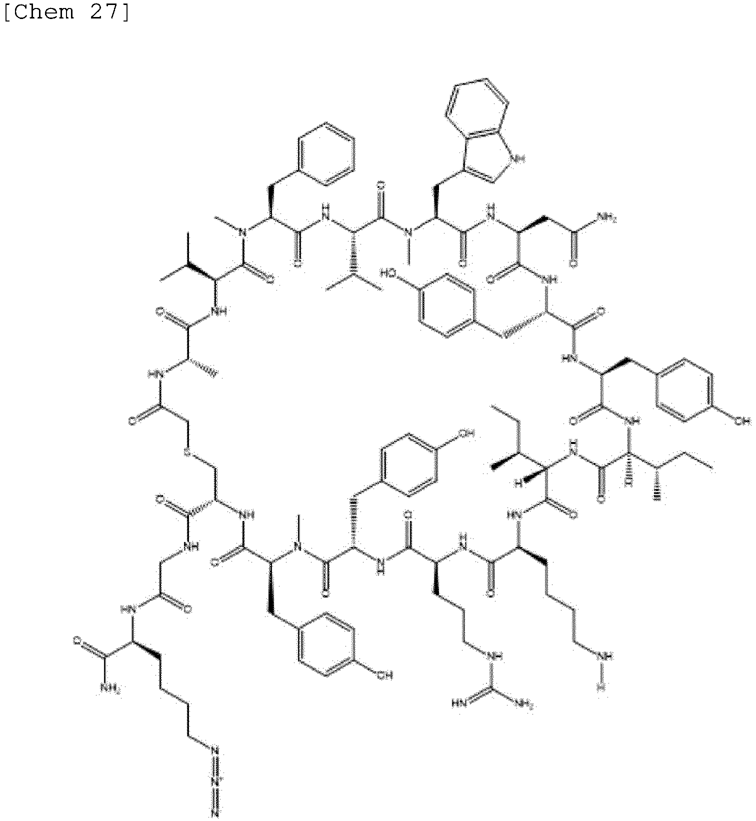

Starting from removal of Fmoc using Sieber amide resin (Watanabe Chemical Industries, Ltd., 0.48 mmol/g, 0.52 g), the target peptide was synthesized. For the synthesis, CEM's Liberty Blue was used as an automatic synthesizer, and the synthesis was performed according to the manufacturer's manual. The synthesis was performed in the same manner as in Example 1.

The obtained crude product was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 7-32% over 3 minutes, then 32-37% over 8 minutes, and then 37-60% over 1 minute; flow rate: 120 mL/min).

The purity of the target product was calculated from the area ratio of the LC/MS (UV wavelength: 225 nm) chromatogram under the following analysis conditions, and the result was 94.20%.
Analysis conditions B: retention time = 4.12 minutes; column: Kinetex EVO C18 2.6 µm, 2.1 × 150 mm, 100Å; mobile phase: A = 0.025% TFA in H₂O, B = 0.025% TFA in MeCN; temperature: 60°C; gradient (%B conc) : 20-60% over 7.15 minutes, then 60-95% over 0.3 minutes, and then 95-95% over 1.55 minutes; flow rate: 0.5 mL/min
ESI-MS (+) measured value m/z = 1147.70 (M+2H)²⁺

### [Example 1-2]

### Synthesis of 894_3143 (SEQ ID NO: 548)

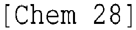

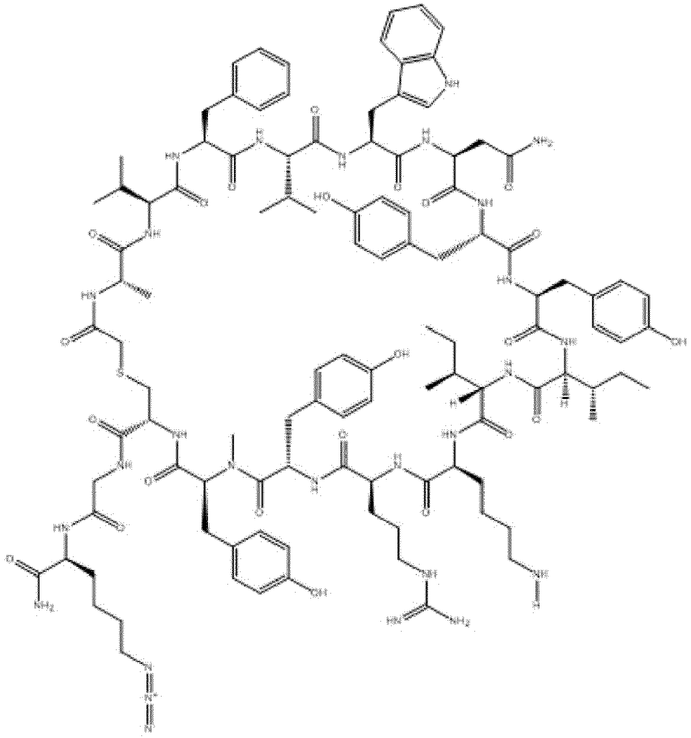

Starting from removal of Fmoc using Sieber amide resin (Watanabe Chemical Industries, Ltd., 0.48 mmol/g, 0.52 g), the target peptide was synthesized. For the synthesis, CEM's Liberty Blue HT was used as an automatic synthesizer, and the synthesis was performed according to the manufacturer's manual. The synthesis was performed in the same manner as in Example 1.

The obtained crude product was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 6-6% over 2 minutes, then 6-31% over 1 minute, then 31-36% over 8 minutes, and then 36-60% over 1 minute; flow rate: 20-20 mL/min over 1 minute, then 20-120 mL/min over 1 minute, and then 120 mL/min).

The purity of the target product was calculated from the area ratio of the LC/MS (UV wavelength: 225 nm) chromatogram under the following analysis conditions, and the result was 97.61%.
Analysis conditions: retention time = 3.75 minutes; column: Kinetex EVO C18 2.6 µm, 2.1 × 150 mm, 100Å; mobile phase: A = 0.025% TFA in H₂O, B = 0.025% TFA in MeCN; temperature: 60°C; gradient (%B conc) : 20-60% over 7.15 minutes, then 60-95% over 0.3 minutes, and then 95-95% over 1.55 minutes; flow rate: 0.5 mL/min
ESI-MS (+) measured value m/z = 1133.66 (M+2H)²⁺

### [Example 1-3]

### Synthesis of 894_3144 (SEQ ID NO: 549)

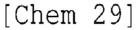

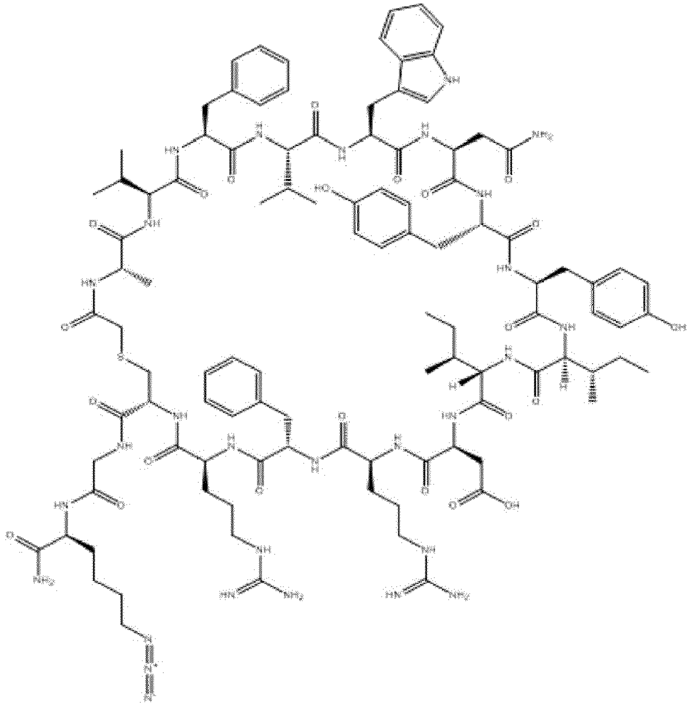

Starting from removal of Fmoc using Sieber amide resin (Watanabe Chemical Industries, Ltd., 0.48 mmol/g, 0.52 g), the target peptide was synthesized. For the synthesis, CEM's Liberty Blue was used as an automatic synthesizer, and the synthesis was performed according to the manufacturer's manual. The synthesis was performed in the same manner as in Example 1.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 8-8% over 2 minutes, then 8-33% over 1 minute, then 33-38% over 8 minutes, and then 38-60% over 1 minute; flow rate: 20-20 mL/min over 1 minute, then 20-120 mL/min over 1 minute, and then 120 mL/min).

The purity of the target product was calculated from the area ratio of the LC/MS (UV wavelength: 225 nm) chromatogram under the following analysis conditions, and the result was 97.52%.
Analysis conditions B: retention time = 3.97 minutes; column: Kinetex EVO C18 2.6 µm, 2.1 × 150 mm, 100Å; mobile phase: A = 0.025% TFA in H₂O, B = 0.025% TFA in MeCN; temperature: 60°C; gradient (%B conc) : 20-60% over 7.15 minutes, then 60-95% over 0.3 minutes, and then 95-95% over 1.55 minutes; flow rate: 0.5 mL/min
ESI-MS (+) measured value m/z = 1108.62 (M+2H)²⁺

### [Example 1-4]

### Synthesis of 894_3145 (SEQ ID NO: 550)

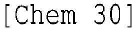

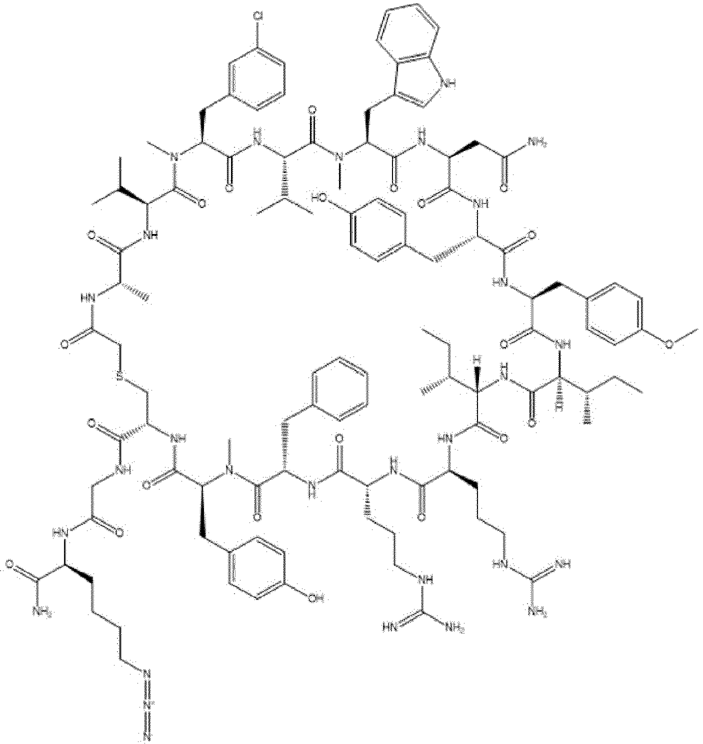

Starting from removal of Fmoc using Sieber amide resin (Watanabe Chemical Industries, Ltd., 0.48 mmol/g, 0.52 g), the target peptide was synthesized. For the synthesis, CEM's Liberty Blue was used as an automatic synthesizer, and the synthesis was performed according to the manufacturer's manual. The synthesis was performed in the same manner as in Example 1.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 13-38% over 3 minutes, then 38-43% over 8 minutes, and then 43-60% over 1 minute; flow rate: 120 mL/min) .

The purity of the target product was calculated from the area ratio of the LC/MS (UV wavelength: 225 nm) chromatogram under the following analysis conditions, and the result was 98.02%.
Analysis conditions: retention time = 4.91 minutes; column: Kinetex EVO C18 2.6 µm, 2.1 × 150 mm, 100Å; mobile phase: A = 0.025% TFA in H₂O, B = 0.025% TFA in MeCN; temperature: 60°C; gradient (%B conc) : 20-60% over 7.15 minutes, then 60-95% over 0.3 minutes, and then 95-95% over 1.55 minutes; flow rate: 0.5 mL/min
ESI-MS (+) measured value m/z = 1177.95 (M+2H)²⁺

### [Example 1-5]

### Synthesis of 894_3147 (SEQ ID NO: 551)

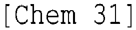

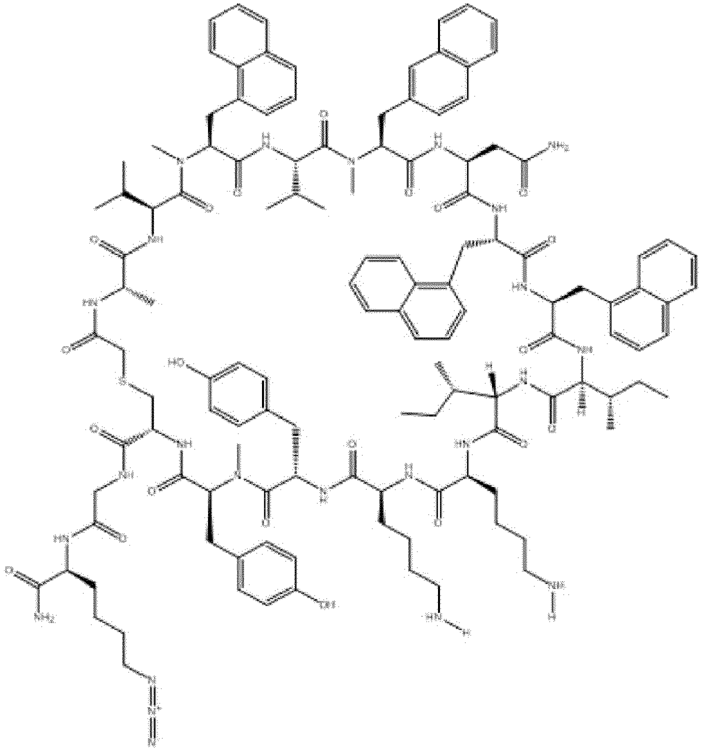

Starting from removal of Fmoc using Sieber amide resin (Watanabe Chemical Industries, Ltd., 0.48 mmol/g, 0.52 g), the target peptide was synthesized. For the synthesis, CEM's Liberty Blue was used as an automatic synthesizer, and the synthesis was performed according to the manufacturer's manual. The synthesis was performed in the same manner as in Example 1.

The obtained crude product was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 21-46% over 3 minutes, then 46-51% over 8 minutes, and then 51-60% over 1 minute; flow rate: 120 mL/min).

The purity of the target product was calculated from the area ratio of the LC/MS (UV wavelength: 225 nm) chromatogram under the following analysis conditions, and the result was 96.34%.
Analysis conditions: retention time = 6.24 minutes; column: Kinetex EVO C18 2.6 µm, 2.1 × 150 mm, 100Å; mobile phase: A = 0.025% TFA in H₂O, B = 0.025% TFA in MeCN; temperature: 60°C; gradient (%B conc) : 20-60% over 7.15 minutes, then 60-95% over 0.3 minutes, and then 95-95% over 1.55 minutes; flow rate: 0.5 mL/min
ESI-MS (+) measured value m/z = 1198.29 (M+2H)²⁺

### [Example 1-6]

### Synthesis of 894_3148 (SEQ ID NO: 552)

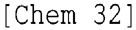

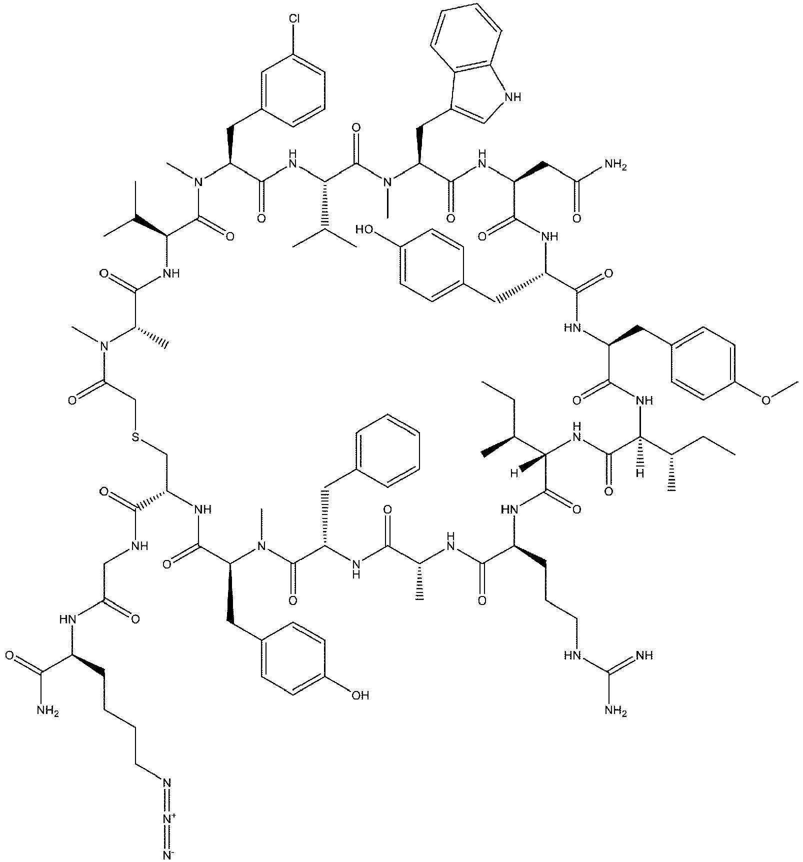

Starting from removal of Fmoc using Sieber amide resin (Watanabe Chemical Industries, Ltd., 0.48 mmol/g, 0.52 g), the target peptide was synthesized. For the synthesis, CEM's Liberty Blue was used as an automatic synthesizer, and the synthesis was performed according to the manufacturer's manual. The synthesis was performed in the same manner as in Example 1.

The obtained crude product was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 18-43% over 3 minutes, then 43-48% over 8 minutes, and then 48-60% over 1 minute; flow rate: 120 mL/min).

The purity of the target product was calculated from the area ratio of the LC/MS (UV wavelength: 225 nm) chromatogram under the following analysis conditions, and the result was 96.03%.
Analysis conditions: retention time = 6.15 minutes; column: Kinetex EVO C18 2.6 µm, 2.1 × 150 mm, 100Å; mobile phase: A = 0.025% TFA in H₂O, B = 0.025% TFA in MeCN; temperature: 60°C; gradient (%B conc) : 20-60% over 7.15 minutes, then 60-95% over 0.3 minutes, and then 95-95% over 1.55 minutes; flow rate: 0.5 mL/min
ESI-MS (+) measured value m/z = 1142.36 (M+2H)²⁺

### [Synthesis Example 1-7]

### Synthesis of 894_variant_61_G_Azi (SEQ ID NO: 543)

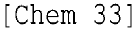

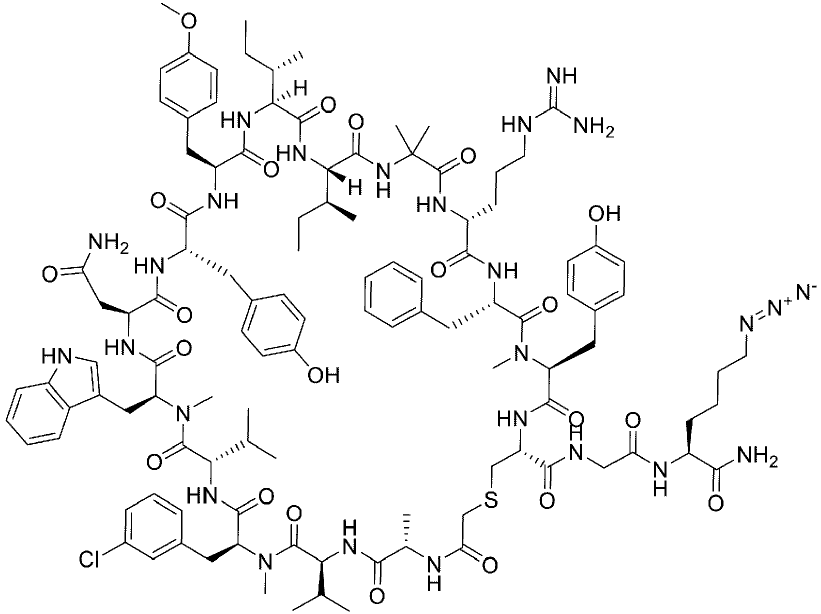

Starting from removal of Fmoc groups using Sieber amide resin (Watanabe Chemical Industries, Ltd., 0.47 mmol/g, 0.21 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA/HATU/DIEA (5.3Eq/5 Eq/10 Eq) per Eq of the resin, the reaction was performed once in DMF at 75°C for 10 minutes. However, for the 2nd and 4th residues, the reaction was performed twice at 75°C for 30 minutes. For the 10th, 11th, and 13th residues, the reaction was repeated twice at 75°C for 10 minutes. For the 12th residue, the reaction was performed twice at 25°C for 20 minutes . For the 15th residue, the reaction was performed once at 25°C for 20 minutes. Further, the basic conditions for Fmoc removal were such that the reaction with a DMF solution of 20% piperidine was performed at 75°C for 3 minutes. However, the Fmoc groups of the 2nd, 4th, and 13th residues were removed by reaction at 25°C for 5 minutes, and then reaction at 25°C for 10 minutes. A chloroacetyl group was introduced in such a manner that after the Fmoc group of the α-amino group was removed by the method described above from the solid phase resin holding the Fmoc-protected peptide obtained in the previous step, 10 Eq of chloroacetic acid, 10 Eq of DIPCI, and 10 Eq of HOSu were stirred in DCM, and the same amount of DMF as DCM was added to prepare a DCM/DMF solution (0.2 M) of ClAcOSu, which was then added to the solid phase resin, followed by shaking at room temperature for 60 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether/hexane (1/1) cooled to 0°C, a white cloudy precipitate was formed. The mixture was subjected to centrifugation separation (9000 rpm, 2 min), and the solution was subjected to decantation, followed by drying under reduced pressure. The obtained solid was used in the subsequent cyclization reaction. In the peptide cyclization reaction, after the solid was dissolved in DMSO so that the peptide final concentration was 5 mM based on the number of moles of the solid phase resin, 5 Eq of triethylamine was added, followed by shaking at room temperature for about 14 hours. The obtained reaction solution was concentrated under reduced pressure using Savant Explorer SpeedVac.

The obtained crude product was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 17-42% over 3 minutes, then 42-47% over 8 minutes, and then 47-60% over 1 minute; flow rate: 120 mL/min).

The purity of the target product was calculated from the area ratio of the LC/MS (UV wavelength: 225 nm) chromatogram under analysis conditions B, and the result was 92.8%. Analysis conditions B: retention time = 6.22 minutes; column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min
ESI-MS (+) measured value m/z = 1142.31 (M+2H)²⁺

### [Synthesis Example 1-8]

### Synthesis of hTfR_894_3m_G_PEG4_gAbu(NHS) (conjugate of linker represented by SEQ ID NO: 635 and peptide represented by SEQ ID NO: 296)

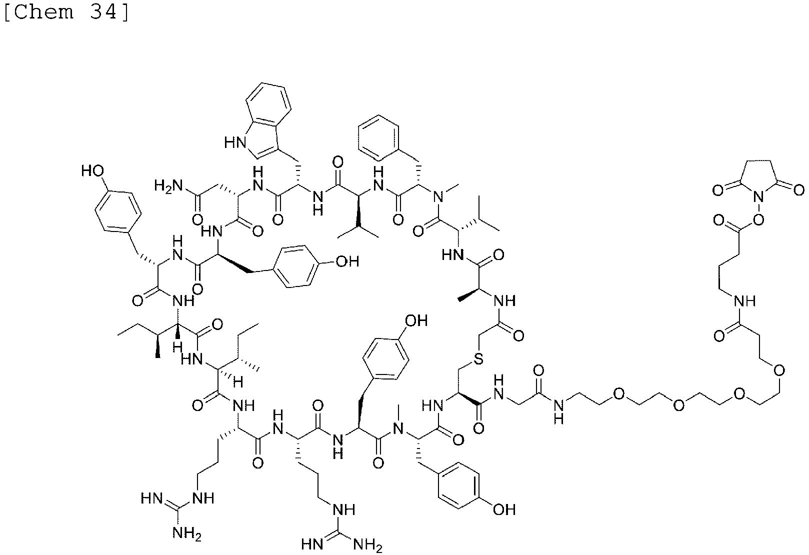

Starting from removal of Fmoc using Fmoc-PEG4c-Abu-Alko resin, which was prepared from Fmoc-Abu (4)-Alko resin (Watanabe Chemical Industries, Ltd., 1.00 mmol/g, 0.1 g) by general solid phase synthesis, according to the general method described above, a C-terminus carboxylic acid peptide was synthesized. For the synthesis, CEM's Liberty Blue was used as a solid phase synthesizer, and the synthesis was performed according to the manufacturer's manual. A chloroacetyl group was introduced in such a manner that after the Fmoc group of the α-amino group was removed by the method described above from the solid phase resin holding the Fmoc-protected peptide obtained in the previous step, 0.2 M chloroacetic acid in DMF (5 Eq), 0.5 M HATU in DMF (5 Eq), and 1 M DIEA in DMF (10 Eq) were added to the solid phase resin, followed by shaking at room temperature for 30 minutes. For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diethyl ether/hexane (1/1) cooled to 0°C, a white cloudy precipitate was formed. The mixture was subjected to centrifugation separation (10000 rpm, 1 min), and the solution was subjected to decantation. After the obtained solid was washed again with a small amount of diethyl ether cooled to 0°C, the obtained solid was dried and used in the subsequent cyclization reaction. In the peptide cyclization reaction, after the solid was dissolved in DMSO so that the peptide final concentration was 5 mM based on the number of moles of the solid phase resin, 10 Eq of triethylamine was added, followed by stirring at room temperature for about 17 hours. The reaction solution was concentrated under reduced pressure using Savant Explorer SpeedVac. The mixture was dissolved in DMSO/H₂O (9/1, 4 mL) so that the final concentration of the obtained C-terminus carboxylic acid peptide was 5 mM based on the number of moles of the solid phase resin, and HOSu (230 mg, 20 Eq) and EDC HCl (383.4 mg, 20 Eq) were added, followed by stirring.

The obtained crude product was purified under the following conditions (column: Waters Xbridge (registered trademark) C18 5 µm (registered trademark) 30 × 150 mm; mobile phase: A = 0.1% TFA in H₂O, B = 0.1% TFA in MeCN; temperature: 40°C; gradient (%B): 9-34% over 3 minutes, then 34-39% over 8 minutes, and then 39-60% over 1 minute; flow rate: 45 mL/min.

The purity of the target product was calculated from the area ratio of the LC/MS (UV wavelength: 225 nm) chromatogram under analysis conditions B, and the result was 87.4%. Analysis conditions B: retention time = 4.25 minutes; column: Kinetex EVO C18 2.6 µm 2.1 × 150 mm, 100Å; mobile phase: A = 0.025% TFA in H₂O, B = 0.025% TFA in MeCN; temperature: 40°C; gradient (%B conc) : 20-60% over 7.2 minutes, then 60-95% over 0.3 minutes, and then 95-95% over 1.6 minutes; flow rate: 0.5 mL/min
ESI-MS (+) measured value m/z = 1292.86 (M+2H)²⁺

### [Synthesis Example 1-9]

### Synthesis of 894_C12NH2 (conjugate of fatty acid linker and SEQ ID NO: 1)

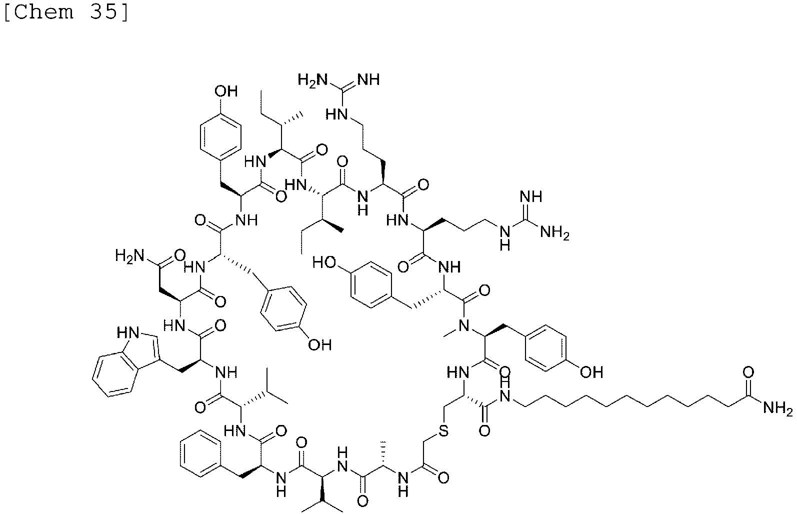

Starting from removal of Fmoc groups using Sieber amide resin (Watanabe Chemical Industries, Ltd., 0.53 mmol/g, 0.21 g) by the general method described above, the target peptide was synthesized. For the synthesis, CEM's Liberty Blue was used as a solid phase synthesizer, and the synthesis was performed according to the manufacturer's manual. For the introduction of each residue, using Fmoc-AA/HATU/DIEA (5.3 Eq/5 Eq/10 Eq) per Eq of the resin, the reaction was performed in DMF at 75°C for 10 minutes. However, for the 1st, 9th, 10th, 13th, and 14th residues, the reaction was performed twice at 75°C for 10 minutes . For the 11th and 12th residues, the reaction was performed twice at 30°C for 20 minutes . For the introduction of the 15th residue, the reaction was performed once at 30°C for 20 minutes. Further, the basic conditions for Fmoc removal were such that the reaction with a DMF solution of 20% piperidine was performed at 75°C for 3 minutes. However, the Fmoc groups of the 11th, 12th, 14th, and 15th residues were removed by reaction at 25°C for 5 minutes, and then reaction at 25°C for 10 minutes. A chloroacetyl group was introduced in such a manner that after the Fmoc group of the α-amino group was removed by the method described above from the solid phase resin holding the Fmoc-protected peptide obtained in the previous step, chloroacetic acid (5 Eq), 5 Eq of DIPCI, and 5 Eq of HOSu were stirred in DCM, and the same amount of DMF as DCM was added to prepare a DCM/DMF solution (0.2 M) of ClAcOSu, which was then added to the solid phase resin, followed by shaking at room temperature for 60 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 60 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether cooled to 0°C, a white cloudy precipitate was formed. The mixture was subjected to centrifugation separation (6000 rpm, 4 min), and the solution was subjected to decantation. The obtained solid was washed again with a small amount of diethyl ether cooled to 0°C, and then dried under reduced pressure . The obtained solid was used in the subsequent cyclization reaction. In the peptide cyclization reaction, after the solid was dissolved in DMSO so that the peptide final concentration was 5 mM based on the number of moles of the solid phase resin, 5 Eq of triethylamine was added, followed by shaking at room temperature for about 2 hours. The obtained reaction solution was concentrated under reduced pressure using Savant Explorer SpeedVac.

The obtained crude product was purified under the following conditions (column: Waters XBridge (registered trademark) C18 5 µm (registered trademark) 30 x 150 mm; mobile phase: A = 0.1% TFA in H₂O, B = 0.1% TFA in MeCN; temperature: 40°C; gradient (%B): 11-36% over 3 minutes, then 36-41% over 3 minutes, and then 41-60% over 1 minute; flow rate: 45 mL/min) .

The purity of the target product was calculated from the area ratio of the LC/MS (UV wavelength: 225 nm) chromatogram under analysis conditions B, and the result was 96.3%. Analysis conditions B: retention time = 12.61 minutes; column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc): 20-60% over 20 minutes, then 60-95% over 1 minute, and then 95% over 5 minutes; flow rate: 0.25 mL/min
ESI-MS (+) measured value m/z = 1140.56 (M+2H)²⁺

### [Synthesis Example 1-10]

### Synthesis of 894_3m_G4 (conjugate of linker represented by SEQ ID NO: 643 and peptide represented by SEQ ID NO: 296)

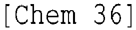

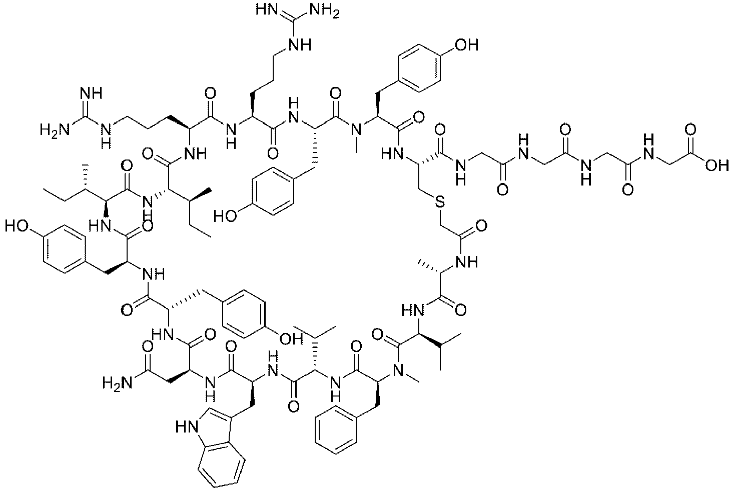

Starting from removal of Fmoc groups using Fmoc-Gly-wang resin (Watanabe Chemical Industries, Ltd., 0.7 mmol/g, 1.43 g) by the general method described above, the target peptide was synthesized. For the synthesis, CEM's Liberty Blue was used as a solid phase synthesizer, and the synthesis was performed according to the manufacturer's manual. The basic conditions for the condensation reaction were such that using HATU as a condensing agent, the reaction was performed once at 75°C for 10 minutes. However, for the introduction of the 15th residue, the reaction was performed once at 25°C for 20 minutes. For the introduction of the 13th residue, the reaction was repeated twice at 75°C for 10 minutes. For the introduction of the 2nd residue, the reaction was repeated twice at 75°C for 45 minutes . Further, the basic conditions for Fmoc removal were such that the reaction with a DMF solution of 20% piperidine was performed at 75°C for 3 minutes. However, the Fmoc groups of the 2nd and 13th residues were removed by reaction at 25°C for 5 minutes, and then reaction for 10 minutes. A chloroacetyl group was introduced in such a manner that after the Fmoc group of the α-amino group was removed by the method described above from the solid phase resin holding the Fmoc-protected peptide obtained in the previous step, a DMF solution (0.2 M) of chloroacetic acid (4 Eq), a DMF solution (0.5 M) of 4 Eq of DIPCI, and a DMF solution (0.5 M) of 4 Eq of HOSu were added, followed by shaking at room temperature for 60 minutes. For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether cooled to 0°C, a white cloudy precipitate was formed. The mixture was collected by filtration, washed with diethyl ether, and then dried under reduced pressure. The obtained solid was used in the subsequent cyclization reaction. In the peptide cyclization reaction, after the solid was dissolved in DMSO/IPA/H₂O (90/5/5) so that the peptide final concentration was 5 mM based on the number of moles of the solid phase resin, 5 Eq of triethylamine was added, followed by shaking at room temperature for about 16 hours. The obtained reaction solution was concentrated under reduced pressure using Genevac HT-12.

The obtained crude product was purified under the following conditions (column: Waters XBridge (registered trademark) C18 5 µm (registered trademark) 30 × 250 mm; mobile phase: A = 0.1% TFA in H₂O, B = 0.1% TFA in MeCN; temperature: 40°C; gradient (%B): 50-7% over 0.1 minutes, then 7-7% over 1.9 minutes, then 7-32% over 3 minutes, then 32-37% over 11 minutes, and then 37-60% over 1 minute; flow rate: 120 mL/min).

The purity of the target product was calculated from the area ratio of the LC/MS (UV wavelength: 225 nm) chromatogram under analysis conditions B, and the result was 90.6%. Analysis conditions B: retention time = 11.34 minutes; gradient (%B conc) : 20-60% over 20 minutes, then 60-95% over 1 minute, and then 95% over 5 minutes
ESI-MS (+) measured value m/z = 1163.59 (M+2H)²⁺

### [Synthesis Example 1-11]

### Synthesis of 894_3m_G4S2_K(Mal) (conjugate of linker represented by SEQ ID NO: 561 and peptide represented by SEQ ID NO: 296)

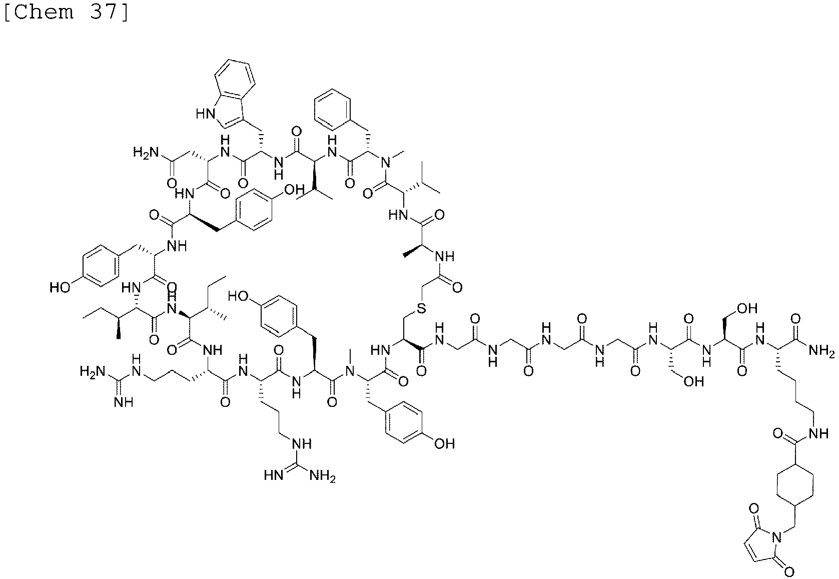

Starting from removal of Fmoc using Sieber amide resin (Watanabe Chemical Industries, Ltd., 0.52 mmol/g, 2.4 g X 3) according to a general method, the target peptide was synthesized. For the synthesis, CEM's Liberty Blue was used as a solid phase synthesizer, and the synthesis was performed according to the manufacturer's manual. The basic conditions for the condensation reaction were such that using DIPCI and Oxyma pure as condensing agents, the reaction was performed once at 75°C for 10 minutes. However, for the 15th residue, the reaction was performed at 50°C for 20 minutes. For the 3rd, 4th, 8th, 10th, 13th, and 14th residues, the reaction was performed twice at 75°C for 10 minutes. For the 11th and 12th residues, the reaction was performed twice at 50°C for 20 minutes . For the 2nd residue, the reaction was performed three times at 75°C for 60 minutes. For the 1st residue, the reaction was performed twice at 75°C for 20 minutes. Further, the basic conditions for Fmoc removal were such that the reaction with a DMF solution of 20% piperidine was performed at 75°C for 3 minutes. However, the Fmoc groups of the 15th, 16th, and 17th residues were removed by reaction at room temperature for 5 minutes, and then reaction at 75°C for 3 minutes. A chloroacetyl group was introduced in such a manner that first, the Fmoc group of the α-amino group was removed by the method described above from the solid phase resin holding the Fmoc-protected peptide obtained in the previous step. Then, chloroacetic acid (5 Eq), DIPCI (5 Eq), and HOSu (5 Eq) were stirred in DCM, and the same amount of DMF as DCM was added to prepare a DCM/DMF solution (0.15 M) of ClAcOSu, which was then added to the solid phase resin, followed by shaking at room temperature for 180 minutes . For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether cooled to 0°C, a white cloudy precipitate was formed. After the mixture was collected by filtration and washed with diethyl ether cooled to 0°C, the obtained solid was used in the subsequent cyclization reaction. In the peptide cyclization reaction, after the solid was dissolved in DMSO so that the peptide final concentration was 4.9 mM based on the number of moles of the solid phase resin, 7 Eq of triethylamine was added, followed by shaking at room temperature for about 1 hour. 1.05 Eq of SMCC was added to the obtained reaction solution, followed by shaking at room temperature for 1.5 hours . The obtained reaction solution was concentrated under reduced pressure using Genevac EZ-2 Elite.

The obtained crude product was purified under the following conditions (column: Waters XBridge (registered trademark) C18 5 µm (registered trademark) 50 × 250 mm; mobile phase: A = 0.1% TFA in H₂O, B = 0.1% TFA in MeCN; temperature: 60°C; gradient (%B): 0-0% over 5.1 minutes, then 0-5% over 1.9 minutes, then 5-29% over 5 minutes, then 29-34% over 13.5 minutes, and then 34-60% over 1.5 minutes; flow rate: 1 mL/min from 0 to 5.1 minutes, 1-119 mL/min from 5.1 minutes to 7.0 minutes, and then 119 mL/min).

The purity of the target product was calculated from the area ratio of the LC/MS (UV wavelength: 225 nm) chromatogram under analysis conditions B, and the result was 94.1%. Analysis conditions B: retention time = 11.33 minutes; gradient (%B conc) : 20-60% over 20 minutes, then 60-95% over 1 minute, and then 95% over 5 minutes
ESI-MS (+) measured value m/z = 1424.0 (M+2H)²⁺

### [Synthesis Example 1-12]

### Synthesis of 894_3m_G4S2C (conjugate of linker represented by SEQ ID NO: 644 and peptide represented by SEQ ID NO: 296)

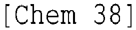

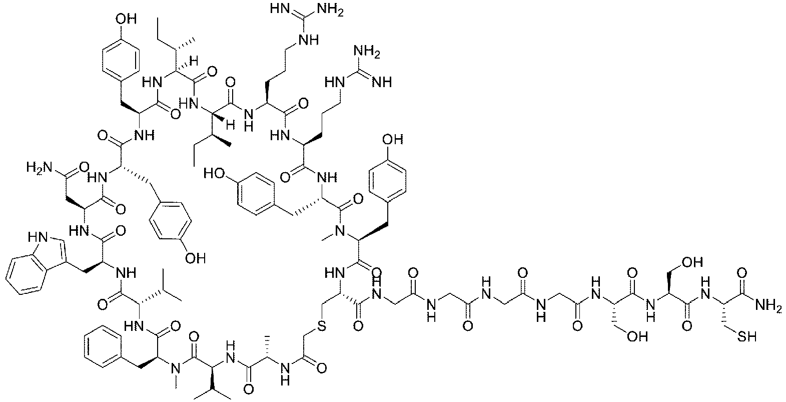

Separately synthesized 894_3m_G (500 mg, 0.21 mmol) was dissolved in DMF (5 mL), followed by stirring with 1.2 Eq of H-Gly-Gly-Gly-Ser(OtBu)-Ser(OtBu)-CyS(Trt)-NH2 (207 mg, 0.25 mmol) synthesized by a known method, 1.2 Eq of EDC (39.1 mg, 0.25 mmol), and 1.2 Eq of DIEA (35.8 mg, 0.25 mmol) at room temperature for 2.5 hours. 0.24 Eq of H-Gly-Gly-Gly-Ser(OtBu)-Ser(OtBu)-CyS(Trt)-NH2 (41.4 mg, 0.05 mmol), 0.24 Eq of 7.8 mg, 0.05 mmol), and 0.24 Eq of DIEA (7.2 mg, 0.05 mmol) were added, followed by stirring at room temperature for 2 hours. After concentration with Biotage V-10, the obtained mixture was reacted in TFA/TIS/H₂O/DODT (92.5/2.5/2.5/2.5) at room temperature for 75 minutes. When the reaction liquid was added to excess diisopropyl ether cooled to 0°C, a white cloudy precipitate was formed. The mixture was subjected to centrifugation separation (9000 rpm, 2 min), and the solution was subjected to decantation. After the obtained solid was washed with diethyl ether cooled to 0°C and subjected to centrifugation separation (9000 rpm, 2 min), the solution was subjected to decantation.

The obtained mixture was purified under the following conditions (column: Waters XBridge (registered trademark) C18 5 µm (registered trademark) 50 × 150 mm; mobile phase: A = 0.1% TFA in H₂O, B = 0.1% TFA in MeCN; temperature: 40°C; gradient (%B): 5-30% over 3 minutes, then 30-35% over 8 minutes, and then 35-60% over 1 minute; flow rate: 120 mL/min).

The purity of the target product was calculated from the area ratio of the LC/MS (UV wavelength: 225 nm) chromatogram under analysis conditions B, and the result was 96.1%. Analysis conditions B: retention time = 3.52 minutes; column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95-95% over 1.6 minutes; flow rate: 0.5 mL/min
ESI-MS (+) measured value m/z = 1301.89 (M+2H)²⁺

### [Synthesis Example 1-13]

### Synthesis of 894_3m_GGRGRS_K(Mal) (conjugate of linker represented by SEQ ID NO: 573 and peptide represented by SEQ ID NO: 296)

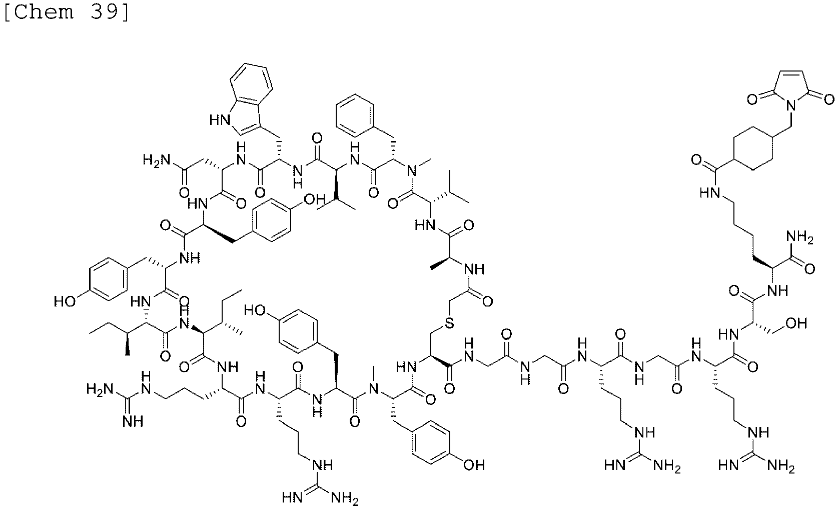

Starting from removal of Fmoc using Sieber amide resin (Watanabe Chemical Industries, Ltd., 0.47 mmol/g, 532 mg) according to a general method, the target peptide was synthesized. For the synthesis, CEM's Liberty Blue HT was used as a solid phase synthesizer, and the synthesis was performed according to the manufacturer's manual. The basic conditions for the condensation reaction were such that using HATU as a condensing agent, the reaction was performed twice at 75°C for 10 minutes. However, for the 2nd residue, the reaction was performed twice at 75°C for 30 minutes. For the 5th, 6th, 7th, 16th, 17th, 19th, 21st, and 22nd residues, the reaction was performed once at 75°C for 10 minutes. For the 11th residue, the reaction was performed twice at 50°C for 15 minutes. For the 12th, 18th, and 20th residues, the reaction was performed once at 50°C for 15 minutes. For the 15th residue, the reaction was performed once at 50°C for 15 minutes. Further, the basic conditions for Fmoc removal were such that the reaction with a DMF solution of 20% piperidine was performed at 75°C for 3 minutes. However, the Fmoc groups of the 2nd and 13th residues were removed by performing the reaction twice at room temperature for 5 minutes. A chloroacetyl group was introduced in such a manner that chloroacetic acid (5 Eq), DIPCI (5 Eq), and HOSu (5 Eq) were stirred in DCM, and the same amount of DMF as DCM was added to prepare a DCM/DMF solution (0.15 M) of ClAcOSu, which was then added to the solid phase resin obtained in the previous step, followed by shaking at room temperature for 60 minutes. For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 150 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether and hexane cooled to 0°C, a white cloudy precipitate was formed. The mixture was subjected to centrifugation separation (9500 rpm, 1 min), the supernatant was subjected to decantation, followed by washing with diethyl ether cooled to 0°C, and the obtained solid was used in the subsequent cyclization reaction. In the peptide cyclization reaction, after the solid was dissolved in DMSO (containing 5% water) so that the peptide final concentration was 5 mM based on the number of moles of the solid phase resin, 7 Eq of triethylamine was added, followed by shaking at room temperature for about 3 hours. 1.1 Eq of SMCC was added to the obtained reaction solution, followed by shaking at room temperature for 3 hours. The obtained reaction solution was concentrated under reduced pressure using Genevac EZ-2 Elite.

The obtained crude product was purified under the following conditions (column: Waters XBridge (registered trademark) C18 5 µm (registered trademark) 50 × 250 mm; mobile phase: A = 0.1% TFA in H₂O, B = 0.1% TFA in MeCN; temperature: 40°C; gradient (%B): 5-29% over 3 minutes, then 29-34% over 8 minutes, and then 34-60% over 1 minute; flow rate: 120 mL/min).

The purity of the target product was calculated from the area ratio of the LC/MS (UV wavelength: 225 nm) chromatogram under analysis conditions B, and the result was 95.4%. Analysis conditions B: retention time = 9.53 minutes; gradient (%B conc) : 20-60% over 20 minutes, then 60-95% over 1 minute, and then 95% over 5 minutes
ESI-MS (+) measured value m/z = 1005.7 (M+3H)³⁺

### [Synthesis Example 1-14]

### Synthesis of 894_3m_PEG12dk(Biotin) (conjugate of linker represented by SEQ ID NO: 613 and peptide represented by SEQ ID NO: 296)

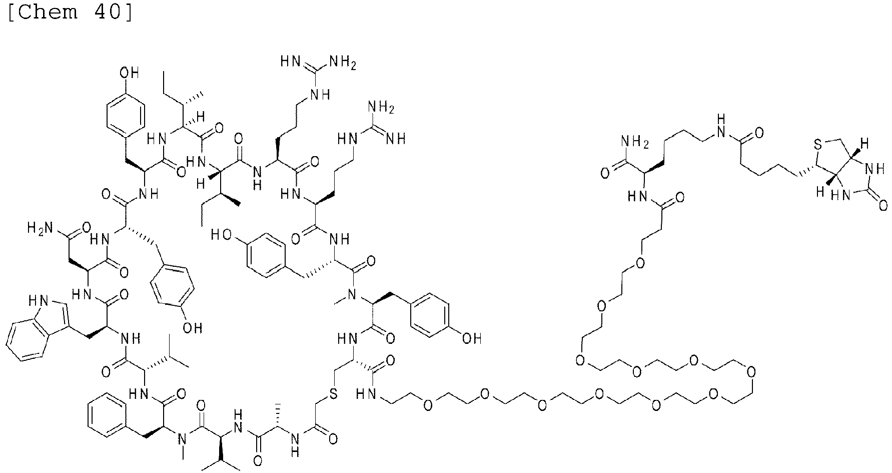

Starting from removal of Fmoc using Sieber amide resin (Watanabe Chemical Industries, Ltd., 0.65 mmol/g, 0.54 g), the target peptide was synthesized. For the synthesis, CEM's Liberty Blue HT was used as an automatic synthesizer, and the synthesis was performed according to the manufacturer's manual. The basic conditions for the introduction of each residue were such that using Fmoc-AA/DIC/Oxyma pure = 5.3 Eq/10 Eq/5 Eq per Eq of the resin, the reaction was performed at 90°C for 3 minutes. However, for the 2nd residue, the reaction was performed twice at 75°C for 30 minutes. For the 11th and 12th residues, the reaction was performed twice at 50°C for 15 minutes. For the 13th residue, the reaction was performed twice at 90°C for 3 minutes. For the 15th residue, the reaction was performed at 50°C for 15 minutes. The basic conditions for Fmoc removal were such that the reaction with a DMF solution of 20% piperidine was performed at 75°C for 3 minutes. However, the Fmoc groups of the 2nd and 13th residues were removed by performing the reaction twice at 25°C for 5 minutes. A chloroacetyl group was introduced in such a manner that after the Fmoc group of the α-amino group was removed by the method described above from the solid phase resin holding the Fmoc-protected peptide obtained in the previous step, 5 Eq of ClAcOH in DMF, 5 Eq of HATU in DMF, and 10 Eq of DIEA in DMF were added to the solid phase resin, followed by shaking at room temperature for 30 minutes. For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 90 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether/hexane (1/1) cooled to 0°C, a white cloudy precipitate was formed. The mixture was subjected to centrifugation separation (10000 rpm, 1 min), and the solution was subjected to decantation. After the obtained solid was washed again with a small amount of diethyl ether cooled to 0°C, the obtained solid was used in the subsequent cyclization reaction. In the peptide cyclization reaction, after the solid was dissolved in 5% water-containing DMSO so that the peptide final concentration was 5 mM based on the number of moles of the solid phase resin, 10 Eq of triethylamine was added, followed by shaking at room temperature for about 16 hours . The obtained reaction solution was concentrated under reduced pressure using Savant Explorer SpeedVac.

The obtained crude intermediate peptide was purified under the following conditions (column: Waters XBridge (registered trademark) C18 5 µm OBD (registered trademark) 50 x 150 mm; mobile phase: A = 0.1% TFA in H₂O, B = 0.1% TFA in MeCN; temperature: 40°C; gradient (%B): 5-30% over 3 minutes, then 30-35% over 8 minutes, and then 35-60% over 1 minute; flow rate: 120 mL/min).

1.3 Eq of Biotin-NHS and 5 Eq of DIEA were added to a DMSO solution (22 mM, 160 µL) of the obtained intermediate peptide, followed by stirring at room temperature. After 2.5 hours, the reaction liquid was quenched with acetic acid.

The obtained crude product was purified under the following conditions (column: Waters XBridge (registered trademark) C18 5 µm OBD (registered trademark) 19 × 150 mm; mobile phase: A = 0.1% TFA in H₂O, B = 0.1% TFA in MeCN; temperature: 60°C; gradient (%B): 10-35% over 3 minutes, then 35-40% over 8 minutes, and then 40-60% over 1 minute; flow rate : 17 mL/min).

The purity of the target product was calculated from the area ratio of the LC/MS (UV wavelength: 225 nm) chromatogram under analysis conditions B, and the result was 98.7%. Analysis conditions B: retention time = 4.30 minutes; column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95% over 1.6 minutes; flow rate: 0.5 mL/min
ESI-MS (+) measured value m/z = 1017.76 (M+3H)³⁺

### [Synthesis Example 1-15]

### Synthesis of 894_11K_3Me_PEG4C_KTrzMal (conjugate of linker represented by SEQ ID NO: 624 and peptide represented by SEQ ID NO: 293)

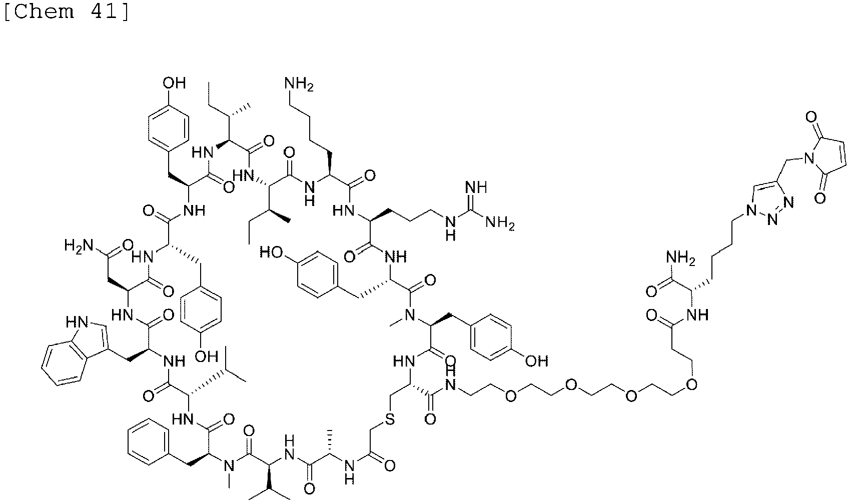

Starting from removal of Fmoc using Sieber amide resin (Watanabe Chemical Industries, Ltd., 0.52 mmol/g, 0.19 g), the target peptide was synthesized. For the synthesis, Biotage Syro I was used as an automatic synthesizer, and the synthesis was performed according to the manufacturer's manual. The basic conditions for the introduction of each residue were such that using Fmoc-AA/DIC/Oxyma pure = 3.2 Eq/3 Eq/6.3 Eq per Eq of the resin, the reaction was performed twice at 75°C for 20 minutes. However, for the 2nd, 13th, and 17th residues, the reaction was performed three times at 75°C for 10 minutes. For the 15th residue, the reaction was performed twice at 25°C for 15 minutes. For the 16th residue, the reaction was performed at 25°C for 60 minutes. Fmoc removal was performed by reaction with a DMF solution of 20% piperidine at 25°C for 5 minutes, and then reaction for 15 minutes. A chloroacetyl group was introduced in such a manner that after the Fmoc group of the α-amino group was removed by the method described above from the solid phase resin holding the Fmoc-protected peptide obtained in the previous step, 5 Eq of chloroacetic acid, 5 Eq of DIPCI, and 5 Eq of HOSu were stirred in DCM, and the same amount of NMP as DCM was added to prepare a DCM/NMP solution (0.2 M) of ClAcOSu, which was then added to the solid phase resin, followed by shaking at room temperature for 90 minutes. For deprotection of the side chain and cleavage from the solid phase resin, first, the resin obtained after the chloroacetyl group introduction step was washed five times with DMF and three times with methylene chloride, and dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 60 minutes. The reaction liquid was collected by frit filtration. The solid phase resin remaining in the reaction vessel was shaken again with a cleavage cocktail, and the solution component was collected by frit and mixed with the above filtrate. When the filtrate was added to excess diisopropyl ether/hexane (1/1) cooled to 0°C, a white cloudy precipitate was formed. The mixture was subjected to centrifugation separation (9000 rpm, 3 min), and the solution was subjected to decantation. After the obtained solid was washed again with small amounts of diethyl ether and hexane cooled to 0°C, the obtained solid was used in the subsequent cyclization reaction. In the peptide cyclization reaction, after the solid was dissolved in 9% water-containing DMSO so that the peptide final concentration was 5 mM based on the number of moles of the solid phase resin, 10 Eq of triethylamine was added, followed by shaking at room temperature for about 1.5 hours. The obtained reaction solution was concentrated under reduced pressure using Savant Explorer SpeedVac.

The obtained crude intermediate peptide was purified under the following conditions (column: Waters XBridge (registered trademark) C18 5 µm OBD (registered trademark) 50 × 150 mm; mobile phase: A = 0.1% TFA in H₂O, B = 0.1% TFA in MeCN; temperature: 40°C; gradient (%B): 9-34% over 3 minutes, then 34-39% over 8 minutes, and then 39-60% over 1 minute; flow rate: 120 mL/min).

After freeze-drying, 2 Eq of a CuSO₄·5H₂O aqueous solution (100 mM) and 10 Eq of an aqueous ascorbic acid solution (500 mM) were added to a DMF solution (15 mM) of the obtained intermediate peptide, and then a DMF solution (100 mM) of 2 Eq of N-propargylmaleimide was added, followed by stirring at room temperature.

The obtained crude product was purified under the following conditions (column: COSMOSIL PBr 10 × 150 mm; mobile phase: A = 0.1% TFA in H₂O, B = 0.1% TFA in MeCN; temperature: 40°C; gradient (%B): 17-42% over 3 minutes, then 42-47% over 8 minutes, and then 47-60% over 1 minute; flow rate: 5 mL/min).

The purity of the target product was calculated from the area ratio of the LC/MS (UV wavelength: 225 nm) chromatogram under analysis conditions B, and the result was 82.7%. Analysis conditions B: retention time = 11.37 minutes; column: Kinetex EVO C18 2.6 µm 2.1 × 150 mm, 100Å; mobile phase: A = 0.025% TFA in H₂O, B = 0.025% TFA in MeCN; temperature: 40°C; gradient (%B conc) : 20-60% over 7.2 minutes, then 60-95% over 0.3 minutes, and then 95% over 1.6 minutes; flow rate: 0.25 mL/min
ESI-MS (+) measured value m/z = 1303.33 (M+3H)³⁺

### [Synthesis Example 1-16]

### Synthesis of 894_variant_03_GKN3 (conjugate of linker represented by SEQ ID NO: 554 and SEQ ID NO: 397)

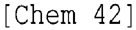

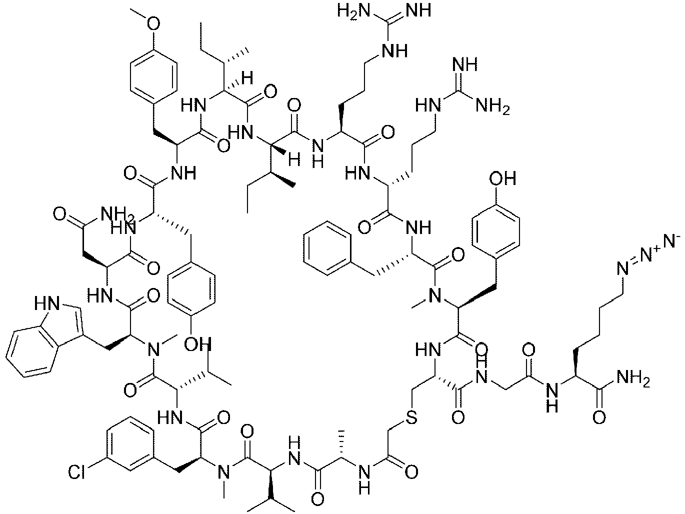

Starting from removal of Fmoc groups using Sieber amide resin (Watanabe Chemical Industries, Ltd., 0.47 mmol/g, 0.21 g) by the general method described above, the target peptide was synthesized. For the synthesis, CEM's Liberty Blue HT was used as a solid phase synthesizer, and the synthesis was performed according to the manufacturer's manual. For the introduction of each residue, using Fmoc-AA/HATU/DIEA (4.2 Eq/4 Eq/8 Eq) per Eq of the resin, the reaction was performed once in DMF at 75°C for 10 minutes. However, for the 2nd and 4th residues, the reaction was performed twice at 75°C for 30 minutes . For the 11th and 12th residues, the reaction was repeated twice at 25°C for 20 minutes. For the 13th residue, the reaction was performed twice at 75°C for 10 minutes. For the 15th and 22nd residues, the reaction was performed once at 25°C for 30 minutes . Further, the basic conditions for Fmoc removal were such that the reaction with a DMF solution of 20% piperidine was performed at 75°C for 3 minutes. However, the Fmoc groups of the 2nd, 4th, and 13th residues were removed by reaction at 25°C for 5 minutes, and then reaction at room temperature for 10 minutes. A chloroacetyl group was introduced in such a manner that after the Fmoc group of the α-amino group was removed by the method described above from the solid phase resin holding the Fmoc-protected peptide obtained in the previous step, 5 Eq of chloroacetic acid, 5 Eq of DIPCI, and 10 Eq of HOSu were stirred in DCM, and the same amount of DMF as DCM was added to prepare a DCM/DMF solution (0.2 M) of ClAcOSu, which was then added to the solid phase resin, followed by shaking at room temperature for 30 minutes . For deprotection of the side chain and cleavage from the solid phase resin, first, the resin obtained after the chloroacetyl group introduction step was washed five times with DMF and three times with methylene chloride, and then dried under reduced pressure. Subsequently, a reagent cocktail-A (a mixture of TFA/H₂O/TIS/DODT at a volume ratio of 92.5:2.5:2.5:2.5) was added to the reaction vessel containing the solid phase resin, followed by shaking at room temperature for 20 minutes. The reaction liquid was collected by frit filtration. The solid phase resin remaining in the reaction vessel was shaken again with a cleavage cocktail, and the solution component was collected by frit and mixed with the above filtrate. When the filtrate was added to an excess mixed solvent of diethyl ether/hexane (1/1) cooled to 0°C, a white cloudy precipitate was formed. The mixture was subjected to centrifugation separation (10000 rpm, 1 min), and the solution was subjected to decantation, followed by drying under reduced pressure. The obtained solid was used in the subsequent cyclization reaction. In the peptide cyclization reaction, after the solid was dissolved in 5% water-containing DMSO so that the peptide final concentration was 5 mM based on the number of moles of the solid phase resin, 6 Eq of triethylamine was added, followed by shaking at room temperature for about 16 hours. The obtained reaction solution was concentrated under reduced pressure using Savant Explorer SpeedVac.

The obtained crude product was purified under the following conditions (column: Waters XBridge (registered trademark) C18 5 µm (registered trademark) 50 x 150 mm; mobile phase: A = 0.1% TFA in H₂O, B = 0.1% TFA in MeCN; temperature: 40°C; gradient (%B): 11-36% over 3 minutes, then 36-41% over 8 minutes, and then 41-60% over 1 minute; flow rate: 120 mL/min) .

The purity of the target product was calculated from the area ratio of the LC/MS (UV wavelength: 225 nm) chromatogram under analysis conditions B, and the result was 95.5%. Analysis conditions B: retention time = 4.84 minutes; column: Kinetex EVO C18 2.6 µm 2.1 x 150 mm, 100Å; mobile phase: A = 0.025% TFA in H₂O, B = 0.025% TFA in MeCN; temperature: 60°C; gradient (%B conc) : 20-60% over 7.2 minutes, then 60-95% over 0.3 minutes, and then 95% over 1.6 minutes; flow rate: 0.5 mL/min.
ESI-MS (+) measured value m/z = 1178.06 (M+2H)²⁺

Novel amino acids were synthesized as described below.

### [Synthesis Example 2-1]

### Synthesis of (S)-2-[[(9H-fluoren-9-ylmethoxy)carbonyl]amino]-3-(7-chloro -1-ethyl-1H-indol-3-yl)propanoic acid (W1Et7Cl)

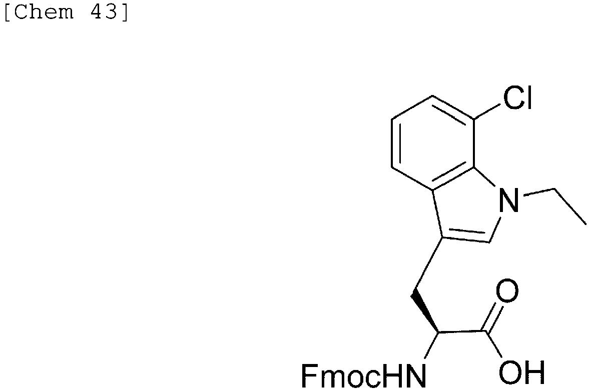

NaH (60 wt%, 8.7 g, 361 mmol) was divisionally added at 0°C to a DMF solution (500 mL) of 7-chloro-3-iodo-1H-indole (18 g, 64.9 mmol). Subsequently, ethyl iodide (10.3 mL, 130.4 mmol) was divisionally added at 0°C, and this was then stirred at room temperature for about 16 hours. The reaction solution was diluted with water and then extracted with ethyl acetate (3 x 200 mL). The combined organic layer was washed three times with water (100 mL), dried over anhydrous sodium sulfate, and filtered. After the organic layer was concentrated, the residue was purified by silica gel column chromatography (ethyl acetate:petroleum ether =1:5).

In a nitrogen atmosphere, iodine (1.6 g, 12.6 mmol) was added to a DMF suspension (50 mL) of zinc (8 g, 122 mmol). Subsequently, methyl (2R)-2-[[(9H-fluoren-9-ylmethoxy)carbonyl]amino]-3-iodoprop anoate (18.5 g, 41 mmol) was added, followed by stirring at room temperature for 30 minutes. A DMF (300 mL) solution of a part of the product (15 g, 49 mmol) obtained above was added to the reaction liquid. Subsequently, SPhos (0.84 g, 2.0 mmol) and Pd₂(dba)₃ (1.1 g, 1.2 mmol) were added, and the mixture was stirred at 50°C for 4 hours . The reaction was stopped with water, the solid was separated by filtration, and the filtrate was extracted four times with ethyl acetate (400 mL). The combined organic layer was washed four times with water (200 mL). The organic layer was dried over anhydrous sodium sulfate, filtered, and then concentrated. The obtained residue was purified by silica gel column chromatography (ethyl acetate:petroleum ether = 2:8).

Hydrogen chloride (60 g, 1.7 mol) was blown into a mixture of a part of the obtained product (18 g, 35.8 mmol) and 1,4-dioxane (200 mL), followed by stirring at 100°C for 4 hours. After the reaction liquid was concentrated, the residue was purified by reverse-phase silica gel column chromatography (water:acetonitrile = 100:0-0:100), thereby obtaining the title substance.
ESI-MS (+) measured value m/z = 489.10 (M+H)⁺

### [Synthesis Example 2-2]

### Synthesis of (S)-2-[[(9H-fluoren-9-ylmethoxy)carbonyl]amino]-3-(2-((tert iary butyloxycarbonyl)amino)pyridin-4-yl)propanoic acid (4Py6NH2)

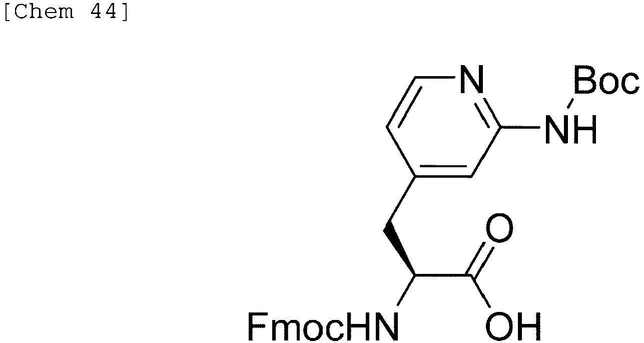

In a nitrogen atmosphere, iodine (4.5 g, 17.7 mmol) was added to a DMF (200 mL) suspension of zinc (5.8 g, 88.6 mmol), and then a DMF solution (50 mL) of methyl (2R)-2-[[(9H-fluoren-9-ylmethoxy)carbonyl]amino]-3-iodoprop anoate (20 g, 44.32 mmol, 1.0 Eq) was added dropwise at room temperature. The mixture was stirred at room temperature for 1 hour. Subsequently, 4-bromopyridin-2-amine (7.7 g, 44.3 mmol, 1 Eq), Pd₂(dba)₃ (2.0 g, 2.2 mmol, 0.05 Eq), and SPhos (1.8 g, 4.4 mmol, 0.1 Eq) were added, and the resultant was then stirred at 50°C for about 16 hours. After 500 mL of water was added to stop the reaction, the solid was separated by filtration. The filtrate was extracted three times with ethyl acetate (300 mL). The combined organic extract was washed three times with a saturated saline solution (300 mL) and dried over anhydrous sodium sulfate, followed by filtration, and the filtrate was concentrated. The obtained residue was purified by silica gel column chromatography (ethyl acetate:petroleum ether = 25:75).

A part of the product (10 g, 24.0 mmol) obtained above was added to BoC₂O (6.3 g, 28.7 mmol) and NaI (4.3 g, 28.7 mmol) in tertiary butyl alcohol (110 mL), followed by stirring at room temperature for about 16 hours. After the reaction liquid was concentrated, the obtained residue was dissolved in 80 mL of ethyl acetate, and then washed three times with a saturated saline solution (100 mL). The organic layer was dried over anhydrous sodium sulfate, filtered, and concentrated.

After CaCl₂ (6.4 g, 58.0 mmol) was added to a 2-propanol (30 mL) solution of a part of the obtained product (3 g, 5.8 mmol), an aqueous solution (5 mL) of LiOH-H₂O (0.28 g, 11.6 mmol) was added at 0°C. The mixed liquid was stirred at room temperature for about 16 hours and then diluted with 60 mL of water, and the solid was separated by filtration. The pH of the filtrate was adjusted to about 6 using an aqueous citric acid solution, and the filtrate was extracted three times with ethyl acetate (40 mL). The combined organic extract was washed three times with a saturated saline solution (50 mL), dried over anhydrous sodium sulfate, then filtered, and concentrated. The residue was purified by silica gel column chromatography (dichloromethane:methanol = 92:8), thereby obtaining the title substance. ESI-MS(+) measured value m/z = 504.15 (M+H)⁺

### [Synthesis Example 2-3]

### Synthesis of (S)-2-[[(9H-fluoren-9-ylmethoxy)carbonyl]amino]-3-(6-((tert iary butyloxycarbonyl)amino)pyridin-3-yl)propanoic acid (3Py6NH2)

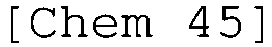

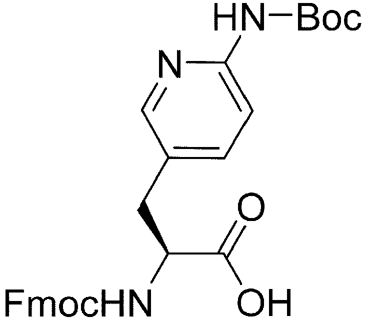

The title substance was obtained in the same manner according to Synthesis Example [1-x] using 5-bromopyridin-2-amine in place of 4-bromopyridin-2-amine.
ESI-MS (+) measured value m/z = 504.15 (M+H)⁺

### [Synthesis Example 2-4]

### Synthesis of N2-[[(9H-fluoren-9-ylmethoxy)carbonyl]-N6-(4-methylpiperazi n-1-carbonyl)-L-lysine (KCOpipzMe)

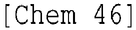

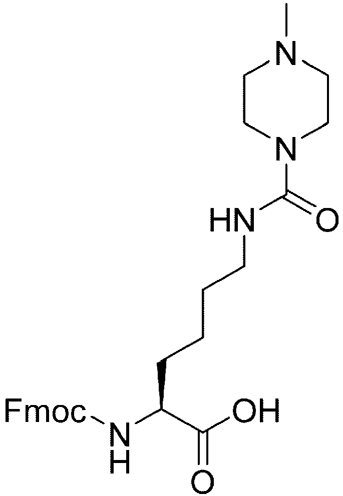

DIPEA (2.3 mL, 13.0 mmol) and triphosgene (1.23 g, 4.2 mmol) were added at 0°C to 1-methylpiperazine (1.4 mL, 12.7 mmol) dissolved in dichloromethane (20 mL). The resulting mixture was stirred at the same temperature for 1 hour and then concentrated. A dichloromethane solution (20 mL) of [(9H-fluoren-9-ylmethoxy) carbonyl]-L-lysine and DIPEA (2.8 mL, 16.3 mmol) was added at 0°C to the obtained residue. The mixed liquid was stirred at 0°C for about 16 hours. After the reaction was stopped with a saturated aqueous sodium bicarbonate solution, the resultant was diluted with dichloromethane, and washed once with water, twice with a 5% aqueous acetic acid solution, once with a saturated aqueous sodium bicarbonate solution, and finally once with a saturated saline solution. The organic layer was dried over anhydrous sodium sulfate and concentrated. The residue was purified by silica gel column chromatography (dichloromethane:methanol = 100:0-80:20), thereby obtaining the title compound. ESI-MS(+) measured value m/z = 495.40 (M+H)⁺

### [Synthesis Example 2-5]

### Synthesis of N-alpha-[(9H-fluoren-9-ylmethoxy)carbonyl]-1-(2-amino-2-oxo ethyl)-L-tryptophan (W1mCON)

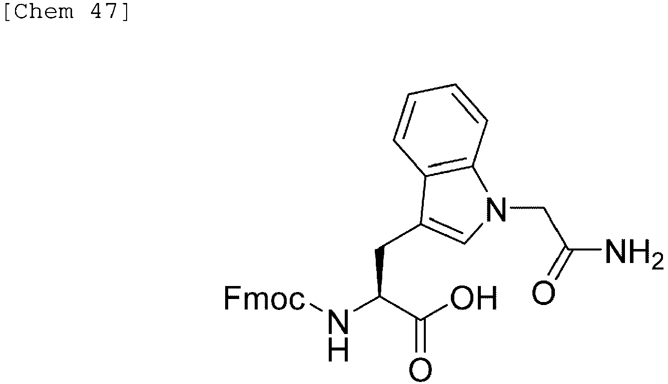

In a nitrogen atmosphere, potassium-tertiary butoxide (3.7 g, 32.9 mmol) was added at 0°C several times to a DMF (30 mL)-THF (30 mL) mixed solution of N-Boc-tryptophan (5 g, 16.4 mmol). After the resulting mixture was stirred at the same temperature for 0.5 hours, 2-chloroacetamide (1.5 g, 16.4 mmol) was added at 0°C several times, after which the mixture was stirred at room temperature for 3 hours. After water (100 mL) was added to stop the reaction, the resultant was extracted three times with ethyl acetate (50 mL). The combined organic layer was washed three times with a saturated saline solution (50 mL), and then dried over anhydrous magnesium sulfate, followed by filtration, and the filtrate was concentrated. The obtained residue was purified by silica gel column chromatography (ethyl acetate:petroleum ether = 90:10).

An ethyl acetate solution (2 M, 30 mL) of hydrogen chloride was added to an ethyl acetate solution (30 mL) of a part of the obtained product (6 g, 16.6 mmol) , followed by stirring at room temperature for about 16 hours. The produced solid was collected by filtration, and this was washed with ethyl acetate and diethyl ether. After the obtained solid was dissolved in 1,4-dioxane (50 mL) and water (10 mL), 2,5-dioxypyrrolidin-1-yl-9H-fluoren-9-yl-methyl carbonate (5.9 g, 17.5 mmol) and sodium hydrogen carbonate (5.9 g, 69.9 mmol) were added at 0°C. The mixture was stirred at room temperature for 3 hours and then diluted with 200 mL of water. After citric acid was added to adjust the pH to 5 to 6, the resultant was extracted three times with ethyl acetate (60 mL) . The combined organic extract was washed three times with a saturated saline solution (100 mL). The organic layer was dried over anhydrous sodium sulfate, filtered, and concentrated. The residue was purified by silica gel column chromatography (dichloromethane/methanol = 92/8), thereby obtaining the title compound. ESI-MS(+) measured value m/z = 484.25 (M+H)⁺

### [Synthesis Example 2-6]

### Synthesis of Fmoc-Glu(d-Pro-O-allyl)-OH(Epryl2RCOO)

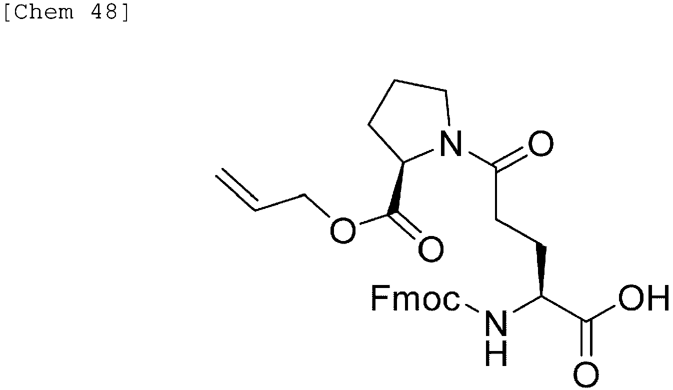

HATU (2.7 g, 7.0 mmol), DIPEA (1.3 mL, 7.5 mmol), and allyl-D-prolinate (0.9 g, 5.80 mmol) were added to a DMF solution (10 mL) of 4-((((9H-fluoren-9-yl)methoxy)carbonyl)amino)-5-(tertiary butoxy)-5-oxopentanoic acid (2.5 g, 5.8 mmol), followed by stirring at 0°C for 1 hour. The reaction solution was diluted with water, and then extracted with a mixed solvent of ethyl acetate and hexane. The organic layer was washed four times with water, then dried over anhydrous sodium sulfate, filtered, and then concentrated under reduced pressure. The residue was purified by silica gel column chromatography.

A part of the product (2.6 g, 4.5 mmol) obtained in the above reaction and dichloromethane/TFA (1:1, 30 mL) were stirred at room temperature for 3 hours. After the reaction solution was concentrated, the residue was dissolved in dichloromethane, and a saturated aqueous sodium bicarbonate solution was added to adjust the pH to 8 or more. Subsequently, hydrochloric acid was added to adjust the pH to 3 to 4, and extraction was performed with ethyl acetate. The organic layer was dried over anhydrous sodium sulfate, filtered, and concentrated under reduced pressure, thereby obtaining the title compound. ESI-MS(+) measured value m/z = 507.40 (M+H)⁺

### [Synthesis Example 2-7]

### Synthesis of Fmoc-Asp(d-Pro-O-allyl)-OH(Dpryl2RCOO)

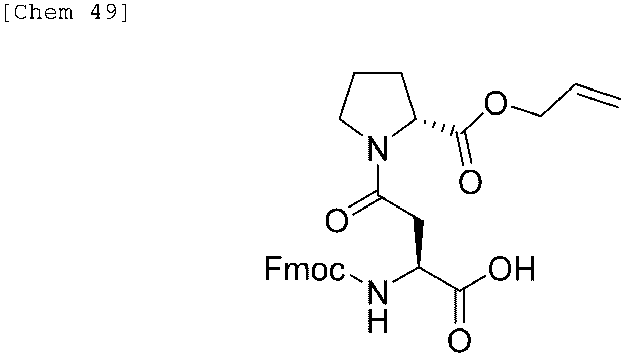

The title substance was obtained in the same manner according to Synthesis Example [1-x] using 3-((((9H-fluoren-9-yl)methoxy)carbonyl)amino)-4-(tertiary butoxy)-4-oxobutanoic acid in place of 4-((((9H-fluoren-9-yl)methoxy)carbonyl)amino)-5-(tertiary butoxy)-5-oxopentanoic acid. ESI-MS(+) measured value m/z = 493.30 (M+H)⁺

### [Synthesis Example 2-8]

### Synthesis of Fmoc-Lys(Gly-O-allyl)-OH(KaAC)

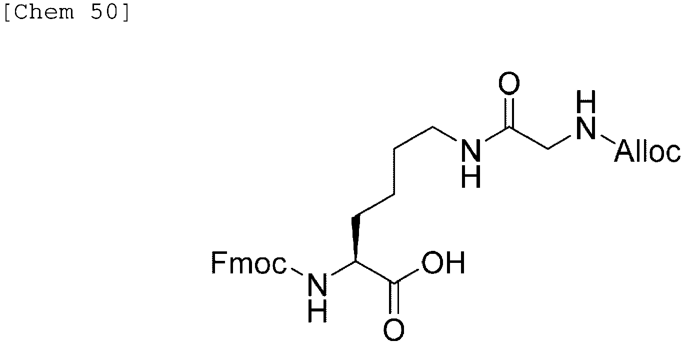

A DMF solution (20 mL) of HATU (1.22g, 3.1 mmol) was added to a DMF solution of tertiary butyl(((9H-fluoren-9-yl)methoxy)carbonyl)-L-lysinemethyl-hy drochloride (1.2 g, 2.6 mmol), ((allyloxy)carbonyl)glycine (0.46 g, 2.9 mmol) , and DIPEA (0.59 mL,3.4 mmol) , followed by stirring at 0°C for 1 hour. After dilution with a mixed solvent of ethyl acetate and hexane, the organic layer was washed four times with water. After the organic layer was dried over anhydrous sodium sulfate and concentrated, the residue was purified by silica gel column chromatography (ethyl acetate:hexane = 0:100-80:20).

TFA (10 mL) was added to a dichloromethane solution (10 mL) of a part of the product (1.5 g, 2.6 mmol) synthesized above, followed by stirring at room temperature for about 16 hours. The reaction solution was concentrated, and an azeotropic operation was conducted three times using toluene. The obtained residue was washed with diisopropyl ether and dried under reduced pressure at 55°C, thereby obtaining the title compound. ESI-MS(+) measured value m/z = 510.40 (M+H)⁺

### [Synthesis Example 2-9]

### Synthesis of N^{α}- (((9H-fluoren-9-yl)methoxy)carbonyl)-1-(2-((tertiary butyldimethylsilyl)oxy)ethyl)-L-tryptophan (Fmoc-WlEtOH-OH)

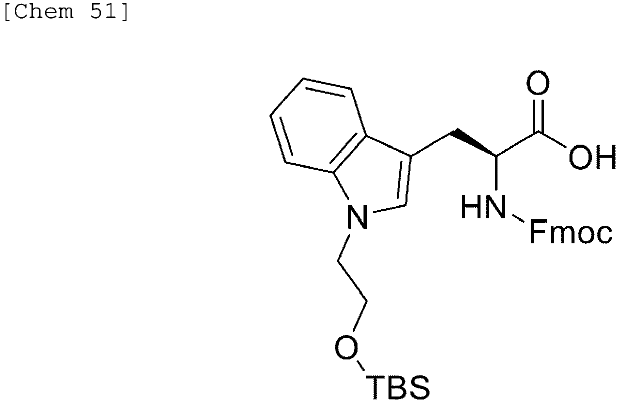

Potassium-tertiary butoxide (14.8 g, 69.0 mmol) was added at 0°C to a THF (100 mL)-DMF (100 mL) mixed liquid of N-Boc-tryptophan (20 g, 64.9 mmol). After stirring for 15 minutes, 2-bromoethoxy (tertiary butyl) dimethylsilane (16.5 g, 69.0 mmol) was added, followed by stirring at room temperature for about 16 hours. An aqueous citric acid solution was added to the reaction solution at 0°C to adjust the pH to 6, and the resultant was diluted with 200 mL of water. The reaction solution was extracted three times with ethyl acetate (100 mL) , and the combined organic extract was washed three times with a saturated saline solution (100 mL). The organic layer was dried over sodium sulfate, filtered, and then concentrated. The residue was purified by silica gel column chromatography (ethyl acetate:hexane = 1:5).

In a nitrogen atmosphere, 2,6-lutidine (20 mL, 172.9 mmol) was added to a dichloromethane (150 mL) solution of a part of the obtained product (16 g, 34.6 mmol), and TMSOTf (25 mL, 112 mmol) was added at 0°C. The mixed liquid was heated to room temperature, followed by stirring for 24 hours. The reaction solution was concentrated. After dioxane (300 mL) and water (150 mL) were added to the obtained mixture, sodium hydrogen carbonate (9.3 g, 110.35 mmol, 3.19 Eq) and 9-fluorenylmethyl-N-succinimidyl carbonate (14.0 g, 41.4 mmol) were added, followed by stirring at room temperature for about 16 hours. The solid was filtered, and citric acid was added to the filtrate to adjust the pH to 7. The resultant was diluted with 200 mL of water and extracted three times with ethyl acetate (200 mL). The combined organic extract was washed three times with a saturated saline solution (200 mL). The organic layer was dried over sodium sulfate, filtered, and then concentrated. The residue was purified by silica gel column chromatography (ethyl acetate:hexane (1:15), thereby obtaining the title compound. ESI-MS(+) measured value m/z = 585.15 (M+H)⁺

### [Industrial Applicability]

This invention can be used in pharmaceutical industries.

## Claims

1. A peptide that binds to a transferrin receptor, the peptide having:
an amino acid sequence represented by SEQ ID NO: 1 (Ala-Val-Phe-Val-Trp-Asn-Tyr-Tyr-Ile-Ile-Ser-Cys); or
an amino acid sequence having substitutions, deletions, additions, and/or insertions of from 1 to 10 amino acid residues in the amino acid sequence represented by SEQ ID NO: 1.

2. The peptide according to claim 1, the peptide having the amino acid sequence with one or more substitutions selected from following groups:
(I) substitution of a 1st alanine residue of SEQ ID NO: 1 with an aliphatic amino acid or a methylated aliphatic amino acid;
(II) substitution of a 2nd valine residue of SEQ ID NO: 1 with a basic amino acid residue or a methylated basic amino acid residue;
(III) substitution of a 3rd phenylalanine residue of SEQ ID NO: 1 with an aromatic amino acid residue, a methylated aromatic amino acid residue, an amino acid residue added with an aromatic ring, or an amino acid residue added with a condensed ring;
(IV) substitution of a 4th valine residue of SEQ ID NO: 1 with a methylated valine residue;
(V) substitution of a 5th tryptophan residue of SEQ ID NO: 1 with an aromatic amino acid residue, a methyltryptophan residue, a methylated aromatic amino acid residue, an amino acid residue added with an aromatic ring, or an amino acid residue added with a condensed ring;
(VI) substitution of a 6th asparagine residue of SEQ ID NO: 1 with a neutral amino acid or a methylated neutral amino acid;
(VII) substitution of 7th and 8th tyrosine residues of SEQ ID NO: 1 with aromatic amino acid residues, methylated aromatic amino acid residues, amino acid residues added with aromatic rings, or amino acid residues added with condensed rings;
(VIII) substitution of a 9th isoleucine residue of SEQ ID NO: 1 with an aliphatic amino acid residue, a methylated aliphatic amino acid residue, or an amino acid residue having a branched chain structure;
(IX) substitution of a 10th isoleucine residue of SEQ ID NO: 1 with any amino acid; and
(X) substitution of a 11th serine residue of SEQ ID NO: 1 with a neutral amino acid residue.

3. The peptide according to claim 1, the peptide having:
an amino acid sequence represented by SEQ ID NO: 2 (Ala-Val-Phe-Val-Trp-Asn-Tyr-Tyr-Ile-Ile-Arg-Arg-Tyr-MeTyr-Cys); or
the amino acid sequence represented by SEQ ID NO: 2 with one or more substitutions selected from following groups:
(I) substitution of a 1st alanine residue of SEQ ID NO: 2 with an aliphatic amino acid or a methylated aliphatic amino acid;
(II) substitution of a 2nd valine residue of SEQ ID NO: 2 with a basic amino acid residue or a methylated basic amino acid residue;
(III) substitution of a 3rd phenylalanine residue of SEQ ID NO: 2 with an aromatic amino acid residue, a methylated aromatic amino acid residue, an amino acid residue added with an aromatic ring, or an amino acid residue added with a condensed ring;
(IV) substitution of a 4th valine residue of SEQ ID NO: 2 with a methylated valine residue;
(V) substitution of a 5th tryptophan residue of SEQ ID NO: 2 with an aromatic amino acid residue, a methylated aromatic amino acid residue, an amino acid residue added with an aromatic ring, or an amino acid residue added with a condensed ring;
(VI) substitution of a 6th asparagine residue of SEQ ID NO: 2 with a neutral amino acid or a methylated neutral amino acid;
(VII) substitution of 7th and 8th tyrosine residues of SEQ ID NO: 2 with aromatic amino acid residues, methylated aromatic amino acid residues, amino acid residues added with aromatic rings, or amino acid residues added with condensed rings;
(VIII) substitution of a 9th isoleucine residue of SEQ ID NO: 2 with an aliphatic amino acid residue, a methylated aliphatic amino acid residue, or an amino acid residue having a branched chain structure;
(IX) substitution of a 10th isoleucine residue of SEQ ID NO: 2 with any amino acid;
(XI) substitution of 11th and 12th arginine residues of SEQ ID NO: 2 with basic amino acid residues;
(XII) substitution of a 13th tyrosine residue of SEQ ID NO: 2 with a hydrophilic amino acid residue;
(XIII) substitution of a 14th methyl tyrosine residue of SEQ ID NO: 2 with a tyrosine residue, an aromatic amino acid residue, or a methylated aromatic amino acid residue; and
(XIV) substitution of a 15th cysteine residue of SEQ ID NO: 2 with a methylated cysteine residue.

4. The peptide according to claim 1, wherein,
provided that a peptide A has a peptide length of from 10 to 17 and includes a 1st to 10th amino acid sequence represented by SEQ ID NO: 18 (Ala-Val-Phe-Val-Trp-Asn-Tyr-Tyr-Ile-Ile-Ser-Cys),
the peptide is any of:
the peptide A;
the peptide A having the amino acid sequence with substitutions, deletions, and/or insertions of from 1 to 6 amino acid residues;
the peptide A having the amino acid sequence in which any of 2nd, 3rd, 5th, 8th, and 10th amino acid residues of SEQ ID NO: 18 is substituted; and
the peptide A having the amino acid sequence in which:
the 2nd amino acid residue of SEQ ID NO: 18 is valine (Val) that may be modified or glutamic acid (Glu) that may be modified;
the 3rd amino acid residue of SEQ ID NO: 18 is phenylalanine (Phe) that may be modified;
the 5th amino acid residue of SEQ ID NO: 18 is tryptophan (Trp) that may be modified;
the 8th amino acid residue of SEQ ID NO: 18 is tyrosine (Tyr) that may be modified; and/or
the 10th amino acid residue of SEQ ID NO: 18 is isoleucine (Ile) that may be modified or valine (Val) that may be modified.

5. The peptide according to claim 4, having a peptide length of from 11 to 13.

6. The peptide according to claim 1, wherein,
provided that a peptide B has a peptide length of from 10 to 19 and includes a 1st to 10th amino acid sequence represented by SEQ ID NO: 15 (Ala-Val-Phe-Val-Trp-Asn-Tyr-Tyr-Ile-Val-Pro-Arg-Asp-Cys),
the peptide is any of:
the peptide B;
the peptide B having the amino acid sequence with substitutions, deletions, and/or insertions of from 1 to 5 amino acid residues;
the peptide B having the amino acid sequence in which any of 2nd, 3rd, 5th, 8th, and 10th amino acid residues of SEQ ID NO: 15 is substituted; and
the peptide B having the amino acid sequence in which:
the 2nd amino acid residue of SEQ ID NO: 15 is valine (Val) that may be modified or glutamic acid (Glu) that may be modified;
the 3rd amino acid residue of SEQ ID NO: 15 is phenylalanine (Phe) that may be modified or tryptophan (Trp);
the 5th amino acid residue of SEQ ID NO: 15 is tryptophan (Trp) that may be modified;
the 8th amino acid residue of SEQ ID NO: 15 is tyrosine (Tyr) that may be modified; and/or
the 10th amino acid residue of SEQ ID NO: 15 is isoleucine (Ile) that may be modified or valine (Val) that may be modified.

7. The peptide according to claim 6, having a peptide length of from 13 to 15.

8. The peptide according to claim 1, wherein,
provided that a peptide C has a peptide length of from 11 to 19 and includes a 1st to 10th amino acid sequence represented by SEQ ID NO: 214 (MeA-Val-MeF3C-Val-MeW-Asn-Tyr-F4OMe-Ile-Ile-Arg-Arg-Phe-Me Y-Cys),
the peptide is any of:
the peptide C;
the peptide C having the amino acid sequence with substitutions, deletions, and/or insertions of from 1 to 5 amino acid residues;
the peptide C having the amino acid sequence in which any of 1st, 3rd, 5th, and 8th amino acid residues of SEQ ID NO: 214 is substituted; and
the peptide C having the amino acid sequence in which:
the 1st amino acid residue of SEQ ID NO: 214 is alanine (Ala) that may be modified or glutamic acid (Glu) that may be modified;
the 3rd amino acid residue of SEQ ID NO: 214 is phenylalanine (Phe) that may be modified;
the 5th amino acid residue of SEQ ID NO: 214 is tryptophan (Trp) that may be modified; and/or
the 8th amino acid residue of SEQ ID NO: 214 is phenylalanine (Phe) that may be modified.

9. The peptide according to claim 1, wherein,
provided that a peptide D has a peptide length of from 11 to 19 and includes a 1st to 10th amino acid sequence represented by SEQ ID NO: 219 (Ala-Glu-Phe-Val-Trp-Asn-Tyr-Tyr-Ile-Ile-Arg-Arg-Tyr-MeY-Cy s),
the peptide is any of:
the peptide D;
the peptide D having the amino acid sequence with substitutions, deletions, and/or insertions of from 1 to 5 amino acid residues;
the peptide D having the amino acid sequence in which any of 2nd, 3rd, 5th, 8th, and 10th amino acid residues of SEQ ID NO: 219 is substituted; and
the peptide D having the amino acid sequence in which:
the 2nd amino acid residue of SEQ ID NO: 219 is valine (Val) that may be modified, glutamic acid (Glu) that may be modified, arginine (Arg) that may be modified, lysine (Lys) that may be modified, aspartic acid (Asp) that may be modified, or phenylalanine (Phe) that may be modified;
the 3rd amino acid residue of SEQ ID NO: 219 is phenylalanine (Phe) that may be modified;
the 5tn amino acid residue of SEQ ID NO: 219 is tryptophan (Trp) that may be modified;
the 8th amino acid residue of SEQ ID NO: 219 is tyrosine (Tyr) that may be modified; and/or
the 10th amino acid residue of SEQ ID NO: 219 is isoleucine (Ile) that may be modified, glutamic acid (Glu) that may be modified, or lysine (Lys) that may be modified.

10. The peptide according to claim 9, having a peptide length of from 15 to 18.

11. The peptide according to claim 1, wherein,
provided that a peptide E has a peptide length of from 15 to 18 and includes a 1st to 15th amino acid sequence represented by SEQ ID NO: 296 (Ala-Val-MeF-Val-Trp-Asn-Tyr-Tyr-Ile-Ile-Arg-Arg-Tyr-MeY-Cy s),
the peptide is:
the peptide E; or
the peptide E having the amino acid sequence with substitutions, deletions, and/or insertions of from 1 to 5 amino acid residues.

12. The peptide according to claim 11, wherein the peptide E has the amino acid sequence in which any of 3rd, 5th, 7th, 8th, 11th, 12th, and 13th amino acid residues of SEQ ID NO: 296 is substituted.

13. The peptide according to claim 11, wherein the peptide E is any of the peptides in which:
the 3rd amino acid residue of SEQ ID NO: 296 is phenylalanine (Phe) that may be modified;
the 5th amino acid residue of SEQ ID NO: 296 is tryptophan (Trp) that may be modified;
the 7th amino acid residue of SEQ ID NO: 296 is tyrosine (Tyr) that may be modified;
the 8th amino acid residue of SEQ ID NO: 296 is tyrosine (Tyr) that may be modified;
the 11th amino acid residue of SEQ ID NO: 296 is arginine (Arg) that may be modified or lysine (Lys) that may be modified;
the 12th amino acid residue of SEQ ID NO: 296 is arginine (Arg) that may be modified or lysine (Lys) that may be modified; and/or
the 13th amino acid residue of SEQ ID NO: 296 is tyrosine (Tyr) that may be modified or phenylalanine (Phe) that may be modified.

14. The peptide according to claim 11, wherein the peptide E is the peptide in which:
the 3rd amino acid residue of SEQ ID NO: 296 is phenylalanine (Phe), methylated phenylalanine (MeF), or N-α-methyl-N-α-chloroacetyl-3-chloro-L-phenylalanine (MeF3C);
the 5th amino acid residue of SEQ ID NO: 296 is tryptophan (Trp) or methylated tryptophan (MeW);
the 8th amino acid residue of SEQ ID NO: 296 is tyrosine (Tyr) or (S)-2-amino-3-(4-methoxyphenyl)propanoic acid (F4OMe);
the 11th amino acid residue of SEQ ID NO: 296 is arginine (Arg) or lysine (Lys);
the 12th amino acid residue of SEQ ID NO: 296 is arginine (Arg) or D-configuration arginine (dr); and/or
the 13th amino acid residue of SEQ ID NO: 296 is tyrosine (Tyr) or phenylalanine (Phe).

15. The peptide according to claim 1, having:
an amino acid sequence of any of SEQ ID NOs: 3 to 200; or
the amino acid sequence of any of SEQ ID NOs: 3 to 200 in which an N-terminus is chloroacetyl-Ala.

16. The peptide according to claim 1, wherein,
the peptide has, as an amino acid sequence site, a 1st to 10th amino acid sequence of:
an amino acid sequence represented by any of SEQ ID NOs: 1 to 552; or
a linker conjugate thereof, the amino acid sequence site including a cyclic structure.

17. The peptide according to claim 16, wherein,
the peptide has, as the amino acid sequence site, a 1st to 15th amino acid sequence of:
an amino acid sequence represented by any of SEQ ID NOs: 2, 9, 21 to 148, 159 to 200, 213 to 448, and 450 to 552; or
a linker conjugate thereof, the amino acid sequence site including the cyclic structure.

18. The peptide according to any of claims 1 to 16, wherein the peptide is the cyclic peptide.

19. The peptide according to claim 16 or 17, wherein the peptide has 15 amino acid residues.

20. The peptide according to claim 1, wherein the peptide is capable of passing through a blood-brain barrier.

21. The peptide according to claim 1, wherein the peptide has directivity to a muscle tissue.

22. The peptide according to claim 1, wherein the peptide has a cell-penetrating property.

23. A conjugate comprising the peptide according to claim 1, a linker bound to the peptide, and a substance bound to the linker.

24. The conjugate according to claim 23, wherein the substance is capable of passing through the blood-brain barrier.

25. The conjugate according to claim 23, wherein the linker has an amino acid length of from 1 to 15 and includes one or more glycine (Gly) or serine (Ser).

26. The conjugate according to claim 23, wherein an N-terminus of the linker is cysteine (Cys) that may be modified or lysine (Lys) that may be modified.

27. The conjugate according to claim 23, wherein the linker has an amino acid length of from 1 to 5 and includes either one or both of D-configuration glutamic acid (de) and methylated glycine (MeG).

28. The conjugate according to claim 23, wherein the N-terminus of the linker is Cys that may be modified or lysine (Lys) that may be modified.

29. The conjugate according to claim 23, wherein the linker is a PEG linker including polyethylene glycol (PEG) or a derivative of polyethylene glycol.

30. The conjugate according to claim 29, wherein the PEG linker further includes glycine (Gly), serine (Ser), glutamic acid (Glu), arginine (Arg), or lysine (Lys).

31. The conjugate according to claim 29 or 30, wherein the N-terminus of the linker is cysteine (Cys) that may modified or lysine (Lys) that may modified.

32. The conjugate according to claim 23, wherein the linker has a sequence represented by any of SEQ ID NOs: 201 and 553 to 642.

33. The conjugate according to claim 22, wherein,
the linker is:
polyethylene glycol (PEG);
a G linker or a GS linker; or
a linker having an amino acid sequence represented by any of SEQ ID NOs: 201 and 553 to 644.

34. A preventive or therapeutic agent for brain-related disease comprising the conjugate according to claim 23, wherein the substance is an active ingredient.

35. A method for producing the preventive or therapeutic agent for brain-related disease comprising a step of obtaining the conjugate according to claim 23.

36. The method according to claim 35, wherein,
the linker is:
polyethylene glycol (PEG);
the G linker or the GS linker; or
the linker having the amino acid sequence represented by any of SEQ ID NOs: 201 and 553 to 644.

37. A diagnostic agent for brain-related disease, comprising the conjugate according to claim 23.

38. The conjugate according to claim 23, having directivity to the muscle tissue.

39. A preventive or therapeutic agent for neuromuscular disease comprising the conjugate according to claim 23, wherein the substance is an active ingredient.

40. A diagnostic agent for neuromuscular disease comprising the conjugate according to claim 23.
